# EUROPEAN PATENT SPECIFICATION

(11) **EP 3 694 856 B1**
(45) Date of publication and mention of the grant of the patent: **25.10.2023**
(21) Application number: 18810716.3
(22) Date of filing: 02.06.2018
(51) Int. Cl.: C07D 471/04, C07D 455/06, A61K 31/435, A61K 31/4375, A61K 31/5025, A61P 31/12, A61P 31/20

(54) **FUSED TRICYCLIC COMPOUNDS AND USES THEREOF IN MEDICINE**
KONDENSIERTE TRICYCLISCHE VERBINDUNGEN UND VERWENDUNGEN DAVON IN DER MEDIZIN
COMPOSÉS TRYCICLIQUES CONDENSÉS ET UTILISATIONS CORRESPONDANTES EN MÉDECINE

(30) Priority: 22.11.2017 CN 201711170576
(43) Date of publication of application: 19.08.2020
(73) Proprietor: Sunshine Lake Pharma Co., Ltd., Dongguan, Guangdong (CN)
(72) Inventor: LIU, Xinchang, Dongguan Guangdong 523871 (CN); REN, Qingyun, Dongguan Guangdong 523871 (CN); HUANG, Jianzhou, Dongguan Guangdong 523871 (CN); XIONG, Zhimin, Dongguan Guangdong 523871 (CN); XIONG, Jinfeng, Dongguan Guangdong 523871 (CN); LI, You, Dongguan Guangdong 523871 (CN); LIU, Yang, Dongguan Guangdong 523871 (CN); ZOU, Zhifu, Dongguan Guangdong 523871 (CN); YAN, Guanghua, Dongguan Guangdong 523871 (CN); GOLDMANN, Siegfried, Dongguan Guangdong 523871 (CN); ZHANG, Yingjun, Dongguan Guangdong 523871 (CN)
(74) Representative: Hoffmann Eitle
(86) International application number: PCT/CN2018/089699
(87) International publication number: WO 2018/219356

(56) References cited:
- WO-A1-2016/071215
- WO-A1-2016/128335
- WO-A1-2017/016921
- WO-A1-2017/017043
- WO-A1-2017/108630
- WO-A1-2017/140821
- CN-A- 105 899 508
- CN-A- 106 459 032
- CN-A- 106 810 548

## Description

### Field

The present invention relates to a fused tricyclic compound and use thereof as a medicament, in particular as a medicament for the treatment and prevention of hepatitis B. The present invention also relates to compositions containing these fused tricyclic compounds and other antiviral agents, and their use for the treatment and prevention of Hepatitis B (HBV) infection.

### BACKGROUND

The hepatitis B virus belongs to the family of hepadnaviridae. It can cause acute and/or persistent or progressive chronic diseases. Many other clinical manifestations in the pathological morphology can also be caused by HBV-in particular chronic hepatitis, cirrhosis and hepatocellular carcinoma. According to estimates by the World Health Organization, 2 billion people in the world have been infected with HBV, and about 350 million people are chronically infected. About 1 million people die each year because of liver failure, cirrhosis and primary hepatocellular carcinoma (Hepatocellular carcinoma, HCC) which result form HBV infection.

Currently, the treatment of chronic hepatitis B (CHB) is mainly based on antiviral therapy. Interferon alpha (IFN-alpha), pegylated IFN-alpha and five nucleoside (acid) analogs (lamivudine, adefovir dipivoxil, entecavir, telbivudine, and tenofovir) have been approved by Food and Drug Administration (FDA) for clinical treatment. Interferon is the earliest FDA-approved anti-HBV drug. It mainly through direct antiviral effect and induction of the body's immune response to achieve the effect of removing the virus, but because of its low response rate, having a variety of side effects, high price and restrictions of the treatment object, *etc,* its application is subject to many restrictions. The common point of nucleoside (acid) analogues in anti-HBV action is specifically acting viral DNA polymerase, which has a strong effect of inhibiting viral replication, and the patient's tolerance to drugs is better than interferon. However, with the widespread long-term use of nucleoside (acid) drugs, mutations in DNA polymerase can be induced to form drug resistance, and leads to the emergence of drug-resistant strains, thus it makes the treatment is far less than that of ideal.

The applications CN106810548A, WO2017140821A1, WO2017108630A1, CN105899508A, WO2017017043A1, WO2016128335A1, CN106459032A, and WO2016071215A1 respectively, disclose some compounds, which can be used in treating HBV infection.

Therefore, there is still a need in the clinic for new compounds that can effectively be used as antiviral drugs, especially these compounds as drugs for treating and/or preventing hepatitis B.

### SUMMARY

The present invention relates to a novel fused tricyclic compound and its use in the preparation of a medicament for the treatment and prevention of HBV infections. The inventor found that the novel fused tricyclic compound of present invention has good pharmacokinetic properties, good solubility, low toxicity, good liver microsome stability, and good inhibitory activity againist HBsAg production or secretion and HBV DNA replication, and other advantages, it has a good application prospects in anti-HBV virus action. In particular, the compounds of the present invention and pharmaceutically acceptable compositions thereof, are effective in inhibiting HBV infection.

In one aspect, the invention relates to a compound having Formula (I) or a stereoisomer, a tautomer, an N-oxide, a solvate, or a pharmaceutically acceptable salt thereof, wherein, 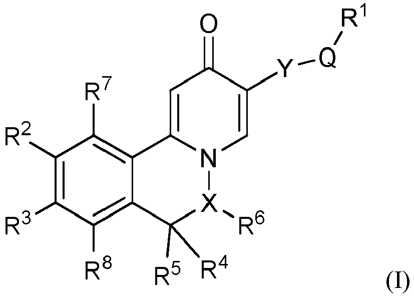
X is N or -CR^{6a}-;
Y is -C(=O)-;
Q is a single bond, -O- or -N(R⁹)-;
R¹ is H, deuterium, methyl, ethyl, n-propyl or isopropyl, wherein each of the methyl, ethyl, *n*-propyl and isopropylis independently unsubstituted or substituted with 1, 2, 3 or 4 R^{v};
R⁹ is H, deuterium, methyl, ethyl, *n*-propyl, isopropyl or C₁₋₆ haloalkyl;
each of R⁴ and R⁵ is independently H, deuterium, C₁₋₄ alkyl, C₁₋₄ alkylamino or C₁₋₄ alkoxy, wherein each of the C₁₋₄ alkyl, C₁₋₄ alkylamino and C₁₋₄ alkoxy is independently unsubstituted or substituted with 1, 2, 3 or 4 R^{y};
R⁶ is H, deuterium, C₁₋₄ alkyl, C₂₋₄ alkenyl or C₃₋₆ cycloalkyl, wherein each of the C₁₋₄alkyl, C₂₋₄ alkenyl and C₃₋₆ cycloalkyl is independently unsubstituted or substituted with 1, 2, 3 or 4 R^{z};
R^{6a} is H, deuterium, F, Cl, Br, CN, OH, C₁₋₄alkyl or C₂₋₄ alkenyl,wherein each of the C₁₋₄ alkyl and C₂₋₄ alkenyl is independently unsubstituted or substituted with 1, 2, 3 or 4 R^{z};
R² is phenyl, 5- to 6-membered heteroaryl, R¹³-O-C(=O)-(CR^{e}R^{f})ₘ-, wherein m is 0, R¹⁴-S(=O)₂-N(R^{g})-C(=O)-, R^{c}C(=O)-, R^{a}R^{b}NC(=O)- or R^{d}OC(=O)-, wherein each of the phenyland 5- to 6-membered heteroaryl is independently unsubstituted or substituted with 1, 2, 3 or 4 R^{w};
each of R⁷ and R⁸ is independently H, deuterium, F, Cl, Br, hydroxy, cyano or C₁₋₆ alkyl;
R³ is 5- to 6-membered heterocyclyl, phenyl, 5- to 6-membered heteroaryl R¹³-(CR^{e}R^{f})ₘ-O- or R¹⁰O-, wherein each of the 5- to 6-membered heterocyclyl, phenyl and 5- to 6-membered heteroaryl is independently unsubstituted or substituted with 1, 2, 3 or 4 R^{w};
R¹⁰ is H, deuterium, C₁₋₆ alkyl, C₂₋₆ alkynyl, C₃₋₆ cycloalkyl, 5- to 6-membered heterocyclyl, phenyl, naphthyl or 5- to 6-membered heteroaryl, wherein each of the C₁₋₆ alkyl, C₂₋₆ alkynyl, C₃₋₆ cycloalkyl, 5- to 6-membered heterocyclyl, phenyl, naphthyl and 5- to 6-membered heteroaryl is independently unsubstituted or substituted with 1, 2, 3 or 4 R^{x};
each R¹ and R¹⁴ is independently C₁₋₁₂ alkyl, C₁₋₁₂ alkoxy, C₃₋₇ cycloalkyl, 5- to 10-membered heteroaryl, 3- to 12-membered heterocyclyl or C₆₋₁₀ aryl, wherein each of the C₁₋₁₂ alkyl, C₁₋₁₂ alkoxy, C₃₋₇ cycloalkyl, 5- to 10-membered heteroaryl, 3- to 12-membered heterocyclyl and C₆₋₁₀ aryl is independently unsubstituted or substituted with 1, 2, 3 or 4 R^{h};
each R^{a}, R^{b}, R^{c}, R^{d}, R^{e}, R^{f} and R^{g} is independently H, deuterium, OH, C₁₋₄ haloalkyl, C₁₋₆ alkyl, C₁₋₄ alkoxy, C₂₋₄ alkenyl, C₂₋₄ alkynyl, C₃₋₆ cycloalkyl, phenyl, naphthyl, 5- to 6-membered heterocyclyl or 5- to 6-membered heteroaryl, wherein each of the C₁₋₄ haloalkyl, C₁₋₆ alkyl, C₁₋₄ alkoxy, C₂₋₄ alkenyl, C₂₋₄ alkynyl, C₃₋₆ cycloalkyl, phenyl, naphthyl, 5- to 6-membered heterocyclyl and 5- to 6-membered heteroaryl is independently unsubstituted or substituted with 1, 2, 3 or 4 substituents independently selected from F, Cl, Br, CN, OH, amino, C₁₋₄ alkyl, C₁₋₄ haloalkyl, C₁₋₄ alkoxy or C₁₋₄ alkylamino;
or R^{a} and R^{b}, together with the nitrogen atom to which they are attached, form a 5- to 6-membered heterocyclyl or 5- to 6-membered heteroaryl, wherein each of the 5- to 6-membered heterocyclyl and 5- to 6-membered heteroaryl is independently unsubstituted or substituted with 1, 2, 3 or 4 substituents independently selected from F, Cl, Br, CN, OH, amino, C₁₋₄ alkyl, C₁₋₄ haloalkyl, C₁₋₄ alkoxy or C₁₋₄ alkylamino;
each R^{v}, R^{w}, R^{x}, R^{y}, R^{z} and R^{h} is independently F, Cl, Br, CN, OH, -COOH, amino, C₁₋₆ alkyl, C₁₋₆ haloalkyl, C₁₋₆ alkoxy, C₁₋₆ haloalkoxy, C₁₋₆ alkylthio, C₁₋₆ alkylamino, C₂₋₄ alkenyl, C₂₋₄ alkynyl, C₃₋₆ cycloalkyl, 5- to 6-membered heterocyclyl, phenyl, naphthyl or 5- to 6-membered heteroaryl, wherein each of the amino, C₁₋₆ alkyl, C₁₋₆ haloalkyl, C₁₋₆ alkoxy, C₁₋₆ haloalkoxy, C₁₋₆ alkylthio, C₁₋₆ alkylamino, C₂₋₄ alkenyl, C₂₋₄ alkynyl, C₃₋₆ cycloalkyl, 5- to 6-membered heterocyclyl, phenyl, naphthyl and 5- to 6-membered heteroaryl is independently unsubstituted or substituted with 1, 2, 3 or 4 substituents independently selected from F, Cl, Br, CN, OH, =O, -COOH, amino, C₁₋₄ alkyl, C₁₋₄ haloalkyl, C₁₋₄ alkoxy, C₁₋₄ alkylthio, C₁₋₄ haloalkoxy, C₁₋₄ alkylamino, C₂₋₄ alkenyl, C₂₋₄ alkynyl, phenyl, naphthyl, C₃₋₆ cycloalkyl, 5- to 6-membered heteroaryl, 5- to 6-membered heterocyclyl, C₁₋₄ alkoxy-C₁₋₄-alkyl or C₁₋₄ alkylamino-C₁₋₄-alkyl;
m is 0, 1, 2, 3, 4, 5, 6, 7, 8, 9 or 10.

In other embodiments, R² is furyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidinyl, phenyl, R¹³-O-C(=O)-(CR^{e}R^{f})ₘ-, wherein each of the furyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidinyl and phenyl is independently unsubstituted or substituted with 1, 2, 3 or 4 R^{w}; and
each R¹³, R^{e}, R^{f} and R^{w} is as defined herein.

In some embodiments, each of R⁷ and R⁸ is independently H, deuterium, F, Cl, Br, hydroxy, cyano, methyl, ethyl, *n*-propyl, isopropyl, *n*-butyl, isobutyl, *n*-pentyl or *n*-hexyl;
R³ is pyrrolidinyl, pyrazolidinyl, imidazolidinyl, tetrahydrofuranyl, tetrahydrothiophenyl, tetrahydropyranyl, tetrahydrothiapyranyl, piperidyl, morpholinyl, thiomorpholinyl, piperazinyl, furyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidinyl, phenyl, R¹³-(CR^{e}R^{f})ₘ-O- or R¹⁰O-, and wherein each of the pyrrolidinyl, pyrazolidinyl, imidazolidinyl, tetrahydrofuranyl, tetrahydrothiophenyl, tetrahydropyranyl, tetrahydrothiapyranyl, piperidyl, morpholinyl, thiomorpholinyl, piperazinyl, furyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidinyl and phenyl and is independently unsubstituted or substituted with 1, 2, 3 or 4 R^{w};
R¹⁰ is H, deuterium, methyl, ethyl, *n*-propyl, isopropyl, *n*-butyl, isobutyl, *n*-pentyl, *n*-hexyl, ethynyl, propynyl, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidyl, morpholinyl, thioxomorpholinyl, piperazinyl, furyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidinyl, phenyl or naphthyl, wherein each of the methyl, ethyl, *n*-propyl, isopropyl, *n*-butyl, isobutyl, *n*-pentyl, *n*-hexyl, ethynyl, propynyl, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidyl, morpholinyl, thioxomorpholinyl, piperazinyl, furyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidinyl, phenyl and naphthyl is independently unsubstituted or substituted with 1, 2, 3 or 4 R^{x};
each m, R¹³, R^{e}, R^{f}, R^{x} and R^{w} is as defined herein.

In other embodiments, each of R⁴ and R⁵ is independently H, deuterium, C₁₋₃ alkyl, C₁₋₃ alkylamino or C₁₋₃ alkoxy, wherein each of the C₁₋₃ alkyl, C₁₋₃ alkylamino and C₁₋₃ alkoxy is independently unsubstituted or substituted with 1, 2, 3, or 4 R^{y};
R⁶ is H, deuterium, methyl, ethyl, *n*-propyl, isopropyl, *n*-butyl, isobutyl, *tert*-butyl, vinyl, propenyl, cyclopropyl, cyclobutyl, cyclopentyl or cyclohexylwherein each of the methyl, ethyl, *n*-propyl, isopropyl, *n*-butyl, isobutyl, *tert*-butyl, vinyl, propenyl, cyclopropyl, cyclobutyl and cyclopentyl, is independently unsubstituted or substituted with 1, 2, 3, or 4 R^{z};
R^{6a} is H, deuterium, F, Cl, Br, CN, OH, methyl, ethyl, *n*-propyl, isopropyl, *n*-butyl, isobutyl, *tert*-butyl, vinyl or propenyl, wherein each of the methyl, ethyl, *n*-propyl, isopropyl, *n*-butyl, isobutyl, *tert*-butyl, vinyl and propenyl is independently unsubstituted or substituted with 1, 2, 3, or 4 R^{z}; and
each R^{z} and R^{y} is as defined herein.

In some embodiments, each R¹³and R¹⁴ is independently C₁₋₆ alkyl, C₁₋₆ alkoxy, C₃₋₆ cycloalkyl, 5- to 6-membered heteroaryl, 5- to 6-membered heterocyclyl, phenyl or naphthyl, wherein each of the C₁₋₆ alkyl, C₁₋₆ alkoxy, C₃₋₆ cycloalkyl, 5- to 6-membered heteroaryl, 5- to 6-membered heterocyclyl, phenyl and naphthyl is independently unsubstituted or substituted with 1, 2, 3 or 4 R^{h};
each R^{h} is as defined herein.

In other embodiments, each R¹³and R¹⁴ is independently methyl, ethyl, *n*-propyl, isopropyl, *n*-butyl, isobutyl, *tert*-butyl, methoxy, ethoxy, propoxy, isopropoxy, *n*-butoxy, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidyl, morpholinyl, thiomorpholinyl, piperazinyl, furyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidinyl, phenyl or naphthyl, wherein each of the methyl, ethyl, *n*-propyl, isopropyl, *n*-butyl, isobutyl, *tert*-butyl, methoxy, ethoxy, propoxy, isopropyl, *n*-butoxy, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidyl, morpholinyl, thiomorpholinyl, piperazinyl, furyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidinyl, phenyl and naphthyl is independently unsubstituted or substituted with 1, 2, 3, or 4 R^{h};
each R^{h} is as defined herein.

In other embodiments, each R^{a}, R^{b}, R^{c}, R^{d}, R^{e}, R^{f}and R^{g} is independently H, deuterium, OH, C₁₋₃ haloalkyl, methyl, ethyl, *n*-propyl, isopropyl, *n*-butyl, isobutyl, *tert*-butyl, *n*-pentyl, 3-pentyl, methoxy, ethoxy, propoxy, isopropoxy, *n*-butoxy, ethenyl, propenyl, ethynyl, propynyl, propargyl, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidyl, morpholinyl, thiomorpholinyl, piperazinyl, furyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidinyl, phenyl or naphthyl, wherein each of the C₁₋₃ haloalkyl, methyl, ethyl, *n*-propyl, isopropyl, *n*-butyl, isobutyl, *tert*-butyl, methoxy, ethoxy, propoxy, isopropoxy, *n*-butoxy, ethenyl, propenyl, ethynyl, propynyl, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidyl, morpholinyl, thiomorpholinyl, piperazinyl, furyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidinyl, phenyl and naphthyl is independently unsubstituted or substituted with 1, 2, 3, or 4 substituents independently selected from F, Cl, Br, CN, OH, amino, C₁₋₄ alkyl, C₁₋₄ haloalkyl, C₁₋₄ alkoxy or C₁₋₄ alkylamino;
or, R^{a} and R^{b}, together with the nitrogen atom to which they are attached, form pyrrolidinyl, pyrazolidinyl, imidazolidinyl, piperidyl, morpholinyl, thiomorpholinyl, piperazinyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, pyrazinyl, pyridazinyl or pyrimidinyl, wherein each of the pyrrolidinyl, pyrazolidinyl, imidazolidinyl, piperidyl, morpholinyl, thiomorpholinyl, piperazinyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, pyrazinyl, pyridazinyl and pyrimidinyl is independently unsubstituted or substituted with 1, 2, 3, or 4 substituents independently selected from F, Cl, Br, CN, OH, amino, C₁₋₄ alkyl, C₁₋₄ haloalkyl, C₁₋₄ alkoxy or C₁₋₄ alkylamino.

In other embodiments, each R^{v}, R^{w}, R^{x}, R^{y}, R^{z} and R^{h} is independently F, Cl, Br, CN, OH, -COOH, amino, C₁₋₄ alkyl, C₁₋₄ haloalkyl, C₁₋₄ alkoxy, C₁₋₄ haloalkoxy, C₁₋₄ alkylthio, C₁₋₄ alkylamino, C₂₋₄ alkenyl, C₂₋₄ alkynyl, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidyl, morpholinyl, thiomorpholinyl, piperazinyl, furyl, pyrrolyl, pyridyl, pyrazolyl, imidazolyl, triazolyl, tetrazolyl, oxazolyl, isoxazolyl, dioxazolyl, 1,3,5-triazinyl, thiazolyl, thienyl, pyrazinyl, pyridazinyl, pyrimidinyl, phenyl or naphthyl, wherein each of the cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidyl, morpholinyl, thiomorpholinyl, piperazinyl, furyl, pyrrolyl, pyridyl, pyrazolyl, imidazolyl, triazolyl, tetrazolyl, oxazolyl, isoxazolyl, dioxazolyl, 1,3,5-triazinyl, thiazolyl, thienyl, pyrazinyl, pyridazinyl, pyrimidinyl, phenyl and naphthyl is independently unsubstituted or substituted with 1, 2, 3 or 4 substituents independently selected from F, Cl, Br, CN, OH, =O, -COOH, amino, C₁₋₄ alkyl, C₁₋₄ haloalkyl, C₁₋₄ alkoxy, C₁₋₄ alkylthio, C₁₋₄ haloalkoxy, C₁₋₄ alkylamino, C₂₋₄ alkenyl, C₂₋₄ alkynyl, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidyl, morpholinyl, thiomorpholinyl, piperazinyl, furyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidinyl, phenyl, naphthyl, C₁₋₃ alkoxy-C₁₋₃-alkyl and C₁₋₃ alkylamino-C₁₋₃-alkyl.

In another aspect, provided herein is a pharmaceutical composition comprising the compound of the invention; optionally, wherein the pharmaceutical composition further comprises a pharmaceutically acceptable excipient, or a combination of the excipients.

In some embodiments, the pharmaceutical composition disclosed herein further comprises other anti-HBV drug.

In some embodiments, the pharmaceutical composition disclosed herein, wherein the other anti-HBV drug is a HBV polymerase inhibitor, an immunomodulator or an interferon.

In some embodiments, the pharmaceutical composition disclosed herein, wherein the other anti-HBV agent is lamivudine, telbivudine, tenofovir, entecavir, adefovir dipivoxil, alfaferone, alloferon, celmoleukin, clevudine, emtricitabine, famciclovir, interferon, hepatect CP, intefen, interferon α-1b, interferon α, interferon α-2a, interferon β-1a, interferon α-2, interleukin-2, mivotilate, nitazoxanide, peginterferon alfa-2a, ribavirin, roferon-A, sizofiran, euforavac, ampligen, phosphazid, heplisav, interferon α-2b, levamisole or propagermanium.

In another aspect, provided herein is the compound or the pharmaceutical composition of the invention in the manufacture of a medicament for use in preventing, treating or lessening a disorder or disease caused by a virus infection in a patient.

In some embodiments, the use disclosed herein, wherein the virus disease is hepatitis B infection or a disease caused by hepatitis B infection.

In other embodiments, the use disclosed herein, wherein the disease caused by hepatitis B infection is cirrhosis or hepatocellular carcinogenesis.

In another aspect, provided herein is the compound of the invention in the manufacture of a medicament for ues in preventing, managing or treating a HBV disorder or disease in a patient, or lessening the severity of the HBV disorder or disease in a patient.

In another aspect, provided herein is the pharmaceutical composition compirsing the compound of the invention in the manufacture of a medicament for ues in preventing, managing or treating a HBV disorder or disease in a patient, or lessening the severity of the HBV disorder or disease in a patient.

In another aspect, provided herein is the compound or the pharmaceutical composition of the invention in the manufacture a medicament for use in inhibiting the production or secretion of HBsAg, and/or for inhibiting the production of HBV DNA.

In another aspect, provided herein is the compound or the pharmaceutical composition of the invention for use in preventing, treating or lessening a disorder or disease caused by a virus infection in a patient.

In some embodiments, the compound or the pharmaceutical composition disclosed herein, wherein the virus disease is hepatitis B infection or a disease caused by hepatitis B infection.

In other embodiments, the compound or the pharmaceutical composition disclosed herein, wherein the disease caused by hepatitis B infection is cirrhosis or hepatocellular carcinogenesis.

In another aspect, provided herein is the compound or the pharmaceutical composition of the invention for use in inhibiting the production or secretion of HBsAg, and/or in inhibiting the production of HBV DNA in a patient.

In another aspect, provided herein is a method of preventing, treating or lessening a HBV disorder or disease in a patient comprising administering to the patient a therapeutically effective amount of the compound of the invention.

In another aspect, provided herein is a method of preventing, treating or lessening a HBV disorder or disease in a patient comprising administering to the patient a therapeutically effective amount of the pharmaceutical composition compring the compound of the invention.

In another aspect, provided herein is a method of inhibiting the production or secretion of HBsAg, and/or inhibiting the production of HBV DNA in a patient, comprising administering to the patient a therapeutically effective amount of the compound or the pharmaceutical composition of the invention.

In another aspect, provided herein is a method of inhibitingHBV infection, comprising contacting a cell with an amount of the compound or composition of the invention that is effective to inhibit HBV. In other embodiments, the method further comprises contacting the cell with an anti-HBV agent.

In another aspect, provided herein is a method of treating HBV disorder or disease in a patient, comprising administering to the patient an effective therapeutic amount of the compound or composition of the invention. In other embodiments, the method further comprises administration of other HBV therapy.

In another aspect, provided herein is a method of inhibiting HBV infection in a patient, comprising administering to the patient an effective therapeutic amount of the compound or composition of the invention. In other embodiments, the method further comprises administration of other HBV therapy.

In other aspect, provided herein is a method of preparing, separating or purifying the compound of Formula (I).

The foregoing merely summarizes certain aspects disclosed herein and is not intended to be limiting in nature. These aspects and other aspects and embodiments are described more fully below.

### DETAILED DESCRIPTION OF THE INVENTION

### DEFINITIONS AND GENERAL TERMINOLOGY

Reference will now be made in detail to certain embodiments disclosed herein, examples of which are illustrated in the accompanying structures and formulas. The invention is intended to cover all alternatives, modifications, and equivalents that may be included within the scope disclosed herein. One skilled in the art will recognize many methods and materials similar or equivalent to those described herein, which could be used in the practice of the present invention. The present invention is in no way limited to the methods and materials described herein. In the event that one or more of the incorporated literature, patents, and similar materials differs from or contradicts this application, including but not limited to defined terms, term usage, described techniques, or the like, this application prevails.

As used herein, the following definitions shall be applied unless otherwise indicated. For purposes disclosed herein, the chemical elements are identified in accordance with the *Periodic Table of the Elements,* CAS version, and *the* Handbook of Chemistry and Physics, 75 thEd. 1994. Additionally, general principles of organic chemistry are described in Sorrell et al., "Organic Chemistry", University Science Books, Sausalito: 1999, and "March's Advanced Organic Chemistry", by Michael B. Smith and Jerry March, John Wiley & Sons, New York: 2007.

As described herein, compounds disclosed herein may optionally be substituted with one or more substituents, such as are illustrated generally below, or as exemplified by particular classes, subclasses, and species of the invention. In general, the term "substituted" refers to the replacement of one or more hydrogen radicals in a given structure with the radical of a specified substituent. Unless otherwise indicated, an optionally substituted group may have a substituent at each substitutable position of the group. When more than one position in a given structure can be substituted with more than one substituent selected from a specified group, the substituent may be either the same or different at each position. Wherein the substitutents may be, but are not limited to F, Cl, Br, CN, OH, -COOH, amino, C₁₋₁₂ alkyl, C₁₋₁₂ haloalkyl, C₁₋₁₂ alkoxy, C₁₋₁₂ haloalkoxy, C₁₋₁₂ alkylthio, C₁₋₁₂ alkylamino, C₂₋₆ alkenyl, C₂₋₆ alkynyl, C₃₋₇ cycloalkyl, 3- to 12-membered heterocyclyl, C₆₋₁₀ aryl or 5- to 10-membered heteroarylo, and the like.

At various places in the present specification, substituents of compounds disclosed herein are disclosed in groups or in ranges. It is specifically intended that the invention include each and every individual subcombination of the members of such groups and ranges. For example, the term "C₁₋C₆ alkyl"or "C₁₋₆ alkyl" is specifically intended to individually disclose methyl, ethyl, C₃ alkyl, C₄ alkyl, C₅ alkyl and C₆ alkyl.

Furthermore, what need to be explained is that the phrase "each...is independently", "each... and... is independently" and "each of... and... is independently", unless otherwise stated, should be used exchangeably and broadly understood. The specific options expressed by the same symbol are independent of each other in different groups; or the specific options expressed by the same symbol are independent of each other in same groups.

The term "alkyl" or "alkyl group" refers to a saturated linear or branched-chain monovalent hydrocarbon radical of 1 to 20 carbon atoms, wherein the alkyl radical may be optionally and independently substituted with one or more substituents described herein. In some embodiments, the alkyl group contains 1-12 carbon atoms. In other embodiments, the alkyl group contains 1-8 carbon atoms. In other embodiments, the alkyl group contains 1-6 carbon atoms. In other embodiments, the alkyl group contains 2-6 carbon atoms. In still other embodiments, the alkyl group contains 1-4 carbon atoms. In yet other embodiments, the alkyl group contains 2-4 carbon atoms and in still yet other embodiments, the alkyl group contains 1-3 carbon atoms. Some non-limiting examples of the alkyl group include, methyl (Me, -CH₃), ethyl (Et, -CH₂CH₃), *n*-propyl (*n*-Pr, -CH₂CH₂CH₃), isopropyl (*i*-Pr, -CH(CH₃)₂), *n*-butyl (*n*-Bu, -CH₂CH₂CH₂CH₃), 2-methylpropyl or isobutyl (*i*-Bu, -CH₂CH(CH₃)₂), 1-methylpropyl or *sec*-butyl (*s*-Bu, -CH(CH₃)CH₂CH₃), *tert*-butyl (*t*-Bu, -C(CH₃)₃), *n*-pentyl (-CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-l-butyl (-CH₂CH₂CH(CH₃)₂), 2-methyl-l-butyl (-CH₂CH(CH₃)CH₂CH₃), n-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3, 3-dimethyl-2-butyl (-CH(CH₃)C(CH₃)₃), *n*-heptyl and *n*-octyl, *etc.* The term "alkyl" or the prefix "alk-" is inclusive of both straight chain and branched saturated carbon chain. The term "halogenated aliphatic" as used herein refers to an aliphatic group that is substituted with one or more of the same or different halogen atoms, wherein the aliphatic or alkyl group are as defined herein, and the halogen atom is fluorine, chlorine, bromine or iodine, and such examples include, but are not limited to, trifluoromethyl, trifluoroethyl, and the like.

The terms "haloalkyl", "haloalkenyl" or "haloalkoxy" refer to alkyl, alkenyl or alkoxy, as the case may be, substituted with one or more halogen atoms. In some embodiments, the haloalkyl group contains 1-12 carbon atoms. In other embodiments, the haloalkyl group contains 1-10 carbon atoms. In other embodiments, the haloalkyl group contains 1-8 carbon atoms. In still other embodiments, the haloalkyl group contains 1-6 carbon atoms. In yet other embodiments, the haloalkyl group contains 1-4 carbon atoms; and in still yet other embodiments, the haloalkyl group contains 1-3 carbon atoms. Such examples include, but are not limited to, trifluoromethyl, trifluoroethyl, trifluoromethoxy, 2-fluorovinyl, *etc.* Wherein each of the haloalkyl, haloalkenyl and haloalkoxy may be independently unsubstituted or substituted with one or more substituents described herein.

The terms "carboxy" or "carboxyl", whether used alone or with other terms, such as "carboxyalkyl", refer to -CO₂H or -COOH.

The term "carbonyl", whether used alone or with other terms, such as "aminocarbonyl", "acyloxy", denotes -(C=O)-.

The term "alkylamino" refers to "*N*-alkylamino" and "*N,N-*dialkylamino" wherein amino groups are independently substituted with one alkyl radical or two alkyl radicals, respectively. In some embidiments, the alkylamino radical is "lower alkylamino" radical having one or two C₁₋₁₂ alkyl groups attached to a nitrogen atom. In some embodiments, the alkylamino radical refers to C₁₋₆ lower alkylamino group. In some embodiments, the alkylamino radical refers to C₁₋₄ lower alkylamino group. The suitable alkylamino group include monoalkylamino or dialkylamino, such examples include, but are not limited to *N*-methylamino, *N*-ethylamino, *N,N*-dimethylamino, *N*,*N*-diethylamino, *N*-propylamino, *N*,*N*-dipropylamino, and the like, wherein the alkylamino group may be independently unsubstituted or substituted with one or more substituents described herein.

The term "alkylene" refers to a saturated divalent hydrocarbon group derived from a straight or branched chain saturated hydrocarbon by the removal of two hydrogen atoms. Unless otherwise specified, the alkylene group contains 1-12 carbon atoms. In other embodiments, the alkylene group contains 1-6 carbon atoms. In other embodiments, the alkylene group contains 1-4 carbon atoms. In still other embodiments, the alkylene group contains 1-3 carbon atoms. In yet other embodiments, the alkylene group contains 1-2 carbon atoms. Examples of such group include, methylene (-CH₂-), ethylene (-CH₂CH₂-), propylene (-CH₂CH₂CH₂-) , isopropylene (-CH(CH₃)CH₂-), butylene (-CH₂CH₂CH₂CH₂-), pentylene (-CH₂CH₂CH₂CH₂CH₂-), hexylene (-CH₂CH₂CH₂CH₂CH₂CH₂-), heptylene (-CH₂CH₂CH₂CH₂CH₂CH₂CH₂-), octylene (-CH₂CH₂CH₂CH₂CH₂CH₂CH₂CH₂-), and the like. Wherein the alkylene group may independently be unsubstituted or substituted by one or more substituents described herein.

The term "alkenyl" refers to a linear or branched chain monovalent hydrocarbon radical of 2 to 12 carbon atoms, or 2 to 8 carbon atoms, or 2 to 6 carbon atoms, or 2 to 4 carbon atoms, with at least one site of unsaturation, *i*.*e*., a carbon-carbon, sp² double bond, wherein the alkenyl radical may be independently unsubstituted or substituted with one or more substituents described herein, and includes radicals having "*cis*" and "*trans*" orientations, or alternatively, "*E*" and "*Z*" orientations. Specific examples of the alkenyl group include, but are not limited to, vinyl (-CH=CH₂), allyl (-CH₂CH=CH₂), and the like. Wherein the alkenyl group may independently be unsubstituted or substituted by one or more substituents described herein.

The term "alkynyl" refers to a linear or branched chain monovalent hydrocarbon radical of 2 to 12 carbon atoms, or 2 to 8 carbon atoms, or 2 to 6 carbon atoms, or 2 to 4 carbon atoms, with at least one site of unsaturation, i.e., a carbon-carbon, sp triple bond, wherein the alkynyl radical may be independently unsubstituted or substituted with one or more substituents described herein. Specific examples of the alkynyl group include, but are not limited to, ethynyl (-C=CH), propargyl (-CH₂C≡CH), 1-propinyl (-C≡C-CH₃), butyl-1-yne (-CH₂CH₂C≡CH), butyl-2-yne (-CH₂C≡CCH₃), butyl-3-yne (-C≡CCH₂CH₃), and the like. The alkynyl group may independently be unsubstituted or substituted by one or more substituents described herein.

The term "alkoxy" refers to an alkyl group, as previously defined, attached to the parent molecular moiety via an oxygen atom. Unless otherwise specified, the alkoxy group contains 1-20 carbon atoms. In other embodiments, the alkoxy group contains 1-12 carbon atoms. In other embodiments, the alkoxy group contains 1-8 carbon atoms. In still other embodiments, the alkoxy group contains 1-6 carbon atoms. In yet other embodiments, the alkoxy group contains 1-4 carbon atoms; and in still yet other embodiments, the alkoxy group contains 1-3 carbon atoms.

Some non-limiting examples of alkoxy group include, methoxy (MeO, -OCH₃), ethoxy (EtO, -OCH₂CH₃), 1-propoxy (*n*-PrO, *n*-propoxy, -OCH₂CH₂CH₃), 2-propoxy (*i*-PrO, *i*-propoxy, -OCH(CH₃)₂), 1-butoxy (*n*-BuO, *n*-butoxy, -OCH₂CH₂CH₂CH₃), 2-methyl-l-propoxy (*i*-BuO, *i*-butoxy, -OCH₂CH(CH₃)₂), 2-butoxy (*s*-BuO, *s*-butoxy, -OCH(CH₃)CH₂CH₃), 2-methyl-2-propoxy (*t*-BuO, *t*-butoxy, -OC(CH₃)₃), 1-pentoxy (*n*-pentoxy, -OCH₂CH₂CH₂CH₂CH₃), 2-pentoxy (-OCH(CH₃)CH₂CH₂CH₃), 3-pentoxy (-OCH(CH₂CH₃)₂), 2-methyl-2-butoxy (-OC(CH₃)₂CH₂CH₃), 3-methyl-2-butoxy (-OCH(CH₃)CH(CH₃)₂), 3-methyl-l-butoxy (-OCH₂CH₂CH(CH₃)₂), 2-methyl-l-butoxy (-OCH₂CH(CH₃)CH₂CH₃), and the like. Wherein the alkoxy group is independently unsubstituted or substituted with one or more substitutents disclosed herein.

The term "carbocycle", "carbocyclyl", "carbocyclic" or "carbocyclic ring" used interchangeablely herein refers to a non-aromatic carbocyclic ring system having 3 to 14 ring carbon atoms, which is saturated or contains one or more units of unsaturation. In some embodiments, the number of carbon atom is 3 to 12; in other embodiments, the number of carbon atom is 3 to 10; in other embodiments, the number of carbon atom is 3 to 8; in other embodiments, the number of carbon atom is 3 to 6; in other embodiments, the number of carbon atom is 5 to 6; in other embodiments, the number of carbon atom is 5 to 8; in other embodiments, the number of carbon atom is 6 to 8. The "carbocyclyl" includes a monocyclic, bicyclic, or polycyclic fused ring, spiro ring or bridged ring system, and a polycyclic ring system containg one carbocyclic ring fused with one or more non-aromatic carbocyclic ring or heterocyclic ring, or one or more aromatic ring, or a combination thereof, wherein the linked group or point exsits on carbocyclic ring. The bicyclic carbocyclyl groups includes bridged bicyclic carbocyclyl, fused bicyclic carbocyclyl and spiro bicyclic carbocyclyl group, and fused bicyclic system contains two rings which share two adjacent ring atoms. Bridged bicyclic group contains two rings which share three or four adjacent ring atoms. Spiro bicyclic system contains two rings which share one ring atom. Some non-limiting examples of the cycloaliphatic group include cycloalkyl, cycloalkenyl and cycloalkynyl. Further examples of carbocyclyl groups include cyclopropyl, cyclobutyl, cyclopentyl, 1-cyclopent-l-enyl, l-cyclopent-2-enyl, 1-cyclopent-3-enyl, cyclohexyl, 1-cyclohex-l-enyl, l-cyclohex-2-enyl, l-cyclohex-3-enyl, cyclohexadienyl, cycloheptyl, cyclooctyl, cyclononyl, cyclodecyl, cycloundecyl, cyclododecyl, and the like. Bridged bicyclic carbocyclyl group includes, but are not limited to, bicyclo[2.2.2]octyl, bicyclo[2.2.1]heptyl, bicyclo[3.3.1]nonyl, bicyclo[3.2.3]nonyl, and the like.

The term "cycloalkyl" refers to a saturated ring having 3 to 12 ring carbon atoms as a monocyclic, bicyclic, or tricyclic ring system, which has one or more attachements attaching to the rest of the molecule. In some embodiments, the cycloalkyl group contains 3 to 10 ring carbon atoms. In other embodiments, the cycloalkyl group contains 3 to 8 ring carbon atoms. In other embodiments, the cycloalkyl group contains 3 to 7 ring carbon atoms. In other embodiments, the cycloalkyl group contains 5 to 8 ring carbon atoms. In still other embodiments, the cycloalkyl group contains 3 to 6 ring carbon atoms. In yet other embodiments, the cycloalkyl group contains 5 to 6 ring carbon atoms. Examples of cycloalkyl group include, but are not limited to, cyclopropyl, cyclobutyl, cyclopentyl, cyclohexyl, and the like. The cycloalkyl radical is independently unsubstituted or substituted with one or more substituents described herein.

The term "heterocycle", "heterocyclyl", or "heterocyclic ring" as used interchangeably herein refers to a saturated or partially unsaturated non-aromatic monocyclic, bicyclic or tricyclic ring containing 3-12 ring atoms of which at least one ring atom is selected from nitrogen, sulfur and oxygen, and of which may has one ore more attachments attached to the rest of the molecule. The term "heterocyclyl" includes a monocyclic, bicyclic, or polycyclic fused, spiro, bridged heterocyclic ring system, and a polycyclic ring system containg one heterocyclic ring fused with one or more non-aromatic carbocyclic ring or heterocyclic ring, or one or more aromatic ring, or a combination thereof, wherein the linked group or attachment exsits on heterocyclic ring. Biheterocyclyl radical includes bridged biheterocyclyl, fused biheterocyclyl and spiro biheterocyclyl. Unless otherwise specified, the heterocyclyl group may be carbon or nitrogen linked, and a -CH₂- group can be optionally replaced by a -C(=O)- group. In which, the sulfur can be optionally oxygenized to *S*-oxide. and the nitrogen can be optionally oxygenized to *N*-oxide. In some embodiments, the heterocyclyl group is a 3- to 12-membered ring system; in other embodiments, the heterocyclyl group is a 3- to 8-membered ring system; in other embodiments, the heterocyclyl group is a 3- to 6-membered ring system; in other embodiments, the heterocyclyl group is a 5- to 7-membered ring system; in other embodiments, the heterocyclyl group is a 5- to 8-membered ring system; in other embodiments, the heterocyclyl group is a 6- to 8-membered ring system; in other embodiments, the heterocyclyl group is a 5- to 6-membered ring system; in other embodiments, the heterocyclyl group is a 3-membered ring system; in other embodiments, the heterocyclyl group is a 4-membered ring system; in other embodiments, the heterocyclyl group is a 5-membered ring system; in other embodiments, the heterocyclyl group is a 6-membered ring system; in other embodiments, the heterocyclyl group is a 7-membered ring system; in other embodiments, the heterocyclyl group is a 8-membered ring system.

Examples of heterocyclyl group include, but are not limited to, heterocyclyl group may be a carbon radical or heteroatom radical. The heterocyclyl group also includes a group in which the heterocyclyl group is fused with a saturated or partially unsaturated ring or a heterocyclic ring. Examples of heterocyclyl group include, but are not limited to, pyrrolidinyl, tetrahydrofuranyl, dihydrofuranyl, tetrahydrothiophenyl, tetrahydropyranyl, dihydropyranyl, tetrahydrothiopyranyl, piperidyl, morpholinyl, thiomorpholinyl, thioxanyl, piperazinyl, homopiperazinyl, azetidinyl, oxetanyl, thietanyl, homopiperidyl, glycidyl, azacycloheptyl, oxacycloheptyl, thiacycloheptyl, oxazepinyl, diazepinyl, thiazepinyl, 2-pyrrolinyl, 3-pyrrolinyl, dihydroindolyl, 2H-pyranyl, 4H-pyranyl, dioxanyl, 1,3-dioxolanyl, pyrazolinyl, dithianyl, dithiolanyl, dihydrothiophenyl, pyrazolidinyl, imidazolinyl, imidazolidinyl, 1,2,3,4-tetrahydroisoquinolinyl, 3-azabicyclo[3.1.0]hexyl, 3-azabicyclo[4.1.0]heptyl, azabicyclo[2.2.2]hexyl, 3*H*-indolylquinolizinyl and *N*-pyridyl urea. Examples of heterocyclyl group also include, 1,1-dioxythiomorpholinyl; wherein examples of ring carbon atom is replaced with oxo (=O) group include, but are not limited to, pyrimidinyl-dione, 1,2,4-thiadiazol-5(4*H*)-yl-one, 1,2,4-oxadiazol-5(4*H*)-yl-one, 1*H*-1,2,4-triazol-5 (4*H* )-yl-one, and the like; examples of the ring carbon atom is replaced with a =S group include, but are not limited to, 1,2,4-oxadiazol-5(4*H*)-yl-thione, 1,3,4-oxadiazol-2(3*H*)-yl-thione and so on. The heterocyclyl group may be optionally substituted with one or more substituents disclosed herein.

The terms "heterocyclylalkylene" and "heterocyclylalkyl" are used interchangeably herein to refer that an alkyl group is substituted with 1, 2, 3, or 4 heterocyclyl groups, wherein the heterocyclyl group, alkyl and alkylene group are as defined herein. Some non-limiting examples of such group include pyrrol-2-ylmethyl, morpholin-4-ylmethyl, *etc.*

The term "heterocyclylalkoxy" refers that an alkoxy group is substituted with 1, 2, 3, or 4 heterocyclyl groups, wherein the heterocyclyl group and alkoxy group are as defined herein. Some non-limiting examples of such group include pyrrol-2-ylmethoxy, piperid-2-ylethoxy, *etc.*

The term "heterocyclylalkylamino" refers to an alkylamino group substituted with heterocyclyl group, wherein the nitrogen atom attaches to the rest of the molecule; and wherein the heterocyclyl and alkylamino are as defined herein. Examples of such groups include, but are not limited to, piperazin-2-ylethylamino, morpholin-4-ylpropoxy, morpholin-4-ylethylamino, and the like.

The term "heteroatom" refers to one or more of oxygen (O), sulfur (S), nitrogen (N), phosphorus (P) and silicon (Si), including any oxidized form of nitrogen (N), sulfur (S), or phosphorus (P); primary, secondary, tertiary or quaternary ammonium salts; or a substitutable nitrogen of a heterocyclic ring, for example, N (as in 3,4-dihydro-2*H*-pyrrolyl), NH (as in pyrrolidinyl) or NR (as in *N*-substituted pyrrolidinyl).

The term "halogen" or "halogen atom" refers to fluorine (F), chlorine (Cl), bromine (Br), or iodine (I).

The term "unsaturated" refers to a moiety having one or more units of unsaturation.

The term "aryl" used alone or as a great part of "arylalkyl", "arylalkoxy", or "aryloxyalkyl" refers to monocyclic, bicyclic and tricyclic carbocyclic ring systems having a total of 6 to 14 ring carbon atoms, or 6 to 12 ring carbon atoms, or 6 to 10 ring carbon atoms, wherein at least one ring in the system is aromatic, wherein each ring in the system contains 3 to 7 ring carbon atoms and that has a single point or multipoint of attachment to the rest of the molecule. The term "aryl" may be used interchangeably with the term "aryl ring" or "aromatic ring". Some non-limiting examples of the aryl group include phenyl, naphthyl and anthracene. The aryl group may be optionally unsubstituted or substituted with one or more substituents disclosed herein.

The term "heteroaryl" used alone or as a great part of "heteroarylalkyl" or "heteroarylalkoxy", refers to monocyclic, bicyclic or tricyclic ring system having a total of five to sixteen ring members, wherein at least one ring in the system is aromatic, and in which at least one ring member is selected from heteroatom, and wherein each ring in the system contains 5 to 7 ring members and that has a single point or multipoint of attachment to the rest of the molecule. The term "hetreroaryl" and "heteroaromatic ring" or "heteroaromatic compound" can be used interchangeably herein. In one embodiment, the heteroaryl group is a 5- to 14-membered heteroaryl comprising 1, 2, 3 or 4 heteroatoms independently selected from O, S and N. In another embodiment, the heteroaryl group is a 5- to 12-membered heteroaryl comprising 1, 2, 3 or 4 heteroatoms independently selected from O, S and N. In another embodiment, the heteroaryl group is a 5- to 10-membered heteroaryl comprising 1, 2, 3 or 4 heteroatoms independently selected from O, S and N. In another embodiment, the heteroaryl group is a 5- to 8-membered heteroaryl comprising 1, 2, 3 or 4 heteroatoms independently selected from O, S and N. In another embodiment, the heteroaryl group is a 5- to 7-membered heteroaryl comprising 1, 2, 3 or 4 heteroatoms independently selected from O, S and N. In another embodiment, the heteroaryl group is a 5- to 6-membered heteroaryl comprising 1, 2, 3 or 4 heteroatoms independently selected from O, S and N. In another embodiment, the heteroaryl group is a 5-membered heteroaryl comprising 1, 2, 3 or 4 heteroatoms independently selected from O, S and N. In another embodiment, the heteroaryl group is a 6-membered heteroaryl comprising 1, 2, 3 or 4 heteroatoms independently selected from O, S and N.

In othe embodiments, some non-limiting examples of heteroaryl rings include the following monocyclic ring: 2-furanyl, 3-furanyl, *N*-imidazolyl, 2-imidazolyl, 4-imidazolyl, 5-imidazolyl, 3-isoxazolyl, 4-isoxazolyl, 5-isoxazolyl, 2-oxazolyl, 4-oxazolyl, 5-oxazolyl, *N*-pyrrolyl, 2-pyrrolyl, 3-pyrrolyl, 2-pyridyl, 3-pyridyl, 4-pyridyl, 2-pyrimidinyl, 4-pyrimidinyl, 5-pyrimidinyl, pyridazinyl (*e*.*g*., 3-pyridazinyl), 2-thiazolyl, 4-thiazolyl, 5-thiazolyl, tetrazolyl (e.g., 5*H*-tetrazolyl, 2*H*-tetrazolyl), triazolyl (e.g., 2-triazolyl, 5-triazolyl, 4*H*-1,2,4-triazolyl, 1*H*-1,2,4-triazolyl, 1,2,3-triazolyl), 2-thienyl, 3-thienyl, pyrazolyl (*e*.*g*., 2-pyrazolyl and 3-pyrazolyl), isothiazolyl, 1,2,3-oxadiazolyl, 1,2,5-oxadiazolyl, 1,2,4-oxadiazolyl, 1,3,4-oxadiazolyl, 1,2,3-thiodiazolyl, 1,3,4-thiodiazolyl, 1,2,5-thiodiazolyl, pyrazinyl, 1,3,5-triazinyl, and the following non-limiting bicycles or tricycles: benzimidazolyl, benzofuryl, benzothiophenyl, indolyl (*e*.*g*., 2-indolyl), purinyl, quinolinyl (*e.g.,* 2-quinolinyl, 3-quinolinyl, 4-quinolinyl), isoquinolinyl (*e*.*g*., 1-isoquinolinyl, 3-isoquinolinyl or 4-isoquinolinyl), phenoxathiinyl, dibenzoimidazolyl, dibenzofuranyl, or dibenzothiophenyl, *etc.* The heteroaryl group is optionally substituted with one or more substituents disclosed herein.

The term "heteroarylalkyl" refers to an alkyl group substituted with one or more heteroaryl groups, wherein the alkyl group and heteroaryl group are as defined herein. Some non-limiting examples of the heteroarylalkyl group include (pyrid-2-yl)ethyl, (thiazol-2-yl)methyl, (imidazol-2-yl)ethyl, (pyrimidin-2-yl)propyl, and the like.

The term "sulfonyl", whether used alone or in combination with other terms such as "alkylsulfonyl", respectively denotes the divalent group -SO₂-. The term "alkylsulfonyl" refers to an alkyl-substituted sulfonyl group forming an alkylsulfonyl group (*e*.*g*., -SO₂CH₃).

The term "alkylthio" refers to a linear or branched C₁₋₁₂ alkyl chain binding to a bivalent sulphur atom, wherein the alkyl group is as defined herein. In some embodiments, the alkylthio group is a lower C₁₋₆ alkylthio. In other embodiments, the alkylthio group is a lower C₁₋₆ alkylthio. Some non-limiting examples of such groups include methylthio (CH₃S-), ethylthio, and the like.

The term "aralkyl" or "arylalkyl" refers to an alkyl group substituted with one or more aryl radicals, wherein the aryl and the alkyl group are as defined herein. In some embodiments, the aralkyl group refers to a "lower aralkyl" group having aryl radical(s) attached to a C₁₋₆ alkyl radical. In some embodiments, the "aralkyl" group or "arylalkyl" group is a "phenylalkylene" having C₁₋₃ alkyl radical. Some non-limiting examples of such group include benzyl, diphenylmethyl, phenylethyl, and the like. The aralkyl group may be optionally unsubstituted or substituted with one or more substituents disclosed herein.

The term "haloalkyl-substituted aryl" includes aryl groups that may be substituted with one or more of the same or different haloalkyl groups, wherein the haloalkyl and aryl groups are as described herein. Such examples include, but are not limited to, 2-trifluoromethylphenyl, 3,5-bis(trifluoromethyl)phenyl, 3-trifluoromethylphenyl, 4-trifluoromethylphenyl, 2,6-bis(trifluoromethyl)phenyl and the like.

The term "halo-substituted aryl" includes aryl groups that may be substituted with one or more of the same or different halogen atoms, wherein the halogen atom and aryl groups are as described herein. Such examples include, but are not limited to, fluorophenyl, difluorophenyl, trifluorophenyl, chlorophenyl, dichlorophenyl, trichlorophenyl, bromophenyl, tribromophenyl, dibromophenyl, fluorochlorophenyl, fluorobromophenyl, chlorobromophenyl, and the like.

The term "cycloalkylalkyl" refers to an alkyl group substituted with one or more cycloalkyl groups, wherein the cycloalkyl and alkyl groups are as defined herein. Some non-limiting examples of such group include cyclohexylmethyl, cyclopropylethyl and cyclopropylpropyl, *etc.* The cycloalkyl group may be independently unsubstituted or substituted with one or more substituents disclosed herein.

Furthermore, unless otherwise stated, the phrase "each...is independently" is used interchangeably with the phrase "each (of)...and...is independently". It should be understood broadly that the specific options expressed by the same symbol are independently of each other in different radicals; or the specific options expressed by the same symbol are independently of each other in same radicals. Such as Formula (p1) and Formula (p2), specific options of two m are independent of each other, specific options of two R¹³ are independent of each other, specific option of each R^{e} is independent of each other, specific option of each R^{f} is independent of each other.

R¹³-C(=O)-N(R^{g})-(CR^{e}R^{f})ₘ- Formula (p1);

and

R¹³-O-C(=O)-(CR^{e}R^{f})ₘ- Formula (p2).

Unless otherwise stated, structures depicted herein are also meant to include all isomeric (*e*.*g*., enantiomeric, diastereomeric, and geometric (or conformational)) forms of the structure; for example, the *R* and *S* configurations for each asymmetric center, (*Z*) and (*E*) double bond isomers, and (*Z*) and (*E*) conformational isomers. Therefore, single stereochemical isomers as well as enantiomeric, diastereomeric, or geometric (or conformational)) mixtures of the present compounds are within the scope disclosed herein.

An "*N*-oxide" refers to one or more than one nitrogen atoms oxidised to form an *N*-oxide or *N*-oxides, where a compound contains several amine functions. Particular examples of *N*-oxides are the *N*-oxides of a tertiary amine or a nitrogen atom of a nitrogen-containing heterocycle. *N*-oxides can be formed by treatment of the corresponding amine with an oxidizing agent such as hydrogen peroxide or a per-acid (*e.g*., a peroxycarboxylic acid) (See, Advanced Organic Chemistry, by Jerry March, 4th Edition, Wiley Interscience, pages). More particularly, N-oxides can be made by the procedure of L. W. Deady (Syn. Comm. 1977, 7, 509-514) in which the amine compound is reacted with m-chloroperoxybenzoic acid (MCPBA), for example, in an inert solvent such as dichloromethane.

The term "prodrug" refers to a compound that is transformed in vivo into a compound of Formula (I). Such a transformation can be affected, for example, by hydrolysis of the prodrug form in blood or enzymatic transformation to the parent form in blood or tissue. Prodrugs of the compounds disclosed herein may be, for example, esters. Some common esters which have been utilized as prodrugs are phenyl esters, aliphatic (C₁-C₂₄) esters, acyloxymethyl esters, carbonates, carbamates and amino acid esters. For example, a compound disclosed herein that contains a hydroxy group may be acylated at this position in its prodrug form. Other prodrug forms include phosphates, such as, those phosphate compounds derived from the phosphonation of a hydroxy group on the parent compound. A thorough discussion of prodrugs is provided in T. Higuchi and V. Stella, Pro-drugs as Novel Delivery Systems, Vol. 14 of the A.C.S. Symposium Series, Edward B. Roche, ed., Bioreversible Carriers in Drug Design, American Pharmaceutical Association and Pergamon Press, 1987, J. Rautio et al., Prodrugs: Design and Clinical Applications, Nature Review Drug Discovery, 2008, 7, 255-270, and S. J. Hecker et al., Prodrugs of Phosphates and Phosphonates, Journal of Medicinal Chemistry, 2008, 51, 2328-2345.

Unless otherwise stated, all tautomeric forms of the compounds disclosed herein are within the scope of the invention. Additionally, unless otherwise stated, structures depicted herein are also meant to include compounds that differ only in the presence of one or more isotopically enriched atoms.

A "metabolite" is a product produced through metabolism in the body of a specified compound or salt thereof. The metabolites of a compound may be identified using routine techniques known in the art and their activities may be determined using tests such as those described herein. Such products may result for example from oxidation, reduction, hydrolysis, amidation, deamidation, esterification, deesterification, enzyme cleavage, and the like, of the administered compound. Accordingly, the invention includes metabolites of compounds disclosed herein, including metabolites produced by contacting a compound disclosed herein with a mammal for a sufficient time period.

Stereochemical definitions and conventions used herein generally follow S. P. Parker, Ed., McGraw-Hill Dictionary of Chemical Terms (1984) McGraw-Hill Book Company, NewYork; and Eliel, E. and Wilen, S., "Stereochemistry of Organic Compounds", John Wiley&Sons, Inc., New York, 1994. The compounds disclosed herein may contain asymmetric or chiral centers, and therefore exist in different stereoisomeric forms. It is intended that all stereoisomeric forms of the compounds disclosed herein, including, but not limited to, diastereomers, enantiomers and atropisomers, as well as mixtures thereof such as racemic mixtures, form part of the present invention. Many organic compounds exist in optically active forms, *i*.*e*., they have the ability to rotate the plane of plane-polarized light. In describing an optically active compound, the prefixes *D* and *L*, or *R* and *S*, are used to denote the absolute configuration of the molecule about its chiral center(s). The prefixes *d* and *1* or (+) and (-) are employed to designate the sign of rotation of plane-polarized light by the compound, with (-) or l meaning that the compound is levorotatory. A compound prefixed with (+) or d is dextrorotatory. For a given chemical structure, these stereoisomers are identical except that they are mirror images of one another. A specific stereoisomer may also be referred to as an enantiomer, and a mixture of such isomers is often called an enantiomeric mixture. A 50:50 mixture of enantiomers is referred to as a racemic mixture or a racemate, which may occur where there has been no stereoselection or stereospecificity in a chemical reaction or process. The term "racemic mixture" or "racemate" refers to an equimolar mixture of two enantiomeric species, devoid of optical activity.

The term "tautomer" or "tautomeric form" refers to structural isomers of different energies which are interconvertible via a low energy barrier. Some non-limiting examples of proton tautomers (also known as prototropic tautomers) include interconversions via migration of a proton, such as keto-enol and imine-enamine isomerizations. Valence tautomers include interconversions by reorganization of some of the bonding electrons.

A "pharmaceutically acceptable salts" refers to organic or inorganic salts of a compound disclosed herein. Pharmaceutically acceptable salts are well known in the art. For example, Berge et al., describe pharmaceutically acceptable salts in detail in J. Pharmacol Sci, 1977, 66: 1-19, 1-19, 1977. Some non-limiting examples of pharmaceutically acceptable and nontoxic salts include salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid and malonic acid or by using other methods used in the art such as ion exchange. Other pharmaceutically acceptable salts include adipate, 2-hydroxy propionate, alginate, ascorbate, aspartate, benzenesulfonate, benzoate, bisulfate, borate, butyrate, camphorate, camphorsulfonate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, laurylsulfate, malate, malonate, methanesulfonate, 2-naphthalenesulfonate, nicotinate, nitrate, oleate, palmitate, pamoate, pectinate, persulfate, 3-phenylpropionate, picrate, pivalate, propionate, stearate, thiocyanate, p-toluenesulfonate, undecanoate, valerate salts, and the like. If the compound disclosed herein is an acid, the desired salt may be prepared by any suitable method, for example, treatment of the free acid with an inorganic or organic base, such as an amine (primary, secondary or tertiary), an alkali metal hydroxide, ammonium, N⁺(R¹⁴)₄ salt or alkaline earth metal hydroxide, and the like. Some non-limiting examples of suitable salts include organic salts derived from amino acids, such as glycine and arginine; ammonia, such as primary, secondary and tertiary amine, N⁺(R¹⁴)₄ salt, wherein R¹⁴ is H, C₁₋₄ alkyl, C₆₋₁₀ aryl, C₆₋₁₀ aryl-C₁₋₄-alkyl, and the like; and cyclic amines, such as piperidine, morpholine and piperazine, and inorganic salts derived from sodium, calcium, potassium, magnesium, manganese, iron, copper, zinc, aluminum, lithium, and the like, and further include, when appropriate, nontoxic ammonium, quaternary ammonium and amine cations formed using counterions such as halide, hydroxide, carboxylate, sulfate, phosphate, nitrate, C₁₋₈ sulfonate or aryl sulfonate.

The term "solvate" refers to an association or complex of one or more solvent molecules and a compound disclosed herein. Some non-limiting examples of the solvent that form solvates include water, isopropanol, ethanol, methanol, dimethylsulfoxide (DMSO), ethyl acetate, acetic acid and ethanolamine. The term "hydrate" refers to the complex where the solvent molecule is water.

The term "protecting group" or "Pg" refers to a substituent that is commonly employed to block or protect a particular functionality while reacting with other functional groups on the compound. For example, an "amino-protecting group" is a substituent attached to an amino group that blocks or protects the amino functionality in the compound. Suitable amino-protecting groups include acetyl, trifluoroacetyl, *t*-butoxy-carbonyl (BOC, Boc), benzyloxycarbonyl (CBZ, Cbz) and 9-fluorenylmethylenoxy-carbonyl (Fmoc). Similarly, a "hydroxy-protecting group" refers to a substituent of a hydroxy group that blocks or protects the hydroxy functionality. Suitable protecting groups include acetyl and silyl. A "carboxy-protecting group" refers to a substituent of the carboxy group that blocks or protects the carboxy functionality. Common carboxy-protecting groups include -CH₂CH₂SO₂Ph, cyanoethyl, 2-(trimethylsilyl)ethyl, 2-(trimethylsilyl)ethoxy-methyl, 2-(*p*-toluenesulfonyl)ethyl, 2-(*p*-nitrophenylsulfonyl)-ethyl, 2-(diphenylphosphino)ethyl, nitroethyl and the like. For a general description of protecting groups and their use, see T. W. Greene, Protective Groups in Organic Synthesis, John Wiley & Sons, New York, 1991; and P. J. Kocienski, Protecting Groups, Thieme, Stuttgart, 2005.

### DESCRIPTION OF COMPOUNDS OF THE INVENTION

The compounds of the present invention and pharmaceutically acceptable compositions thereof are effective in inhibiting HBV infection.

In one aspect, the invention relates to a compound having Formula (I) or a stereoisomer, a tautomer, an N-oxide, a solvate, or a pharmaceutically acceptable salt thereof, wherein, 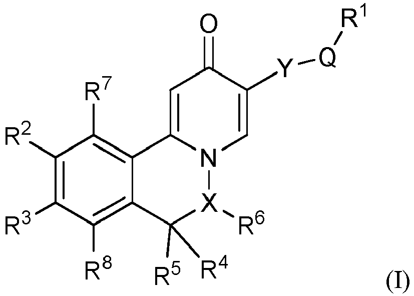
X is N or -CR^{6a}-;
Y is -C(=O)-;
Q is a single bond, -O- or -N(R⁹)-;
R¹ is H, deuterium, methyl, ethyl, *n*-propyl or isopropyl, wherein each of the methyl, ethyl, *n*-propyl and isopropyl is independently unsubstituted or substituted with 1, 2, 3 or 4 R^{v};
R⁹ is H, deuterium, methyl, ethyl, *n*-propyl, isopropyl or C₁₋₆ haloalkyl;
each of R⁴ and R⁵ is independently H, deuterium, C₁₋₄ alkyl, C₁₋₄ alkylamino or C₁₋₄ alkoxy, wherein each of the C₁₋₄ alkyl, C₁₋₄ alkylamino and C₁₋₄ alkoxy is independently unsubstituted or substituted with 1, 2, 3 or 4 R^{y};
R⁶ is H, deuterium, C₁₋₄ alkyl, C₂₋₄ alkenyl or C₃₋₆ cycloalkyl -, wherein each of the C₁₋₄ alkyl, C₂₋₄ alkenyl and C₃₋₆ cycloalkyl is independently unsubstituted or substituted with 1, 2, 3 or 4 R^{z};
R^{6a} is H, deuterium, F, Cl, Br, CN, OH, C₁₋₄alkyl or C₂₋₄ alkenyl, wherein each of the C₁₋₄alkyl and C₂₋₄ alkenyl is independently unsubstituted or substituted with 1, 2, 3 or 4 R^{z};
R² is phenyl, 5- to 6-membered heteroaryl, R¹³-O-C(=O)-(CR^{e}R^{f})ₘ-, wherein m is 0, R¹⁴-S(=O)₂-N(R^{g})-C(=O)-, R^{c}C(=O)-, R^{a}R^{b}NC(=O)- or R^{d}OC(=O)-, wherein each of the phenyland 5- to 6-membered heteroaryl is independently unsubstituted or substituted with 1, 2, 3 or 4 R^{w};
each of R⁷ and R⁸ is independently H, deuterium, F, Cl, Br, hydroxy, cyano or C₁₋₆ alkyl;
R³ is 5- to 6-membered heterocyclyl, phenyl, 5- to 6-membered heteroaryl R¹³-(CR^{e}R^{f})ₘ-O- or R¹⁰O-, wherein each of the 5- to 6-membered heterocyclyl, phenyl and 5- to 6-membered heteroaryl is independently unsubstituted or substituted with 1, 2, 3 or 4 R^{w};R¹⁰ is H, deuterium, C₁₋₆ alkyl, C₂₋₆ alkynyl, C₃₋₆ cycloalkyl, 5- to 6-membered heterocyclyl, phenyl, naphthyl or 5- to 6-membered heteroaryl, wherein each of C₁₋₆ alkyl, C₂₋₆ alkynyl, C₃₋₆ cycloalkyl, 5- to 6-membered heterocyclyl, phenyl, naphthyl and 5- to 6-membered heteroaryl is independently unsubstituted or substituted with 1, 2, 3 or 4 R^{x};
each R¹³ and R¹⁴ is independently C₁₋₁₂ alkyl, C₁₋₁₂ alkoxy, C₃₋₇ cycloalkyl, 5- to 10-membered heteroaryl, 3- to 12-membered heterocyclyl or C₆₋₁₀ aryl, wherein each of the C₁₋₁₂ alkyl, C₁₋₁₂ alkoxy, C₃₋₇ cycloalkyl, 5- to 10-membered heteroaryl, 3- to 12-membered heterocyclyl and C₆₋₁₀ aryl is independently unsubstituted or substituted with 1, 2, 3 or 4 R^{h};
each R^{a}, R^{b}, R^{c}, R^{d}, R^{e}, R^{f}, R^{g}, R^{k}, Rⁱ, R^{m} and Rⁿ is independently H, deuterium, OH, C₁₋₄ haloalkyl, C₁₋₆ alkyl, C₁₋₄ alkoxy, C₂₋₄ alkenyl, C₂₋₄ alkynyl, C₃₋₆ cycloalkyl, phenyl, naphthyl, 5-to 6-membered heterocyclyl or 5- to 6-membered heteroaryl, wherein each of the C₁₋₄ haloalkyl, C₁₋₆ alkyl, C₁₋₄ alkoxy, C₂₋₄ alkenyl, C₂₋₄ alkynyl, C₃₋₆ cycloalkyl, phenyl, naphthyl, 5- to 6-membered heterocyclyl and 5- to 6-membered heteroaryl is independently unsubstituted or substituted with 1, 2, 3 or 4 substituents independently selected from F, Cl, Br, CN, OH, amino, C₁₋₆ alkyl, C₁₋₆ haloalkyl, C₁₋₆ alkoxy or C₁₋₆ alkylamino;
or, R^{a} and R^{b}, together with the nitrogen atom to which they are attached, form a 5- to 6-membered heterocyclyl or 5- to 6-membered heteroaryl, wherein each of the 5- to 6-membered heterocyclyl and 5- to 6-membered heteroaryl is independently unsubstituted or substituted with 1, 2, 3 or 4 substituents independently selected from F, Cl, Br, CN, OH, amino, C₁₋₄ alkyl, C₁₋₄ haloalkyl, C₁₋₄ alkoxy or C₁₋₄ alkylamino;
each R^{v}, R^{w}, R^{x}, R^{y}, R^{z} and R^{h} is independently F, Cl, Br, CN, OH, -COOH, amino, C₁₋₆ alkyl, C₁₋₆ haloalkyl, C₁₋₆ alkoxy, C₁₋₆ haloalkoxy, C₁₋₆ alkylthio, C₁₋₆ alkylamino, C₂₋₄ alkenyl, C₂₋₄ alkynyl, C₃₋₆ cycloalkyl, 5- to 6-membered heterocyclyl, phenyl, naphthyl or 5- to 6-membered heteroaryl, wherein each of the amino, C₁₋₆ alkyl, C₁₋₆ haloalkyl, C₁₋₆ alkoxy, C₁₋₆ haloalkoxy, C₁₋₆ alkylthio, C₁₋₆ alkylamino, C₂₋₄ alkenyl, C₂₋₄ alkynyl, C₃₋₆ cycloalkyl, 5- to 6-membered heterocyclyl, phenyl, naphthyl and 5- to 6-membered heteroaryl is independently unsubstituted or substituted with 1, 2, 3 or 4 substituents independently selected from F, Cl, Br, CN, OH, =O, -COOH, amino, C₁₋₄ alkyl, C₁₋₄ haloalkyl, C₁₋₄ alkoxy, C₁₋₄ alkylthio, C₁₋₄ haloalkoxy, C₁₋₄ alkylamino, C₂₋₄ alkenyl, C₂₋₄ alkynyl, phenyl, naphthyl, C₃₋₆ cycloalkyl, 5- to 6-membered heteroaryl, 5- to 6-membered heterocyclyl, C₁₋₄ alkoxy-C₁₋₄-alkyl or C₁₋₄ alkylamino-C₁₋₄-alkyl;
m is independently 0, 1, 2, 3, 4, 5, 6, 7, 8, 9 or 10.

In other embodiments, R² is furyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidinyl, phenyl, R¹³-O-C(=O)-(CR^{e}R^{f})ₘ-, wherein each of the furyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidinyl and phenyl is independently unsubstituted or substituted with 1, 2, 3 or 4 R^{w}; and
each R¹³, R^{e}, R^{f} and R^{w} is as defined herein.

In some embodiments, each of R⁷ and R⁸ is independently H, deuterium, F, Cl, Br, hydroxy, cyano, methyl, ethyl, *n*-propyl, isopropyl, *n*-butyl, isobutyl, *n*-pentyl or *n*-hexyl;
R³ is pyrrolidinyl, pyrazolidinyl, imidazolidinyl, tetrahydrofuranyl, tetrahydrothiophenyl, tetrahydropyranyl, tetrahydrothiapyranyl, piperidyl, morpholinyl, thiomorpholinyl, piperazinyl, furyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidinyl, phenyl, R¹³-(CR^{e}R^{f})ₘ-O- or R¹⁰O-, and wherein each of the pyrrolidinyl, pyrazolidinyl, imidazolidinyl, tetrahydrofuranyl, tetrahydrothiophenyl, tetrahydropyranyl, tetrahydrothiapyranyl, piperidyl, morpholinyl, thiomorpholinyl, piperazinyl, furyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidinyl and phenyl is independently unsubstituted or substituted with 1, 2, 3 or 4 R^{w};
R¹⁰ is H, deuterium, methyl, ethyl, *n*-propyl, isopropyl, *n*-butyl, isobutyl, *n*-pentyl, *n*-hexyl, ethynyl, propynyl, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidyl, morpholinyl, thioxomorpholinyl, piperazinyl, furyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidinyl, phenyl or naphthyl, wherein each of the methyl, ethyl, *n*-propyl, isopropyl, *n*-butyl, isobutyl, *n*-pentyl, *n*-hexyl, ethynyl, propynyl, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidyl, morpholinyl, thioxomorpholinyl, piperazinyl, furyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidinyl, phenyl and naphthyl is independently unsubstituted or substituted with 1, 2, 3 or 4 R^{x};
each m, R¹³, R^{e}, R^{f}, R^{x} and R^{w} is as defined herein.

In other embodiments, each of R⁴ and R⁵ is independently H, deuterium, C₁₋₃ alkyl, C₁₋₃ alkylamino or C₁₋₃ alkoxy, wherein each of the C₁₋₃ alkyl, C₁₋₃ alkylamino and C₁₋₃ alkoxy is independently unsubstituted or substituted with 1, 2, 3, or 4 R^{y};
R⁶ is H, deuterium, methyl, ethyl, *n*-propyl, isopropyl, *n*-butyl, isobutyl, *tert*-butyl, vinyl, propenyl, cyclopropyl, cyclobutyl, cyclopentyl or cyclohexyl, wherein each of the methyl, ethyl, *n*-propyl, isopropyl, *n*-butyl, isobutyl, *tert*-butyl, vinyl, propenyl, cyclopropyl, cyclobutyl and cyclopentyl is independently unsubstituted or substituted with 1, 2, 3, or 4 R^{z};
R^{6a} is H, deuterium, F, Cl, Br, CN, OH, methyl, ethyl, *n*-propyl, isopropyl, *n*-butyl, isobutyl, *tert*-butyl, vinyl or propenyl, wherein each of the methyl, ethyl, *n*-propyl, isopropyl, *n*-butyl, isobutyl, *tert*-butyl, vinyl, propenyl, cyclopropyl, cyclobutyl and cyclopentyl is independently unsubstituted or substituted with 1, 2, 3, or 4 R^{z}; and
each q, R¹⁷, R^{e}, R^{f}, R^{z} and R^{y} is as defined herein.

In some embodiments, each R¹³ and R¹⁴ is independently C₁₋₆ alkyl, C₁₋₆ alkoxy, C₃₋₆ cycloalkyl, 5- to 6-membered heteroaryl, 5- to 6-membered heterocyclyl, phenyl or naphthyl, wherein each of the C₁₋₆ alkyl, C₁₋₆ alkoxy, C₃₋₆ cycloalkyl, 5- to 6-membered heteroaryl, 5- to 6-membered heterocyclyl, phenyl and naphthyl is independently unsubstituted or substituted with 1, 2, 3 or 4 R^{h};
each R^{h} is as defined herein.

In other embodiments, each R¹³ and R¹⁴ is independently methyl, ethyl, *n*-propyl, isopropyl, *n*-butyl, isobutyl, *tert*-butyl, methoxy, ethoxy, propoxy, isopropoxy, *n*-butoxy, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidyl, morpholinyl, thiomorpholinyl, piperazinyl, furyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidinyl, phenyl or naphthyl, wherein each of the methyl, ethyl, *n*-propyl, isopropyl, *n*-butyl, isobutyl, *tert*-butyl, methoxy, ethoxy, propoxy, isopropyl, *n*-butoxy, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidyl, morpholinyl, thiomorpholinyl, piperazinyl, furyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidinyl, phenyl and naphthyl is independently unsubstituted or substituted with 1, 2, 3, or 4 R^{h};
each R^{h} is as defined herein.

In other embodiments, each R^{a}, R^{b}, R^{c}, R^{d}, R^{e}, R^{f} and R^{g} is independently H, deuterium, OH, C₁₋₃ haloalkyl, methyl, ethyl, *n*-propyl, isopropyl, *n*-butyl, isobutyl, *tert*-butyl, *n*-pentyl, 3-pentyl, methoxy, ethoxy, propoxy, isopropoxy, *n*-butoxy, ethenyl, propenyl, ethynyl, propynyl, propargyl, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidyl, morpholinyl, thiomorpholinyl, piperazinyl, furyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidinyl, phenyl or naphthyl, wherein each of the C₁₋₃ haloalkyl, methyl, ethyl, *n*-propyl, isopropyl, *n*-butyl, isobutyl, *tert*-butyl, methoxy, ethoxy, propoxy, isopropoxy, *n*-butoxy, ethenyl, propenyl, ethynyl, propynyl, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidyl, morpholinyl, thiomorpholinyl, piperazinyl, furyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidinyl, phenyl and naphthyl is independently unsubstituted or substituted with 1, 2, 3, or 4 substituents independently selected from F, Cl, Br, CN, OH, amino, C₁₋₄ alkyl, C₁₋₄ haloalkyl, C₁₋₄ alkoxy or C₁₋₄ alkylamino;
or, R^{a} and R^{b}, together with the nitrogen atom to which they are attached, form pyrrolidinyl, pyrazolidinyl, imidazolidinyl, piperidyl, morpholinyl, thiomorpholinyl, piperazinyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, pyrazinyl, pyridazinyl or pyrimidinyl, wherein each of the pyrrolidinyl, pyrazolidinyl, imidazolidinyl, piperidyl, morpholinyl, thiomorpholinyl, piperazinyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, pyrazinyl, pyridazinyl and pyrimidinyl is independently unsubstituted or substituted with 1, 2, 3, or 4 substituents independently selected from F, Cl, Br, CN, OH, amino, C₁₋₄ alkyl, C₁₋₄ haloalkyl, C₁₋₄ alkoxy or C₁₋₄ alkylamino.

In other embodiments, each R^{v}, R^{w}, R^{x}, R^{y}, R^{z} and R^{h} is independently F, Cl, Br, CN, OH, -COOH, amino, C₁₋₄ alkyl, C₁₋₄ haloalkyl, C₁₋₄ alkoxy, C₁₋₄ haloalkoxy, C₁₋₄ alkylthio, C₁₋₄ alkylamino, C₂₋₄ alkenyl, C₂₋₄ alkynyl, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidyl, morpholinyl, thiomorpholinyl, piperazinyl, furyl, pyrrolyl, pyridyl, pyrazolyl, imidazolyl, triazolyl, tetrazolyl, oxazolyl, isoxazolyl, dioxazolyl, 1,3,5-triazinyl, thiazolyl, thienyl, pyrazinyl, pyridazinyl, pyrimidinyl, phenyl or naphthyl, wherein each of the cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidyl, morpholinyl, thiomorpholinyl, piperazinyl, furyl, pyrrolyl, pyridyl, pyrazolyl, imidazolyl, triazolyl, tetrazolyl, oxazolyl, isoxazolyl, dioxazolyl, 1,3,5-triazinyl, thiazolyl, thienyl, pyrazinyl, pyridazinyl, pyrimidinyl, phenyl and naphthyl is independently unsubstituted or substituted with 1, 2, 3 or 4 substituents independently selected from F, Cl, Br, CN, OH, =O, -COOH, amino, C₁₋₄ alkyl, C₁₋₄ haloalkyl, C₁₋₄ alkoxy, C₁₋₄ alkylthio, C₁₋₄ haloalkoxy, C₁₋₄ alkylamino, C₂₋₄ alkenyl, C₂₋₄ alkynyl, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidyl, morpholinyl, thiomorpholinyl, piperazinyl, furyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidinyl, phenyl, naphthyl, C₁₋₃ alkoxy-C₁₋₃-alkyl and C₁₋₃ alkylamino-C₁₋₃-alkyl.

In other embodiments, the invention relates to a compound having one of the following structures, or a stereoisomer, a tautomer, an *N*-oxide or a pharmaceutically acceptable salt thereof, but is not limited to: 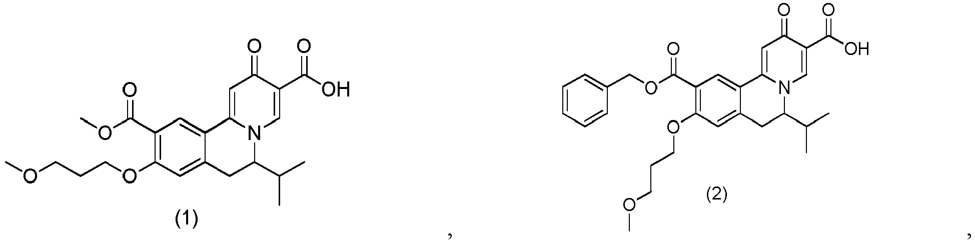 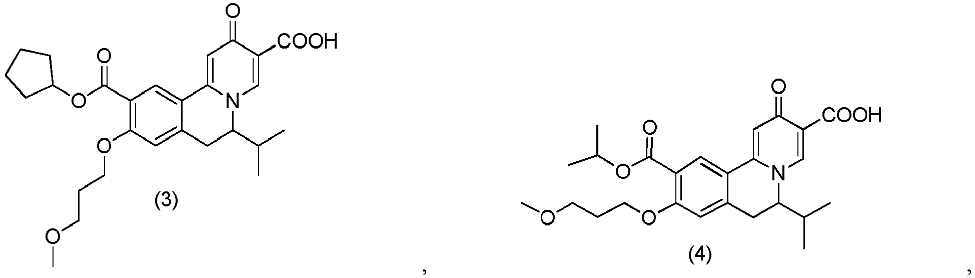 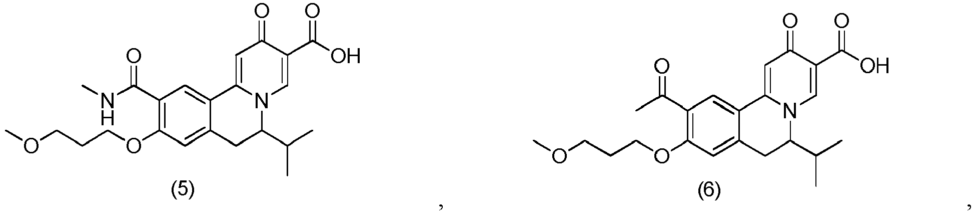 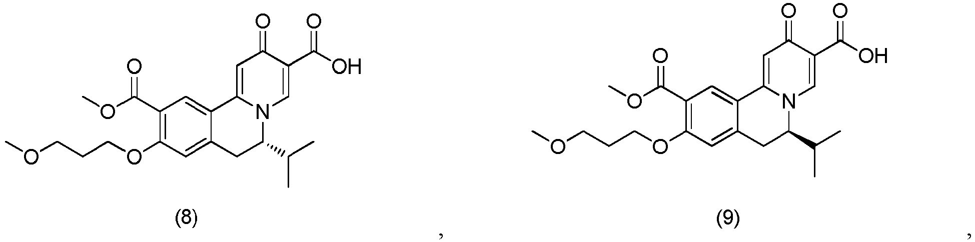 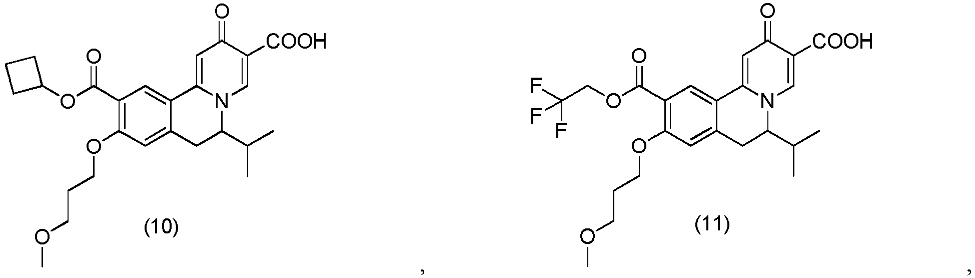 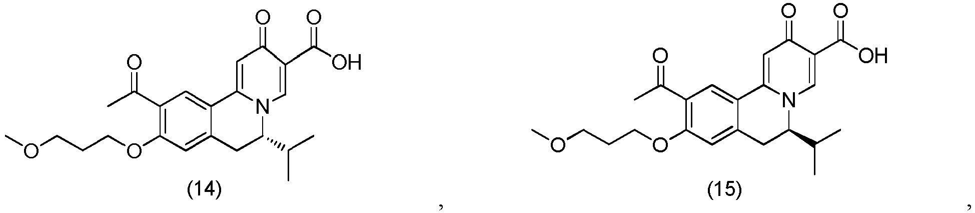 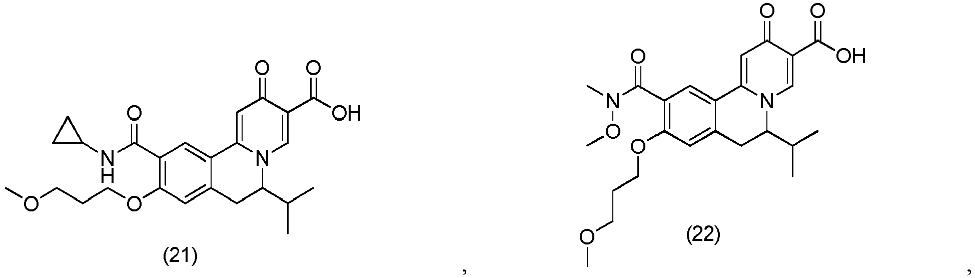 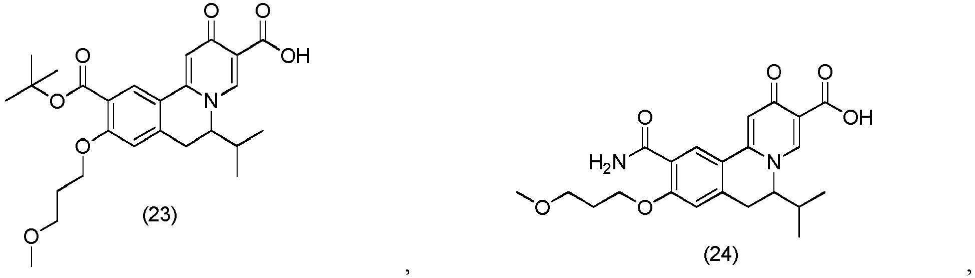 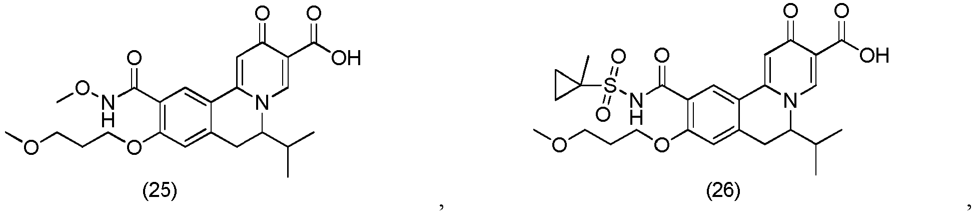 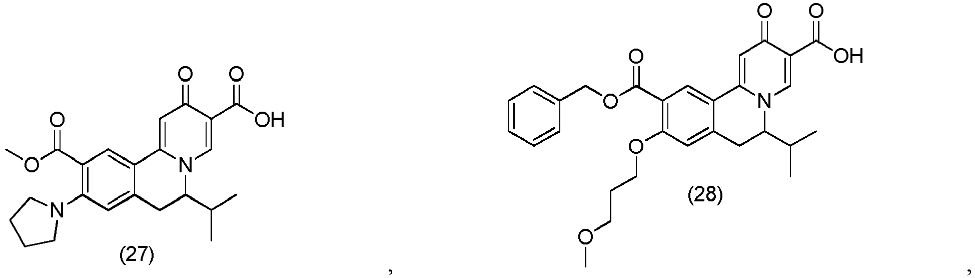 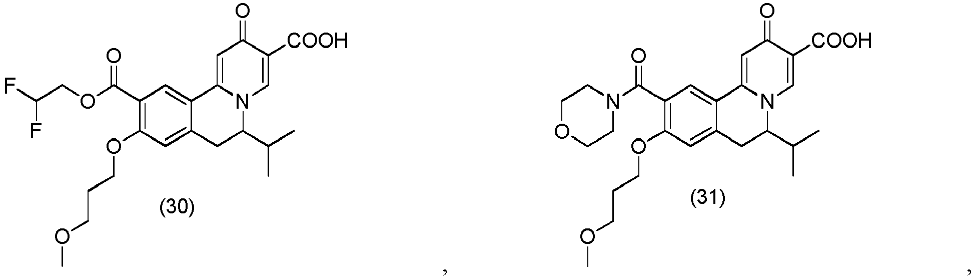 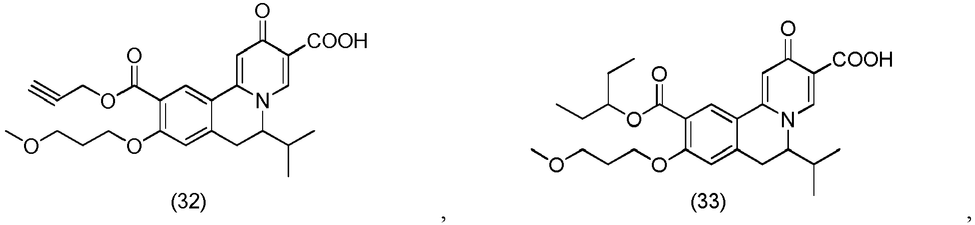 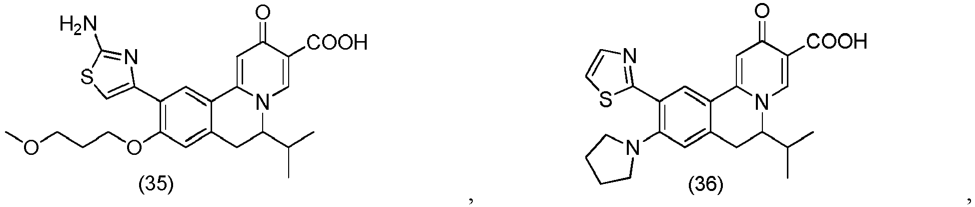 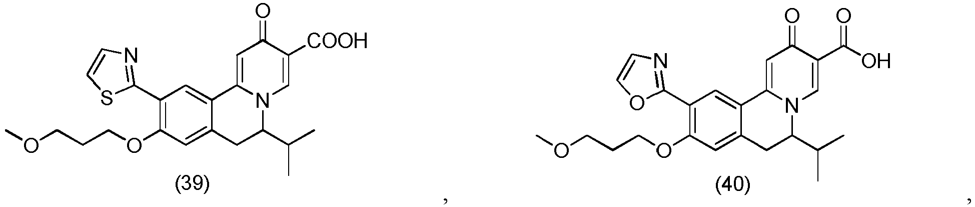 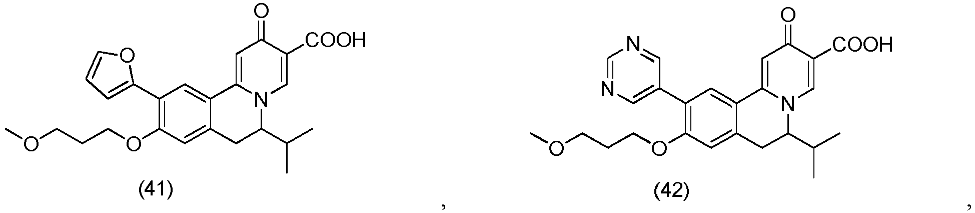 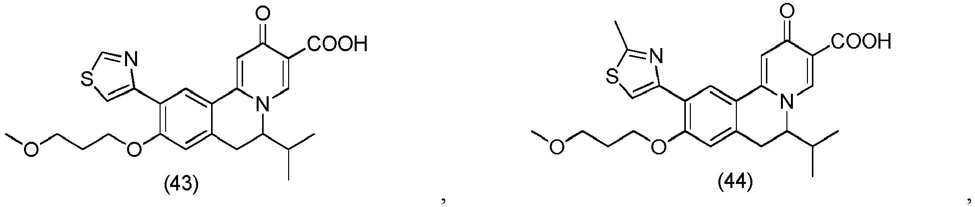 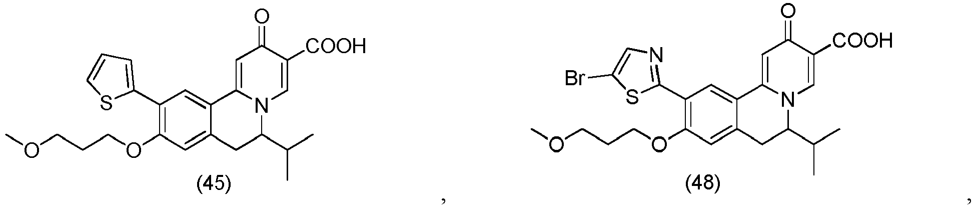 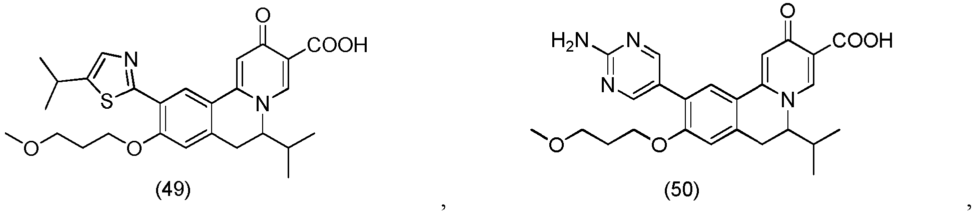 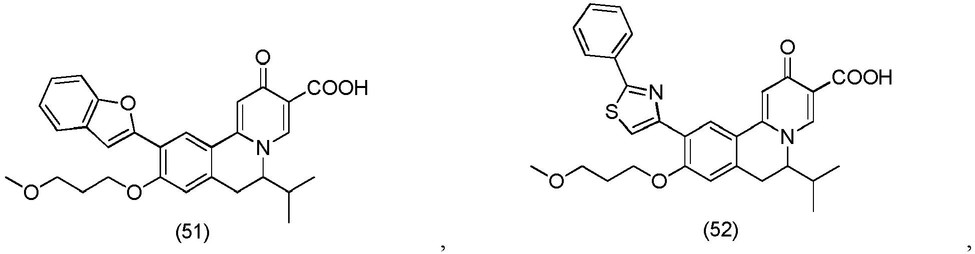 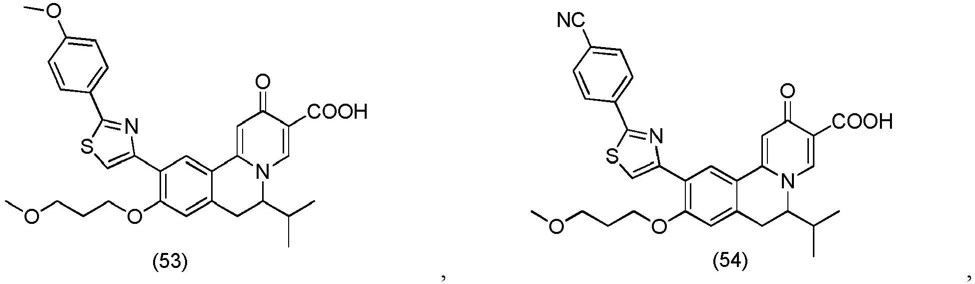 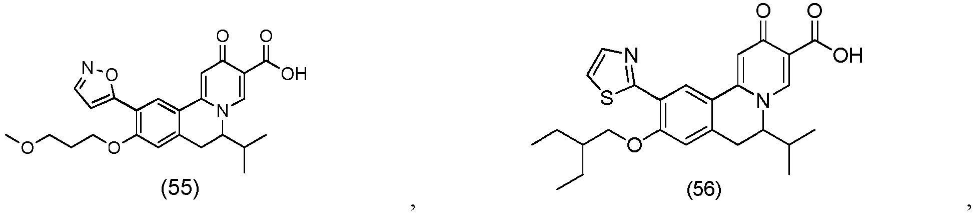 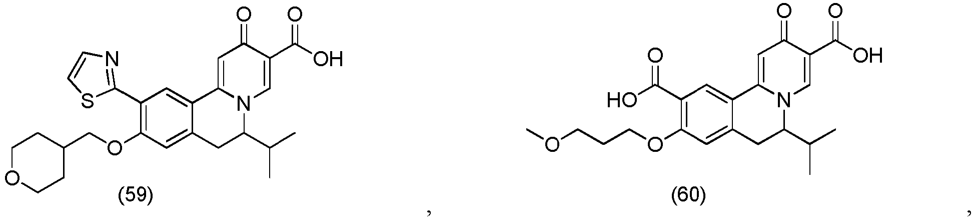 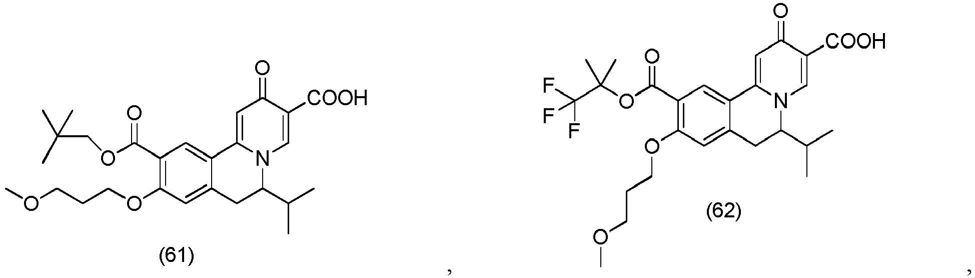 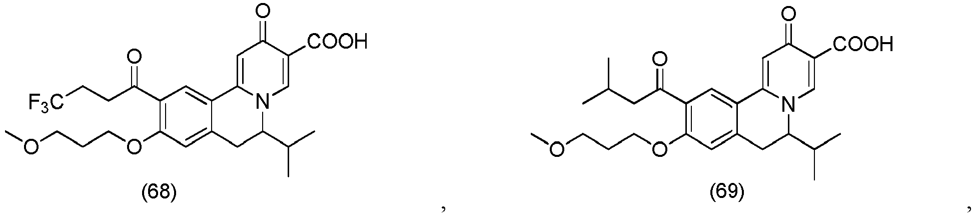 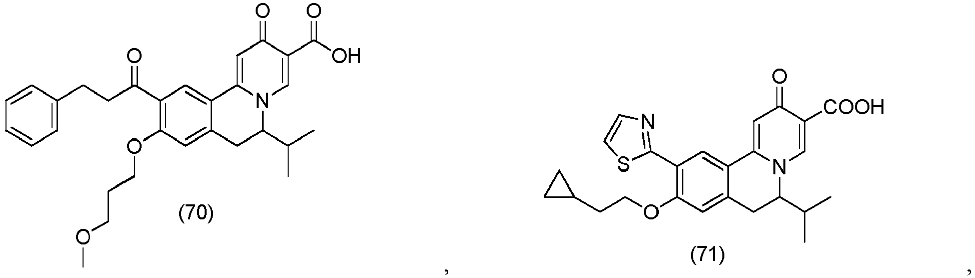 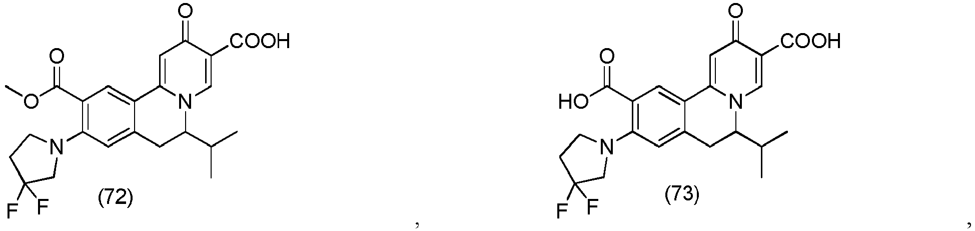 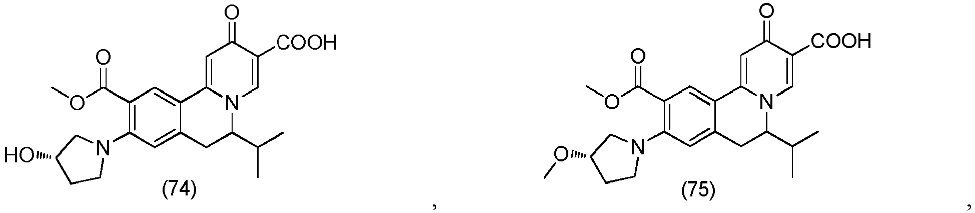 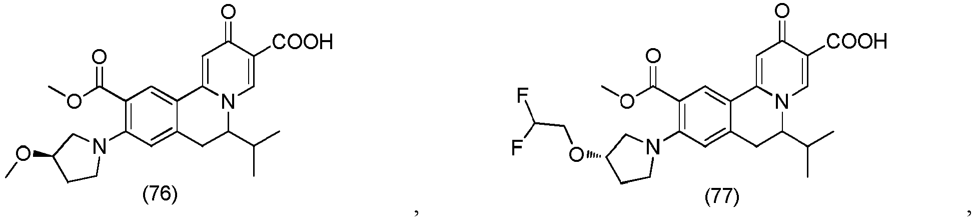 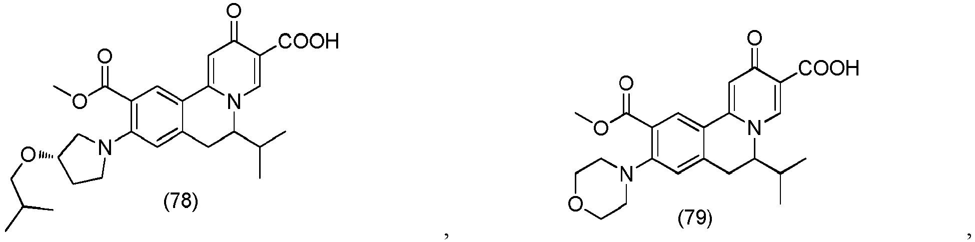 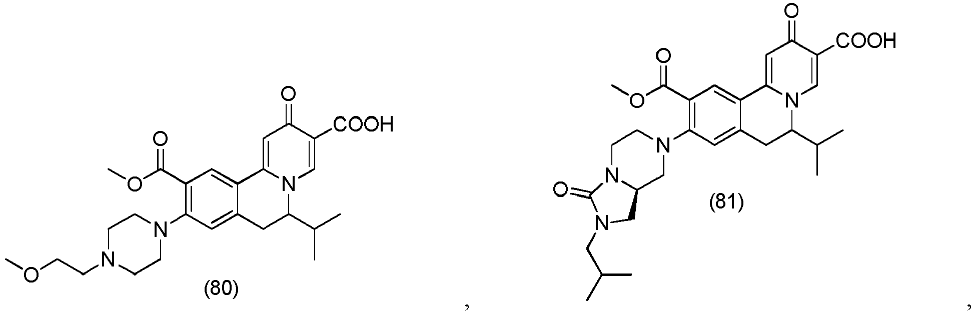 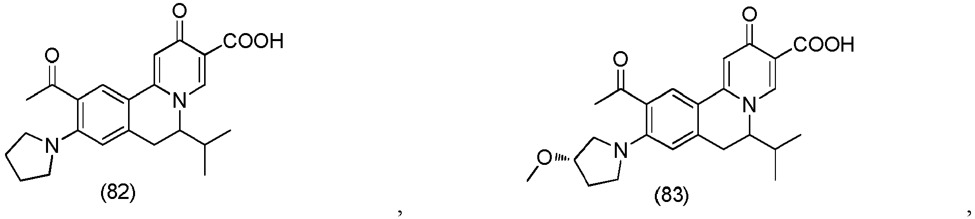 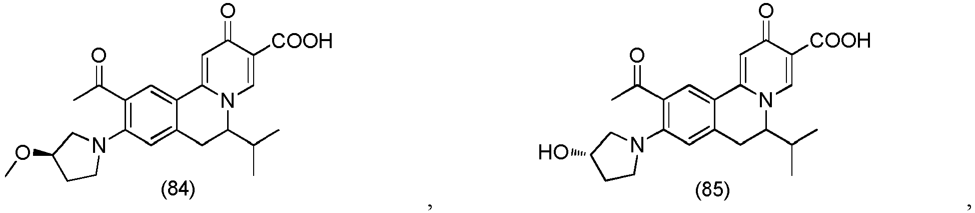 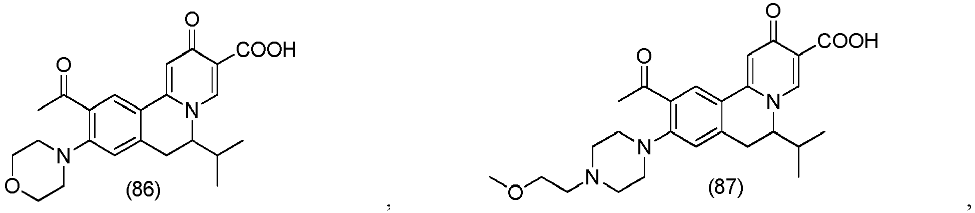 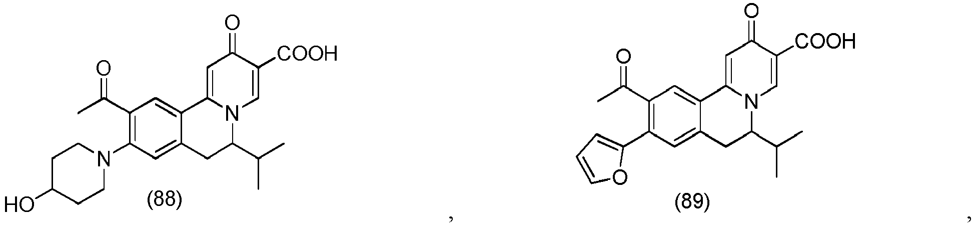 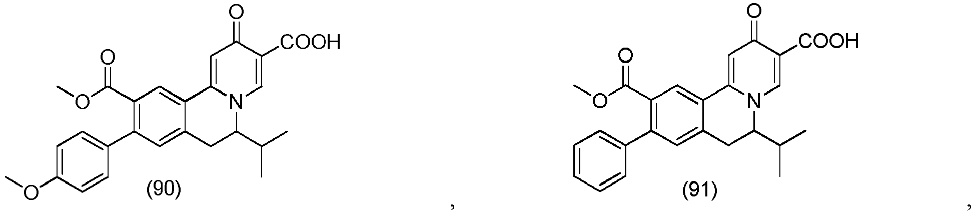 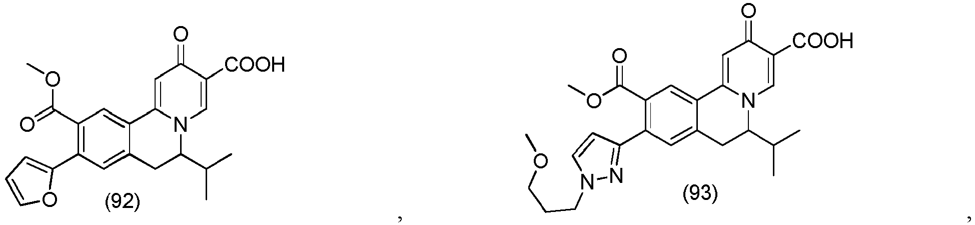 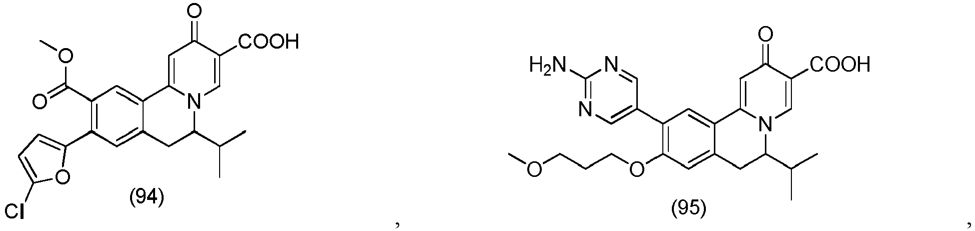 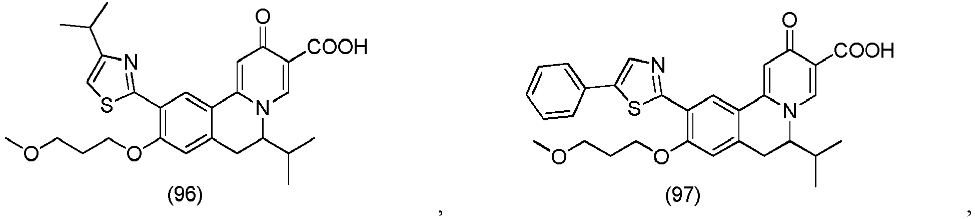 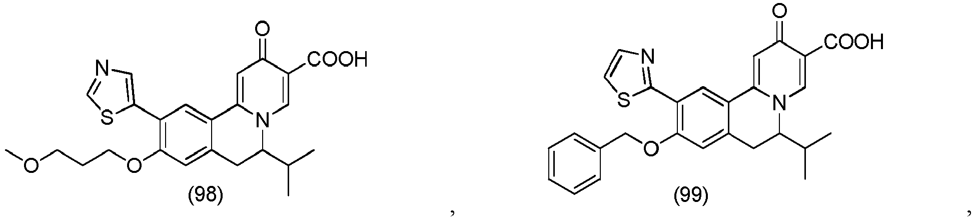 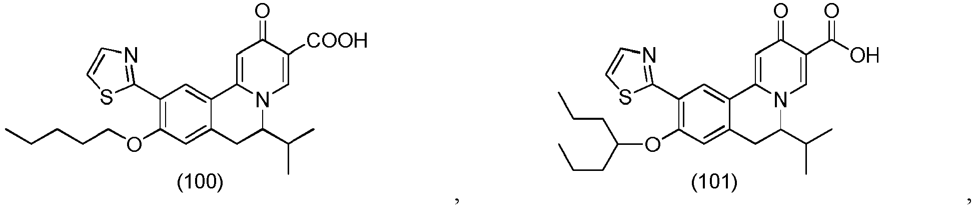 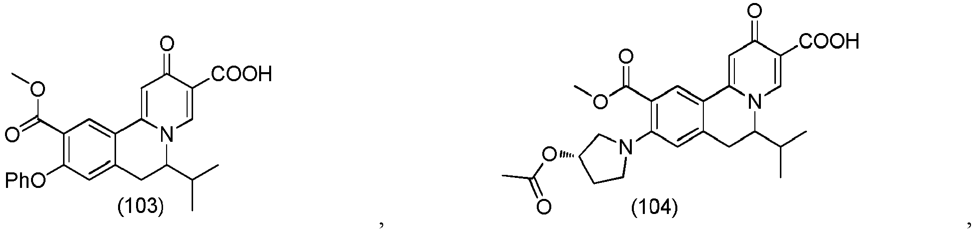 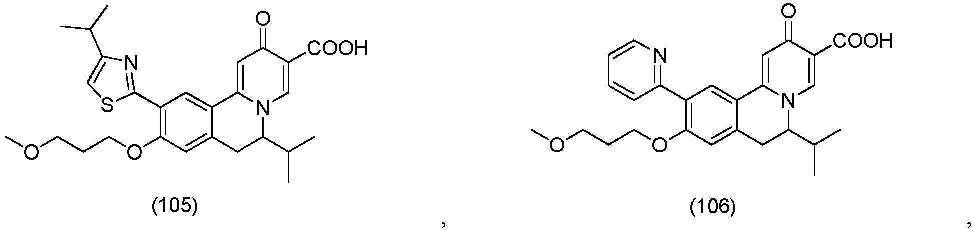 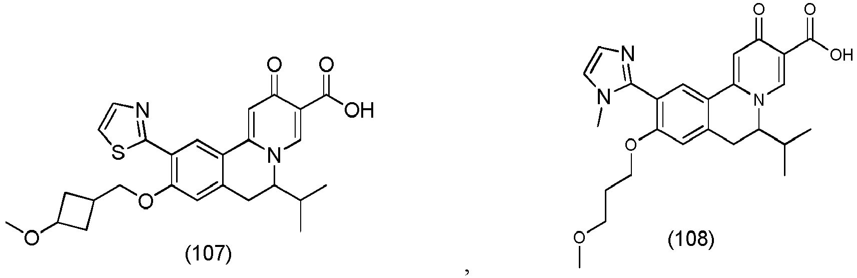 or

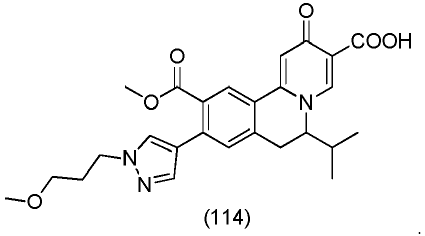

Unless otherwise specified, all stereoisomers, tautomers, *N*-oxides, pharmaceutically acceptable salts of the compound having formula (I) are within the scope of the present invention.

In another aspect, provided herein is a pharmaceutical composition comprising the compound of the invention; optionally, wherein the pharmaceutical composition further comprises a pharmaceutically acceptable excipient, or a combination of the excipients.

In some embodiments, the pharmaceutical composition disclosed herein further comprises other anti-HBV drug.

In some embodiments, the pharmaceutical composition disclosed herein, wherein the other anti-HBV drug is a HBV polymerase inhibitor, an immunomodulator or an interferon.

In some embodiments, the pharmaceutical composition disclosed herein, wherein the other anti-HBV agent is lamivudine, telbivudine, tenofovir, entecavir, adefovir dipivoxil, alfaferone, alloferon, celmoleukin, clevudine, emtricitabine, famciclovir, interferon, hepatect CP, intefen, interferon α-1b, interferon α, interferon α-2a, interferon β-1a, interferon α-2, interleukin-2, mivotilate, nitazoxanide, peginterferon alfa-2a, ribavirin, roferon-A, sizofiran, euforavac, ampligen, phosphazid, heplisav, interferon α-2b, levamisole or propagermanium.

In another aspect, provided herein is the compound or the pharmaceutical composition of the invention in the manufacture of a medicament for use in preventing, treating or lessening a disorder or disease caused by a virus infection in a patient.

In some embodiments, the use disclosed herein, wherein the virus disease is hepatitis B infection or a disease caused by hepatitis B infection.

In other embodiments, the use disclosed herein, wherein the disease caused by hepatitis B infection is cirrhosis or hepatocellular carcinogenesis.

In another aspect, provided herein is the compound of the invention in the manufacture of a medicament for use in preventing, managing or treating a HBV disorder or disease in a patient, or lessening the severity of the HBV disorder or disease in a patient.

In another aspect, provided herein is the pharmaceutical composition compring the compound of the invention in the manufacture of a medicament for ues in preventing, managing or treating a HBV disorder or disease in a patient, or lessening the severity of the HBV disorder or disease in a patient.

In another aspect, provided herein is the compound or the pharmaceutical composition of the invention in the manufacture a medicament for ues in inhibiting the production or secretion of HBsAg, and/or for inhibiting the production of HBV DNA.

In another aspect, provided herein is the compound or the pharmaceutical composition of the invention for use in preventing, treating or lessening a disorder or disease caused by a virus infection in a patient.

Unless otherwise stated, all stereoisomers, tautomers, *N*-oxides, solvates or pharmaceutically acceptable salts the compounds disclosed herein are within the scope of the invention.

In certain embodiments, the salt is a pharmaceutically acceptable salt. The phrase "pharmaceutically acceptable" refers to that the substance or composition must be compatible chemically and/or toxicologically, with the other ingredients comprising a formulation, and/or the mammal being treated therewith.

The salts of the compounds also include salts that may be useful for preparing and/or purifying the intermediates of compounds represented by Formula (I), compounds represented by Formula (I), and/or enantiomers of compounds represented by Formula (I), which are not necessarily pharmaceutically acceptable salts.

The term as used herein, "pharmaceutically acceptable" means a substance is acceptable for pharmaceutical applications from the standpoint of toxicology and does not adversely interact with active ingredients.

The salts of the compounds also include salts that may be useful for preparing and/or purifying the intermediates of compounds represented by Formula (I), and/or separated enantiomers of compounds represented by Formula (I), which are not necessarily pharmaceutically acceptable salts.

If the compound of the invention is basic, the desired salt can be prepared by any suitable method provided in the literature, for example, using an inorganic acid such as hydrochloric acid, hydrobromic acid, sulfuric acid, nitric acid, phosphoric acid, and the like; or using an organic acid, such as acetic acid, maleic acid, succinic acid, mandelic acid, fumaric acid, malonic acid, pyruvic acid, malic acid, 2-hydroxypropionic acid, citric acid, oxalic acid, glycolic acid and salicylic acid; a pyranosidyl acid, such as glucuronic acid and galacturonic acid; an alpha-hydroxy acid, such as citric acid and tartaric acid; an amino acid, such as aspartic acid and glutamic acid; an aromatic acid, such as benzoic acid and cinnamic acid; a sulfonic acid, such as *p*-toluenesulfonic acid, benzenesulfonic acid, methanesulfonic acid, ethanesulfonic acid, trifluoromethanesulfonic acid, and the like; or the combination thereof.

If the compound of the present invention is acidic, the desired salt can be prepared by an appropriate method. Inorganic bases, such as the lithium salt, sodium salt, potassium salt, calcium salt, magnesium salt, aluminum salt, iron salts, ferrous salts, manganese salts, manganous salts, copper salts, zinc salts and ammonium salts of the compound represented by formula (I) are obtained; inorganic bases, such as the compounds represented by the formula (I) and methylamine, dimethylamine, trimethylamine, ethylamine, diethylamine, triethylamine, tromethamine, diethylaminoethanol, isopropylamine, 2-ethylaminoethanol, pyridine, picoline, ethanolamine, diethanolamine, ammonium, dimethylethanolamine, tetramethylammonium, tetraethylammonium, triethanolamine, piperidine, piperazine, morpholine, imidazole salts, lysine, arginine, *L*-arginine, histidine, *N*-methylglucamine, dimethylglucosamine, ethylglucosamine, dicyclohexylamine, 1,6-hexamethylenediamine, ethylenediamine, glucosamine, sarcosine, serinol, aminopropylene glycol, 1-aminobutan-2,3,4-triol, *L*-lysine, ornithine and the like.

### COMPOSITION OF THE COMPOUND OF THE INVENTION, PREPARATIONS,

### ADMINISTRATION, AND USES OF COMPOUND AND COMPOSITION

The invention features pharmaceutical compositions that include a compound of Formula (I) or a compound listed in Examples, or a stereoisomer, a tautomer, an *N*-oxide, a solvate, or a pharmaceutically acceptable salt thereof, and a pharmaceutically acceptable excipient. Chronic viral diseases caused by HBV may cause serious pathological changes. Chronic hepatitis B virus infection can lead to cirrhosis and/or hepatocellular carcinogenesis in many cases. The compounds in the composition of the present invention can effectively inhibit hepatitis B virus. Diseases caused by viruses are especially acute and chronic persistent HBV viral infections.

Areas of indication which may be mentioned for the compounds of the invention are, for example: the treatment of acute and chronic viral infections which may lead to infectious hepatitis, for example infections with hepatitis B viruses. The compounds of the invention are particularly suitable for the treatment of chronic hepatitis B infections and the treatment of acute and chronic hepatitis B viral infections.

The present invention includes pharmaceutical preparations which, besides nontoxic, inert pharmaceutically suitable excipients, comprise one or more compounds having Formula (I) or a composition of the invention.

The pharmaceutical preparations mentioned above may also comprise other active pharmaceutical ingredients apart from the compounds having Formula (I).

It will also be appreciated that certain of the compounds disclosed herein can exist in free form for treatment, or where appropriate, as a pharmaceutically acceptable derivative thereof. Some non-limiting examples of the pharmaceutically acceptable derivative include pharmaceutically acceptable prodrugs, salts, esters, salts of such esters, or any other adducts or derivatives which upon administration to a patient in need is capable of providing, directly or indirectly, a compound as otherwise described herein, or a metabolite or residue thereof.

As described above, the pharmaceutically compositions disclosed herein comprise any one of the compound having Formula (I), and further comprise a pharmaceutically acceptable excipient, which, as used herein, includes any and all solvents, diluents, solid excipients, diluent, adhesives, disintegrant or other liquid vehicle, dispersion, flavoring agents or suspension aids, surface active agents, isotonic agents, thickening or emulsifying agents, preservatives, solid binders, lubricants and the like, as suited to the particular dosage form desired. As the following described: *In* Remington: The Science and Practice of Pharmacy, 21st edition, 2005, ed. D.B. Troy, Lippincott Williams& Wilkins, Philadelphia, and Encyclopedia of Pharmaceutical Technology, eds. J. Swarbrick and J. C. Boylan, 1988-1999, Marcel Dekker, New York, discloses various excipients used in formulating pharmaceutically acceptable compositions and known techniques for the preparation thereof. Except insofar as any conventional adjuvant incompatible with the compounds disclosed herein, such as by producing any undesirable biological effect or otherwise interacting in a deleterious manner with any other components of the pharmaceutically acceptable composition, its use is contemplated to be within the scope of this invention.

Some non-limiting examples of materials which can serve as pharmaceutically acceptable adjuvants include ion exchangers; aluminium; aluminum stearate; lecithin; serum proteins such as human serum albumin; buffer substances such as phosphates; glycine; sorbic acid; potassium sorbate; partial glyceride mixtures of saturated vegetable fatty acids; water; salts or electrolytes such as protamine sulfate, disodium hydrogen phosphate, potassium hydrogen phosphate, sodium chloride and zinc salts; colloidal silica; magnesium trisilicate; polyvinyl pyrrolidone; polyacrylates; waxes; polyethylene-polyoxypropylene-block polymers; wool fat; sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil, safflower oil, sesame oil, olive oil, corn oil and soybean oil; glycols such as propylene glycol and polyethylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol; and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants.

The pharmaceutical composition of the compound disclosed herein may be administered in any of the following routes: orally, inhaled by spray, locally, rectally, nasally, vaginally, parenterally such as subcutaneous, intravenous, intramuscular, intraperitoneal, intrapulmonary, intrathecal, intraventricular, intrasternal, or intracranial injection or infusion, or administered with the aid of an explanted reservoir. Administration routes by orally, intramuscular, intraperitoneal or intravenous injection are preferred.

The compound and pharmaceutically composition thereof of the invention may be administered in a unit dosage form. The dosage form may be in a liquid form, or a solid form. The liquid form includes true solutions, colloids, particulates, suspensions. Other dosage forms include tablets, capsules, dropping pills, aerosols, pills, powders, solutions, suspensions, emulsions, granules, suppositories, freeze-dried powder injection, clathrates, implants, patches, liniments, and the like.

Oral tablets and capsules may comprise excipients, *e*.*g*., binders, such as syrup, arabic gum, sorbitol, tragacanth or polyvinylpyrrolidone; fillers, such as lactose, sucrose, corn starch, calcium phosphate, sorbitol, glycine; lubricants such as magnesium stearate, talc, polyethylene glycol, silica; disintegrating agents, such as potato starch;, or acceptable moisturizing agents such as sodium lauryl sulfate. Tablets may be coated by using known methods in pharmaceutics.

Oral solution may be made as a suspension of water and oil, a solution, an emulsion, syrup or an elixir, or made as a dried product to which water or other suitable medium is added before use. This liquid preparation may comprise conventional additives, *e.g*., suspending agents such sorbitol, cellulose methyl ether, glucose syrup, gel, hydroxyethyl cellulose, carboxymethyl cellulose, aluminum stearate gel, hydrogenated edible greases; emulsifying agents such as lecithin, sorbitan monoleate, Arabic gum; or non-aqueous carriers (possibly including edible oil), such as almond oil, grease such as glycerin, ethylene glycol, or ethanol; antiseptics such as methyl or propyl *p*-hydroxybenzoate, sorbic acid. If desired, a flavoring agent or a colorant may be added.

Suppositories may comprise a conventional suppository base, such as cocoa butter or other glyceride.

For parenteral administration, the liquid dosage form is usually made from the compound and a sterilized excipient. Water is the preferred excipient. According to the difference of selected excipient and drug concentration, the compound can be either dissolved in the excipient or made into a supernatant solution. When being made into a solution for injection, the compound is firstly dissolved in water, and then filtered and sterilized before being packaged into an sealed bottle or an ampoule.

For application topically to the skin, the compound disclosed herein may be made into a suitable form of ointments, lotions or creams, wherein the active ingredient is suspended or dissolved in one or more excipient(s). Wherein excipients used for an ointment preparation include, but are not limited to: mineral oil, liquid vaseline, white vaseline, propylene glycol, polyoxyethylene, polyoxypropylene, emulsified wax and water; excipients used for a lotion and a cream include, but are not limited to: mineral oil, sorbitan monostearate, Tween 60, cetyl ester wax, hexadecylene aromatic alcohol, 2-octyl dodecanol, benzyl alcohol and water.

In general, it has proved to be advantageous in either human medicine or veterinary medicine, the total administrated dose of the active compound disclosed herein is about 0.01 to 500 mg/kg body weight every 24 hours, preferably 0.01 to 100 mg/kg body weight. If appropriate, the drug is administrated in single dose for multiple times, to achieve the desired effect. The amount of the active compound in a single dose is preferably about 1 to 80 mg, more preferably 1 to 50 mg/kg body weight. Nevertheless, the dose may also be varied according to the kind and the body weight of treatment objects, the nature and the severity of diseases, the type of preparations and the method of administration of drugs, and administration period or time interval.

The pharmaceutical composition provided herein further comprises anti-HBV drugs, and the anti-HBV drug is an HBV polymerase inhibitor, immunomodulator, interferon or other emerging anti-HBV agents such as HBV RNA replication inhibitors, HBsAg secretion inhibitors, HBV capsid inhibitors, antisense oligomers, siRNA, HBV therapeutic vaccines, HBV preventive vaccines, HBV antibody therapy (monoclonal or polyclonal) and agonists for the treatment or prevention of HBV.

The HBV agent is lamivudine, telbivudine, tenofovir, entecavir, adefovir dipivoxil, alfaferone, alloferon, celmoleukin, clevudine, emtricitabine, famciclovir, feron, hepatect CP, intefen, interferon α-1b, interferon α, interferon α-2a, interferon β-1a, interferon α-2, interleukin-2, mivotilate, nitazoxanide, peginterferon alfa-2a, ribavirin, roferon-A, sizofiran, euforavac, rintatolimod, phosphazid, heplisav, interferon α-2b, levamisole, or propagermanium, and the like.

In one aspect, provided herein is use of the compound disclosed herein or pharmaceutical compositions thereof in the manufacture of a medicament for preventing, managing, treating or lessening HBV diseases in a patient. The HBV disease is a hepatic disease caused by hepatitis B virus infection or hepatitis B infection, including acute hepatitis, chronic hepatitis, cirrhosis and hepatocellular carcinoma. The symptoms of acute hepatitis B virus infection may be asymptomatic or manifested as acute hepatitis symptoms. A patient with chronic virus infection suffers an active disease, which can progress to cirrhosis and liver cancer.

The compounds or pharmaceutical compositions of the present invention may be used for inhibiting the production or secretion of HBsAg,comprising administering to a patient a pharmaceutically acceptable effective dose.

The compounds or pharmaceutical compositions of the present invention may be used for inhibiting the production of HBV DNA, comprising administering to a patient a pharmaceutically acceptable effective dose.

In one aspect, the compounds or pharmaceutical compositions of the present invention may be used for inhibiting the production of HBV genetic expression, including administering to a patient a pharmaceutically acceptable effective dose.

Those anti-HBV agents may be administered separately from the composition containing the compound disclosed herein as part of a multiple dosage regimen. Alternatively, those agents may be part of a single dosage form, mixed together with the compound disclosed herein in a single composition. If administered as part of a multiple dosage regimen, the two active agents may be submitted simultaneously, sequentially or within a period of time from one another which would result in the desired activity of the agents.

The amount of both the compound and the additional therapeutic agent (in those compositions which comprise an additional therapeutic agent as described above) that may be combined with the carrier materials to produce a single dosage form will vary depending upon the host treated and the particular mode of administration. Normally, the amount of the compositions disclosed herein will be no more than the amount of the composition comprising the only active agent as therapeutic agent.

The compounds of the present invention show a strong antiviral effect. Such compounds have unexpected antiviral activity against HBV and are therefore suitable for the treatment of various diseases caused by viruses, in particular diseases caused by acute and chronic persistent HBV viral infections. Chronic viral diseases caused by HBV can lead to various syndromes of varying severity. It is well known that chronic hepatitis B virus infection can lead to cirrhosis and/or hepatocellular carcinoma.

Examples of indications that may be treated with the compounds of the present invention are: treatment of acute and chronic viral infections that can cause infectious hepatitis, such as heterosexual hepatitis virus infections. Particularly preferred treatment are the treatment of chronic hepatitis B infection and the treatment of acute hepatitis B virus infection.

The invention also relates to the use of the compounds and compositions of the invention in the manufacture of a medicament for the treatment and prevention of viral diseases, in particular hepatitis B.

### GENERAL SYNTHETIC PROCEDURES

For the purpose of describing the invention, the following examples are listed. It should be understood that, the invention is not limited to these examples, and the present invention only provide the method to practice the invention.

Generally, the compounds disclosed herein may be prepared by methods described herein, wherein the substituents are as defined for Formula (I), except where further noted. The following non-limiting schemes and examples are presented to further exemplify the invention.

Persons skilled in the art will recognize that the chemical reactions described may be readily adapted to prepare a number of other compounds disclosed herein, and alternative methods for preparing the compounds disclosed herein are deemed to be within the scope disclosed herein. For example, the synthesis of non-exemplified compounds according to the invention may be successfully performed by modifications apparent to those skilled in the art, e.g., by appropriately protecting interfering groups, by utilizing other suitable reagents known in the art other than those described, and/or by making routine modifications of reaction conditions. Alternatively, other reactions disclosed herein or known in the art will be recognized as having applicability for preparing other compounds disclosed herein.

In the examples described below, unless otherwise indicated all temperatures are set forth in degrees Celsius (°C). Reagents were purchased from commercial suppliers such as Aldrich Chemical Company, Arco Chemical Company and Alfa Chemical Company, and were used without further purification unless otherwise indicated. Common solvents were purchased from commercial suppliers such as Shantou XiLong Chemical Factory, Guangdong Guanghua Reagent Chemical Factory Co. Ltd., Guangzhou Reagent Chemical Factory, Tianjin YuYu Fine Chemical Ltd., Qingdao Tenglong Reagent Chemical Ltd., Qingdao Ocean Chemical Factory.

Nuclear magnetic resonance (NMR) spectra were recorded by a Bruker Avance 400 MHz spectrometer or Bruker Avance III HD 600 spectrometer, using CDCl₃, DMSO-d₆, CD₃OD or acetone-d*₆* (reported in ppm) as solvent, and using TMS (0 ppm) or chloroform (7.25 ppm) as the reference standard. When peak multiplicities were reported, the following abbreviations were used: s (singlet), s, s (singlet, singlet), d (doublet), t (triplet), m (multiplet), br (broadened), dd (doublet of doublets), ddd (doublet of doublet of doublets), dt (doublet of triplets), ddt (doublet of doublet of triplets), td (triplet of doublets), br.s (broadened singlet). Coupling constants, when given, were reported in Hertz (Hz).

Low-resolution mass spectral (MS) data were determined by an Agilent 6320 Series LC-MS spectrometer equipped with a G1312A binary pump and a G1316A TCC (column was operated at 30 °C). G1329A autosampler and G1315B DAD detector were applied in the analysis, and an ESI source was used in the LC-MS spectrometer.

Low-resolution mass spectral (MS) data were determined by an Agilent 6120 Series LC-MS spectrometer equipped with a G1311A quaternary pump and a G1316A TCC (column was operated at 30 °C). G1329A autosampler and G1315B DAD detector were applied in the analysis, and an ESI source was used in the LC-MS spectrometer.

Both LC-MS spectrometers above were equipped with an Agilent Zorbax SB-C18 column, 2.1 × 30 mm, 5 µm. Injection volume was decided by the sample concentration. The flow rate was 0.6 mL/min. The HPLC peaks were recorded by UV-Vis wavelength at 210 nm and 254 nm. The mobile phase was 0.1% formic acid in acetonitrile (phase A) and 0.1% formic acid in ultrapure water (phase B). The gradient elution conditions were shown in Table 1:

**Table 1 The gradient elution conditions**

| Time (min) | A (CH₃CN,0.1% HCOOH) | B (H₂O,0.1% HCOOH) |
|---|---|---|
| 0-3 | 5- 100 | 95 - 0 |
| 3-6 | 100 | 0 |
| 6-6.1 | 100 - 5 | 0-95 |
| 6.1 - 8 | 5 | 95 |

Purities of compounds were assessed by Agilent 1100 Series High Performance Liquid Chromatography (HPLC) with UV detection at 210 nm and 254 nm on a Zorbax SB-C18 column with a size of 2.1 × 30 mm, 4 µm, 10 minutes; the flow rate was 0.6 mL/min; the mobile phase was 5-95% (0.1% formic acid in acetonitrile) in (0.1% formic acid in water) and the column temperature was maintained at 40 °C.

The following abbreviations are used throughout the specification:
- AcOK: potassium acetate
- MeCN, CH₃CN: acetonitrile
- MeOH: methanol
- DCM, CH₂Cl₂: dichloromethane
- D₂O: deuteroxide
- DME: dimethyl ether
- DMSO: dimethylsulfoxide
- DMF: dimethylformamide
- DMAP: 4-dimethylaminopyridine
- DIBAH: diisobutylaluminium hydride
- CHCl₃: chloroform, trichloromethane
- CDCl₃: chloroform-*d*, Deuterated chloroform
- CCl₄: tetrachloromethane
- BOC, Boc: *tert*-butoxycarbonyl
- Boc₂O: di-*tert*-butyl dicarbonate
- Bn: benzyl
- PE: petroleum ether
- Pd(dba)₂: bis(dibenzylideneacetone)palladium
- Pd₂(dba)₃: tris(dibenzylideneacetone)dipalladium
- Ph: phenyl
- PTSA: *p*-toluenesulfonic acid
- EA, EtOAc: ethyl acetate
- EtOH: ethanol
- HCl: hydrochloric acid
- K₂CO₃: potassium carbonate
- NaHCO₃: sodium bicarbonate
- NH₄OAc: ammonium acetate
- NaOH: sodium hydroxide
- NaBH₃CN: sodium cyanoborohydride
- NaCl: sodium chloride
- Na₂SO₄: sodium sulfate
- Et₃N, TEA: triethylamine
- NBS: *N*-bromosuccinimide
- H₂O: water
- mL, ml: milliliter
- min: minute, minutes
- *m*-CPBA: *m*-chloroperoxybenzoic acid
- h: hour, hours
- RT, rt: room temperature
- Rt: retention time
- H₂: hydrogen
- HATU: o-(7-azabenzotriazol-1-yl)-*N,N,N',N'*-te-tramethyluronium hexafluorophosphate
- HCl/EtOAc: a solution of hydrogen chloride in ethyl acetate
- HOAt: 1-hydroxy-7-azabenzotriazole
- DIPEA: *N,N*-diisopropylethylamine
- DCC: *N,N'*-dicylohexylcarbodiimide
- DMF: dimethylformamide
- DME: dimethyl ether
- THF: tetrahydrofuran
- TFA: trifluoroacetic acid
- Tf: trifluoromethylsulfonyl
- LiOH.HzO: lithium hydroxide monohydrate
- NaBH₃CN: sodium cyanoborohydride
- IPA: isopropanol
- DMSO: dimethylsulfoxide
- CuCN: cuprous cyanide
- CH₃OH, MeOH: methanol
- N₂: nitrogen
- NH₄Cl: ammonium chloride
- NH₄OAc: ammonium acetate
- Ac₂O: acetic anhydride
- Xantphos: 4,5-bis(diphenylphosphino)-9,9-dimethylxanthene
- t_{1/2}: half-time
- *t*-BuOH: *tert*-butanol
- AUC: area under the curve
- Vss: apparent volume of distribution at steady state
- CL, clearance: clearance
- *F*, absolute bioavailability: bioavailability
- Dose: dosage
- Tmax: time to peak
- Cₘₐₓ: maximum concentration
- hr^{∗}ng/mL: plasma concentration*time

### Synthetic method

The following schemes list the synthetic steps of the compounds of the invention, wherein, each R¹, R³, R⁴, R⁵, R^{6a}, R⁷, R⁸, R^{10a}, R¹⁰, R¹³, R^{a} and R^{b} are as defined in the invention, X is halogen.

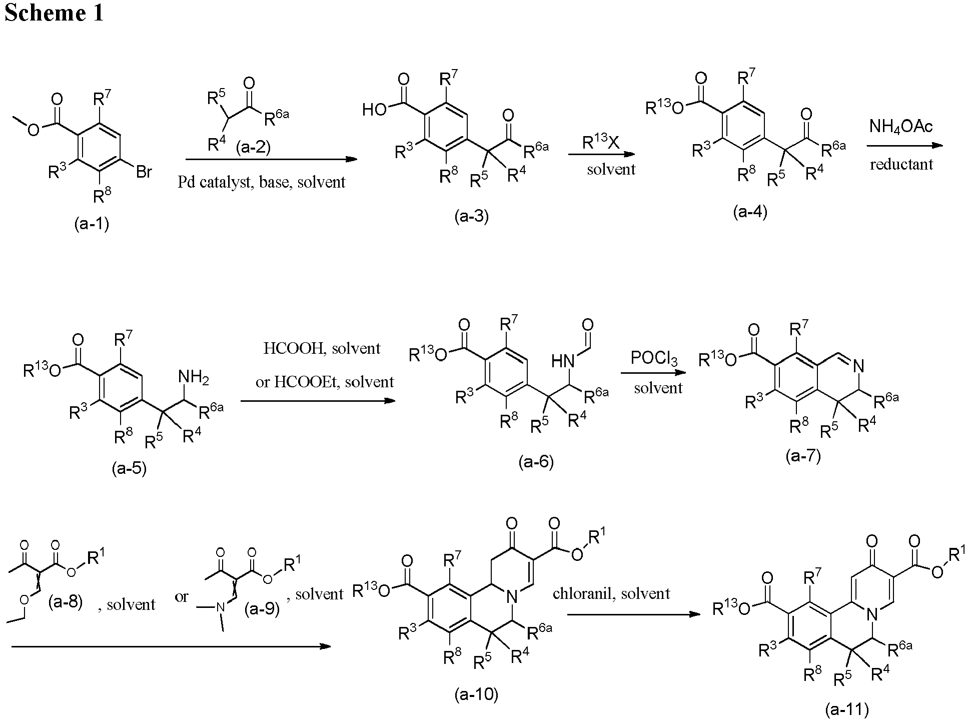

Compound having Formula (a-11) can be prepared by the process illustrated in **scheme 1**, wherein R³ is as defined in the invention. Firstly, compound (a-1) with compound (a-2) can undergo coupling reaction in the presence of a palladium catalyst (such as Pd(dba)₂, Pd₂(dba)₃, *etc.),* a ligand (such as Xantphos, *etc.),* a suitable base (such as sodium tert-butoxide, *etc.)* and a suitable solvent (such as THF, toluene, and the like) to give compound (a-3). Compound (a-3) with R¹³X can undergo nucleophilic substitution in the presence of a base (such as K₂CO₃, and the like) in a suitable solvent (such as CH₃CN, and the like) to give compound (a-4). Compound (a-4) with NH₄OAc can undergo reductive amination in the presence of a reductant (such as NaBH₃CN, and the like) in a suitable solvent (such as methanol, and the like) to give compound (a-5). Compound (a-5) can react with formic acid or ethyl formate in a suitable solvent (such as 1,4-dioxane, tetrahydrofuran, and the like) to give compound (a-6). Then, compound (a-6) can react with phosphorus oxychloride in a suitable solvent (such as DCM, and the like) to give compound (a-7). Nextly, compound (a-7) with compound (a-8) or compound (a-9) in a suitable solvent (such as isopropanol, ethanol, DMSO, and the like) can undergo cyclization to give compound (a-10). Lastly, compound (a-10) with chloranil can undergo dehydrogenation reaction in a suitable solvent (such as DME, and the like) to give compound (a-11).

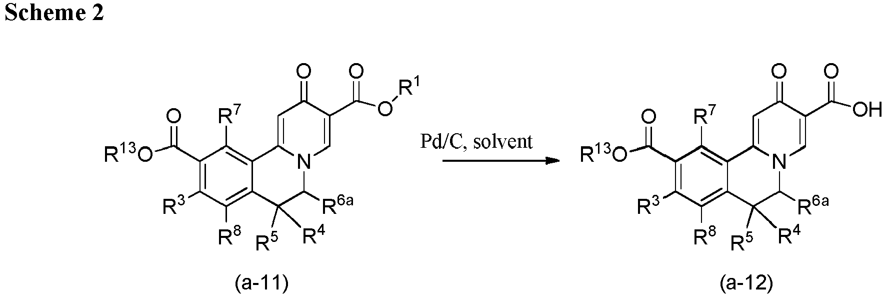

When R¹ is benzyl and R¹³ is not benzyl, compound having Formula (a-12) can be prepared by the process illustrated in **scheme 2**, wherein R³ is as defined in the invention. Benzyl group of compound (a-11) can be removed in the presence of Pd/C catalyst in a suitable solvent (such as THF, methano, and the like) in hydrogen atomosphere (0.1 MPa) to give compound having Formula (a-12).

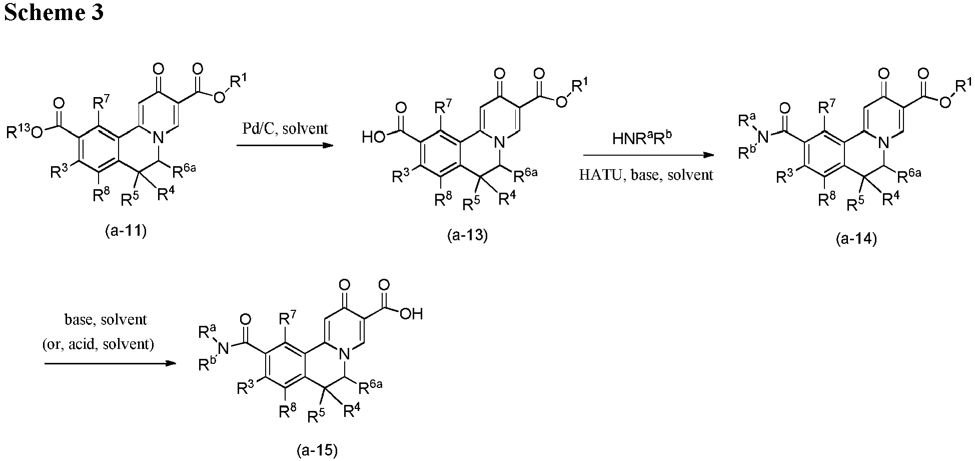

When R¹³ is benzyl and R¹ is not benzyl, compound having Formula (a-15) can be prepared by the process illustrated in **scheme 3**, wherein R³ is as defined in the invention. Firstly, benzyl group of compound (a-11) can be removed in the presence of Pd/C catalyst in a suitable solvent (such as THF, methanol, and the like) in hydrogen atomosphere (0.1 MPa) to give compound having Formula (a-13). Then, compound (a-13) can react with HNR^{a}R^{b} in the presence of HATU and a base (such as DIPEA, and the like) in a suitable solvent (such as DMF, and the like) to give compound (a-14). Lastly, compound (a-14) can undergo ester hydrolysis in the presence of a base (such as lithium hydroxide, sodium hydroxide, and the like) in a suitable solvent (such as THF/H₂O, EtOH/H₂O, MeOH/H₂O, MeOH/THF, H₂O, and the like), or in the presence of an acid (such as TFA, and the like) in a suitable solvent (such as DCM, and the like) to give compound (a-15).

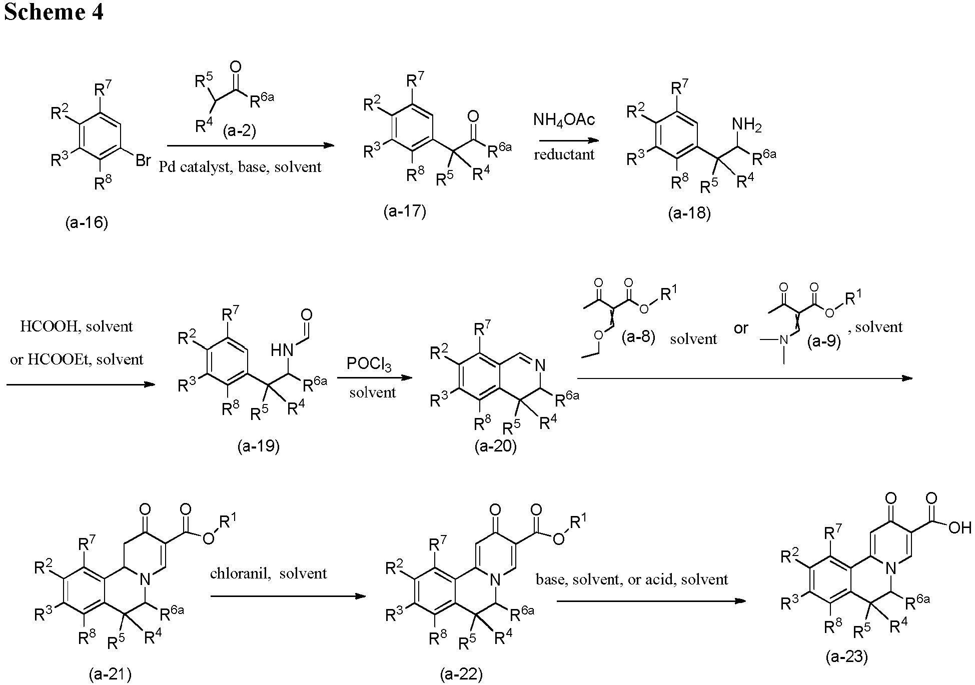

Compound having Formula (a-23) can be prepared by the process illustrated in **scheme 4**, wherein R³ and R² are as defined herein, and R³ can also be I or -NBn₂, R² can also be I, phenoxy or -NBn₂. Firstly, compound (a-16) with compound (a-2) can undergo coupling reaction in the presence of a palladium catalyst (such as Pd(dba)₂, Pd₂(dba)₃, *etc.),* a ligand (such as Xantphos, *etc.),* a suitable base (such as sodium tert-butoxide, and the like) in a suitable solvent (such as THF, toluene, *etc.)* to give compound (a-17). Compound (a-17) with NH₄OAc can undergo reductive amination in the presence of a reductant (such as NaBH₃CN, and the like) in a suitable solvent (such as methanol, and the like) to give compound (a-18). Compound (a-18) can react with formic acid or ethyl formate in a suitable solvent (such as 1,4-dioxane, tetrahydrofuran, and the like) to give compound (a-19). Then, compound (a-19) can react with phosphorus oxychloride in a suitable solvent (such as DCM, and the like) to give compound (a-20). Nextly, compound (a-20) with compound (a-8) or compound (a-9) in a suitable solvent (such as isopropanol, ethanol, DMSO, and the like) can undergo cyclization to give compound (a-21). Lastly, compound (a-21) with chloranil can undergo dehydrogenation reaction in a suitable solvent (such as DME, and the like) to give compound (a-22). Lastly, compound (a-22) can undergo _{ester hydrolysis in the presence of a base (such as} lithium hydroxide, sodium hydroxide, and the like) in a suitable solvent (such as THF/H₂O, EtOH/H₂O, MeOH/H₂O, MeOH/THF, H₂O, and the like), or _{in the presence of a acid} (such as TFA, and the like) in a suitable solvent (such as DCM, and the like) to give compound (a-23).

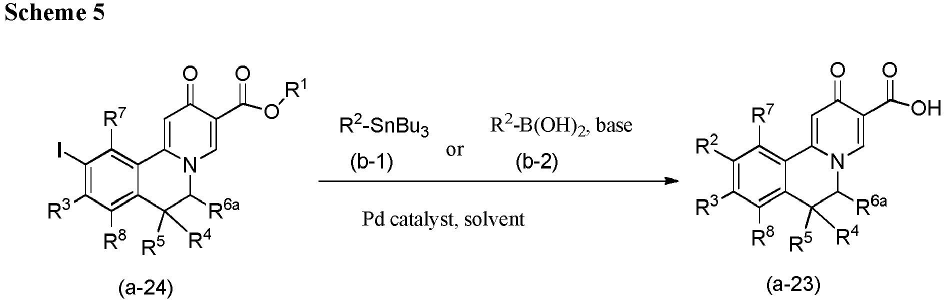

Compound having Formula (a-23) can be prepared by the process illustrated in **scheme 5**, wherein R³ is as defined herein; R² is cyclopropyl, C₄₋₇ cycloalkyl, 3- to 12-membered heterocyclyl, C₆₋₁₀ aryl or 5- to 10-membered heteroaryl, each of which is unsubstituted or substituted with 1, 2, 3 or 4 R^{w}; and R^{w} is as defined herein. Compound (a-24) with compound (b-1) or compound (b-2) can undergo coupling reaction in the presence of a Pd catalyst (such as bis(triphenylphosphine)palladium(II) chloride, and the like) in a suitable solvent (such as dioxane, and the like) to give compound (a-23).

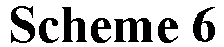

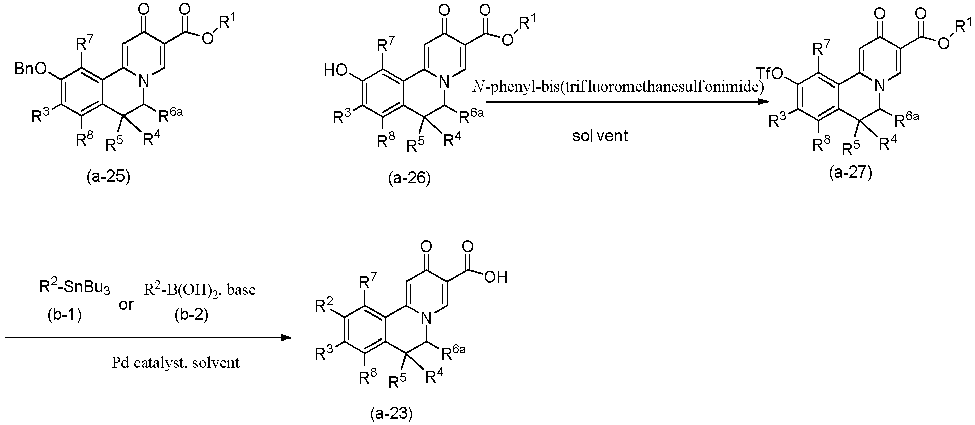

Compound having Formula (a-23) can be prepared by the process illustrated in **scheme 6,** wherein R³ is as defined herein; R² is cyclopropyl, C₄₋₇ cycloalkyl, 3- to 12-membered heterocyclyl, C₆₋₁₀ aryl or 5- to 10-membered heteroaryl, each of which is unsubstituted or substituted with 1, 2, 3 or 4 R^{w}; and R^{w} is as defined herein. Firstly, benzyl group on compound (a-25) can be removed to give compound (a-26), then compound (a-26) can react with *N*-phenyl-bis(trifluoromethanesulfonimide) under a base condition (such as in the presence of triethylamine, and the like) in a suitable solvent (such as dichloromethane, and the like) to give compound (a-27). Lastly, compound (a-27) with compound (b-1) can undergo coupling reaction in the presence of a Pd catalyst (such as bis(triphenylphosphine)palladium(II) chloride, and the like) in a suitable solvent (such as 1,4- dioxane, and the like) to give compound (a-23); or compound (a-27) with compound (b-2) can undergo coupling reaction in the presence of a Pd catalyst (such as tetrakis(triphenylphosphine)palladium(0), and the like), a suitable solvent (such as 1,4-dioxane, and the like) and a suitable base (such as sodium carbonate, potassium phosphate, potassium carbonate, and the like) to give compound (a-23).

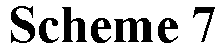

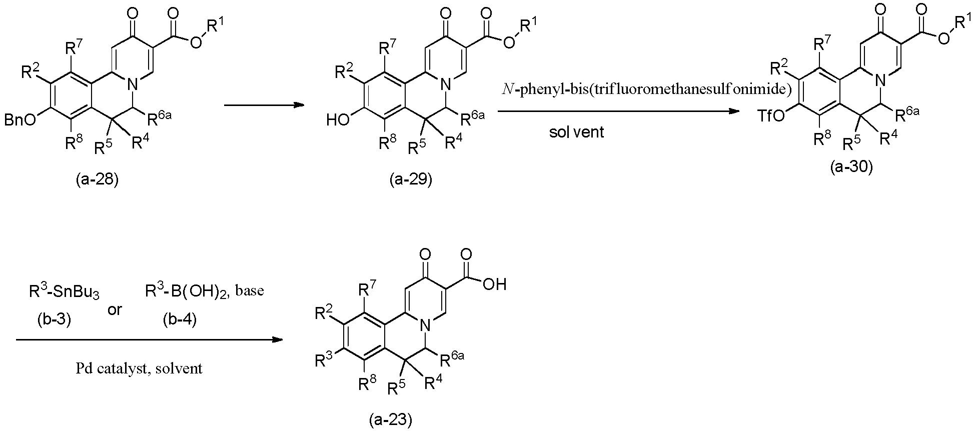

Compound having Formula (a-23) can be prepared by the process illustrated in **scheme 7**, wherein R² is as defined herein; R³ is cyclopropyl, C₄₋₇ cycloalkyl, 3- to 12-membered heterocyclyl, C₆₋₁₀ aryl or 5- to 10-membered heteroaryl, each of which is unsubstituted or substituted with 1, 2, 3 or 4 R^{w}; and R^{w} is as defined herein. Firstly, benzyl group on compound (a-28) can be removed to give compound (a-29), then compound (a-29) can react with *N*-phenyl-bis(trifluoromethanesulfonimide) under a base condition (such as in the presence of triethylamine, and the like) in a suitable solvent (such as dichloromethane, and the like) to give compound (a-30). Lastly, compound (a-30) with compound (b-3) can undergo coupling reaction in the presence of a Pd catalyst (such as bis(triphenylphosphine)palladium(II) chloride, and the like) in a suitable solvent (such as 1,4-dioxane, and the like) to give compound (a-23); or compound (a-30) with compound (b-4) can undergo coupling reaction in the presence of a Pd catalyst (such as tetrakis(triphenylphosphine)palladium(0), and the like), a suitable solvent (such as 1,4-dioxane, and the like) and a suitable base (such as sodium carbonate, potassium phosphate, potassium carbonate, and the like) to give compound (a-23).

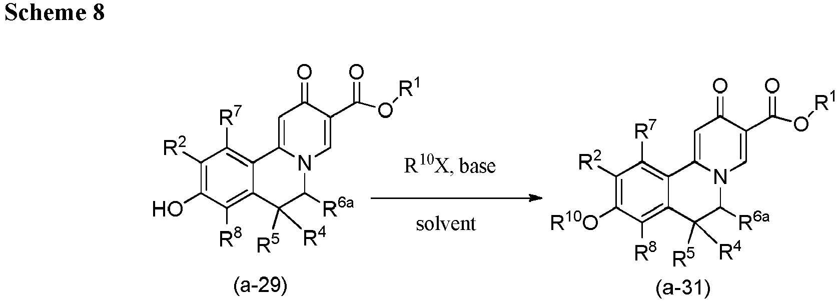

Compound having Formula (a-31) can be prepared by the process illustrated in **scheme 8**, wherein X is halogen. Compound (a-29) can react with R¹⁰X in the presence of a suitable base (such as potassium carbonate, triethylamine, and the like) in a suitable solvent (such as acetonitrile, DMF, DCM, and the like) at a suitable temperature to give compound (a-31).

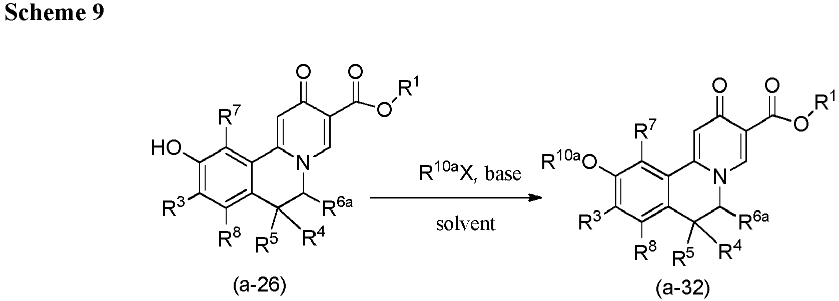

Compound having Formula (a-31) can be prepared by the process illustrated in **scheme 9,** wherein X is halogen. Compound (a-26) can react with R¹⁰X in the presence of a suitable base (such as potassium carbonate, triethylamine, and the like) in a suitable solvent (such as acetonitrile, DMF, DCM, and the like) at a suitable temperature to give compound (a-32).

### DESCRIPTION OF THE PREFERRED EMBODIMENTS

The following examples are used for illustrating the invention, but can not be construed to limit the scope of the invention.

### PREPARATION EXAMPLES

In the following preparation examples, the inventors took a part of the compounds of the present invention as examples to describe in detail the preparation process of the compounds of the present invention.

### Example 1: 6-isopropyl-10-(methoxycarbonyl)-9-(3-methoxypropoxy)-2-oxo-6,7-dihydro -2H-pyrido[2,1-a]isoquinoline-3-carboxylic acid

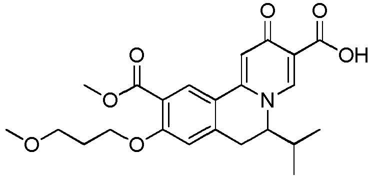

### Step 1: methyl 4-bromo-2-hydroxybenzoate

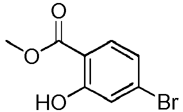

To a three-neck flask were added 4-bromo-2-hydroxybenzoic acid (20 g, 92.16 mmol) and CH₃OH (200 mL). After the mixture was stirred well, to the mixture was added DMF (0.5 mL). The reaction mixture was cooled in an ice-bath, then thionyl chloride (8.02 mL, 111 mmol) was added dropwise slowly into the mixture. After addition, the resulting mixture was heated to reflux and stirred for 12 hours. After the reaction was completed, the mixture was concentrated *in vacuo,* and the residue was diluted with water (300 mL) and EtOAc (500 mL), then the resulting mixture was stiring, and 5% aqueous sodium hydroxide solution was then added to adjust pH to 6-8, then the mixture was stood for partition. The separated organic layer was washed with saturated brine, dried over anhydrous sodium sulfate, and filtered. The filtrate was concentrated *in vacuo* to give the title compound as a pale brown solid (20 g, 93.932%).
¹H NMR (400 MHz, CDCl₃): δ 10.83 (s, 1H), 7.70 (d, *J* = 8.53 Hz, 1H), 7.20 (s, 1H), 7.04 (d, *J*= 8.50 Hz, 1H), 3.97 (s, 3H).

### Step 2: methyl 4-bromo-2-(3-methoxypropoxy)benzoate

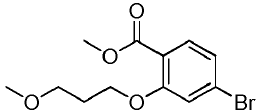

To a 1 L single-neck flask were added methyl 4-bromo-2-hydroxybenzoate (44.5 g, 193 mmol) and CH₃CN (450 mL). After the solid was dissolved completely by stirring, K₂CO₃ (39.9 g, 289 mmol) and 1-bromo-3-methoxypropane (45.1 g, 289 mmol) were added in turn. The resulting mixture was heated to 80 °C and stirred for 12 hours at 80 °C. After the reaction was completed, the reaction mixture was cooled to 25 °C and filtered. The filter cake was washed with acetonitrile (100 mL). The combined filtrates was concentrated *in vacuo,* and the residue was diluted with EtOAc (500 mL). The mixture was washed with saturated brine, dried over anhydrous sodium sulfate and filtered. The filtrate was concentrated *in vacuo* to give the title compound as orange oil (58 g, 99.3%).
MS (ESI, pos.ion) *m*/*z:* 303.2 [M+H] ⁺.

### Step 3: 2-(3-methoxypropoxy)-4-(3-methyl-2-oxobutyl)benzoic acid

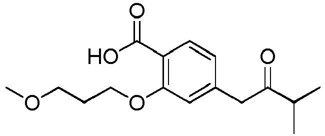

To a three-neck flask were added sodium tert-butoxide (56 g, 666.0 mmol) and THF (600 mL) in turn. After stirring well, to the mixture was added 4,5-bis(diphenylphosphino)-9,9-dimethylxanthene (3.4 g, 5.9 mmol) and Pd₂(dba)₃ (4.4 g, 4.8 mmol). The reaction mixture was degassed and refilled with nitrogen for three times, then 3-methylbutan-2-one (33 g, 383.1 mmol) and methyl 4-bromo-2-(3-methoxypropoxy)benzoate (58 g, 191.32 mmol) were added in turn. The mixture was degassed and refilled with nitrogen for three times, then heated to 55 °C and stirred at this temperature for 4 hours. After the additon, the reaction mixture was filtered and the filter cake was washed with THF (100 mL). To the filtrate was added water (100 mL), and the mixture was concentrated *in vacuo* to remove the solvent. To the residue was added water (800 mL), and the mixture was stirred, then extracted with EtOAc (250 mL × 4), and the oragnic layer was discarded. To the aqueous layer was added EtOAc (800 mL), then the resulting mixture was stirred, and concentrated hydrochloric acid was then added to adjust pH to 5-6, then the mixture was stood for partition. The separated organic layer was washed with saturated brine, dried over anhydrous sodium sulfate, and filtered. The filtrate was concentrated *in vacuo* to give the title compound as brown oil (32 g, 56.83%).
MS (ESI, pos.ion) *m*/*z:* 295.1 [M+H] ⁺.

### Step 4: methyl 2-(3-methoxypropoxy)-4-(3-methyl-2-oxobutyl)benzoate

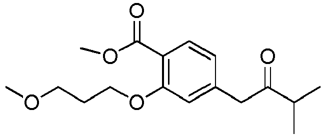

To a 500 mL single-neck flask were added 2-(3-methoxypropoxy)-4-(3-methyl-2-oxobutyl)benzoic acid (10 g, 33.98 mmol) and CH₃CN (150 mL). After the solid was dissolved by stirring, K₂CO₃ (7.1 g, 51 mmol) and iodomethane (2.75 mL, 44.2 mmol) were added. The reaction mixture was heated to reflux and stirred for 8 hours, then cooled to 25 °C. The mixture was filtered, and the filtrate was concentrated *in vacuo.* The residue was diluted with EtOAc (200 mL), and the mixture was washed with saturated brine, dired over anydrous sodium sulfate and filtered. The filtrate was concentrated *in vacuo,* and the residue was purified by silica gel column chromatography (PE/EtOAc (V/V)=15/1) to give the title compound as light yellow oil (8.9 g, 85%).
MS (ESI, pos.ion) *m*/*z:* 309.3 [M+H] ⁺.

### Step 5: methyl 4-(2-amino-3-methylbutyl)-2-(3-methoxypropoxy)benzoate

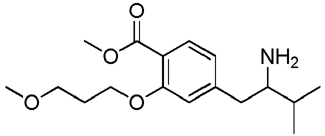

Methyl 2-(3-methoxypropoxy)-4-(3-methyl-2-oxobutyl)benzoate (6.2 g, 20 mmol) was dissolved in CH₃OH (60 mL), then CH₃COONH₄ (15 g, 194.6 mmol) was added into the mixture. The resulting mixture was stirred for 30 minitues, then cooled to 0 °C, and NaBH₃CN (2.5 g, 40 mmol) was added. The reaction mixture was stirred at 25 °C for 20 hours. After the reaction was completed, the reaction mixture was concentrated *in vacuo* and the residue was diluted with EtOAc (200 mL). The organic layer was washed with saturated brine, dried over anhydrous sodium sulfate and filtered. The filtrate was concentrated *in vacuo* to give the title compound as colorless oil (6.2 g, 100%).
MS (ESI, pos.ion) *m*/*z:* 310.3 [M+H]⁺.

### Step 6: methyl 4-(2-formamido-3-methylbutyl)-2-(3-methoxypropoxy)benzoate

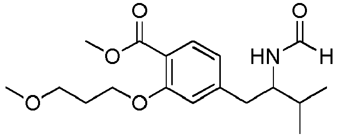

Methyl 4-(2-amino-3-methylbutyl)-2-(3-methoxypropoxy)benzoate (1.0 g, 3.23 mmol) was dissolved in dioxane (10 mL), then to the mixture was added formic acid (2.2 g, 42 mmol). The mixture was heated and refluxed for 21 hours. After the reaction was completed, the reaction mixture was cooled to 50 °C, and then concentrated *in vacuo.* The residue was diluted with EtOAc (100 mL). The organic layer was washed with saturated brine, dried over anhydrous sodium sulfate and filtered. The filtrate was concentrated *in vacuo* to give the title compound as light yellow oil (1.09 g, 100%).
MS (ESI, pos.ion) *m*/*z:* 338.2[M+H]⁺.

### Step 7: methyl 3-isopropyl-6-(3-methoxypropoxy)-3,4-dihydroisoquinoline-7-carboxylate

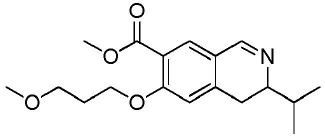

To a 100 mL single-neck flask were added dichloromethane (5 mL) and methyl 4-(2-formamido-3-methylbutyl)-2-(3-methoxypropoxy)benzoate (0.45 g, 1.3 mmol), then the mixture was stirred and cooled to 0 °C, and then phosphorus oxychloride (0.2 mL, 2 mmol) was added. The resulting mixture was heated to reflux for 2 hours, then cooled to 0 °C, and DCM (50 mL) was added to dilute the mixture. The resulting mixture was added slowly into the water (20 mL), then the mixture was adjusted with ammonium hydroxide to pH 7-8, and then stood for partition. The aqueous layer was extracted with DCM (30 mL × 2), and the combined organic layers was washed with saturated brine, dried over anhydrous sodium sulfate, and filtered. The filtrate was cocentrated *in vacuo* to give the title compound as a light yellow solid (0.4 g, 90%).
MS (ESI, pos.ion) *m*/*z:* 320.2 [M+H]⁺.

### Step 8: 3-benzyl 10-methyl 6-isopropyl-9-(3-methoxypropoxy)-2-oxo-2,6,7,11b-tetrahydro -1H-pyrido[2,1-a]isoquinoline-3,10-dicarboxylate

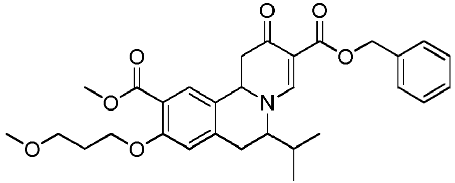

To a dried flask were added ethanol (10 mL), methyl 3-isopropyl-6-(3-methoxypropoxy)-3,4-dihydroisoquinoline-7-carboxylate (0.5 g, 1.57 mmol) and benzyl 2-(ethoxymethylene)-3-oxobutanoate (583 mg, 2.348 mmol) in turn. The reaction mixture was heated and refluxed for 17 hours. After the reaction was completed, the mixture was concentrated *in vacuo,* and the residue was purified by silica gel column chromatography (DCM/CH₃OH(V/V)=50/1) to give the title compound as brown oil (0.8 g, 98%).
MS (ESI, pos.ion) *m*/*z:* 522.2 [M+H]⁺.

### Step 9: 3-benzyl 10-methyl 6-isopropyl-9-(3-methoxypropoxy)-2-oxo-6,7-dihydro -2H-pyrido[2,1-a]isoquinoline-3,10-dicarboxylate

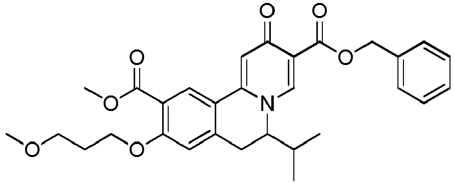

To a dried flask were added 1,2-dimethoxyethane (10 mL) and 3-benzyl 10-methyl 6-isopropyl-9-(3-methoxypropoxy)-2-oxo-2,6,7,11b-tetrahydro-1*H*-pyrido[2,1-*a*]isoquinoline-3, 10-dicarboxylate (0.8 g,1.534 mmol). The mixture was stirred well, then chloranil (380 mg, 1.53 mmol) was added. The reaction mixture was heated and reluxed for 3 hours, then concentrated *in vacuo,* and the residue was purified by silica gel column chromatography (DCM/CH₃OH(V/V)=50/1) to give the title compound as a gray solid (400 mg, 50.19%).
MS (ESI, pos.ion) *m*/*z:* 520.2 [M+H]⁺.

### Step 10: 6-isopropyl-10-(methoxycarbonyl)-9-(3-methoxypropoxy)-2-oxo-6,7-dihydro-2H-pyrido[2,1-a]isoquinoline-3-carboxylic acid

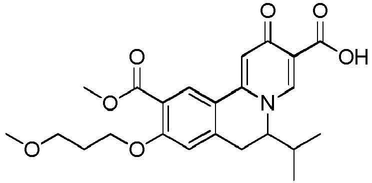

To a 50 mL reaction flask were added 3-benzyl 10-methyl 6-isopropyl-9-(3-methoxypropoxy)-2-oxo-6,7-dihydro-2*H*-pyrido[2,1-*a*]isoquinoline-3,10-dicarb oxylate (400 mg, 0.77 mmol), methanol (5 mL) and Pd/C (40 mg). The reaction mixture was stirred at rt for 24 hours in a hydrogen atmosphere, then filtered. The filter cake was washed with methanol (10 mL). The filtrate was concentrated, and the residue was purified by silica gel column chromatography (MeOH/DCM(V/V)=50/1) to give the title compound as a gray solid (90mg, 27.2%).
MS (ESI, pos.ion) *m*/*z:* 430.1[M+H]⁺;
¹H NMR (400 MHz, CDCl₃) δ 15.93 (s, 1H), 8.47 (s, 1H), 8.26 (s, 1H), 7.16 (s, 1H), 6.92 (s, 1H), 4.29 - 4.20 (m, 2H), 3.95 (s, 3H), 3.93 - 3.90 (m, 1H), 3.67 - 3.60 (m, 2H), 3.44 - 3.39 (m, 4H), 3.22 (d, *J* = 16.28 Hz), 1H), 2.19 - 2.13 (m, 2H), 1.80 - 1.73 (m, 1H), 0.97 (d, *J* = 6.64 Hz, 3H), 0.85 (d, *J=* 6.71 Hz, 3H).

### Example 2: 10-((benzyloxy)carbonyl)-6-isopropyl-9-(3-methoxypropoxy)-2-oxo-6,7 -dihydro-2H-pyrido[2,1-a]isoquinoline-3-carboxylic acid

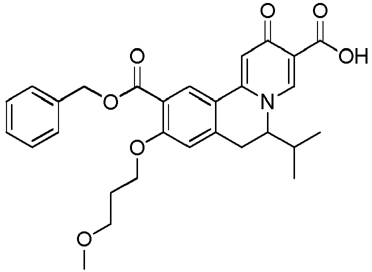

### Step 1: benzyl 2-(3-methoxypropoxy)-4-(3-methyl-2-oxobutyl)benzoate

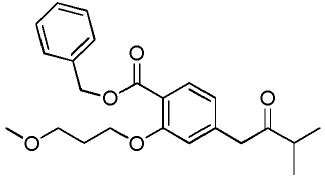

To a 500 mL single-neck flask were added 2-(3-methoxypropoxy)-4-(3-methyl-2-oxobutyl)benzoic acid (14.8 g, 50.3 mmol) and CH₃CN (150 mL). After the solid was dissolved completely by stirring, K₂CO₃ (10.4 g, 75.2 mmol) and benzyl bromide (6.3 mL, 53 mmol) were added in turn. The reaction mixture was heated and refluxed for 8 hours, then cooled to 25 °C, and filtered. The filtrate was concentrated *in vacuo,* and the residue was diluted with EtOAc (300 mL), then the mixture was washed with saturated brine. The organic layer was dried over anydrous sodium sulfate, and filtered. The filtrate was concentrated *in vacuo,* and the residue was purified by silica gel column chromatography (PE/EtOAc(V/V)=10/1) to give the title compound as light yellow oil (18 g, 93.1%).
MS (ESI, pos.ion) *m*/*z:* 385.6 [M+H] ⁺.

### Step 2: benzyl 4-(2-amino-3-methylbutyl)-2-(3-methoxypropoxy)benzoate

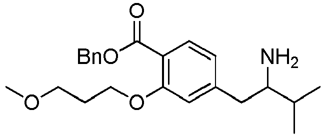

To a 500 mL single-neck flask were added benzyl 2-(3-methoxypropoxy)-4-(3-methyl-2-oxobutyl)benzoate (16.27 g, 42.31 mmol) and CH₃OH (250 mL). After the solid was dissolved completely by stirring, CH₃COONH₄ (33 g, 428.1 mmol) was added into the mixture. The reaction mixture was stirred for 30 minutes, then cooled to 0 °C, and NaBH₃CN (4 g, 63.65 mmol) was added in three portions. After addition, the reaction mixture was warmed to 25 °C and stirred for 24 hours. After the reaction was completed, the reaction mixture was concentrated *in vacuo,* and the residue was diluted with EtOAc (400 mL), then the mixture was washed with saturated brine. The organic layer was dried over anydrous sodium sulfate, and filtered. The filtrate was concentrated *in vacuo,* and the residue was purified by silica gel column chromatography (DCM/CH₃OH(V/V)=100/1) to give the title compound as colorless oil (14 g, 85.82%).
MS (ESI, pos.ion) *m*/*z:* 386.6 [M+H]⁺.

### Step 3: benzyl 4-(2-formamido-3-methylbutyl)-2-(3-methoxypropoxy)benzoate

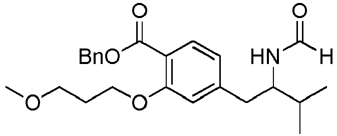

To a 50 mL single-neck flask were added benzyl 4-(2-amino-3-methylbutyl)-2-(3-methoxypropoxy)benzoate (2.7 g, 7.0 mmol), dioxane (12 mL) and formic acid (5.2 g, 110 mmol). The mixture was stirred at 110 °C for 24 hours under N₂ protection. After the reaction was completed, the reaction mixture was concentrated *in vacuo,* and to the residue was added saturated brine (15 mL). The residue was extracted with DCM (30 mL × 4). The combined organic layers were washed with saturated brine, dried over anhydrous sodium sulfate and filtered. The filtrate was concentrated *in vacuo* to give the title compound as brown oil (2.9 g, 100%).
MS (ESI, pos.ion) *m*/*z:* 414.2[M+H]⁺.

### Step 4: benzyl 3-isopropyl-6-(3-methoxypropoxy)-3,4-dihydroisoquinoline-7-carboxylate

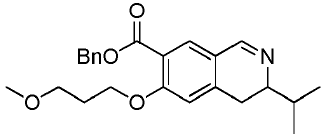

To a 100 mL single-neck flask were added benzyl 4-(2-formamido-3-methylbutyl)-2-(3-methoxypropoxy)benzoate (2.9 g, 7.0 mmol) and DCM (30 mL). The mixture was stirred well, then cooled to 0 °C, and POCl₃ (1.3 mL, 14 mmol) was added into the mixture. The reaction mixture was heated to 50 °C under N₂ protection, then stirred for 3 hours. After the reaction was completed, the reaction mixture was concentrated *in vacuo,* and to the residue was added ice-water (40 mL). The resulting mixture was extracted with EtOAc (50 mL×3). The combined organic layers were washed with saturated brine, dried over anydrous sodium sulfate, and filtered. The filtrate was concentrated *in vacuo* to give the title compound as a light yellow solid (2.7 g, 97%).
MS (ESI, pos.ion) *m*/*z:* 396.5[M+H]⁺.

### Step 5: 10-benzyl 3-tert-butyl 6-isopropyl-9-(3-methoxypropoxy)-2-oxo-2,6,7,11b -tetrahydro-1H-pyrido[2,1-a]isoquinoline-3,10-dicarboxylate

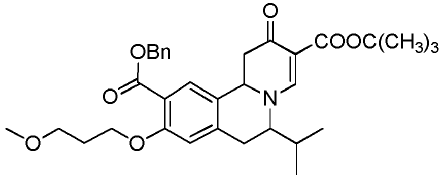

To a 100 mL single-neck flask were added benzyl 3-isopropyl-6-(3-methoxypropoxy)-3,4-dihydroisoquinoline-7-carboxylate (1.9 g, 4.8 mmol), tert-butyl 2-((dimethylamino)methylene)-3-oxobutanoate (1.5 g, 7 mmol) and tert-butanol (50 mL) in turn. The reaction mixture was stirred at 85 °C for 24 hours under N₂ protection, then concentrated *in vacuo.* The residue was purified by silica gel column chromatography (EtOAc/MeOH (V/V) = 20/1) to give the title compound as a light brown solid (1.62 g, 60%).
MS (ESI, pos.ion) *m*/*z:* 564.3 [M+H]⁺.

### Step 6: 10-benzyl 3-tert-butyl 6-isopropyl-9-(3-methoxypropoxy)-2-oxo-6,7-dihydro -2H-pyrido[2,1-a]isoquinoline-3,10-dicarboxylate

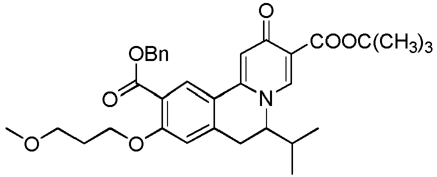

To a 250 mL three-neck flask were added 10-benzyl 3-tert-butyl 6-isopropyl-9-(3-methoxypropoxy)-2-oxo-2,6,7,11b-tetrahydro-1*H*-pyrido[2,1-*a*]isoquinoline-3, 10-dicarboxylate (12.2 g, 21.6 mmol) and DME (90 mL). After stirring well, chloranil (5.0 g, 20 mmol) was added into the mixture, and the reaction mixture was stirred at 25 °C for 12 hours. After the reaction was completed, the mixture was concentrated *in vacuo,* and the residue was purified by silica gel column chromatography (DCM/MeOH(V/V)=10/1) to give the title compound as a light brown solid (12.2 g, 100%).
MS (ESI, pos.ion) *m*/*z:* 562.4[M+H]⁺.

### Step 7: 10-((benzyloxy)carbonyl)-6-isopropyl-9-(3-methoxypropoxy)-2-oxo-6,7-dihydro-2H -pyrido[2,1-a]isoquinoline-3-carboxylic acid

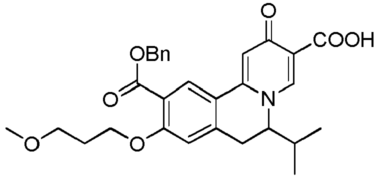

To a 50 mL single-neck flask were added 10-benzyl 3-*tert*-butyl 6-isopropyl-9-(3-methoxypropoxy)-2-oxo-6,7-dihydro-2*H*-pyrido[2,1-*a*]isoquinoline-3,10-dicarb oxylate (0.4 g, 0.71 mmol) and DCM (5 mL). After dissolving completely by stirring, to the reaction mixture was added TFA (3 mL). The reaction mixture was stirred at 25 °C for 12 hours, then concentrated *in vacuo.* The residue was diluted with EtOAc (100 mL), and the mixture was washed with saturated brine. The organic layer was dried over anydrous sodium sulfate, and filtered. The filtrate was concentrated *in vacuo,* and the residue was purified by silica gel column chromatography (DCM/CH₃OH(V/V)=50/1) to give the title compound as a white solid (0.2 g, 56%).
MS (ESI, pos.ion) *m*/*z:* 506.6 [M+H]⁺;
¹H NMR (400 MHz, CDCl₃) δ 15.95 (s, 1H), 8.47 (s, 1H), 8.26 (s, 1H),7.49 - 7.41 (m, 5H), 7.13 (s, 1H), 6.91 (s, 1H), 5.39 (s, 2H), 4.24 - 4.19 (m, 2H), 3.96 - 3.91 (m,1H), 3.55- 3.45 (m, 2H), 3.44 - 3.39 (m, 1H), 3.33 (s,3H), 3.19 (d, *J =* 15.90 Hz, 1H), 2.10 - 2.08 (m, 3H), 0.96 (d, *J* = 6.19 Hz 3H), 0.84 (d, *J=* 6.35 Hz, 3H).

### Example 3: 10-((cyclopentyloxy)carbonyl)-6-isopropyl-9-(3-methoxypropoxy)-2-oxo-6,7 -dihydro-2H-pyrido[2,1-a]isoquinoline-3-carboxylic acid

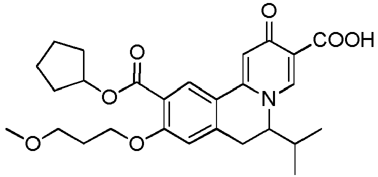

### Step 1: 3-(tert-butoxycarbonyl)-6-isopropyl-9-(3-methoxypropoxy)-2-oxo-6,7-dihydro -2H-pyrido[2,1-a]isoquinoline-10-carboxylic acid

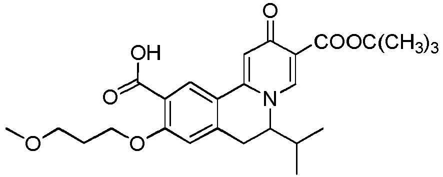

To a 100 mL single-neck flask were added 10-benzyl 3-*tert*-butyl 6-isopropyl-9-(3-methoxypropoxy)-2-oxo-6,7-dihydro-2*H*-pyrido[2,1-*a*]isoquinoline-3,10-dicarb oxylate (4.0 g, 7.1 mmol), THF (40 mL) and Pd/C (0.4 g, 10%). The reaction mixture was stirred at 25 °C for 12 hours under an hydrogen atomosphere of 0.1 MPa. After the reaction was completed, the mixture was filtered, and the filter cake was washed with DCM (50 mL). The filtrate was concentated *in vacuo,* and the residue was purified by silica gel column (DCM/MeOH(V/V)=10/1) to give the title compound as a light brown solid (3.0 g, 89%).
MS (ESI, pos.ion) *m*/*z:* 472.3 [M+H]⁺.

### Step 2: 3-tert-butyl 10-cyclopentyl 6-isopropyl-9-(3-methoxypropoxy)-2-oxo-6,7-dihydro-2H -pyrido[2,1-a]isoquinoline-3,10-dicarboxylate

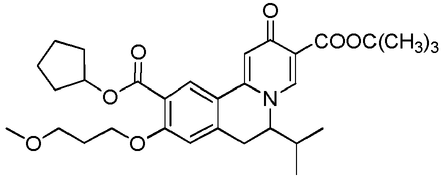

To a 50 mL single-neck flask were added 3-(*tert*-butoxycarbonyl)-6-isopropyl-9-(3-methoxypropoxy)-2-oxo-6,7-dihydro-2*H*-pyrido[2,1-*a*] isoquinoline-10-carboxylic acid (0.3 g, 0.6 mmol), bromocyclopentane (0.379 g, 2.54 mmol), K₂CO₃ (0.351 g, 2.54 mmol) and DMF (5 mL). The reaction mixture was heated to 50 °C, and stirred at this temperature for 12 hours, then cooled to 0 °C and diluted with water (10 mL). The mixture was extracted with DCM (20 mL × 4). The combined organic layers were washed with saturated brine, dried over anhydrous sodium sulfate and filtered. The filtrate was concentrated *in vacuo* to give the title compound as a light brown solid (0.343 g, 100%).
MS (ESI, pos.ion) *m*/*z:* 540.2 [M+H]⁺.

### Step 3: 10-((cyclopentyloxy)carbonyl)-6-isopropyl-9-(3-methoxypropoxy)-2-oxo-6,7 -dihydro-2H-pyrido[2,1-a]isoquinoline-3-carboxylic acid

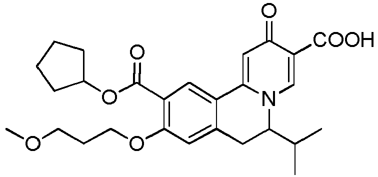

To a 50 mL single-neck flask were added 3-tert-butyl 10-cyclopentyl 6-isopropyl-9-(3-methoxypropoxy)-2-oxo-6,7-dihydro-2H-pyrido[2,1-a]isoquinoline-3,10-dicarb oxylate (0.343 g, 0.636 mmol), DCM (3 mL) and TFA (3 mL). The reaction mixture was stirred at 50 °C for 12 hours in a hydrogen atmosphere, then concentrated *in vacuo,* and the residue was purified by silica gel column chromatography (DCM/MeOH(V/V)=10/1) to give the title compound as a gray solid (120 mg, 39.0%).
MS (ESI, pos.ion) *m*/*z:* 484.3[M+H]⁺;
¹H NMR (400 MHz, CDCl₃) δ 15.98 (s, 1H), 8.48 (s, 1H), 8.17 (s, 1H), 7.14 (s, 1H), 6.90 (s, 1H), 4.47 - 4.35 (m, 1H), 4.27 - 4.17 (m, 2H), 3.96 - 3.91 (s, 1H), 3.70 - 3.55 (m, 2H), 3.44-3.33 (m, 4H), 3.23- 3.14 (m,1H), 2.16 - 2.13 (m,2H), 2.08 - 1.94 (m, 3H), 1.90 - 1.78 (m, 6H), 0.96 (d, *J* = 5.77 Hz , 3H), 0.84 (d, *J=* 5.95 Hz, 3H).

### Example 4: 10-(isopropoxycarbonyl)-6-isopropyl-9-(3-methoxypropoxy)-2-oxo-6,7-dihydro -2H-pyrido[2,1-a]isoquinoline-3-carboxylic acid

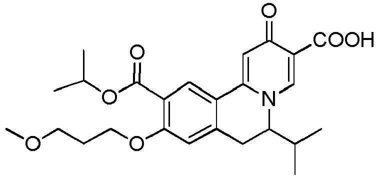

### Step 1: 3-tert-butyl 10-isopropyl 6-isopropyl-9-(3-methoxypropoxy)-2-oxo-6,7-dihydro -2H-pyrido[2,1-a]isoquinoline-3,10-dicarboxylate

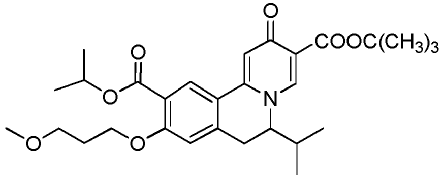

The title compound was prepared according to the synthetic method of step 2 in example 3 by using 3-(tert-butoxycarbonyl)-6-isopropyl-9-(3-methoxypropoxy) -2-oxo-6,7-dihydro-2*H*-pyrido[2,1-*a*]isoquinoline-10-carboxylic acid (0.47 g, 1 mmol), isopropyl iodide (0.34 g, 2 mmol), potassium carbonate (0.28 g, 2 mmol) and DMF (10 mL) as raw materials to give the title compound as a gray solid (0.33 g, 64%).
MS (ESI, pos.ion) *m*/*z:* 514.3 [M+H]⁺.

### Step 2: 10-(isopropoxycarbonyl)-6-isopropyl-9-(3-methoxypropoxy)-2-oxo-6,7-dihydro -2H-pyrido[2,1-a]isoquinoline-3-carboxylic acid

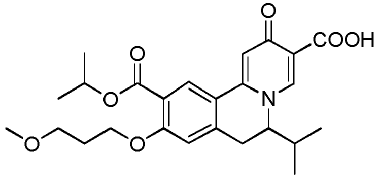

The title compound was prepared according to the synthetic method of step 3 in example 3 by using 3-tert-butyl 10-isopropyl 6-isopropyl-9-(3-methoxypropoxy)-2-oxo -6,7-dihydro -2*H*-pyrido[2,1-*a*]isoquinoline-3,10-dicarboxylate (0.26 g, 0.5 mmol), DCM (3 mL) and TFA (3 mL) as raw materials to give the title compound as an offwhite solid (112 mg, 49%).
MS (ESI, pos.ion) *m*/*z:* 458.1 [M+H]⁺;
¹H NMR (400 MHz, CDCl₃) δ 16.07 (s, 1H), 8.53 (s, 1H), 8.15 (s, 1H), 7.12 (s, 1H), 6.90 (s, 1H), 5.32 -5.23 (m, 1H), 4.25 - 4.16 (m, 2H), 4.04 (dd, *J=* 9.4, 4.4 Hz,1H), 3.64 - 3.584 (m, 2H), 3.43 (dd, *J =* 4.84, 16.46 Hz, 1H), 3.35 (s, 3H), 3.18 (d, *J =* 16.32 Hz, 1H), 2.15 - 2.09 (m, 2H), 1.78-1.69 (m, 1H), 1.39 - 1.35 (m, 6H), 0.94 (d, 3H), 0.80 (d, 3H).

### Example 5: 6-isopropyl-9-(3-methoxypropoxy)-10-(methylcarbamoyl)-2-oxo-6,7-dihydro -2H-pyrido[2,1-a]isoquinoline-3-carboxylic acid

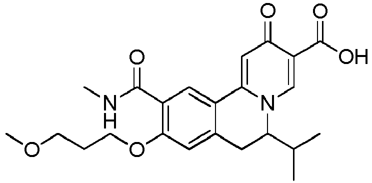

### Step 1: 10-benzyl 3-ethyl 6-isopropyl-9-(3-methoxypropoxy)-2-oxo-2,6,7,11b-tetrahydro -1H-pyrido[2,1-a]isoquinoline-3,10-dicarboxylate

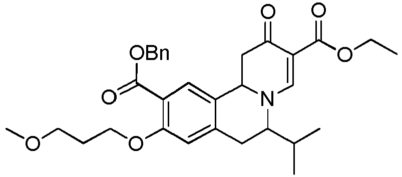

The title compound was prepared according to the synthetic method of step 8 in example 1 by using benzyl 3-isopropyl-6-(3-methoxypropoxy)-3,4-dihydroisoquinoline -7-carboxylate (1.9 g, 4.8 mmol), ethyl 2-(ethoxymethylene)-3-oxobutanoate (1.34 g, 7.2 mmol) and anhydrous ethanol (15 mL) as raw materials to give light yellow oil (1.8 g, 70%).
MS (ESI, pos.ion) *m*/*z:* 536.2 [M+H]⁺.

### Step 2: 10-benzyl 3-ethyl 6-isopropyl-9-(3-methoxypropoxy)-2-oxo-6,7-dihydro-2H -pyrido[2,1-a]isoquinoline-3,10-dicarboxylate

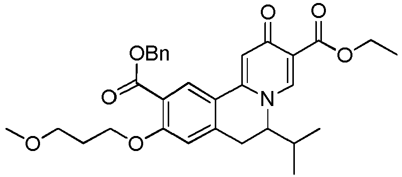

The title compound was prepared according to the synthetic method of step 9 in example 1 by using 10-benzyl 3-ethyl 6-isopropyl-9-(3-methoxypropoxy)-2-oxo-2,6,7,11b -tetrahydro-1*H*-pyrido[2,1-*a*]isoquinoline-3,10-dicarboxylate (1.6 g, 3 mmol), chloranil (0.74 g, 3mmol) and DME (20 mL) as raw materials to give a brown solid (1.31 g, 82%).
MS (ESI, pos.ion) *m*/*z:* 534.1 [M+H]⁺.

### Step 3: 3-(ethoxycarbonyl)-6-isopropyl-9-(3-methoxypropoxy)-2-oxo-6,7-dihydro-2H -pyrido[2,1-a]isoquinoline-10-carboxylic acid

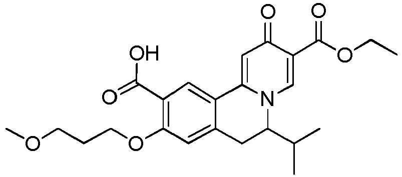

The title compound was prepared according to the synthetic method of step 10 in example 1 by using 10-benzyl 3-ethyl 6-isopropyl-9-(3-methoxypropoxy)-2-oxo-6,7-dihydro -2H-pyrido[2,1-a]isoquinoline-3,10-dicarboxylate (1.3 g, 2.44 mmol) and Pd/C (0.2 g, 10%) as raw materials to give a gray solid (0.97 g, 90%).
MS (ESI, pos.ion) *m*/*z:* 444.1 [M+H]⁺.

### Step 4: ethyl 6-isopropyl-9-(3-methoxypropoxy)-10-(methylcarbamoyl)-2-oxo-6,7-dihydro -2H-pyrido[2,1-a]isoquinoline-3-carboxylate

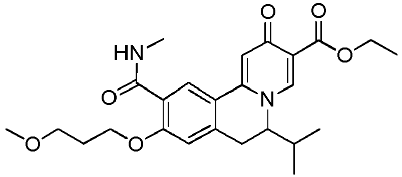

To a 100 mL single-neck flask were added 3-(ethoxycarbonyl)-6-isopropyl-9-(3-methoxypropoxy)-2-oxo-6,7-dihydro-2*H*-pyrido[2,1-*a*]isoq uinoline-10-carboxylic acid (0.55 g, 1.2 mmol), DMF (12 mL), DIPEA (0.41 mL, 2.5 mmol), HATU (0.57 g, 1.5 mmol) and methylamine hydrochloride (0.13 g, 1.9 mmol). The reaction mixture was stirred at 25 °C for 12 hours, then to the reaction mixture was added EtOAc (150 mL) and water (100 mL). The resulting mixture was stirred, and concentrated hydrochloric acid was then added to adjust pH to 6-7, then the mixture was stood for partition. The separated organic layer was washed with saturated brine, dried over anhydrous sodium sulfate, and filtered. The filtrate was concentrated *in vacuo,* and the residue was purified by silica gel column chromatography (DCM/CH₃OH(V/V)=50/1) to give the title compound as a purply solid (0.25 g, 44%).
MS (ESI, pos.ion) *m*/*z:* 457.3 [M+H] ⁺.

### Step 5: 6-isopropyl-9-(3-methoxypropoxy)-10-(methylcarbamoyl)-2-oxo-6,7-dihydro-2H -pyrido[2,1-a]isoquinoline-3-carboxylic acid

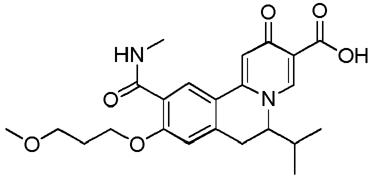

To a 100 mL single-neck flask were added ethyl 6-isopropyl-9-(3-methoxypropoxy)-10-(methylcarbamoyl)-2-oxo-6,7-dihydro-2*H*-pyrido[2,1-*a*]i soquinoline-3-carboxylate (0.24 g, 0.53 mmol), ethanol (6 mL) and THF (6 mL). The reaction mixture was stirred to disolve the solid, and then a solution of NaOH (0.11 g, 2.8 mmol) in H₂O (4 mL) was added. The reaction mixture was continued to stir for 2 hours at room temperature. After the reaction was completed, the reaction mixture was concentrated *in vacuo,* and the residue was diluted with water (30 mL) and EtOAc (50 mL), then the resulting mixture was stirred, and concentrated hydrochloric acid was then added to adjust pH to 5-7. Then the mixture was stood for partition. The separated organic layer was washed with saturated brine, dried over anhydrous sodium sulfate, and filtered. The filtrate was concentrated *in vacuo.* To the residue was added IPA (2 mL), then the mixture was treated by ultraphonic, and then filtered. The filter cake was washed with IPA (1 mL) to give the title compound as a white solid (100 mg, 44%).
MS (ESI, pos.ion) *m*/*z:* 429.3 [M+H] ⁺;
¹H NMR (400 MHz, CDCl₃) δ 16.12 (s, 1H), 8.61 (s, 1H), 8.46 (s, 1H), 8.15 (br, 1H), 7.17 (s, 1H), 6.90 (s, 1H), 4.33 (t, *J=* 5.8 Hz, 2H), 3.98 (dd, *J=* 9.1, 4.8 Hz, 1H), 3.64 (t, *J=* 5.4 Hz, 2H), 3.47 (dd, *J=* 16.4, 4.8 Hz, 1H), 3.40 (s, 3H), 3.20 (d, 1H), 3.02 (d, 3H), 2.24 - 2.18 (m, 2H), 1.77 - 1.70 (m, 1H), 0.95 (d, *J* = 6.6 Hz, 3H), 0.81 (d, *J* = 6.7 Hz, 3H).

### Example 6: 10-acetyl-6-isopropyl-9-(3-methoxypropoxy)-2-oxo-6,7-dihydro-2H -pyrido[2,1-a]isoquinoline-3-carboxylic acid

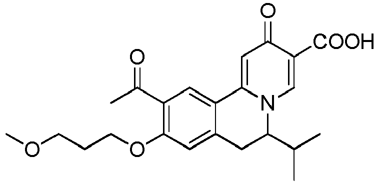

### Step 1: 2-(4-bromo-2-(3-methoxypropoxy)phenyl)-2-methyl-1,3-dioxolane

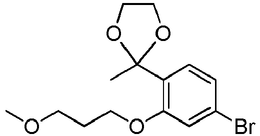

To a dried reaction flask were added 1-(4-bromo-2-(3-methoxypropoxy) phenyl)ethanone (5.0 g, 17 mmol), ethanediol (8.4 mL), *p*-toluenesulfonic acid (0.5 g), trimethyl orthoformate (12 mL) and toluene (25 mL) in turn. The reaction mixture was heated to 60 °C and stirred for 8 hours. After the reaction was completed, the reaction mixture was cooled to 25 °C, then saturated aqueous sodium bicarbonate solution (50 mL) was added. The reaction mixture was stood for partition, and the aqueous layer was extracted with EtOAc (50 mL). The combined organic layer was dried over anhydrous sodium sulfate and filtered. The filtrate was concentrated *in vacuo* to give the title compound as light yellow oil (3.93 g, 70%).
MS (ESI, pos.ion) *m*/*z:* 331.0.[M+H]⁺.

### Step 2: 1-(3-(3-methoxypropoxy)-4-(2-methyl-1,3-dioxolan-2-yl)phenyl)-3-methylbutan-2-one

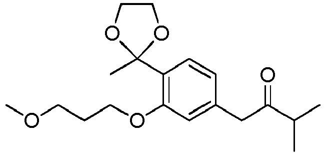

To a dried flask were added 2-(4-bromo-2-(3-methoxypropoxy) phenyl)-2-methyl-1,3-dioxolane (6.0 g, 18 mmol), 3-methylbutan-2-one (3.9 g, 45 mmol), THF (50 mL), 4,5-bis(diphenylphosphino)-9,9-dimethylxanthene (0.39 g, 0.67 mmol), sodium tert-butoxide (5.0 g, 52 mmol) and Pd(dba)₂ (0.35 g, 0.61 mmol) in turn. The reaction mixture was stirred at rt for 1 hour under nitrogen protection, then heated to 60 °C and stirred for 3 hours. After the reaction was completed, the reaction mixture was cooled to rt and concentrated *in vacuo.* To the residue were added EtOAc (50 mL) and aqueous NaOH solution (50 mL,1 M). The mixture was stood for partition, and the aqueous layer was extracted with EtOAc (50 mL×3). The combined organic layers were dried over anydrous sodium sulfate and filtered. The filtrate was concentrated *in vacuo,* and the residue was purified by silica gel column chromatography (PE/EtOAc (V/V)=2/1) to give the title compound as light brown oil (5.0 g, 82%).
MS (ESI, pos.ion) *m*/*z:* 337.5[M+H]⁺.

### Step 3: 1-(3-(3-methoxypropoxy)-4-(2-methyl-1,3-dioxolan-2-yl)phenyl)-3-methylbutan -2-amine

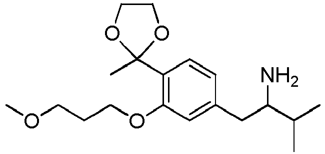

The title compound was prepared according to the synthetic method of step 5 in example 1 by using 1-(3-(3-methoxypropoxy)-4-(2-methyl-1,3-dioxolan-2-yl) phenyl)-3-methylbutan-2-one (1.7 g, 5.1 mmol), NH₄OAc (4.5 g, 58 mmol), MeOH (10 mL) and NaBH₃CN (0.91 g, 14 mmol) as raw materials to give light yellow oil (1.7 g, 100%).
MS: (ESI, pos.ion) *m*/*z:* 338.6. [M+H]⁺.

### Step 4: N-(1-(4-acetyl-3-(3-methoxypropoxy)phenyl)-3-methylbutan-2-yl)formamide

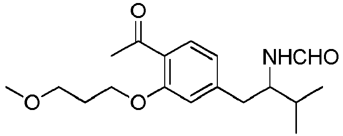

The title compound was prepared according to the synthetic method of step 6 in example 1 by using 1-(3-(3-methoxypropoxy)-4-(2-methyl-1,3-dioxolan-2-yl)phenyl) -3-methylbutan-2-amine (1.7 g, 5.0 mmol), dioxane (8 mL) and formic acid (3.7 g, 80 mmol) as raw materials to give light brown oil (1.03 g, 64%).
MS (ESI, pos.ion) *m*/*z:* 322.2. [M+H]⁺.

### Step 5: 1-(3-isopropyl-6-(3-methoxypropoxy)-3,4-dihydroisoquinolin-7-yl)ethanone

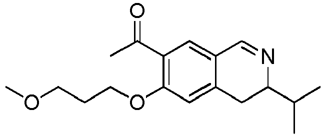

The title compound was prepared according to the synthetic method of step 7 in example 1 by using N-(1-(4-acetyl-3-(3-methoxypropoxy)phenyl)-3-methylbutan-2-yl) formamide (0.21 g, 0.65 mmol), DCM (6 mL) and POCl₃ (0.12 mL, 1.3 mmol) as raw materials to give light brown oil (0.18 g, 91%).
MS (ESI, pos.ion) *m*/*z:* 304.5[M+H]⁺.

### Step 6: ethyl 10-acetyl-6-isopropyl-9-(3-methoxypropoxy)-2-oxo-2,6,7,11b-tetrahydro -1H-pyrido[2,1-a]isoquinoline-3-carboxylate

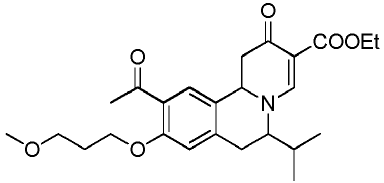

The title compound was prepared according to the synthetic method of step 8 in example 1 by using 1-(3-isopropyl-6-(3-methoxypropoxy)-3,4-dihydroisoquinolin-7-yl)ethanone (0.20 g, 0.66 mmol), ethyl 2-(ethoxymethylene)-3-oxobutanoate (0.2 g, 1 mmol) and EtOH (5 mL) as raw materials to give a gray solid (0.18 g, 62%).
MS (ESI, pos.ion) *m*/*z:* 444.2[M+H]⁺.

### Step 7: ethyl 10-acetyl-6-isopropyl-9-(3-methoxypropoxy)-2-oxo-6,7-dihydro-2H -pyrido[2,1-a]isoquinoline-3-carboxylate

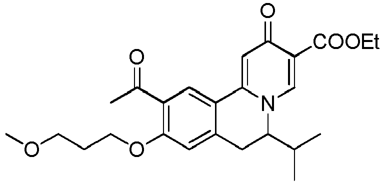

The title compound was prepared according to the synthetic method of step 9 in example 1 by using ethyl 10-acetyl-6-isopropyl-9-(3-methoxypropoxy)-2-oxo-2,6,7,11b -tetrahydro-1*H*-pyrido[2,1-*a*]isoquinoline-3-carboxylate (0.37 g, 0.834 mmol), THF (6 mL) and chloranil (205 mg, 0.834 mmol) as raw materials to give an offwhite solid (0.32 g, 87%).
MS (ESI, pos.ion) *m*/*z:* 442.1. [M+H]⁺.

### Step 8: 10-acetyl-6-isopropyl-9-(3-methoxypropoxy)-2-oxo-6,7-dihydro-2H-pyrido[2,1-a] isoquinoline-3-carboxylic acid

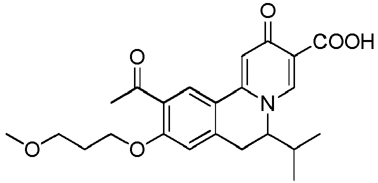

The title compound was prepared according to the synthetic method of step 5 in example 5 by using ethyl 10-acetyl-6-isopropyl-9-(3-methoxypropoxy)-2-oxo-6,7-dihydro -2*H*-pyrido[2,1-*a*]isoquinoline-3-carboxylate (0.15 g, 0.34 mmol), THF (2 mL), EtOH (1 mL), H₂O (0.5 mL) and LiOH.H₂O (57 mg, 1.36 mmol) as raw materials to give an offwhite solid (32 mg, 23%).
MS (ESI, pos.ion) *m*/*z:* 414.4 [M+H]⁺;
¹H NMR (400 MHz, CDCl₃) δ 15.98 (br, 1H), 8.47 (s, 1H), 8.20 (s, 1H), 7.17 (s, 1H), 6.92 (s, 1H), 4.34 - 4.25 (m, 2H), 3.97 - 3.89 (m, 1H), 3.66 - 3.58 (m, 2H), 3.45 - 3.40 (m, 4H), 3.21 (d, *J* = 16.52 Hz, 1H), 2.67 (s, 3H), 2.24 - 2.15 (m, 2H), 1.81 - 1.71 (m, 1H), 0.97 (d, *J* = 6.28 Hz, 3H), 0.84 (d, *J=* 6.30 Hz, 3H).

### Example 8: 10-(2-aminothiazol-4-yl)-6-isopropyl-9-(3-methoxypropoxy)-2-oxo-6,7-dihydro -2H-pyrido[2,1-a]isoquinoline-3-carboxylic acid

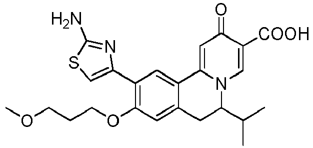

### Step 1: 1-(4-bromo-2-(3-methoxypropoxy)phenyl)ethanone

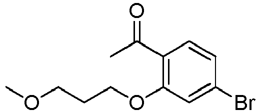

To a 100 mL single-neck flask were added 1-(4-bromo-2-hydroxyphenyl)ethanone (5 g, 23.251 mmol), DMF (20 mL), 1-bromo-3-methoxypropane (11.06 g, 72.28 mmol) and K₂CO₃ (4.813 g, 34.88 mmol). The mixture was stirred at rt overnight. The reaction monitored by TLC was completed, then water (100 mL) was added into reaction mixture. The resulting mixture was extracted with ethyl acetate (100 mL × 4), and the combined organic layer was washed with saturated brine (100 mL × 2). The organic layer was concentrated *in vacuo,* and the residue was purified by silica gel column chromatography (PE/EA (V/V) = 1/1) to give the title compound as colorless oil (6.6 g, 99 %).
MS (ESI, Pos.ion) *m*/*z:* 287.4 [M+H]⁺.

### Step 2: 2-bromo-1-(4-bromo-2-(3-methoxypropoxy)phenyl)ethanone

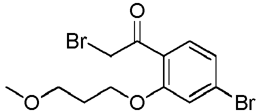

To a 50 mL single-neck flask were added 1-(4-bromo-2-(3-methoxypropoxy)phenyl)ethanone (6 g, 20.90 mmol), then CHCl₃ (30 mL), ethyl acetate (30 mL) and cupric bromide (9.33 g, 41.79 mmol) were added. The mixture was heated to 85 °C under nitrogen protection and stirred for 3 hours. The reaction monitored by TLC was completed, and the reaction mixture was filtered to remove the solid. The filtrate was concentrated *in vacuo* to give the title compound as a white solid (7.64 g, 99 %), which was used in the next step without further purification.
MS (ESI, pos.ion) *m*/*z:* 366.9 [M+H]⁺.

### Step 3: 4-(4-bromo-2-(3-methoxypropoxy)phenyl)thiazol-2-amine

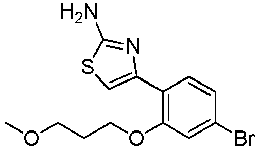

2-Bromo-1-(4-bromo-2-(3-methoxypropoxy)phenyl)ethanone (7.64 g, 20.9 mmol) and thiourea (1.75 g, 23.0 mmol) were added into a 100 mL single-neck flask, then EtOH (50 mL) was added, and the mixture was stirred overnight at room temperature. The reaction monitored by TLC was completed. The reaction mixture was diluted with ethyl acetate (200 mL) and saturated aqueous sodium bicarbonate solution (100 mL), and the resulting mixture was extracted with EtOAc (200 mL × 4). The combined organic layers were washed with saturated brine (100 mL), dried over anhydrous sodium sulfate, and filtered. The filtrate was concentrated *in vacuo,* and the residue was purified by silica gel column chromatography (PE/EA (V/V) = 1/1) to give the title compound as a light yellow solid (5.8 g, 17 mmol, 81%).
MS (ESI, pos.ion) *m*/*z:* 343.0 [M+H]⁺.

### Step 4: tert-butyl (4-(4-bromo-2-(3-methoxypropoxy)phenyl)thiazol-2-yl)carbamate

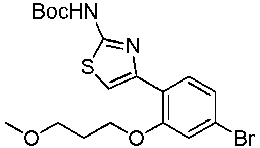

4-(4-Bromo-2-(3-methoxypropoxy)phenyl)thiazol-2-amine (5.1 g, 15 mmol) was added into a 100 mL single-neck flask, then THF (30 mL), pyridine (20 mL), BoczO (4.9 g, 22 mmol) and DMAP (50 mg, 0.41 mmol) were added. The reaction mixture was stirred at 50 °C for 48 hours. After the reaction was completed, the reaction mixture was cooled to room temperature, and concentrated *in vacuo.* The residue was diluted with ethyl acetate (50 mL) and water (50 mL) in turn, then the mixture was stood for partition. The separated organic layer was washed with saturated brine (50 mL), dried over anhydrous sodium sulfate, and filtered. The filtrate was concentrated *in vacuo,* and the residue was purified by silica gel column chromatography (PE/EA (V/V) = 8/1) to give the title compound as a white solid (5.23 g, 11.8 mmol, 79%).
MS (ESI, pos.ion) *m*/*z:* 443.0 [M+H]⁺.

### Step 5: tert-butyl (4-(2-(3-methoxypropoxy)-4-(3-methyl-2-oxobutyl)phenyl)thiazol -2-yl)carbamate

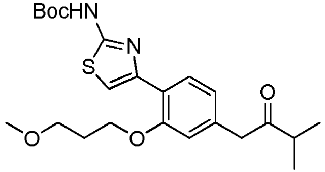

tert-Butyl (4-(4-bromo-2-(3-methoxypropoxy)phenyl)thiazol-2-yl)carbamate (5.2 g, 12 mmol) was added into a 50 mL two-neck flask, then THF (52 mL), 3-methylbutan-2-one (3.0 g, 35 mmol), XantPhos (0.31 g, 0.54 mmol), sodium tert-butoxide (3.9 g, 41 mmol) and Pd(dba)₂ (0.27 g, 0.47 mmol) were added. The reaction mixture was stirred at room temperature for 1 hour under nitrogen protection, then heated to 55 °C and stirred for 2 hours, then the reaction monitored by TLC was completed. The mixture was cooled to rt, and poured into water (100 mL). The mixture was extracted with ethyl acetate (100 mL×4). The combined organic layers were dried over anydrous sodium sulfate and filtered. The filtrate was concentrated *in vacuo,* and the residue was purified by silica gel column chromatography (PE/EA (V/V) = 1/1) to give the title compound as light yellow oil (4.39 g, 9.79 mmol, 83%).
MS (ESI, pos.ion) *m*/*z:* 449.1 [M+H]⁺.

### Step 6: tert-butyl (4-(4-(2-amino-3-methylbutyl)-2-(3-methoxypropoxy)phenyl)thiazol -2-yl)carbamate

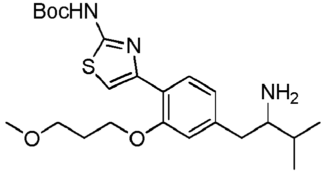

*tert*-Butyl (4-(2-(3-methoxypropoxy)-4-(3-methyl-2-oxobutyl)phenyl)thiazol-2-yl) carbamate (1.45 g, 3.23 mmol) was added into a 20 mL single-neck flask, then NH₄OAc (2.99 g, 38.8 mmol) and MeOH (15 mL) were added. The reaction mixture was stirred at rt for 1 hours, then cooled to 0 °C and NaBH₃CN (0.609 g, 9.69 mmol) was added. The mixture was continued to stir overnight at 55 °C. After the reaction was completed, the reaction mixture was concentrated *in vacuo,* then ethyl acetate (50 mL) and aqueous NaOH solution (1 M, 30 mL) was added in turn to dilute the residue. The mixture was stood for partition, and the aqueous layer were extracted with ethyl acetate (30 mL × 3). The combined organic layer was concentrated *in vacuo* to give the title compound as light yellow oil (1.4 g, 3.1 mmol, 96%).
MS (ESI, pos.ion) *m*/*z:* 450.6 [M+1]⁺.

### Step 7: tert-butyl (4-(4-(2-formamido-3-methylbutyl)-2-(3-methoxypropoxy)phenyl)thiazol -2-yl)carbamate

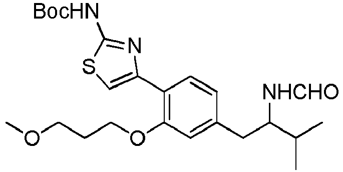

*tert*-Butyl (4-(4-(2-amino-3-methylbutyl)-2-(3-methoxypropoxy)phenyl)thiazol-2-yl) carbamate (0.724 g, 1.61 mmol) was added into a 25 mL single-neck flask, then methyl acetate (15 mL) was added. The mixture was refluxed overnight under nitrogen protection. After the reaction was completed, the reaction mixture was concentrated *in vacuo* to give the title compound as a light yellow solid (0.75 g, 1.6 mmol, 98%), which was used in the next step without further purificacion.
MS (ESI, pos.ion) *m*/*z:* 478.1 [M+H]⁺.

### Step 8: tert-butyl (4-(3-isopropyl-6-(3-methoxypropoxy)-3,4-dihydroisoquinolin-7-yl)thiazol -2-yl)carbamate

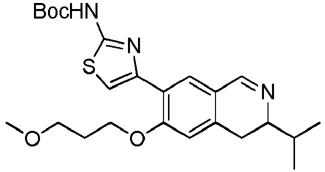

*tert*-Butyl (4-(4-(2-formamido-3-methylbutyl)-2-(3-methoxypropoxy)phenyl)thiazol -2-yl)carbamate (2.31 g, 4.84 mmol) was added in a 100 mL sigle-neck flask, then DCM (23 mL) was added. The mixture was cooled to 0 °C, and POCl₃ (0.902 mL, 9.68 mmol) was added. The resulting mixture was degassed and filled with nitrogen for three times, then heated to relux and stirred for 3 hours. After the reaction was completed, the reaction mixture was cooled to rt, and ammonium hydroxide (15 mL) was added. The mixture was extracted with ethyl acetate (30 mL × 3). The combined organic layers were dried over anydrous sodium sulfate, and filtered. The filtrate was concentrated *in vacuo* to give the title compound as light yellow oil (2.0 g, 4.4 mmol, 90%), which was used directly in the next step.
MS (ESI, pos.ion) *m*/*z:* 460.1 [M+1]⁺.

### Step 9: ethyl 10-(2-((tert-butoxycarbonyl)amino)thiazol-4-yl)-6-isopropyl-9-(3-methoxypropoxy -2-oxo-2,6,7,11b-tetrahydro-1H-pyrido[2,1-a]isoquinoline-3-carboxylate

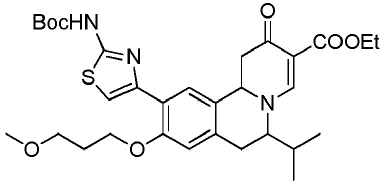

tert-Butyl (4-(3-isopropyl-6-(3-methoxypropoxy)-3,4-dihydroisoquinolin-7-yl)thiazol -2-yl) carbamate (0.5 g, 1 mmol) was added into a 50 mL single-neck flask, then ethyl 2-(ethoxymethylene)-3-oxobutanoate (0.4 g, 2 mmol) and EtOH (5 mL) were added. The mixture was refluxed overnight under nitrogen protection. After the reaction was completed, the mixture was concentrated *in vacuo,* and the residue was purified by silica gel column chromatography (EA) to give the title compound as a brown solid (0.12 g, 0.20 mmol, 20%).
MS (ESI, pos.ion) *m*/*z:* 600.1 [M+1]⁺.

### Step 10: ethyl 10-(2-((tert-butoxycarbonyl)amino)thiazol-4-yl)-6-isopropyl-9-(3 -methoxypropoxy)-2-oxo-6,7-dihydro-2H-pyrido[2,1-a]isoquinoline-3-carboxylate

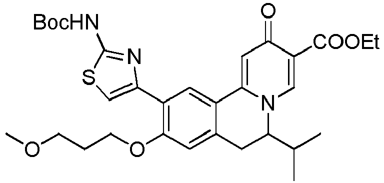

Ethyl 10-(2-((tert-butoxycarbonyl)amino)thiazol-4-yl)-6-isopropyl-9-(3-methoxy propoxy)-2-oxo-2,6,7,11b-tetrahydro-1*H*-pyrido[2,1-*a*]isoquinoline-3-carboxylate (0.290 g, 0.484 mmol) was added into a 25 mL single-neck flask, then DME (10 mL) and chloranil (0.119 g, 0.484 mmol) were added. The mixture was heated to 90 °C and stirred for 1 hour. The reaction mixture was cooled to rt, and concentrated *in vacuo.* The residue was purified by silica gel column chromatography (DCM/MeOH (V/V) = 10/1) to give the title compound as a brown solid (0.20 g, 0.33 mmol, 69%).
MS (ESI, pos.ion) *m*/*z:* 598.2 [M+1]⁺.

### Step 11: ethyl 10-(2-aminothiazol-4-yl)-6-isopropyl-9-(3-methoxypropoxy)-2-oxo-6,7 -dihydro-2H-pyrido[2,1-a]isoquinoline-3-carboxylate

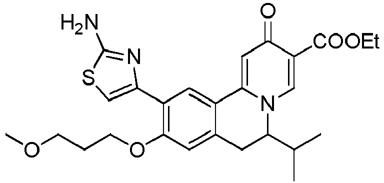

Ethyl 10-(2-((tert-butoxycarbonyl)amino)thiazol-4-yl)-6-isopropyl-9-(3-methoxy propoxy)-2-oxo-6,7-dihydro-2H-pyrido[2,1-a]isoquinoline-3-carboxylate (0.30 g, 0.50 mmol) was added into a 25 mL single-neck flask, then a solution of hydrogen chloride in 1,4-dioxane (5 mL) was added. The mixture was stirred at rt for 3 hours under nitrogen protection. After the reaction was completed, the rection mixture was concentrated *in vacuo,* and the residue was dissolved in DCM (20 mL). The mixture was washed with saturated aqueous sodium bicarbonate (10 mL × 2) and saturated brine (10 mL), dried over anhydrous sodium sulfate, and filtered. The filtrate was concentrated *in vacuo,* and the residue was purified by silica gel column chromatography column chromatography (DCM/MeOH (V/V) = 10/1) to give the title compound as a brown solid (0.21 g, 0.42 mmol, 84%).
MS (ESI, pos.ion) *m*/*z:* 498.3 [M+1]⁺.

### Step 12: 10-(2-aminothiazol-4-yl)-6-isopropyl-9-(3-methoxypropoxy)-2-oxo-6,7-dihydro -2H-pyrido[2,1-a]isoquinoline-3-carboxylic acid

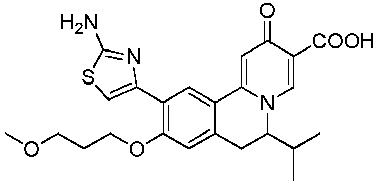

Ethyl 10-(2-aminothiazol-4-yl)-6-isopropyl-9-(3-methoxypropoxy)-2-oxo-6,7-dihydro-2H-pyrido[2,1-a]isoquinoline-3-carboxylate (0.21 g, 0.42 mmol) was added into a 25 mL single-neck flask, then THF (2 mL), EtOH (1 mL), H₂O (0.5 mL) and LiOH.HzO (71 mg, 1.69 mmol) was added. The reaction mixture was stirred at rt for 5 hours, then concentrated *in vacuo,* and the residue was adjusted with hydrochloric acid (1 M) to pH = 3, then the mixture was extracted with DCM (30 mL × 2). The comibined oragnic layers were concentrated *in vacuo,* and the residue was recrystallized from methanol (2 mL) to give the title compound as an offwhite solid (50 mg, 0.10 mmol, 25%).
MS (ESI, pos.ion) *m*/*z:* 470.3 [M+1]⁺;
¹H NMR (400 MHz, CDCl₃) δ 16.197 (br, 1H), 8.665 (s, 1H), 8.458 (s, 1H), 7.292 (s, 1H), 6.866 (s, 1H), 4.996 (s, 2H). 4.345 - 4.257 (m, 2H), 3.932 - 3.873 (m, 1H), 3.63(t, *J=* 6 Hz, 2H), 3.460 - 3.391 (m, 4H), 3.189 - 3.112 (m, 1H), 2.255 - 2.194 (m, 2H), 1.879 - 1.786 (m, 1H), 0.963 (d, *J* =6.4Hz, 3H), 0.828 (d, *J=* 6.8Hz, 3H).

### Example 9: 6-isopropyl-9-(3-methoxypropoxy)-2-oxo-10-(thiazol-2-yl)-6,7-dihydro-2H -pyrido[2,1-a]isoquinoline-3-carboxylic acid

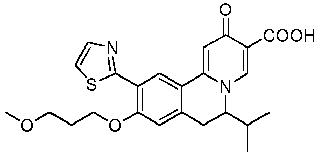

### Step 1: 4-bromo-2-(3-methoxypropoxy)-1-nitrobenzene

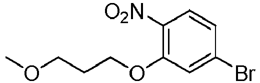

5-Bromo-2-nitrophenol (5.0 g, 23 mmol) was added into a 100 mL single-neck flask, then DMF (50 mL), K₂CO₃ (7.9 g, 57 mmol) and 1-bromo-3-methoxypropane (5.3 g, 35 mmol) were added in turn. The reaction mixture was stirred at 50 °C overnight. After the reaction was completed, the reaction mixture was cooled to room temperature, and diluted with water (50 mL), then the mixture was extracted with EA (40 mL × 4). The combined organic layers were concentrated *in vacuo,* and the residue was purified by silica gel column chromatography (PE/EA (V/V) = 4/1) to give the title compound as brown oil (6.7 g, 23 mmol, 99 %).
MS (ESI, pos.ion) *m*/*z:* 290.0 [M+H]⁺.

### Step 2: 4-bromo-2-(3-methoxypropoxy)aniline

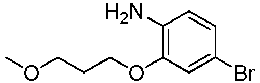

4-Bromo-2-(3-methoxypropoxy)-1-nitrobenzene (2.4 g, 8.3 mmol), H₂O (6 mL) and MeOH (12 mL) were added into a 100 mL two-neck flask, then ferrous powder (3.9 g, 70 mmol) and NH₄Cl (3.9 g, 74 mmol) were added in turn. The reaction mixture was heated to 80 °C and stirred for 40 minutes under nitrogen protection. After the reaction was completed, the reaction mixture was cooled to rt and filtered by suction. The filter cake was washed with EA/MeOH(V/V = 1/1), and the combined filtrates were concentrated. The residue was dissolved in ethyl acetate (50 mL), and the mixture was washed with saturated brine (10 mL). The organic layer was dried over anydrous sodium sulfate, filtered, and the filtrate was concentrated *in vacuo* to give the title compound as a gray solid (1.7 g, 6.5 mmol, 78%).
MS (ESI, pos.ion) *m*/*z:* 260.0 [M+H]⁺.

### Step 3: N,N-dibenzyl-4-bromo-2-(3-methoxypropoxy)aniline

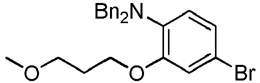

4-Bromo-2-(3-methoxypropoxy)aniline (1.2 g, 4.6 mmol) was added into a 100 mL single-neck flask, then DMF (10 mL), Na₂CO₃ (1.9 g, 14 mmol), benzyl bromide (1.7 g, 10.1 mmol) and THF (10 mL) were added in turn. The reaction mixture was stirred for 10 min, then heated to 80 °C and stirred overnight. After the reaction was completed, to the reaction mixture was added water (50 mL), and the resulting mixture was extracted with ethyl acetate (50 mL × 3). The combined organic layers were dried over anhydrous sodium sulfate, filtered and the filtrate was concentrated *in vacuo.* The residue was purified by silica gel column chromatography (PE/EA (V/V) = 10/1) to give the title compound as colorless oil (1.98 g, 4.50 mmol, 97%).
MS (ESI, pos.ion) *m*/*z:* 440.2 [M+H]⁺.

### Step 4: 1-(4-(dibenzylamino)-3-(3-methoxypropoxy)phenyl)-3-methylbutan-2-one

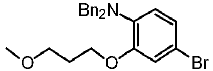

N,N Dibenzyl-4-bromo-2-(3-methoxypropoxy)aniline (1.00 g, 2.27 mmol) was added into a 50 mL single-neck flask, then 3-methylbutan-2-one (0.59 g, 6.9 mmol), THF (10 mL), XantPhos (59 mg, 0.10 mmol), sodium tert-butoxide (0.75 g, 7.8 mmol) and Pd(dba)₂ (46 mg, 0.08 mmol) were added. The reaction mixture was degassed and filled with nitrogen for three times, and then stirred at rt for 30 min, then heated to 60 °C and stirred for 3 hours. After the reaction was completed, the reaction mixture was cooled to rt, and ethyl acetate (50 mL) was added into the mixture, then aqueous NaOH solution (1 M, 50 mL) was added. The resulting mixture was partitioned, and the aqueous layer was extracted with ethyl acetate (50 mL × 3). The combined organic layers were dried over anhydrous sodium sulfate, filtered and the filtrate was concentrated. The residue was purified by silica gel column chromatography (PE/EA (V/V) = 10/1) to give the title compound as colorless oil (0.70 g, 1.6 mmol, 69%).
MS (ESI, pos.ion) *m*/*z:* 446.4 [M+H]⁺.

### Step 5: 4-(2-amino-3-methylbutyl)-A,A-dibenzyl-2-(3-methoxypropoxy)aniline

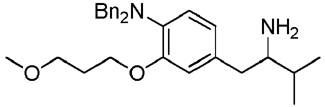

1-(4-(Dibenzylamino)-3-(3-methoxypropoxy)phenyl)-3-methylbutan-2-one (0.70 g, 1.6 mmol) was added into a 50 mL single-neck flask, then NH₄OAc (1.5 g, 19 mmol) and MeOH (10 mL) were added. The reaction mixture was stirred at rt for 30 minites, then cooled to 0 °C and NaBH₃CN (0.30 g, 4.8 mmol) was added. The mixture was gradually heated to 50 °C and stirred overnight. After the reaction, the reaction mixture was concentrated *in vacuo,* and the residue was diluted with water (30 mL), then the mixture was extracted with ethyl acetate (30 mL × 3). The combined organic layers were dried over anydrous sodium sulfate, and filtered. The filtrate was concentrated *in vacuo* to give the title compound as light yellow oil (0.7 g, 2 mmol, 100%), which was directly used in the next step.
MS (ESI, pos.ion) *m*/*z:* 447.4 [M+H]⁺.

### Step 6: N-(1-(4-(dibenzylamino)-3-(3-methoxypropoxy)phenyl)-3-methylbutan-2-yl)formamide

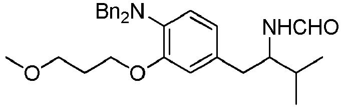

To a 50 mL single-flask were added 4-(2-amino-3-methylbutyl)-N,N-dibenzyl-2-(3-methoxypropoxy)aniline (0.58 g, 1.3 mmol), then 1,4-dioxane (5 mL) and formic acid (0.96 g, 21 mmol) were added. The reaction mixture was heated to 110 °C and stirred for 24 hours under nitrogen protection. After the reaction was completed, the reaction mixture was concentrated *in vacuo,* and the residue was dissolved in EA (20 mL). Then the mixture was washed with saturated brine (20 mL), and the aqueous layer was extracted with EA (20 mL). The organic layers were combined, dried over anydrous sodium sulfate and filtered. The filtrated was concentrated *in vacuo* to give the titile compound as a white solid (0.62 g, 1.3 mmol, 100%).
MS (ESI, pos.ion) *m*/*z:* 475.2 [M+H]⁺.

### Step 7: N-(1-(4-amino-3-(3-methoxypropoxy)phenyl)-3-methylbutan-2-yl)formamide

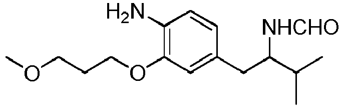

*N-*(1-(4-(Dibenzylamino)-3-(3-methoxypropoxy)phenyl)-3-methylbutan-2-yl)formami de (0.17 g, 0.36 mmol) was added into a 50 mL single-neck flask, then THF (20 mL) and Pd/C (17 mg, 10%) was added in turn. The reaction mixture was heated to 60 °C and stirred overnight under hydrogen atomosphere. After the reaction was completed, the reaction mixture was filtered. The filter cake was washed with EA (30 mL), and the filtrate was concentrated *in vacuo* to give the title compound as a gray solid (0.1 g, 0.3 mmol, 90%), which was directly used in the next step.
MS (ESI, pos.ion) *m*/*z:* 295.2 [M+H]⁺.

### Step 8: N-(1-(4-iodo-3-(3-methoxypropoxy)phenyl)-3-methylbutan-2-yl)formamide

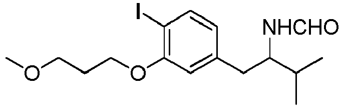

*N-*(1-(4-Amino-3-(3-methoxypropoxy)phenyl)-3-methylbutan-2-yl)formamide (8.00 g, 27.2 mmol) was added into a 100 mL single-neck flask, then H₂O (40 mL) and H₂SO₄ (13.3 g, 136 mmol) was added in turn. The reaction mixture was stirred for 10 min, then cooled to 0 °C, and a solution of NaNO₂ (1.87 g, 27.1 mmol) in H₂O (10 mL) was added. The mixture was stirred at 0 °C for 20 min, then transfered to a dropping funnel. A dried 500 mL single-neck flask was took, then KI (5.87 g, 35.4 mmol), NaHSO₃ (792 mg, 7.6110 mmol) and H₂O (40 mL) were added into the flask. The mixture was heated to 40 °C, and the mixure in the dropping funnel was added dropwise slowly into the flask for about 15 min. After addition, the reaction mixture was heated to 80 °C and stirred for 1 hour. After the reaction was completed, the reaction mixture was cooled to rt, and extracted with ethyl acetate (100 mL × 4). The combined organic layer was concentrated. The residue was purified by silica gel column chromatography (PE/EA (V/V) = 1/1) to give the title compound as red brown oil (4.5 g, 11 mmol, 41%).
MS (ESI, pos.ion) *m*/*z:* 406.1 [M+H]⁺.

### Step 9: 7-iodo-3-isopropyl-6-(3-methoxypropoxy)-3,4-dihydroisoquinoline

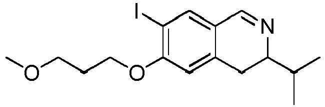

To a 50 mL dried single-neck flask were added N-(1-(4-iodo-3-(3-methoxypropoxy)phenyl)-3-methylbutan-2-yl)formamide (0.34 g, 0.85 mmol) and DCM (6 mL). The mixture was cooled to 0 °C, then POCl₃ (0.159 mL, 1.71 mmol) was added. After addition, the reaction mixture was heated to 50 °C and stirred for 2 hours. After the reaction was completed, the reaction mixture was cooled to rt, and poured into ice-water (20 mL). The mixture was extracted with ethyl acetate (30 mL×3), and the combined organic layers were washed with saturated brine (10 mL), dried over anydrous sodium sulfate, and filtered. The filtrate was concentrated *in vacuo* to give the title compound as brown oil (0.282 g, 0.728 mmol, 85.1%), which was directly used in the next step.
MS (ESI, pos.ion) *m*/*z:* 387.9 [M+H]⁺.

### Step 10: ethyl 10-iodo-6-isopropyl-9-(3-methoxypropoxy)-2-oxo-2,6,7,11b-tetrahydro-1H -pyrido[2,1-a]isoquinoline-3-carboxylate

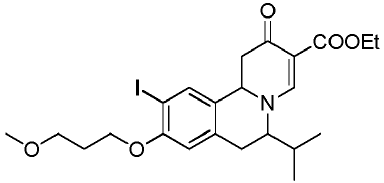

7-Iodo-3-isopropyl-6-(3-methoxypropoxy)-3,4-dihydroisoquinoline (0.282 g, 0.7281 mmol), ethyl 2-(ethoxymethylene)-3-oxobutanoate (0.393 g, 2.11 mmol) and EtOH (10 mL) were added into a 100 mL single-neck flask. The mixture was stirred at 90 °C overnight under nitrogen protection. After the reaction was completed, the reaction mixture was cooled to rt and concentrated *in vacuo.* The residue was purified by silica gel column chromatography (EA) to give the title compound as brown oil (0.195 g, 0.370 mmol, 50.8%).
MS (ESI, pos.ion) *m*/*z:* 528.0 [M+H]⁺.

### Step 11: ethyl 10-iodo-6-isopropyl-9-(3-methoxypropoxy)-2-oxo-6,7-dihydro-2H -pyrido[2,1-a]isoquinoline-3-carboxylate

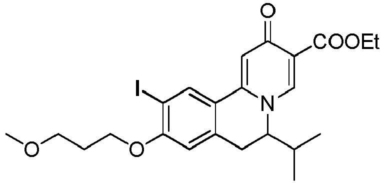

To a 100 mL single-neck flask were added ethyl 10-iodo-6-isopropyl-9-(3-methoxypropoxy)-2-oxo-2,6,7,11b-tetrahydro-1*H*-pyrido[2,1-*a*]isoquin oline-3-carboxylate (0.20 g, 0.38 mmol), DME (10 mL) and chloranil (0.09 g, 0.38 mmol). The reaction mixture was heated to 90 °C and stirred for 1 hour, then concentrated *in vacuo.* The residue was purified by silica gel column chromatography (DCM/MeOH (V/V) = 6 /1) to give the title compound as brown oil (0.199 g, 0.38 mmol, 99.9%).
MS (ESI, pos.ion) *m*/*z:* 526.2 [M+H]⁺.

### Step 12: 6-isopropyl-9-(3-methoxypropoxy)-2-oxo-10-(thiazol-2-yl)-6,7-dihydro-2H -pyrido[2,1-a]isoquinoline-3-carboxylic acid

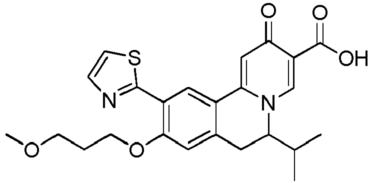

To a 25 mL single-neck flask were added ethyl 10-iodo-6-isopropyl-9-(3-methoxypropoxy)-2-oxo-6,7-dihydro-2*H*-pyrido[2,1-*a*]isoquinoline-3-carboxylate (0.3 g, 0.57 mmol), 2-(tributylstannyl)thiazole (0.427 g, 1.14 mmol), anhydrous dioxane (10 mL) and bis(triphenylphosphine)palladium(II) chloride (99 mg, 0.14 mmol). The reaction mixture was heated to 110 °C and stirred for 24 h under nitrogen protection. After the reaction was completed, the reaction mixture was concentrated *in vacuo.* The residue was purified by silica gel column chromatography (DCM/MeOH (V/V) = 10/1) to give the title compound as a white solid (50 mg, 0.11 mmol, 19.3%).
MS (ESI, pos.ion) *m*/*z:* 455.1 [M+H]⁺;
¹H NMR (400 MHz, CDCl₃) δ 16.106 (br, 1H), 8.915 (s, 1H), 8.470 (s, 1H), 7.971 (d, *J* = 2.4 Hz, 1H), 7.477 (d, *J* = 2.4 Hz, 1H), 7.320 (s, 1H), 6.974 (s, 1H), 4.467 - 4.357 (m, 2H), 3.991 - 3.877 (m, 1H), 3.760 - 3.660 (m, 2H), 3.515 - 3.352 (m, 4H), 3.258 - 3.166 (m, 1H), 2.358 - 2.248 (m, 2H), 2.089 - 1.973 (m, 1H), 0.978 (d, *J=* 6.4 Hz, 3H), 0.847 (d, *J* = 6.0 Hz, 3H).

### Example 10: 6-isopropyl-9-(3-methoxypropoxy)-10-(oxazol-2-yl)-2-oxo-6,7-dihydro-2H -pyrido[2,1-a]isoquinoline-3-carboxylic acid

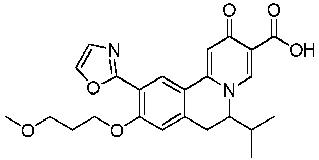

To a 25 mL single-neck flask were added ethyl 10-iodo-6-isopropyl-9-(3-methoxypropoxy)-2-oxo-6,7-dihydro-2H-pyrido[2,1-a]isoquinoline-3-carboxylate (0.330 g, 0.63 mmol), 2-(tributylstannyl)oxazole (0.562 g, 1.57 mmol), anhydrous dioxane (10 mL) and bis(triphenylphosphine)palladium(II) chloride (0.11 g, 0.16 mmol). The reaction mixture was heated to 110 °C and stirred for 24 h under nitrogen protection. After the addition, the reaction mixture was concentrated *in vacuo.* The residue was purified by silica gel column chromatography (DCM/MeOH (V/V) = 10/1) to give the title compound as a white solid (40 mg, 0.11 mmol, 15%).
MS (ESI, pos.ion) *m*/*z:* 438.9 [M+H]⁺;
¹H NMR (400 MHz, CDCl₃) δ 15.988 (br, 1H), 8.476 (s, 1H), 8.407 (s, 1H), 7.802 (d, 1H), 7.325 (d, 1H),7.221 (s, 1H), 6.966 (s, 1H), 4.347 - 4.256 (m, 2H), 3.954 - 3.919 (m, 1H), 3.693 - 3.610 (m, 2H), 3.470 - 3.416 (m, 1H), 3.377 (s, 3H), 3.243 - 3.173 (m, 1H), 2.220 - 2.151 (m, 2H), 2.053 - 2.004 (m, 1H), 0.985 (d, *J* =6.8Hz, 3H), 0.858 (d, *J* =6.8 Hz, 3H).

### Example 11: 10-(furan-2-yl)-6-isopropyl-9-(3-methoxypropoxy)-2-oxo-6,7-dihydro -2H-pyrido[2,1-a]isoquinoline-3-carboxylic acid

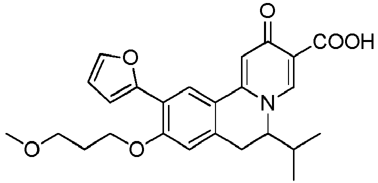

### Step 1: 2-(benzyloxy)-5-bromobenzaldehyde

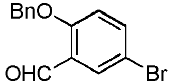

5-Bromo-2-hydroxybenzaldehyde (10.045 g, 49.970 mmol), benzyl bromide (12.82 g, 74.96 mmol), K₂CO₃ (13.79 g, 99.94 mmol) and DMF (50 mL) were added into a 100 mL two-neck flask. The reaction mixture was heated to 60 °C and stirred for 12 h under nitrogen protection. After the reaction was completed, to the reaction mixture was added water (100 mL), and the resulting mixture was extracted with ethyl acetate (50 mL × 4). The combined organic layers were washed with saturated brine (50 mL × 2), dried over anhydrous sodium sulfate, and filtered. The filtrate was concentrated *in vacuo* to give the title compound as a white solid (14.2 g, 48.8 mmol, 97.6%).
MS (ESI, pos.ion) *m*/*z:* 312.9 [M+Na]⁺.

### Step 2: 2-(benzyloxy)-5-bromophenyl formate

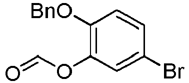

To a 500 mL single-neck flask was added 2-(benzyloxy)-5-bromobenzaldehyde (13.2 g, 45.3 mmol) and ethyl acetate (130 mL), then the mixture was cooled to 0 °C, and m-CPBA (11.7 g, 67.8 mmol) were added into the flask in portions. After addition, the reaction mixture was stirred for 30 min at 0 °C, then heated to rt and stirred overnight. After the reaction was completed, the reaction mixture was diluted with ethyl acetate (50 mL), then washed with aqueous NaOH solution (mass percent: 3%, 100 mL), and the aqueous layer was extracted with ethyl acetate (50 mL × 3). The combined organic layers were washed with saturated brine (50 mL × 3), dried over anhydrous sodium sulfate and filtered. The filtrate was concentrated *in vacuo,* and the residue was purified by silica gel column chromatography (PE/EA (V/V) = 1/1) to give the title compound as brown oil (13.31 g, 43.34 mmol, 95.6%).
MS (ESI, pos.ion) *m*/*z:* 329.0 [M+Na]⁺.

### Step 3: 2-(benzyloxy)-5-bromophenol

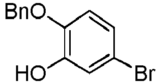

2-(Benzyloxy)-5-bromophenyl formate (13.31 g, 43.34 mmol) was added into a 500 mL single-neck flask, then the solvent in the flask was degassed and filled with nitrogen for three times, then anydrous THF (130 mL) was added. The mixture was cooled to 0 °C, and DIBAH (46 mL, 69 mmol) was added into the mixture. The reaction mixture was stirred at 0 °C for 1 hour, then ethyl acetate (100 mL) was added into the mixture, and water (100 mL) was added to quench the reaction. The mixture was adjusted with concentrated hydrochloric acid to pH = 7, then extracted with ethyl acetate (200 mL × 3). The combined organic layers were concentrated *in vacuo,* and the residue was purified by silica gel column chromatography (PE/EA (V/V) = 1/1) to give the title compound as colorless oil (11.58 g, 41.49 mmol, 95.73%).
MS (ESI, neg.ion) *m*/*z:* 276.9 [M-H]⁻.

### Step 4: 1-(benzyloxy)-4-bromo-2-(3-methoxypropoxy)benzene

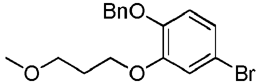

2-(Benzyloxy)-5-bromophenol (11.58 g, 41.49 mmol) was added into a 100 mL single-neck flask, then DMF (60 mL), K₂CO₃ (11.45 g, 82.97 mmol) and 1-bromo-3-methoxypropane (9.523 g, 62.23 mmol) were added in turn. The reaction mixture was stirred at 50 °C overnight, then diluted with water (50 mL), and the mixture was extracted with ethyl acetate (50 mL × 3). The combined organic layers were washed with saturated brine (30 mL × 2), dried over anhydrous sodium sulfate and filtered. The filtrate was concentrated *in vacuo,* and the residue was purified by silica gel column chromatography (PE/EA (V/V) = 10/1) to give the title compound as colorless oil (14.3 g, 40.7 mmol, 98.1%).
MS (ESI, pos.ion) *m*/*z:* 351.1 [M+H]⁺.

### Step 5: 1-(4-(benzyloxy)-3-(3-methoxypropoxy)phenyl)-3-methylbutan-2-one

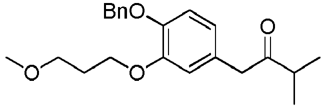

1-(Benzyloxy)-4-bromo-2-(3-methoxypropoxy)benzene (12.50 g, 35.59 mmol), Pd(dba)₂ (0.818 g, 1.42 mmol), sodium tert-butoxide (11.73 g, 122.1 mmol), XanPhos (0.926 g, 1.60 mmol), THF (100 mL) and 3-methylbutan-2-one (9.917 g, 115.1 mmol) were added into a 250 mL two-neck flask. The reaction mixture was stirred at rt for 30 min under nitrogen protection, then heated to 60 °C and stirred overnight. After the reaction was completed, the mixture was diluted with water (100 mL), and the mixture was extracted with EA (200 mL × 3). The combined organic layers were concentrated *in vacuo,* and the residue was purified by silica gel column chromatography (EA) to give the title compound as yellow oil (12.0 g, 33.7 mmol, 94.6%).
MS (ESI, pos.ion) *m*/*z:* 357.2 [M+H]⁺.

### Step 6: 1-(4-(benzyloxy)-3-(3-methoxypropoxy)phenyl)-3-methylbutan-2-amine

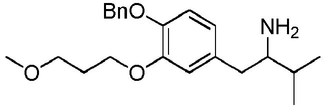

1-(4-(Benzyloxy)-3-(3-methoxypropoxy)phenyl)-3-methylbutan-2-one (13.28 g, 37.25 mmol) was added into a 500 mL single-neck flask, then NH₄OAc (34.46 g, 447.1 mmol) and MeOH (130 mL) were added. The reaction mixture was stirred at rt to dissolve the solid, then cooled to 0 °C and NaBH₃CN (7.023 g, 111.8 mmol) was added. The reaction mixture was stirred at rt for 12 h. After the reaction was completed, the mixture was concentrated *in vacuo,* and to the residue was added saturated aqueous ammonium chloride solution (50 mL), EA (100 mL) and saturated sodium chloride (50 mL). The mixture was partitioned, and the aqueous layer was extracted with ethyl acetate (100 mL × 3). The combined organic layers were concentrated *in vacuo,* and the residue was purified by silica gel column chromatography (DCM/MeOH (V/V) = 10/1) to give the title compound as yellow oil (13.0 g, 36.4 mmol, 97.6 %).
MS (ESI, pos.ion) *m*/*z:* 358.3 [M+H]⁺.

### Step 7: N-(1-(4-(benzyloxy)-3-(3-methoxypropoxy)phenyl)-3-methylbutan-2-yl)formamide

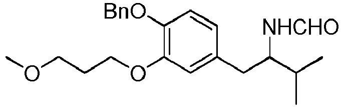

1-(4-(Benzyloxy)-3-(3-methoxypropoxy)phenyl)-3-methylbutan-2-amine (13 g, 36.36 mmol) was added into a 500 mL single-neck flask, then dioxane (130 mL) and formic acid (26.78 g, 581.9 mmol) were added in turn. The reaction mixture was heated to 110 °C and stirred for 12 hours under nitrogen protection. After the reaction was completed, the reaction mixture was concentrated *in vacuo,* and the residue was diluted with water (50 mL). The mixture was extracted with EA (100 mL × 4). The combined organic layers were concentrated *in vacuo,* and the residue was purified by silica gel column chromatography (PE/EA (V/V) = 1/1) to give the title compound as a white solid (9.5 g, 25 mmol, 68%).
MS (ESI, pos.ion) *m*/*z:* 386.4 [M+H]⁺.

### Step 8: 7-(benzyloxy)-3-isopropyl-6-(3-methoxypropoxy)-3,4-dihydroisoquinoline

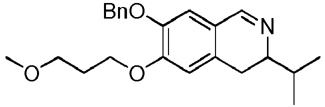

*N*-(1-(4-(benzyloxy)-3-(3-methoxypropoxy)phenyl)-3-methylbutan-2-yl)formamide (5.0 g, 13 mmol) was added into a 100 mL two-neck flask, then DCM (50 mL) was added. The reaction mixture was cooled to 0 °C, then POCl₃ (2.4 mL, 26 mmol) was added, and the mixture was stirred at 50 °C for 3 hours. After the reaction was completed, the reaction mixture was concentrated *in vacuo,* and the residue was diluted with ethyl acetate (50 mL) and water (50 mL). The mixture was partitioned, and the aqueous layer was extracted with EA (50 mL × 4). The combined organic layers were concentrated *in vacuo,* and the residue was purified by silica gel column chromatography (EA) to give the title compound as a yellow solid (3.8 g, 10 mmol, 80%).
MS (ESI, pos.ion) *m*/*z:* 368.2 [M+H]⁺.

### Step 9: ethyl 10-(benzyloxy)-6-isopropyl-9-(3-methoxypropoxy)-2-oxo-2,6,7,11b-tetrahydro -1H-pyrido[2,1-a]isoquinoline-3-carboxylate

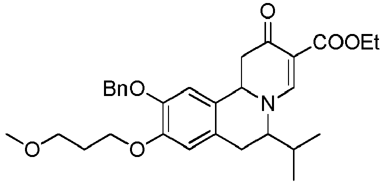

7-(Benzyloxy)-3-isopropyl-6-(3-methoxypropoxy)-3,4-dihydroisoquinoline (4.5 g, 12 mmol), EtOH (10 mL) and ethyl 2-(ethoxymethylene)-3-oxobutanoate (3.4 g, 18 mmol) were added into a 100 mL single-neck flask. The mixture was stirred at 90 °C overnight under nitrogen protection. After the reaction was completed, the reaction mixture was concentrated *in vacuo,* and the residue was purified by silica gel column chromatography (EA) to give the title compound as a brown solid (4.3 g, 8.5 mmol, 69%).
MS (ESI, pos.ion) *m*/*z:* 508.3 [M+H]⁺.

### Step 10: ethyl 10-(benzyloxy)-6-isopropyl-9-(3-methoxypropoxy)-2-oxo-6,7-dihydro-2H -pyrido[2,1-a]isoquinoline-3-carboxylate

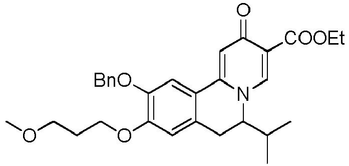

Ethyl 10-(benzyloxy)-6-isopropyl-9-(3-methoxypropoxy)-2-oxo-2,6,7,11b-tetrahydro -1*H*-pyrido[2,1-*a*]isoquinoline-3-carboxylate (3.648 g, 7.187 mmol) was added into a 100 mL single-neck flask, then DME (36 mL) and chloranil (1.767 g, 7.186 mmol) were added. The reaction mixture was stirred at 90 °C for 1 h under nitrogen protection. After the reaction was completed, the reaction mixture was concentrated *in vacuo,* and the residue was purified by silica gel column chromatography (DCM/MeOH (V/V) = 10/1) to give the title compound as a brown solid (3.2 g, 6.3 mmol, 88%).
MS (ESI, pos.ion) *m*/*z:* 506.4 [M+H]⁺.

### Step 11: ethyl 10-hydroxy-6-isopropyl-9-(3-methoxypropoxy)-2-oxo-6,7-dihydro -2H-pyrido[2,1-a]isoquinoline-3-carboxylate

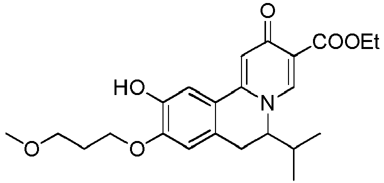

Ethyl 10-(benzyloxy)-6-isopropyl-9-(3-methoxypropoxy)-2-oxo-6,7-dihydro-2*H* -pyrido[2,1-a]isoquinoline-3-carboxylate (2.347 g, 4.642 mmol), Pd/C (0.237 g) and THF (12 mL) were added into a 100 mL single-neck flask. The mixture was stirred at 50 °C overnight under nitrogen protection. After the reaction was completed, the reaction mixture was filtered through a celite pad, and the residue was purified by silica gel column chromatography (DCM/MeOH (V/V) = 10/1) to give the title compound as a brown solid (1.9 g, 4.6 mmol, 99%).
MS (ESI, pos.ion) *m*/*z:* 416.3 [M+H]⁺.

### Step 12: ethyl 6-isopropyl-9-(3-methoxypropoxy)-2-oxo-10-(((trifluoromethyl)sulfonyl) oxy)-6,7-dihydro-2H-pyrido[2,1-a]isoquinoline-3-carboxylate

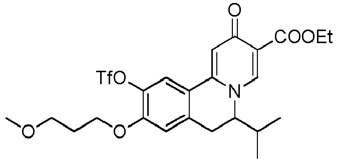

To a 50 mL dried single-neck flask were added ethyl 10-hydroxy-6-isopropyl-9-(3-methoxypropoxy)-2-oxo-6,7-dihydro-2H-pyrido[2,1-a]isoquinolin e-3-carboxylate (250 mg, 0.60 mmol), TEA (0.16 mL, 1.2 mmol) and DCM (5 mL). The reaction mixture was cooled to 0 °C, then *N*,*N*-bis(trifluoromethylsulfonyl)aniline (429 mg, 1.201 mmol) was added. The resulting mixture was warmed to rt and stirred overnight. After the reaction was completed, the reaction mixture was washed with hydrochloric acid (20 mL, 1 M) and saturated brine (20 mL), dried over anydrous sodium sulfate, and filtered. The filtrate was concentrated *in vacuo,* and the residue was purified by silica gel column chromatography (DCM/CH₃OH(V/V)=50/1 to 30/1) to give the title compound as gray powder (310 mg, 94.10%).
MS (ESI, pos.ion) *m*/*z:* 547.8 [M+H]⁺.

### Step 13: 10-(furan-2-yl)-6-isopropyl-9-(3-methoxypropoxy)-2-oxo-6,7-dihydro-2H -pyrido[2,1-a]isoquinoline-3-carboxylic acid

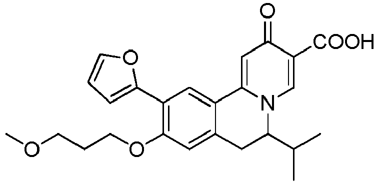

To a 50 mL single-neck flask were added 2-furanboronic acid (81 mg, 0.72 mmol), ethyl 6-isopropyl-9-(3-methoxypropoxy)-2-oxo-10-(((trifluoromethyl)sulfonyl)oxy)-6,7 -dihydro-2H-pyrido[2,1-a]isoquinoline-3-carboxylate (200 mg, 0.36 mmol), tetrakis(triphenylphosphine)palladium (42 mg, 0.036 mmol) and sodium carbonate (193 mg, 1.82 mmol). The reaction mixture was degassed and filled with nitrogen for three times, then 1,4-dioxane (4 mL) and water (1 mL) were added. The resulting mixture was stirred at 100 °C overnight. After the reaction was completed, the reaction mixture was adjusted with hydrochloric acid (1 M) to pH 5, then the mixture was extracted with ethyl acetate (10 mL × 3). The combined organic layer was concentrated *in vacuo.* The residue was purified by silica gel column chromatography (DCM/MeOH (V/V) = 10/1) to give the title compound as an off-white solid (100 mg, 0.23 mmol, 62.57%).
MS (ESI, pos.ion) *m*/*z:* 438.3 [M+ H]⁺;
¹H NMR (400 MHz, CDCl₃) δ 8.58 (s, 1H), 8.28 (s, 1H), 7.54 (s, 1H), 7.30 (s, 1H), 6.99 (d, *J* = 3.2 Hz, 1H), 6.89 (s, 1H), 6.54 (dd, *J=* 3.1, 1.7 Hz, 1H), 4.39 - 4.24 (m, 2H), 4.02 (s, 1H), 3.66 (t, *J=* 5.3 Hz, 2H), 3.48 - 3.36 (m, 4H), 3.17 (d, *J=* 16.2 Hz, 1H), 2.29 - 2.19 (m, 2H), 1.84 (dd, *J=* 15.8, 6.7 Hz, 1H), 0.98 (d, *J=* 6.6 Hz, 3H), 0.84 (d, *J=* 6.6 Hz, 3H).

### Example 14: 6-isopropyl-9-(3-methoxypropoxy)-10-(2-methylthiazol-4-yl)-2-oxo -6,7-dihydro -2H-pyrido[2,1-a]isoquinoline-3-carboxylic acid

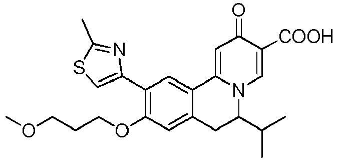

### Step 1: methyl 4-bromo-2-(3-methoxypropoxy)benzoate

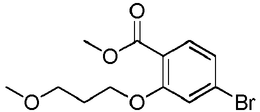

To a dried reaction flask were added DMF (50 mL), methyl 4-bromo-2-hydroxybenzoate (10 g, 43.283 mmol), 1-bromo-3-methoxypropane (7.95 g, 52 mmol) and potassium carbonate (9.06 g, 64.9 mmol). The mixture was heated to 50 °C and stirred for 16 hours. After the reaction was completed, the reaction mixture was diluted with water, and the mixture was extracted with ethyl acetate (50 mL × 3). The combined organic layers were dried over anhydrous sodium sulfate and filtered. The filtrate was concentrated *in vacuo* to give the title compound as colorless oil (13 g, 99.1%).
MS (ESI, pos.ion) *m*/*z:* 303.2 [M+H]⁺.

### Step 2: 2-(3-methoxypropoxy)-4-(3-methyl-2-oxobutyl)benzoic acid

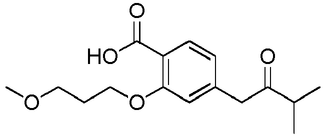

To a 250 mL three-neck flask were added methyl 4-bromo-2-(3-methoxypropoxy)benzoate (6 g, 19.792 mmol), 3-methyl-2-butan-one (2.56 g, 29.72 mmol), tetrahydrofuran (60 mL), xantphos (354 mg, 0.593 mmol) and sodium *tert*-butoxide (5.882 g, 59.36 mmol). The reaction mixture was degassed and filled with nitrogen for three times, and Pd(dba)₂ (440 mg, 0.466 mmol) was added into the mixture under nitrogen protection, then the resulting mixture was degassed and filled with nitrogen for three times. The mixture was heated to 55 °C and stirred for 2 hours. After the reaction was completed, the reaction mixture was diluted with water (100 mL), and washed with ethyl acetate (50 mL) and ethyl acetate (20 mL) in turn. The aqueous layer was diluted with ethyl acetate (80 mL), and the mixture was adjusted with diluted aqueous HCl solution (mass percent: 10%) to pH = 5-7. The mixture was partitioned, and the aqueous layer was extracted with ethyl acetate (30 mL × 2). The combined organic layers were dried over anhydrous sodium sulfate and filtered. The filtrate was concentrated *in vacuo* to give the title compound as colorless oil (3.2 g, 10.88 mmol, 55 %).
MS (ESI, pos.ion) *m*/*z:* 317.1 [M+Na]⁺.

### Step 3: N-methoxy-2-(3-methoxypropoxy)-N-methyl-4-(3-methyl-2-oxobutyl)benzamide

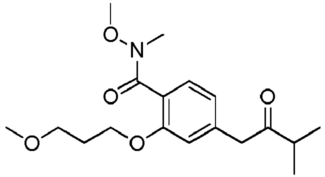

To a 50 mL single-neck flask were added 2-(3-methoxypropoxy)-4-(3-methyl-2-oxobutyl)benzoic acid (1 g, 3.398 mmol), DMF (6 mL), DIPEA (1.78 mL, 10.2 mmol), N,O-dimethylhydroxylamine hydrochloride (500 mg, 5.126 mmol) and HATU (2 g, 5.2598 mmol). The reaction mixture was stirred at rt for 24 h, then to the mixture was added hydrochloric acid (1 M) to adjust pH = 5. The mixture was extracted with ethyl acetate (20 mL × 3). The combined organic layers were dried over anydrous sodium sulfate and filtered. The filtrate was concentrated *in vacuo,* and the residue was purified by silica gel column chromatography (PE/EA (V/V) = 1/1) to give the title compound as colorless oil (200 mg, 0.59 mmol, 17.45%).
MS (ESI, pos.ion) *m*/*z:* 338.3 [M+H]⁺.

### Step 4: 4-(2-amino-3-methylbutyl)-A-methoxy-2-(3-methoxypropoxy)-A-methylbenzamide

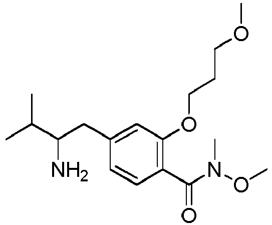

To a 100 mL single-neck flask were added methanol (45 mL), A-methoxy-2-(3-methoxypropoxy)-A-methyl-4-(3-methyl-2-oxobutyl)benzamide (4.5 g, 13 mmol) and ammonium acetate (7.2 g, 93 mmol). The reaction mixture was stirred at rt for 1 h, then cooled to 0 °C, and sodium cyanoborohydride (1.7 g, 27 mmol) was added slowly. The resulting mixture was stirred at rt for 24 h. After the reaction was completed, the reaction mixture was concentrated *in vacuo* to remove the solvent, the reaction mixture was quenched with saturated aqueous sodium bicarbonate solution (50 mL), and extracted with ethyl acetate (50 mL × 3). The combined organic layer was concentrated *in vacuo.* The combined organic layers were dried over anydrous sodium sulfate and filtered. The filtrate was concentrated *in vacuo* to give the title compound as colorless oil (4 g, 11.82 mmol, 89%).
MS (ESI, pos.ion) *m*/*z:* 339.1 [M+H]⁺.

### Step 5: tert-butyl (1-(4-(methoxy(methyl)carbamoyl)-3-(3-methoxypropoxy)phenyl)-3 -methylbutan-2-yl)carbamate

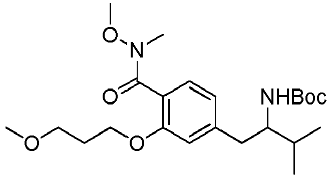

To a 250 mL single-neck flask were added 4-(2-amino-3-methylbutyl)-N-methoxy-2-(3-methoxypropoxy)-N-methylbenzamide (4 g, 11.82 mmol), TEA (3.7 mL, 27 mmol), DCM (50 mL) in turn, then BoczO (5.8 g, 27 mmol) was added dropwise. The resulting mixture was stirred at rt for 2 h. After the reaction was completed, the reaction mixture was concentrated *in vacuo.* To the residue was added hydrochloric acid (1 M) to pH = 5, then the mixture was extracted with ethyl acetate (20 mL × 3). The combined organic layers were dried over anydrous sodium sulfate and filtered. The filtrate was concentrated *in vacuo.* The residue was purified by silica gel column chromatography (PE/EA (V/V) = 1/1) to give the title compound as colorless oil (3.2 g, 7.3 mmol, 62%).
¹H NMR (600 MHz, CDCl₃) δ 7.20 (d, *J* = 7.3 Hz, 1H), 6.83 - 6.77 (m, 2H), 4.37 (d, *J* = 9.3 Hz, 1H), 4.10 (t, *J* = 6.2 Hz, 2H), 3.76 (s, 1H), 3.67 - 3.44 (m, 5H), 3.39 - 3.20 (m, 6H), 2.93 (q, *J* = 7.3 Hz, 1H), 2.83 - 2.76 (m, 1H), 2.72 - 2.65 (m, 1H), 2.06 - 2.01 (m, 1H), 1.79 - 1.71 (m, 1H), 1.40 (s, 9H), 0.98 (d, *J=* 6.8 Hz, 3H), 0.93 (d, *J=* 6.8 Hz, 3H).

### Step 6: tert-butyl (1-(4-acetyl-3-(3-methoxypropoxy)phenyl)-3-methylbutan-2-yl)carbamate

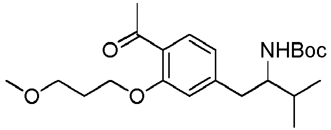

To a dried flask were added tert-butyl (1-(4-(methoxy(methyl)carbamoyl)-3-(3-methoxypropoxy)phenyl)-3-methylbutan-2-yl)carbamat e (21 g, 47.88 mmol) and THF (200 mL). The mixture was degassed and filled with nitrogen for three times, then cooled to -5 °C, and a solution of methyl magnesium bromide in tetrahydrofuran (64 mL, 192 mmol, 3 mol/L) was added dropwise slowly. The reaction mixture was stirred at -5 °C for 12 h, then saturated aqueous ammonium chloride (100 mL), ethyl acetate (200 mL) and water (200 mL) were added. The mixture was partitioned, and the aqueous layer was extracted with ethyl acetate (100 mL). The combined organic layers were concentrated *in vacuo* to remove the solvent, and the residue was purified by silica gel column chromatography (PE/EA (V/V) = 2/1) to give the title compound as a white solid (17 g, 43.20 mmol, 90.23%).
MS (ESI, pos.ion) *m*/*z:* 394.3 [M+H]⁺.

### Step 7: 1-(4-(2-amino-3-methylbutyl)-2-(3-methoxypropoxy)phenyl)ethanone

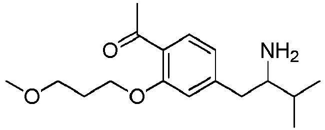

To a 50 mL single-neck flask were added tert-butyl (1-(4-acetyl-3-(3-methoxypropoxy)phenyl)-3-methylbutan-2-yl)carbamate (5 g, 12.71 mmol), DCM (10 mL) and TFA (10 mL). The reaction mixture was stirred at rt for 3 h, then concentrated by rotary evaporation. To the residue was added saturated aqueous sodium bicarbonate solution to pH = 8, then the mixture was extracted with ethyl acetate (20 mL × 3). The combined organic layers were dried over anydrous sodium sulfate and filtered. The filtrate was concentrated *in vacuo* to give the title compound as colorless oil (3.3 g, 11 mmol, 89%).
MS (ESI, pos.ion) *m*/*z:* 294.3 [M+H]⁺.

### Step 8: N-(1-(4-acetyl-3-(3-methoxypropoxy)phenyl)-3-methylbutan-2-yl)formamide

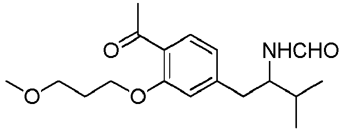

To a 50 mL single-neck flask were added 1-(4-(2-amino-3-methylbutyl)-2-(3-methoxypropoxy)phenyl)ethanone (3.3 g, 11 mmol), 1,4-dioxane (33 mL) and formic acid (6.4 mL, 170 mmol). The mixture was heated and refluxed for 20 hours, then concentrated *in vacuo,* and the residue was purified by silica gel column chromatography (DCM/CH₃OH (V/V) = 20/1) to give the title compound as a white solid (2.35 g, 7.31 mmol, 65%).
MS (ESI, pos.ion) *m*/*z:* 322.3 [M+H]⁺.

### Step 9: N-(1-(4-(2-bromoacetyl)-3-(3-methoxypropoxy)phenyl)-3-methylbutan-2-yl)formamide

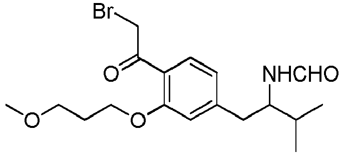

To a 50 mL single-neck flask were added *N*-(1-(4-acetyl-3-(3-methoxypropoxy)phenyl)-3-methylbutan-2-yl)formamide (200 mg, 0.62 mmol), methanol (10 mL) and montmorillonite K10 (20 mg). The mixture was heated to 60 °C, and NBS (130 mg, 0.73 mmol) was added in six portions for 15 min. Then the resulting mixture was stirred for 30 min. After the reaction was completed, the reaction mixture was filtered to remove the solid, and the filtrate was concentrated *in vacuo.* The residue was purified by silica gel column chromatography (PE/EA (V/V) = 2/1) to give the title compound as a white solid (237 mg, 0.5921 mmol, 95.14%).
MS (ESI, pos.ion) *m*/*z:* 400.2 [M+H]⁺.

### Step 10: N-(1-(3-(3-methoxypropoxy)-4-(2-methylthiazol-4-yl)phenyl)-3-methylbutan -2-yl)formamide

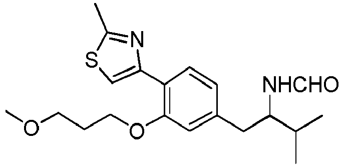

*N*-(1-(4-(2-Bromoacetyl)-3-(3-methoxypropoxy)phenyl)-3-methylbutan-2-yl)formami de (1.22 g, 3.05 mmol), thioacetamide (0.252 g, 3.35 mmol) and EtOH (10 mL) was added into a 100 mL single-neck flask. The mixture was stirred at rt overnight, then concentrated, and the residue was purified by silica gel column chromatography (DCM/CH₃OH(V/V)=10/1) to give the title compound as black brown oil (1.1 g, 2.9 mmol, 96%).
MS (ESI, pos.ion) *m*/*z:* 377.4 [M+H] ⁺.

### Step 11: 4-(3-isopropyl-6-(3-methoxypropoxy)-3,4-dihydroisoquinolin-7-yl)-2-methylthiazole

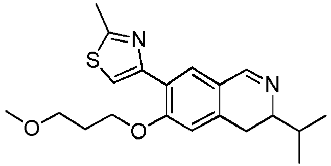

*N*-(1-(3-(3-Methoxypropoxy)-4-(2-methylthiazol-4-yl)phenyl)-3-methylbutan-2-yl) formamide (1.22 g, 3.05 mmol) and DCM (22 mL) was added into a 100 mL single-neck flask. The mixture was cooled to 0 °C, then POCl₃ (0.54 mL, 5.8 mmol) was added into the mixture. The reaction mixture was stirred at rt overnight, and poured into ice-water (20 g). The resulting mixture was adjusted with ammonium hydroxide to pH = 10. The mixture was extracted with ethyl acetate (30 mL × 4). The combined organic layers were washed with saturated brine (30 mL × 2), and the organic layer was concentrated *in vacuo.* The residue was purified by silica gel column chromatography (PE/EA (V/V) = 1/1) to give the title compound as brown oil (0.385 g, 1.07 mmol, 37%).
MS (ESI, pos.ion) *m*/*z:* 359.3 [M+H] ⁺.

### Step 12: ethyl 6-isopropyl-9-(3-methoxypropoxy)-10-(2-methylthiazol-4-yl) -2-oxo-2,6,7,11b-tetrahydro-1H-pyrido[2,1-a]isoquinoline-3-carboxylate

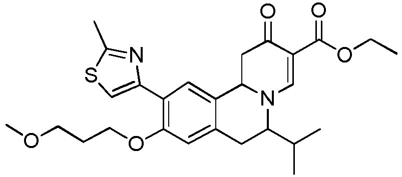

4-(3-Isopropyl-6-(3-methoxypropoxy)-3,4-dihydroisoquinolin-7-yl)-2-methylthiazole (0.385 g, 1.07 mmol), ethyl 2-(ethoxymethylene)-3-oxobutanoate (0.400 g, 2.15 mmol) and EtOH (10 mL) were added into a 100 mL single-neck flask. The mixture was stirred at 85 °C overnight. The reaction mixture was cooled to rt, and concentrated *in vacuo.* The residue was purified by silica gel column chromatography (EA) to give the title compound as brown oil (0.31 g, 0.62 mmol, 50%).
MS (ESI, pos.ion) *m*/*z:* 499.4 [M+H] ⁺.

### Step 13: ethyl 6-isopropyl-9-(3-methoxypropoxy)-10-(2-methylthiazol-4-yl)-2-oxo-6,7 -dihydro-2H-pyrido[2,1-a]isoquinoline-3-carboxylate

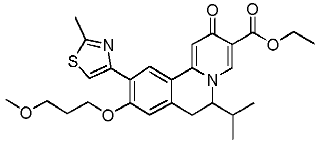

Ethyl 6-isopropyl-9-(3-methoxypropoxy)-10-(2-methylthiazol-4-yl)-2-oxo-2,6,7,11b -tetrahydro-1*H*-pyrido[2,1-*a*]isoquinoline-3-carboxylate (0.31 g, 0.62 mmol) was added into a 50 mL single-neck flask, then chloranil (0.15 g, 0.61 mmol) and 1,2-dimethoxyethane (10 mL) were added. The reaction mixture was heated to 90 °C and stirred for 1 h under nitrogen protection, then concentrated *in vacuo.* The residue was purified by silica gel column chromatography (DCM/MeOH (V/V) = 8/1) to give the title compound as a brown solid (0.30 g, 0.60 mmol, 97%).

MS (ESI, pos.ion) *m*/*z:* 497.3 [M+H] ⁺.

### Step 14: 6-isopropyl-9-(3-methoxypropoxy)-10-(2-methylthiazol-4-yl)-2-oxo-6,7-dihydro -2H-pyrido[2,1-a]isoquinoline-3-carboxylic acid

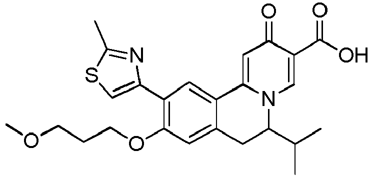

Ethyl 6-isopropyl-9-(3-methoxypropoxy)-10-(2-methylthiazol-4-yl)-2-oxo-6,7 -dihydro-2*H*-pyrido[2,1-*a*]isoquinoline-3-carboxylate (0.300 g, 0.604 mmol) was added into a 50 mL single-neck flask, then THF (6 mL), EtOH (3 mL) and H₂O (1.5 mL) were added. The mixture was stirred to dissolve the solid, then LiOH.HzO (50 mg, 1.1905 mmol) was added. The reaction mixture was stirred at rt for 6 hours, then concentrated *in vacuo,* and the residue was dissolved in water (5 mL). Then the mixture adjusted with hydrochloric acid (1 M) to pH = 2-3, and the mixture was extracted with DCM (20 mL × 4). The combined organic layers were concentrated *in vacuo,* and the residue was recrystallized from methanol (5 mL) to give the title compound as a white solid (170 mg, 0.3628 mmol, 60.1%).
MS (ESI, pos.ion) *m*/*z:* 469.3 [M+H] ⁺;
¹H NMR (400 MHz, CDCl₃) δ 16.172 (br, 1H), 8.796 (s, 1H), 8.463 (s, 1H), 7.821 (s, 1H), 7.322 (s, 1H), 6.904 (s, 1H), 4.270 - 4.361 (m, 2H), 3.911 - 3.889 (m, 1H), 3.669 - 3.593 (m, 2H), 3.469 - 3.406 (m, 1H), 3.388 (s, 3H), 3.321 - 3.122 (m, 1H), 2.810 (s, 3H), 2.261 - 2.200 (m, 2H), 1.886 - 1.787 (m, 1H), 0.973 (d, *J* =6.4Hz, 3H), 0.835 (d, *J* =6.8Hz, 3H).

### Example 15: 6-isopropyl-9-(3-methoxypropoxy)-2-oxo-10-(thiophen-2-yl)-6,7-dihydro-2H -pyrido[2,1-a]isoquinoline-3-carboxylic acid

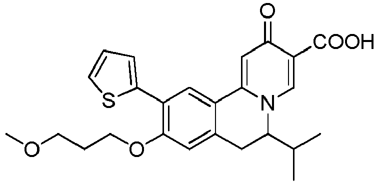

To a 50 mL two-neck flask were added 2-(tributylstannyl)thiazole (0.378 g, 1.01 mmol), ethyl 6-isopropyl-9-(3-methoxypropoxy)-2-oxo-10-(((trifluoromethyl)sulfonyl) oxy)-6,7-dihydro-2H-pyrido[2,1-a]isoquinoline-3-carboxylate (0.22 g, 0.40 mmol), bis(triphenylphosphine)palladium(II) (71 mg, 0.10 mmol) and anhydrous dioxane (10 mL). The reaction mixture was stirred at 110 °C overnight under nitrogen protection, then distilled under reduced pressure to remove the solvent. The residue was recrystallized from methanol (5 mL) to give the title compound as a gray solid (40 mg, 0.09 mmol).
MS (ESI, pos.ion) *m*/*z:* 454.3 [M+H] ⁺;
¹H NMR (400 MHz, CDCl₃) δ 16.049 (br, 1H), 8.489 (s, 1H), 8.040 (s, 1H), 7.559(d, J =2.8Hz, 1H), 7.423(d, J =4.2Hz, 1H),7.185 (s, 1H), 7.168 - 7.146 (m, 1H), 6.907 (s, 1H), 4.341 - 4.248 (m, 2H), 3.948 - 3.914 (m, 1H), 3.691 - 3.636 (m, 2H), 3.461 - 3.405 (m, 1H),3.390 (s, 3H), 3.226 - 3.120 (m, 1H), 2.2594 - 2.198 (m, 2H), 1.902 - 1.799 (m, 1H), 0.991 (d, *J* = 6.8Hz, 3H), 0.864 (d, *J* = 6.8Hz, 3H).

### Example 21: 6-isopropyl-2-oxo-9-((tetrahydro-2H-pyran-4-yl)methoxy)-10-(thiazol-2-yl) -6,7-dihydro-2H-pyrido[2,1-a]isoquinoline-3-carboxylic acid

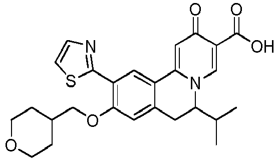

### Step 1: ethyl 6-isopropyl-2-oxo-9-((tetrahydro-2//-pyran-4-yl)methoxy)-10-(thiazol-2-yl) -6,7-dihydro-2H-pyrido[2,1-a]isoquinoline-3-carboxylate

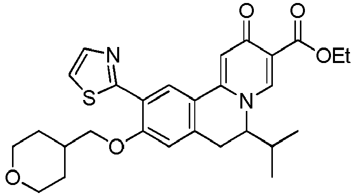

To a single-neck flask were added ethyl 9-hydroxy-6-isopropyl-2-oxo-10-(thiazol-2-yl)-6,7-dihydro-2*H*-pyrido[2,1-*a*]isoquinoline-3-carb oxylate (200 mg, 0.49 mmol), potassium carbonate (270 mg, 1.95 mmol), acetone (3 mL) and 4-bromomethyltetrahydropyrane (220 mg, 1.23 mmol). The reaction mixture was stirred at 80 °C for 8 hours, then concentrated *in vacuo,* and the residue was purified by silica gel column chromatography (DCM/CH₃OH (V/V)=10/1) to give the title compound as a yellow solid (180 mg, 73%).
MS (ESI, pos.ion) *m*/*z:* 509.1 [M+H]⁺.

### Step 2: 6-isopropyl-2-oxo-9-((tetrahydro-2H-pyran-4-yl)methoxy)-10-(thiazol-2-yl) -6,7-dihydro-2H-pyrido[2,1-a]isoquinoline-3-carboxylic acid

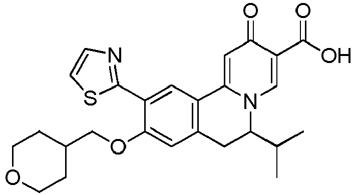

To a single-neck flask were added ethyl 6-isopropyl-2-oxo -9-((tetrahydro-2*H*-pyran-4-yl)methoxy)-10-(thiazol-2-yl)-6,7-dihydro-2*H*-pyrido[2,1-*a*]isoquino line-3-carboxylate (160 mg, 0.32 mmol), lithium hydroxide monohydrate (55 mg, 1.3 mmol) and methanol (4 mL). The mixture was stirred at rt for 8 hours, then hydrochloric acid (1 M) was added to adjust pH to 5. The mixture was filtered to give a yellow solid, and the filter cake was recrystalized from methanol to give the title compound as a light yellow solid (60 mg, 39%).
MS (ESI, pos.ion) *m*/*z:* 481.18 [M+H]⁺;
¹H NMR (400 MHz, CDCl₃) δ 16.08 (s, 1H), 8.91 (s, 1H), 8.47 (s, 1H), 7.97 (d, *J=* 3.2 Hz, 1H), 7.50 (d, *J* = 3.2 Hz, 1H), 7.31 (s, 1H), 6.95 (s, 1H), 4.15 (d, *J* = 6.2 Hz, 2H), 4.10 (dd, *J* = 11.3, 3.2 Hz, 2H), 3.94 (dd, *J* = 9.3, 4.4 Hz, 1H), 3.54 (dd, *J* = 19.2, 7.5 Hz, 2H), 3.45 (d, *J* = 5.1 Hz, 1H), 3.22 (d, *J* = 16.2 Hz, 1H), 2.36 (s, 1H), 1.95 (s, 2H), 1.83 (qd, *J* = 13.4, 6.7 Hz, 1H), 1.62 (s, 2H), 0.99 (d, *J=* 6.6 Hz, 3H), 0.85 (d, *J=* 6.7 Hz, 3H).

### Example 22: 9-(2-cyclopropylethoxy)-6-isopropyl-2-oxo-10-(thiazol-2-yl)-6.7-dihydro-2H -pyrido [2,1-a]isoquinoline-3-carboxylic acid

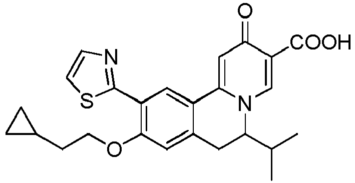

### Step 1: ethyl 9-(2-cyclopropylethoxy)-6-isopropyl-2-oxo-10-(thiazol-2-yl)-6,7-dihydro -2H-pyrido[2,1-a]isoquinoline-3-carboxylate

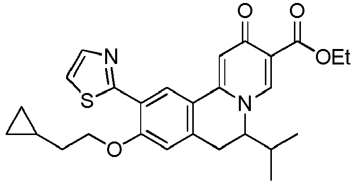

To a single-neck flask were added ethyl 9-hydroxy-6-isopropyl -2-oxo-10-(thiazol-2-yl)-6,7-dihydropyrido[2,1-a]isoquinoline-3-carboxylate (250 mg, 0.61 mmol), potassium carbonate (336 mg, 2.4 mmol), acetonitrile (3 mL) and 2-cyclopropylethylbromide (270 mg, 1.5 mmol). The reaction mixture was stirred at 80 °C for 8 hours, then concentrated *in vacuo,* and the residue was purified by silica gel column chromatography (DCM/CH₃OH (V/V)=10/1) to give the title compound as a yellow solid (180 mg, 73%).
MS (ESI, pos.ion) *m*/*z:* 479.1 [M+H]⁺.

### Step 2: 9-(2-cyclopropylethoxy)-6-isopropyl-2-oxo-10-(thiazol-2-yl)-6,7-dihydro-2H -pyrido[2,1-a]isoquinoline-3-carboxylic acid

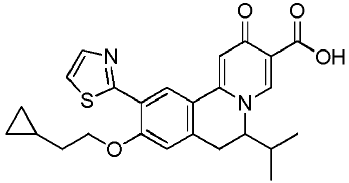

To a single-neck flask were added ethyl 9-(2-cyclopropylethoxy)-6-isopropyl-2-oxo-10-(thiazol-2-yl)-6,7-dihydro-2*H*-pyrido[2,1-*a*]isoqu inoline-3-carboxylate (270 mg, 0.32 mmol), lithium hydroxide monohydrate (95 mg, 2.3 mmol) and methanol (5 mL). The reaction mixture was stirred at rt for 8 h, then adjusted with hydrochloric acid (1 M) to pH = 5, and there was a yellow solid precipitated out. The mixture was filtered, and the filter cake was recrystallized from methanol to give the the title compound as a light yellow solid (120 mg, 47%).
MS (ESI, pos.ion) *m*/*z:* 451.17 [M+H]⁺;
¹H NMR (400 MHz, CDCl₃) δ 16.11 (s, 1H), 8.92 (s, 1H), 8.47 (s, 1H), 7.97 (d, *J=* 3.2 Hz, 1H), 7.48 (d, *J* = 3.2 Hz, 1H), 7.32 (s, 1H), 6.98 (s, 1H), 4.37 (dd, *J* = 10.1, 6.5 Hz, 2H), 4.01 - 3.87 (m, 1H), 3.47 (dd, *J=* 16.4, 4.5 Hz, 1H), 3.22 (d, *J=* 16.1 Hz, 1H), 2.01 - 1.91 (m, 2H), 1.83 (dt, *J=* 13.5, 6.7 Hz, 1H), 0.99 (d, *J=* 6.6 Hz, 3H), 0.85 (d, *J=* 6.7 Hz, 3H), 0.58 (d, *J=* 7.6 Hz, 2H), 0.22 (d, J= 4.9 Hz, 2H).

### Example 23: 9-(2-ethylbutoxy)-6-isopropyl-2-oxo-10-(thiazol-2-yl)-6,7-dihydro-2H-pyrido[2,1-a]isoquinoline-3-carboxylic acid

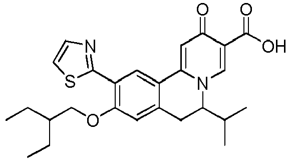

To a single-neck flask were added 9-hydroxy-6-isopropyl-2-oxo-10-(thiazol-2-yl)-6,7-dihydro-2*H*-pyrido[2,1-*a*]isoquinoline-3-carb oxylic acid (200 mg, 0.52 mmol), potassium carbonate (290 mg, 2.1 mmol), acetonitrile (5 mL) and 2-ethyl-1-bromobutane (200 mg, 1.2 mmol). The reaction mixture was stirred at 80 °C for 24 hours, then hydrochloric acid (1 M) was add to adjust the pH to 5. The resulting mixture was concentrated *in vacuo,* and the residue was purified by silica gel column chromatography (DCM/CH₃OH (V/V)=10/1) to give the title compound as a light yellow solid (160 mg, 66%).
MS (ESI, pos.ion) *m*/*z:* 467.20 [M+H]⁺;
¹H NMR (400 MHz, CDCl₃) δ 16.12 (s, 1H), 8.93 (s, 1H), 8.48 (s, 1H), 7.97 (d, *J=* 3.2 Hz, 1H), 7.49 (d, *J=* 3.2 Hz, 1H), 7.33 (s, 1H), 6.97 (s, 1H), 4.18 (dd, *J=* 11.7, 6.9 Hz, 2H), 3.95 (dd, *J=* 9.6, 4.4 Hz, 1H), 3.46 (dd, *J* = 16.3, 5.1 Hz, 1H), 3.22 (d, *J* = 16.2 Hz, 1H), 1.98 - 1.88 (m, 1H), 1.85 (dd, *J* = 6.6, 3.1 Hz, 1H), 1.69 (dd, *J* = 14.4, 7.1 Hz, 4H), 1.06 - 0.96 (m, 9H), 0.85 (d, *J* = 6.7 Hz, 3H).

### Example 24: 10-(5-bromothiazol-2-yl)-6-isopropyl-9-(3-methoxypropoxy)-2-oxo-6,7-dihydro-2H-pyrido[2,1-a]isoquinoline-3-carboxylic acid

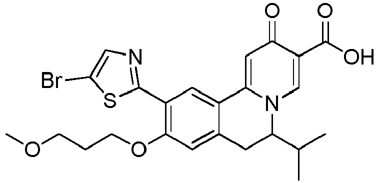

### Step 1: 1-(3-(3-methoxypropoxy)-4-(thiazol-2-yl)phenyl)-3-methylbutan-2-amine

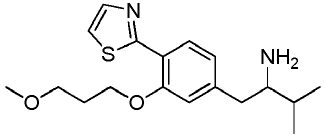

1-(3-(3-Methoxypropoxy)-4-(thiazol-2-yl)phenyl)-3-methylbutan-2-one (4.4 g, 13 mmol), MeOH (45 mL) and NH₄OAc (12 g, 155.7 mmol, mass percent: 100%) were added into a 100 mL single-neck flask. The reaction mixture was stirred at rt for 1 h, then cooled to 0 °C, and NaBH₃CN (2.5 g, 40 mmol) was added. The mixture was warmed to rt and stirred at rt overnight. After the reaction was completed, the reaction mixture was concentrated *in vacuo* to remove the solvent. Ammonium hydroxide (5 mL) and water (10 mL) was added in turn to dilute the residue. The mixture was extracted with EA (30 mL ×4), and the combined organic layers were concentrated *in vacuo* to give the title compound as yellow oil (4.41 g, 13.20 mmol, 100%).
MS (ESI, pos.ion) *m*/*z:* 335.3 [M+H]⁺.

### Step 2: N-(1-(3-(3-methoxypropoxy)-4-(thiazol-2-yl)phenyl)-3-methylbutan-2-yl)formamide

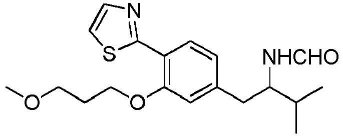

To a 100 mL single-neck flask were added 1-(3-(3-methoxypropoxy)-4-(thiazol-2-yl)phenyl)-3-methylbutan-2-amine (4.415 g, 13.20 mmol), formic acid (9.721 g, 211.2 mmol) and dioxane (20 mL). The mixture was heated to 110 °C and stirred for 12 h, then concentrated *in vacuo* to remove the solvent. The residue was diluted with saturated brine (20 mL), then extracted with EA (30 mL ×4). The combined organic layers were washed with saturated brine (10 mL × 2), and concentrated *in vacuo* to give the title compound as yellow oil (4.785 g, 13.20 mmol, 100.0%).
MS (ESI, pos.ion) *m*/*z:* 363.2 [M+H]⁺.

### Step 3: N-(1-(4-(5-bromothiazol-2-yl)-3-(3-methoxypropoxy)phenyl)-3-methylbutan -2-yl)formamide

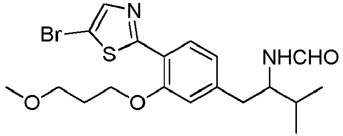

*N*-(1-(3-(3-methoxypropoxy)-4-(thiazol-2-yl)phenyl)-3-methylbutan-2-yl)formamide (0.700 g, 1.93 mmol), NBS (344 mg, 1.93 mmol) and CHCl₃ (10 mL) was added into a 100 mL single-neck flask. The mixture was stirred at rt overnight. After the reaction was completed, the reaction mixture was diluted with water (10 mL), then partitoned. The aqueous layer was extracted with DCM (15 mL × 3). The combined organic layers were washed with water (10 mL × 2), and the organic layer was concentrated *in vacuo.* The residue was purified by silica gel column chromatography (EA) to give the title compound as a white solid (0.82 g, 1.9 mmol, 96%).
MS (ESI, pos.ion) *m*/*z:* 441.1 [M+H]⁺.

### Step4:9-(5-bromothiazol-2-yl)-5-isopropyl-8-(3-methoxypropoxy)-5,6-dihydro-2H-oxazolo[2,3-a]isoquinoline-2,3(10bH)-dione

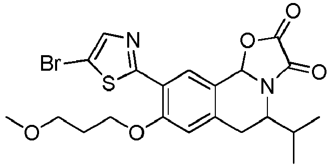

*N-*(1-(4-(5-Bromothiazol-2-yl)-3-(3-methoxypropoxy)phenyl)-3-methylbutan-2-yl) formamide (0.2 g, 0.5 mmol) and DCM (50 mL) were added into a 50 mL two-neck flask, then oxalyl chloride (63 mg, 0.50 mmol) was added. The mixture was stirred at -10 °C under nitrogen protection, then FeCl₃ (87 mg, 0.54 mmol) was added, and the mixture was warmed to rt and stirred overnight. Then to the mixture was added HCl (5 mL, 2 M), and the mixture was stirred for 1 h, and then water (10 mL) was added into the mixture. The mixture was extracted with DCM (30 mL ×4), and the combined organic layers were concentrated *in vacuo* to give the title compound as brownness oil (0.22 g, 0.45 mmol, 100%).
MS (ESI, pos.ion) *m*/*z:* 495.3 [M+H]⁺.

### Step 5: 5-bromo-2-(3-isopropyl-6-(3-methoxypropoxy)-3,4-dihydroisoquinolin-7-yl)thiazole

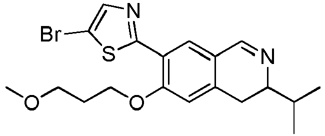

9-(5-Bromothiazol-2-yl)-5-isopropyl-8-(3-methoxypropoxy)-5,6-dihydro-2*H*-oxazolo[ 2,3-*a*]isoquinoline-2,3(10b*H*)-dione (0.2 g, 0.4 mmol) and DCM (10 mL) were added into a 50 mL single-neck flask, then methanol (10 mL) and concentrated sulfuric acid (0.5 mL) were added in turn. The reaction mixture was heated to 70 °C and stirred overnight, then concentrated *in vacuo.* The residue was diluted with water (10 mL) and EA (30 mL) in turn, then partitoned. The organic layer was washed with HCl (10 mL × 2, 2 M), The combined organic layers were adjusted with ammonium hydroxide to pH 9-10, then the mixture was extracted with EA (20 mL ×3). The combined organic layers were concentrated *in vacuo* to give the crude product as a black solid (115 mg, 0.27 mmol, 70%).
MS (ESI, pos.ion) *m*/*z:* 423.1 [M+H]⁺.

### Step 6: ethyl 10-(5-bromothiazol-2-yl)-6-isopropyl-9-(3-methoxypropoxy)-2-oxo-2,6,7,11b-tetrahydro-1H-pyrido[2,1-a]isoquinoline-3-carboxylate

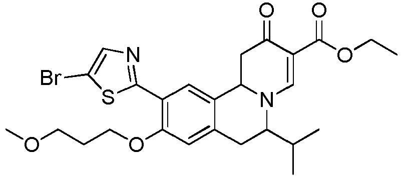

5-Bromo-2-(3-isopropyl-6-(3-methoxypropoxy)-3,4-dihydroisoquinolin-7-yl)thiazole (390 mg, 0.92 mmol), ethyl 2-(ethoxymethylene)-3-oxobutanoate (343 mg, 1.84 mmol) and EtOH (9 mL) were added into a 100 mL single-neck flask. The mixture was stirred at 85 °C for 6 h. After the reaction was completed, the reaction mixture was cooled to rt and concentrated *in vacuo,* and the residue was purified by silica gel column chromatography (DCM/MeOH (V/V) = 10/1) to give the title compound as a brown solid (300 mg, 0.53 mmol, 57.80%).
MS (ESI, pos.ion) *m*/*z:* 563.0 [M+H] ⁺.

### Step 7: ethyl 10-(5-bromothiazol-2-yl)-6-isopropyl-9-(3-methoxypropoxy)-2-oxo-6,7-dihydro -2H-pyridor2, 1-a lisoquinoline-3-carboxylate

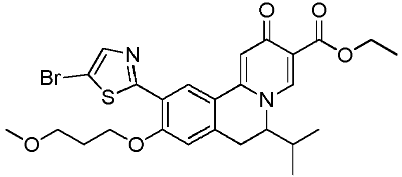

Ethyl 10-(5-bromothiazol-2-yl)-6-isopropyl-9-(3-methoxypropoxy)-2-oxo-2,6,7,11b -tetrahydro-1*H*-pyrido[2,1-*a*]isoquinoline-3-carboxylate (330 mg, 0.59 mmol) was dissolved in dimethoxyethane (9 mL), then chloranil (144 mg, 0.58570 mmol) was added. The mixture was heated to 90 °C and stirred for 1 hour under nitrogen protection, then cooled to rt and concentrated *in vacuo.* The residue was purified by thin-layer chromatography (DCM/CH₃OH (V/V) = 20/1) to give the title compound as a brownness solid (250 mg, 0.45 mmol, 76.03%).
MS (ESI, pos.ion) *m*/*z:* 561.1 [M+H]⁺.

### Step 8: 10-(5-bromothiazol-2-yl)-6-isopropyl-9-(3-methoxypropoxy)-2-oxo-6,7-dihydro -2H-pyridor2, 1-a lisoquinoline-3-carboxylic acid

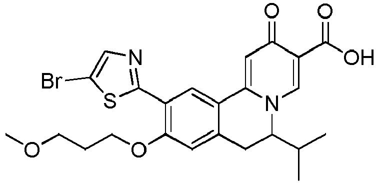

Ethyl 10-(5-bromothiazol-2-yl)-6-isopropyl-9-(3-methoxypropoxy)-2-oxo-6,7-dihydro-2H-pyrido[2,1-a]isoquinoline-3-carboxylate (240 mg, 0.43 mmol), LiOH.H₂O (17 mg, 0.40 mmol) and EtOH (10 mL) was added into a 50 mL single-neck flask. The mixture was stirred at rt for 3 hours, then adjusted with aqueous HCl solution (1 M) to pH 4-5, then concentrated *in vacuo.* The residue was purified by thin-layer chromatography (DCM/CH₃OH (V/V) = 20/1) to give the title compound as a gray solid (125 mg, 0.23 mmol, 54.83%).
MS (ESI, pos.ion) *m*/*z:* 533.1 [M+H] ⁺;
¹H NMR (400 MHz, CDCl₃) δ 16.025 (br, 1H), 8.841 (s, 1H), 8.480 (s, 1H), 7.852 (s, 1H), 7.297 (s, 1H), 6.977 (s, 1H), 4.465 - 4.374 (m, 2H), 3.991 - 3.909 (m,1H), 3.716 - 3.647 (m, 2H), 3.475 - 3.372 (m, 4H), 3.255 - 3.174 (m, 1H), 2.350 - 2.290 (m, 2H), 1.861 - 1.778 (m, 1H), 0.984 (d, *J* = 6.8Hz, 3H), 0.852 (d, J=6.4Hz, 3H).

### Example 25: 10-acetoxy-6-isopropyl-9-(3-methoxypropoxy)-2-oxo-6,7-dihydro -2H-pyrido[2,1-a]isoquinoline-3-carboxylic acid

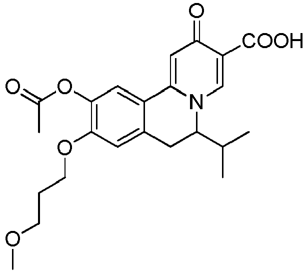

### Step 1: ethyl 10-hydroxy-6-isopropyl-9-(3-methoxypropoxy)-2-oxo-6,7-dihydro-2H-pyrido[2,1-a]isoquinoline-3-carboxylic acid

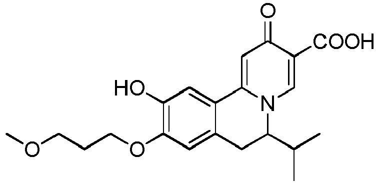

Ethyl 10-hydroxy-6-isopropyl-9-(3-methoxypropoxy)-2-oxo-6,7-dihydro-2*H-*pyrido[2,1-a]isoquinoline-3-carboxylate (1.90 g, 4.57 mmol), THF (6 mL), EtOH (3 mL), H₂O (1.5 mL) and LiOH.H₂O (0.672 g, 16.0 mmol) were added into a 100 mL single-neck flask, then the mixture was stirred at rt for 4 h. After the reaction was completed, the reaction mixture was concentrated *in vacuo,* and to the residue was added water (10 mL). The mixture was extracted with DCM (20 mL × 4). The combined organic layers were dried over anydrous sodium sulfate and filtered. The filtrate was concentrated *in vacuo,* and the residue was purified by silica gel column chromatography (DCM/MeOH (V/V) = 10/1) to give the title compound as a gray solid (1.2 g, 3.1 mmol, 68%).
MS (ESI, neg.ion) *m*/*z:* 388.2 [M+H]⁺.

### Step 2: 10-acetoxy-6-isopropyl-9-(3-methoxypropoxy)-2-oxo-6,7-dihydro-2H-pyrido[2,1-a] isoquinoline-3-carboxylic acid

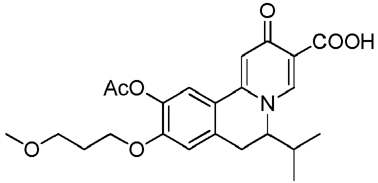

10-Hydroxy-6-isopropyl-9-(3-methoxypropoxy)-2-oxo-6,7-dihydro-2H-pyrido[2,1-a]i soquinoline-3-carboxylic acid (0.7 g, 2 mmol) was added into a 50 mL single-neck flask, then DCM (20 mL) was added. The mixture was cooled to 0 °C, and then acetyl chloride (0.45 mL, 6.32 mmol) and TEA (0.92 g, 9.04 mmol) were added in turn. The reaction mixture was heated to rt and stirred overnight, then poured into ice-water. The mixture was extracted with DCM (30 mL × 3), and the combined organic layers were washed with saturated brine (20 mL), then concentrated *in vacuo.* The residue was purified by silica gel column chromatography (DCM/MeOH (V/V) = 10/1) to give the crude product, which was purified by recrystallization from methanol (10 mL) to give the title compound as a gray solid (0.3 g, 0.7 mmol, 40%).
MS (ESI, pos.ion) *m*/*z:* 430.2 [M+H]⁺;
¹H NMR (400 MHz, CDCl₃) δ 16.025 (br,1H), 8.494 (s, 1H), 7.445 (s, 1H), 7.015 (s, 1H), 6.876 (s, 1H), 4.233 - 4.109 (m, 2H), 4.026 - 3.865 (m, 1H), 3.574 - 3.494 (m, 2H), 3.427 - 3.302 (m, 4H), 3.214 - 3.073 (m, 1H), 2.350(s, 3H), 2.131 - 2.008 (m, 2H), 1.838 - 1.831 (m, 1H), 0.968 (d, *J* =6 Hz, 3H), 0.822 (d, *J=* 6.4 Hz, 3H).

### Example 26: 6-isopropyl-9-(3-methoxypropoxy)-2-oxo-10-(pivaloyloxy)-6,7-dihydro-2H -pyrido[2,1-a]isoquinoline-3-carboxylic acid

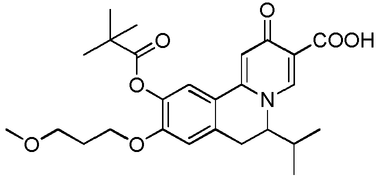

10-Hydroxy-6-isopropyl-9-(3-methoxypropoxy)-2-oxo-6,7-dihydro-2H-pyrido[2,1-a]i soquinoline-3-carboxylic acid (0.26 g, 0.67 mmol) was added into a 50 mL two-neck flask, then DCM (6 mL) was added. The mixture was cooled to 0 °C, and then pivaloyl chloride (0.41 mL, 3.36 mmol) and TEA (0.47 mL, 3.4 mmol) were added in turn. The reaction mixture was stirred for 30 min then warmed to rt and continue to stir overnight. After the reaction was completed, the reaction mixture was poured into ice-water (20 g). The mixture was extracted with DCM (30 mL × 4), The combined organic layers were concentrated *in vacuo.* The residue was purified by thin-layer chromatography (DCM/CH₃OH (V/V) = 20/1) to give the crude product, which was purified twice by recrystallization from methanol (6 mL) to give the title compound as a gray solid (100 mg, 0.21 mmol, 32%).
MS (ESI, pos.ion) *m*/*z:* 472.1 [M+H]⁺;
¹H NMR (400 MHz, CDCl₃) δ 15.994 (br,1H), 8.465 (s, 1H), 7.415(s, 1H), 7.029 (s, 1H), 6.860 (s, 1H), 4.219 - 4.088 (m, 2H), 3.959 - 3.859 (m, 1H), 3.595 - 3.487 (m, 2H), 3.449 - 3.290 (m, 4H), 3.200 - 3.099 (m, 1H), 2.127 - 2.071 (s, 2H), 1.895 - 1.793 (m, 1H), 1.405 (s, 9H), 0.969 (d, *J* = 2.8Hz, 3H), 0.834 (d, *J* = 3.2 Hz, 3H).

### Example 27: 10-(isobutyryloxy)-6-isopropyl-9-(3-methoxypropoxy)-2-oxo-6.7-dihydro-2H -pyrido[2,1-a]isoquinoline-3-carboxylic acid

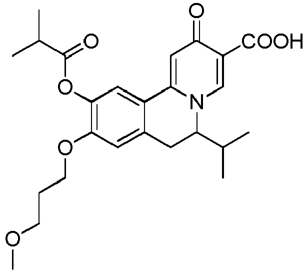

10-Hydroxy-6-isopropyl-9-(3-methoxypropoxy)-2-oxo-6,7-dihydro-2H-pyrido[2,1-a] isoquinoline-3-carboxylic acid (0.21 g, 0.56 mmol) was added into a 50 mL two-neck flask, then DCM (6 mL) was added. The mixture was cooled to 0 °C, and then 2-methylpropionyl chloride (0.29 mL, 2.8 mmol) and TEA (0.39 mL, 2.8 mmol) were added in turn. The reaction mixture was stirred for 30 min then warmed to rt and stirred overnight. After the reaction was completed, the reaction mixture was poured into ice-water (30 mL). The mixture was extracted with DCM (30 mL × 4). The combined organic layers were dried over anydrous sodium sulfate, and filtered. The filtrate was concentrated *in vacuo,* and the residue was purified by thin-layer chromatography (DCM/CH₃OH(V/V)=20/1) to give the title compound as a white solid (50 mg, 0.11 mmol, 20 %).
MS (ESI, pos.ion) *m*/*z:* 458.1 [M+H]⁺;
¹H NMR (400 MHz, CDCl₃) δ 15.971 (br,1H), 8.460 (s, 1H), 7.428(s, 1H), 7.021 (s, 1H), 6.865 (s, 1H), 4.220 - 4.103 (m, 2H), 3.935 - 3.853 (m, 1H), 3.538 (t, *J=* 6.4, 2H), 3.403 - 3.370 (m, 4H), 3.188 - 3.111(m, 1H), 2.920 - 2.839 (m, 1H), 2.101 - 2.011 (s, 2H), 1.889 - 1.802 (m, 1H), 1.364 (d, J=6.0, 6H), 0.973 (d, J=6.8Hz, 3H), 0.839 (d, *J=* 6.8Hz, 3H).

### Example 28: 10-((cyclohexanecarbonyl)oxy)-6-isopropyl-9-(3-methoxypropoxy)-2-oxo-6,7 -dihydro-2H-pyrido[2,1-a]isoquinoline-3-carboxylic acid

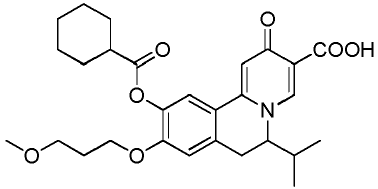

10-Hydroxy-6-isopropyl-9-(3-methoxypropoxy)-2-oxo-6,7-dihydro-2H-pyrido[2,1-a] isoquinoline-3-carboxylic acid (0.26 g, 0.67 mmol) was added into a 50 mL two-neck flask, then DCM (6 mL) was added. The mixture was cooled to 0 °C, and then cyclohexanecarbonyl chloride (0.45 mL, 3.36 mmol) and TEA (0.47 mL, 3.4 mmol) were added in turn. The mixture was stirred at this temperature for 30 min, then warmed to rt and stirred overnight. After the reaction was completed, the reaction mixture was poured into ice-water (20 g), and the mixture was extracted wtih DCM (30 mL × 3). The combined organic layers were concentrated *in vacuo.* The residue was purified by thin-layer chromatography (DCM/CH₃OH (V/V) = 20/1) to give the crude product, which was purified by recrystallization from methanol (6 mL) to give the title compound as a gray solid (100 mg, 0.2 mmol, 30%).
MS (ESI, pos.ion) *m*/*z:* 498.2 [M+H]⁺;
¹H NMR (400 MHz, CDCl₃) δ 15.997 (br,1H), 8.474 (s, 1H), 7.415(s, 1H), 7.022 (s, 1H), 6.864 (s, 1H), 4.197 - 4.129 (m, 2H), 3.966 - 3.862 (m, 1H), 3.589 - 3.794 (m, 2H), 3.456 - 3.313 (m, 4H), 3.200 - 3.096 (m, 1H), 2.691 - 2.597 (m, 1H), 2.085 - 2.037 (m, 3H), 1.869 - 1.848 (m, 3H), 1.767 - 1.719 (m, 2H), 1.666 - 1.608 (m, 3H), 1.441 - 1.380 (m,2H), 0.973 (d, *J* = 4.4Hz, 3H), 0.836 (d, *J* = 4.4Hz, 3H).

### Example 30: 10-acetyl-6-isopropyl-2-oxo-9-(pyrrolidin-1-yl)-6,7-dihydro-2H-pyrido[2,1-a] isoquinoline-3-carboxylic acid

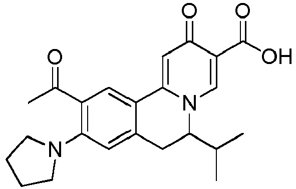

### Step 1: 1-(2-(benzyloxy)-4-bromophenyl)ethanone

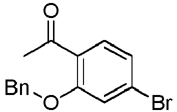

To a single-neck flask were added 1-(4-bromo-2-hydroxyphenyl)ethanone (20 g, 93 mmol), potassium carbonate (25.7 g, 186 mmol), acetonitrile (100 mL) and benzyl bromide (12.2 mL, 103 mmol). The reaction mixture was heated to 70 °C and stirred for 2 h, then cooled to rt, and filtered to remove the solid. The filtrate was concentrated *in vacuo* to give the title compound as a white solid (28 g, 99%).

### Step 2: 2-(2-(benzyloxy)-4-bromophenyl)-2-methyl-L3-dioxolane

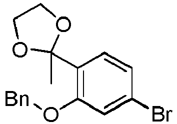

To a reaction flask were added 1-(2-(benzyloxy)-4-bromophenyl)ethanone (28.1 g, 92 mmol), ethanediol (10 mL, 184 mmol), cyclohexane (200 mL), triethyl orthoformate (31 mL, 190 mmol) and *p*-toluene sulfonic acid (1.8 g, 9.2 mmol). The reaction mixture was heated to 40 °C and stirred for 24 h, then concentrated *in vacuo.* The residue was diluted with saturated aqueous sodium bicarbonate solution (100 mL), then extracted with EA (200 mL ×2). The combined organic layers were washed with saturated aqueous sodium chloride solution (30 mL), dried over anhydrous sodium sulfate, and concentrated *in vacuo* to give the title compound as a light yellow oil (32 g, 99%).
MS (ESI, pos.ion) *m*/*z:* 371.1 [M+Na]⁺.

### Step 3: 1-(3-(benzyloxy)-4-(2-methyl-1,3-dioxolan-2-yl)phenyl)-3-methylbutan-2-one

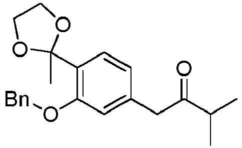

To a single-neck flask were added 2-(2-(benzyloxy)-4-bromophenyl) -2-methyl-1,3-dioxolane (32.0 g, 91.6 mmol), 3-methyl-butan-2-one (19.7 mL, 184 mmol), sodium *tert*-butoxide (17.6 g, 183 mmol), tetrahydrofuran (400 mL), tris(dibenzylideneacetone)dipalladium (3.76 g, 6.41 mmol) and XantPhos (3.83 g, 6.42 mmol). The reaction mixture was degassed and filled with nitrogen for three times, then heated to 60°C and stirred for 4 hours, then concentrated *in vacuo,* and the residue was purified by silica gel column chromatography (PE/EA (V/V) = 10/1) to give the title compound as a light yellow solid (23.34 g, 72%).
MS (ESI, pos.ion) *m*/*z:* 355.1 [M+H]⁺.

### Step 4: 1-(3-(benzyloxy)-4-(2-methyl-1,3-dioxolan-2-yl)phenyl)-3-methylbutan-2-amine

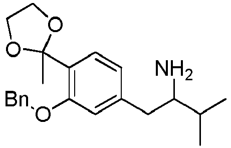

To a single-neck flask were added 1-(3-(benzyloxy)-4-(2-methyl-1,3-dioxolan-2-yl) phenyl)-3-methylbutan-2-one (30 g, 84.7 mmol), methanol (150 mL) and ammonium acetate (32.6 g, 423 mmol). The mixture was stirred at rt for 1 h under nitrogen protection, then NaBH₃CN (8 g, 130 mmol) was added at 0 °C. The resulting mixture was stirred at 0 °C for 24 h. After the reaction was completed, the mixture was concentrated *in vacuo,* and the residue was diluted with water (100 mL) and aqueous sodium hydroxide solution (30 mL, 10%), then extracted with EA (200 mL ×3). The combined organic layers were washed with saturated aqueous sodium chloride solution (50 mL), dried over anhydrous sodium sulfate, and concentrated *in vacuo* to give the title compound as colorless oil (30.0 g, 99.7%).
MS (ESI, pos.ion) *m*/*z:* 356.1 [M+H]⁺.

### Step 5: N-(1-(3-(benzyloxy)-4-(2-methyl-1,3-dioxolan-2-yl)phenyl)-3-methylbutan -2-yl)formamide

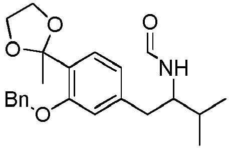

To a single-neck flask were added 1-(3-(benzyloxy)-4-(2-methyl-1,3 -dioxolan-2-yl)phenyl)-3-methylbutan-2-amine (0.69 g, 1.9 mmol) and ethyl formate (10 mL). The reaction mixture was heated to reflux and stirred for 12 hours, then concentrated *in vacuo,* and the residue was purified by silica gel column chromatography (PE/EA (V/V) = 1/1) to give the title compound as colorless oil (0.3 g, 40%).
MS (ESI, pos.ion) *m*/*z:* 384.4 [M+H]⁺.

### Step 6: 1-(6-(benzyloxy)-3-isopropyl-3,4-dihydroisoquinolin-7-yl)ethanone

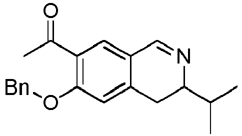

To a single-neck flask were added *N*-(1-(3-(benzyloxy)-4-(2-methyl-1,3-dioxolan -2-yl)phenyl)-3-methylbutan-2-yl)formamide (0.30 g, 0.78 mmol) and dichloromethane (5 mL). The mixture was cooled to 0 °C under a cold-bath condition, then phosphorus oxychloride (0.36 mL, 3.9 mmol) was added into the mixture. The reaction mixture was stirred at rt for 12 h, then concentrated *in vacuo,* and the residue was purified by silica gel column chromatography (DCM/MeOH(V/V)=20/1) to give the title compound as brown viscous product (0.20 g, 80%).
MS (ESI, pos.ion) *m*/*z:* 322.3 [M+H]⁺.

### Step 7: ethyl 10-acetyl-9-(benzyloxy)-6-isopropyl-2-oxo-2,6,7,11b-tetrahydro -1H-pyrido[2,1-a]isoquinoline-3-carboxylate

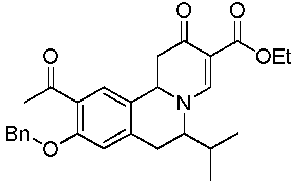

To a single-neck flask were added 1-(6-(benzyloxy)-3-isopropyl-3,4-dihydroisoquinolin-7-yl)ethanone (3.1 g, 9.6 mmol), ethyl 2-(ethoxymethylene)-3-oxobutanoate (3.6 g, 19.3 mmol) and ethanol (30 mL). The reaction mixture was heated to 90 °C and stirred for 12 h. After the reaction was completed, the reaction mixture was concentrated *in vacuo,* and the residue was purified by silica gel column chromatography (PE/EA (V/V) = 1/1) to give the title compound as a brown solid (4.0 g, 90%).
MS (ESI, pos.ion) *m*/*z:* 462.4 [M+H]⁺.

### Step 8: ethyl 10-acetyl-9-(benzyloxy)-6-isopropyl-2-oxo-6,7-dihydro-2H- pyrido[2,1-a] isoquinoline-3-carboxylate

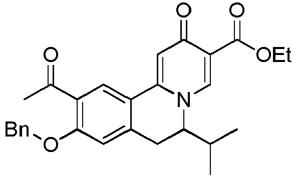

To a single-neck flask were added ethyl 10-acetyl-9-(benzyloxy)-6-isopropyl-2-oxo-2,6,7,11b-tetrahydro-1H-pyrido[2,1-*a*]isoquinoline-3 -carboxylate (4.50 g, 9.75 mmol), chloranil (4.53 g, 18.4 mmol) and dimethoxyethane (50 mL). The reaction mixture was stirred at 80 °C for 2 hours, then concentrated *in vacuo* to remove the solvent. The residue was purified by silica gel column chromatography (DCM/MeOH(V/V)=10/1) to give the title compound as a gray solid (3.9 g, 87%).
MS (ESI, pos.ion) *m*/*z:* 460.1 [M+H]⁺.

### Step 9: ethyl 1 0-acetyl-9-hydroxy-6-isopropyl-2-oxo-6, 7 -dihydro-2H-pyridor2, 1-a lisoquinoline -3-carboxylate

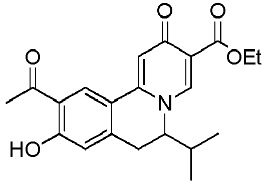

To a single-neck flask were added ethyl 10-acetyl-9-(benzyloxy)-6-isopropyl-2-oxo-6,7-dihydro-2*H*-pyrido[2,1-*a*]isoquinoline-3-carboxy late (3.1 g) and methanol (30 mL). The mixture was degassed and filled with hydrogen for three times, then stirred at rt for 8 h equiped with a hydrogen balloon. The mixture was filtered through a celite pad to remove Pd/C, and the filtrate was concentrated *in vacuo.* The residue was purified by silica gel column chromatography (DCM/MeOH(V/V)=10/1) to give the title compound as a brownish black solid (2.7 g, 86%).
MS (ESI, pos.ion) *m*/*z:* 370.1 [M+H]⁺.

### Step 10: ethyl 10-acetyl-6-isopropyl-2-oxo-9-(((trifluoromethyl(sulfonyl)oxy)-6,7-dihydro -2H-pyrido[2,1-a]isoquinoline-3-carboxylate

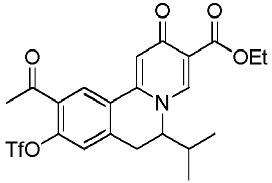

To a two-neck flask were added DCM (10 mL), ethyl 10-acetyl-9-hydroxy-6-isopropyl-2-oxo-6,7-dihydro-2*H*-pyrido[2,1-*a*]isoquinoline-3-carboxylate (0.50 g, 1.4 mmol) and TEA (0.38 mL, 2.7 mmol). The reaction mixture was cooled to 0 °C, then N phenyl-bis(trifluoromethanesulfonimide) (0.53 g, 1.5 mmol) was added in portions. Then the mixture was stirred at rt for 12 h, and concentrated *in vacuo.* The residue was purified by silica gel column chromatography (DCM/MeOH(V/V)=10/1) to give the title compound as a yellow solid (0.65 g, 95%).
MS (ESI, pos.ion) *m*/*z:* 502.1 [M+ H]⁺.

### Step 11: ethyl 1 0-acetyl-6-isopropyl-2-oxo-9-(pyrrolidin-1-yl)-6, 7 -dihydro-2H-pyrido[2,1-a]isoquinoline-3-carboxylate

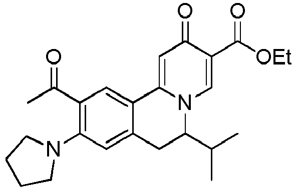

To a single-neck flask were added ethyl 10-acetyl-6-isopropyl-2-oxo-9-(((trifluoromethyl)sulfonyl)oxy)-6,7-dihydro-2H-pyrido[2,1-a]iso quinoline-3-carboxylate (650 mg, 1.3 mmol), cesium carbonate (1.056 g, 3.2 mmol), toluene (10 mL), tertiary butanol (2 mL), tetrahydropyrrole (0.163 mL, 1.94 mmol), X-PHOS (0.16 g, 0.33 mmol) and palladium acetate (30 mg, 0.13 mmol). The reaction mixture was degassed and filled with nitrogen for 4 times, then heated to 90 °C and stirred for 12 h, and then concentrated *in vacuo.* The residue was diluted with water (10 mL), then the mixture was extracted with dichloromethane (20 mL ×2). The combined organic layers were washed with saturated aqueous sodium chloride solution (20 mL), dried over anydrous sodium sulfate, and filtered. The filtrate was concentrated *in vacuo,* and the residue was purified by thin-layer chromatography (DCM/MeOH (V/V) = 20/1) to give the title compound as a yellow solid (0.320 g, 58.4%).
MS (ESI, pos.ion) *m*/*z:* 423.2 [M+H]⁺.

### Step 12: 10-acetyl-6-isopropyl-2-oxo-9-(pyrrolidin-1-yl)-6,7-dihydro-2H-pyrido[2,1-a] isoquinoline-3-carboxylic acid

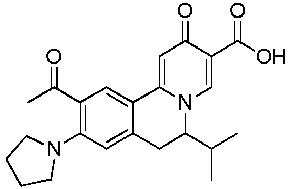

To a single-neck flask were added ethyl 10-acetyl-6-isopropyl-2-oxo-9-(pyrrolidin-l-yl)-6,7-dihydro-2J7-pyrido[2,l-a]isoquinoline-3-car boxylate (0.23 g, 0.544 mmol), lithium hydroxide monohydrate (0.069 g, 1.6 mmol), methanol (1 mL) and THF (1 mL). The reaction mixture was stirred at rt for 5 h, then hydrochloric acid (1 M) was added to adjust the pH to 5. The mixture was filtered to give the title compound as a yellow solid (0.16 g 77%).
MS (ESI, pos.ion) *m*/*z:* 395.20 [M+H]⁺;
¹H NMR (400 MHz, DMSO-*d*₆) δ 8.72 (s, 1H), 8.16 (s, 1H), 7.42 (s, 1H), 6.83 (s, 1H), 4.43 (dd, *J* = 9.3, 3.6 Hz, 1H), 3.29 (d, *J* = 4.5 Hz, 2H), 3.11 (d, *J* = 3.3 Hz, 4H), 2.68 (s, 3H), 1.90 (s, 4H), 1.65 - 1.54 (m, 1H), 0.89 (d, *J* = 6.6 Hz, 3H), 0.69 (d, *J* = 6.7 Hz, 3H).

### Example 31: 10-acetyl-9-(furan-2-yl)-6-isopropyl-2-oxo-6,7-dihydro-2H-pyrido[2,1-a] isoquinoline-3-carboxylic acid

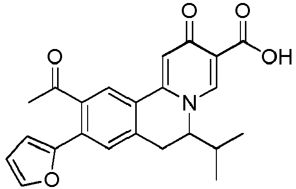

### Step 1: ethyl 10-acetyl-9-(furan-2-yl)-6-isopropyl-2-oxo-6,7-dihydro-2H-pyrido[2,1-a] isoquinoline-3-carboxylate

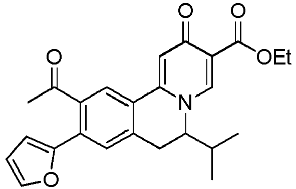

To a single-neck flask were added ethyl 10-acetyl-6-isopropyl-2-oxo-9-(((trifluoromethyl)sulfonyl)oxy)-6,7-dihydro-2H-pyrido[2,1-a]iso quinoline-3-carboxylate (250 mg, 0.50 mmol), potassium carbonate (0.132 g, 1.25 mmol), dioxane (5 mL), ethanol (2 mL), 2-furanboric acid (0.084 g, 0.75 mmol) and tetrakispalladium (58 mg, 0.05 mmol). The reaction mixture was degassed and filled with nitrogen for 4 times, then heated to 90 °C and stirred for 2 h, and then concentrated *in vacuo.* The residue was diluted with water (10 mL), then the mixture was extracted with dichloromethane (20 mL ×2). The combined organic layers were washed with saturated aqueous sodium chloride solution (20 mL), dried over anydrous sodium sulfate, and filtered. The filtrate was concentrated *in vacuo,* and the residue was purified by thin-layer chromatography (DCM/MeOH (V/V) = 20/1) to give the title compound as a yellow solid (0.20 g, 96%).
MS (ESI, pos.ion) *m*/*z:* 423.2 [M+H]⁺.

### Step 2: 1 0-acetyl-9-(furan-2-yl)-6-isopropyl-2-oxo-6, 7 -dihydro-2H-pyridor2, 1-a lisoquinoline -3-carboxylic acid

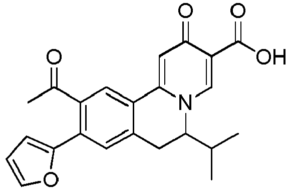

To a single-neck flask were added ethyl 10-acetyl-9-(furan-2-yl)-6-isopropyl-2-oxo-6,7-dihydro-2H-pyrido[2,1-a]isoquinoline-3-carboxy late (0.23 g, 0.55 mmol), lithium hydroxide monohydrate (0.069 g, 1.6 mmol) and methanol (1 mL). The reaction mixture was stirred at rt for 5 h, then hydrochloric acid (1 M) was added to adjust the pH to 5. The mixture was filtered to give the title compound as a yellow solid (0.163 g, 76%).
MS (ESI, pos.ion) *m*/*z:* 392.1497 [M+ H]⁺;
¹H NMR (400 MHz, CDCl₃) δ 15.73 (s, 1H), 8.50 (s, 1H), 7.78 (s, 1H), 7.59 (d, *J* = 1.2 Hz, 1H), 7.56 (s, 1H), 7.20 (s, 1H), 6.75 (d, *J* = 3.3 Hz, 1H), 6.58 (dd, *J* = 3.4, 1.8 Hz, 1H), 3.96 (dd, *J* = 9.7, 4.2 Hz, 1H), 3.43 (dd, *J* = 16.4, 4.7 Hz, 1H), 3.28 (d, *J* = 16.3 Hz, 1H), 2.37 (s, 3H), 1.77 (qd, *J* = 13.4, 6.7 Hz, 1H), 0.97 (d, *J* = 6.6 Hz, 3H), 0.85 (d, *J* = 6.7 Hz, 3H).

### Example 32: 9-(furan-2-yl)-6-isopropyl-10-(methoxycarbonyl)-2-oxo-6,7-dihydro-2H-pyrido[2,1-a]isoquinoline-3-carboxylic acid

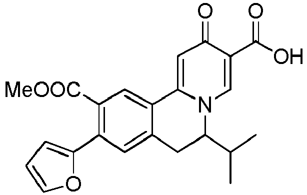

### Step 1: methyl 2-bromo-4-iodobenzoate

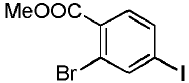

To a 250 mL single-neck flask were added 2-bromo-4-iodobenzoic acid (20.0 g, 61.2 mmol), DMF (100 mL) and K₂CO₃ (16.89 g, 122.4 mmol), then CH₃I (5.71 mL, 91.7 mmol) was added. The reaction mixture was stirred at rt overnight, then diluted with water (100 mL).The mixture was extracted with EA (100 mL ×4). The combined organic layers were washed with saturated brine (50 mL × 3), and concentrated *in vacuo* to give the title compound as black brown oil (20.6 g, 60.4 mmol, 98.8%), which was directly used in the next step.
MS (ESI, pos.ion) *m*/*z:* 341.0 [M+H]⁺.

### Step 2: 2-bromo-4-(3-methyl-2-oxobutyl)benzoic acid

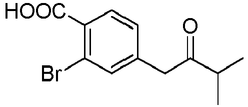

To a 500 mL two-neck flask were added methyl 2-bromo-4-iodobenzoate (20.6 g, 60.4 mmol), 3-methylbutan-2-one (10.4 g, 121 mmol), Pd(dba)₂ (869 mg, 1.5113 mmol), XantPHOS (1.4 g, 2.4 mmol), sodium tert-butoxide (19.9 g, 207 mmol) and dixoane (200 mL). The mixture was stirred at 90 °C overnight under nitrogen protection. After the reaction was completed, the reaction mixture was cooled to rt, and diluted with water (100 mL) and EA (100 mL) in turn, then partitoned. The organic layer was washed with water (50 mL ×3). The combined aqueous layers were adjusted with HCl (1 M) to pH 3-4, then extracted with EA (100 mL × 4). The combined organic layers were concentrated *in vacuo.* The residue was purified by silica gel column chromatography (EA) to give the title compound as brown oil (17.23 g, 60.43 mmol, 100%).
MS (ESI, pos.ion) *m*/*z:* 285.0 [M+H]⁺.

### Step 3: methyl 2-bromo-4-(3-methyl-2-oxobutyl)benzoate

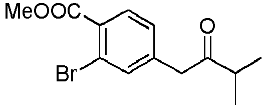

2-Bromo-4-(3-methyl-2-oxobutyl)benzoic acid (17.23 g, 60.43 mmol) was dissolved in MeOH (200 mL), then the solution was stirred and cooled to 0 °C, and thionyl chloride (43.8 mL, 604 mmol) was added. The mixture was heated to 70 °C and stirred for 8 h, then cooled natually to rt and stirred overnight. After the reaction was completed, the mixture was concentrated *in vacuo,* and to the residue was added EA (100 mL) and water (100 mL). The mixture was partitioned, and the aqueous layer was extracted with ethyl acetate (100 mL × 3). The combined organic layers were concentrated *in vacuo,* and the residue was purified by silica gel column chromatography (PE/EA (V/V) = 20/1) to give the title compound as yellow oil (13.9 g, 46.5 mmol, 76.9%).
MS (ESI, pos.ion) *m*/*z:* 299.0 [M+H]⁺.

### Step 4: methyl 4-(2-amino-3-methylbutyl)-2-bromobenzoate

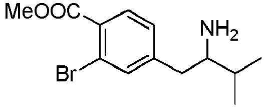

To a 500 mL single-neck flask were added methyl 2-bromo-4-(3-methyl-2-oxobutyl)benzoate (13.9 g, 46.5 mmol), MeOH (150 mL) and NH₄OAc (35.8 g, 464 mmol). The mixture was stirred at rt for 1 h, then cooled to 0 °C and NaBH₃CN (8.76 g, 139 mmol) was added. Then the mixture was warmed to rt and stirred ovenight. After the reaction was completed, the mixture was concentrated *in vacuo,* and to the residue was added water (100 mL), EA (100 mL) and ammonium hydroxide (3 mL). The mixture was partitioned, and the aqueous layer was extracted with ethyl acetate (100 mL × 3). The combined organic layers were concentrated *in vacuo* to give the title compound as brown oil (13.95 g, 46.47 mmol, 100%), which was directly used in the next step.
MS (ESI, pos.ion) *m*/*z:* 300.1 [M+H]⁺.

### Step 5: methyl 2-bromo-4-(2-formamido-3-methylbutyl)benzoate

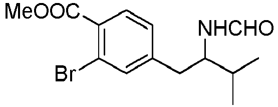

To a 500 mL single-neck flask were added methyl 4-(2-amino-3-methylbutyl)-2-bromobenzoate (13.95 g, 46.47 mmol) and ethyl formate (140 mL). The mixture was stirred at 70 °C overnight, then concentrated *in vacuo,* and the residue was purified by silica gel column chromatography (PE/EA (V/V) = 1/1) to give the title compound as a white solid (10.0 g, 30.5 mmol, 65.6%).
MS (ESI, pos.ion) *m*/*z:* 351.9 [M+Na]⁺.

### Step 6: methyl 8-bromo-5-isopropyl-2,3-dioxo-3,5,6,10b-tetrahydro-2H -oxazolo[2,3-a]isoquinoline-9-carboxylate

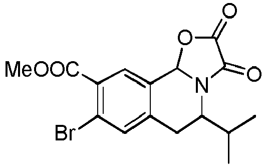

To a 50 mL single-neck flask were added methyl 2-bromo-4-(2-formamido-3-methylbutyl)benzoate (0.256 g, 0.780 mmol) and DCM (10 mL). The mixture was cooled to 0 °C, then oxalyl chloride (0.149 g, 1.17 mmol) was added. The reaction mixture was stirred at rt for 1 h, then cooled to -10 °C, and FeCl₃ (201 mg, 1.2497 mmol) was added. The reaction mixture was stirred at rt overnight. Then to the mixture was added HCl (10 mL, 2 M), and the mixture was stirred for 30 min. The mixture was extracted with DCM (15 mL × 3), and the combined organic layers were concentrated *in vacuo* to give the title compound as brownness oil (0.29 g, 0.76 mmol, 97.5%), which was used derectly in the next step.
MS (ESI, pos.ion) *m*/*z:* 382.1 [M+Na]⁺.

### Step 7: methyl 6-bromo-3-isopropyl-3,4-dihydroisoquinoline-7-carboxylate

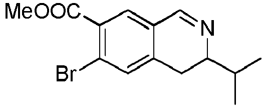

Methyl 8-bromo-5-isopropyl-2,3-dioxo-3,5,6,10b-tetrahydro-2*H*-oxazolo[2,3-*a*] isoquinoline-9-carboxylate (0.29 g, 0.76 mmol) was dissolved in MeOH (10 mL), then concentrated H₂SO₄ (0.5 mL) was added. The mixture was heated to 70 °C and stirred for 5 h. After the reaction was completed, the reaction mixture was cooled to rt and concentrated *in vacuo.* The residue was diluted with EA (20 mL), and the mixture was partitioned. The organic layer was washed with HCl (20 mL × 3, 2 M), and the combined organic layers were cooled to 0 °C, adjusted with ammonium hydroxide to pH 9-10. The resulting mixture was extracted with EA (30 mL × 4), and the combined organic layers were concentrated *in vacuo.* The residue was purified by thin-layer chromatography (PE/EA (V/V) = 1/2) to give the title compound as light yellow oil (0.12 g, 0.39 mmol, 51%).
MS (ESI, pos.ion) *m*/*z:* 310.0 [M+H]⁺.

### Step 8: methyl 6-(furan-2-yl)-3-isopropyl-3,4-dihydroisoquinoline-7-carboxylate

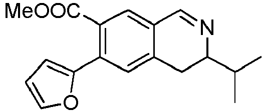

To 50 mL two-neck flask were added methyl 6-bromo-3-isopropyl-3,4-dihydroisoquinoline-7-carboxylate (0.273 g, 0.880 mmol), 2-furanboric acid (0.148 g, 1.32 mmol), tetrakis(triphenylphosphine)palladium (102 mg, 0.0884 mmol), H₂O (2 mL), K₂CO₃ (0.364 g, 2.64 mmol) and dioxane (10 mL). The mixture was heated to 100 °C and stirred for 5 h under nitrogen protection. After the reaction was completed, the mixture was cooled to rt, and to the mixture were added saturated brine (20 mL) and EA (30 mL). The mixture was partitoned, and the aqueous layer was extracted with ethyl acetate (30 mL × 3). The combined organic layers were concentrated *in vacuo,* and the residue was purified by silica gel column chromatography (PE/EA (V/V) = 1/1) to give the title compound as light yellow oil (0.260 g, 0.874 mmol, 99.3%).
MS (ESI, pos.ion) *m*/*z:* 298.2 [M+H]⁺.

### Step 9: 3-tert-butyl 10-methyl 9-(furan-2-yl)-6-isopropyl-2-oxo-2,6,7,11b-tetrahydro-1H -pyrido[2,1-a]isoquinoline-3,10-dicarboxylate

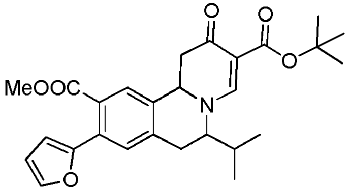

*Tert*-butyl 2-((dimethylamino)methylene)-3-oxobutanoate (367 mg, 1.7208 mmol), t-BuOH (15 mL) and methyl 6-(furan-2-yl)-3-isopropyl-3,4-dihydroisoquinoline-7-carboxylate (260 mg, 0.8742 mmol) were added into a 100 mL single-neck flask. The reaction mixture was heated to 100 °C and stirred for 5 h, then the reaction was stopped. The reaction mixture was cooled to rt and concentrated *in vacuo,* and the residue was purified by thin-layer chromatography (PE/acetone (V/V) = 2/1) to give the title compound as brown oil (0.407 g, 0.874 mmol, 100%).
MS (ESI, pos.ion) *m*/*z:* 466.3 [M+H]⁺.

### Step 10: 9-(furan-2-yl)-6-isopropyl-10-(methoxycarbonyl)-2-oxo-6,7-dihydro-2H -pyrido[2,1-a]isoquinoline-3-carboxylic acid

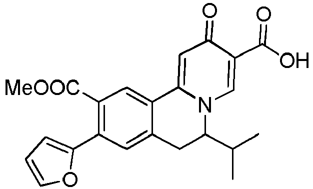

To a 100 mL single-neck flask were added 3-*tert*-butyl 10-methyl 9-(furan-2-yl)-6-isopropyl-2-oxo-2,6,7,11b-tetrahydro-1*H*-pyrido[2,1-*a*]isoquinoline-3,10-dicarb oxylate (0.41 g, 0.87 mmol), dimethoxyethane (15 mL) and chloranil (322 mg, 1.31 mmol). The reaction mixture was stirred at 90 °C for 6 hours, then concentrated *in vacuo* to remove the solvent. The residue was purified by silica gel column chromatography (DCM/MeOH(V/V)=10/1) to give the title compound as a white solid (108 mg, 30.3%).
MS (ESI, pos.ion) *m*/*z:* 408.1 [M+H]⁺;
¹H NMR (400 MHz, DMSO-*d*₆) δ 8.86 (s, 1H), 8.29 (s, 1H), 7.87 (s, 2H), 7.52 (s, 1H), 6.95 (s, 1H), 6.68 (s, 1H), 4.61 - 4.53 (m, 1H), 3.85 (s, 3H), 3.51 - 3.42 (m, 2H), 1.64 - 1.56 (m, 1H), 0.93 - 0.69 (m, 6H).

### Example 33: 6-isopropyl-9-(3-methoxypropoxy)-2-oxo-10-(thiazol-5-yl)-6,7-dihydro -2H-pyrido[2,1-a]isoquinoline-3-carboxylic acid

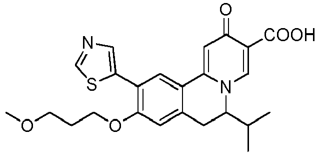

To a 25 mL single-neck flask were added ethyl 6-isopropyl-9-(3-methoxyphenoxy)-2-oxo-10-(((trifluoromethyl)sulfonyl)oxy)-6,7-dihydro-2*H*-p yrido[2,1-*a*]isoquinoline-3-carboxylate (0.310 g, 0.570 mmol), 5-(tributylstannyl)thiazole (198 mg, 0.53 mmol), anhydrous dioxane (10 mL) and bis(triphenylphosphine)palladium(II) chloride (99 mg, 0.14 mmol). The reaction mixture was stirred at 110 °C for 12 hours under a nitrogen protection, then concentrated *in vacuo,* and the residue was purified by silica gel column chromatography (DCM/MeOH(V/V)=15/1) to give the title compound as a gray solid (40 mg, 0.566 mmol, 16%).
MS (ESI, pos.ion) *m*/*z:* 455.3 [M+H]⁺;
¹H NMR (400 MHz, CDCl₃) δ 15.942 (br, 1H) , 8.878 (s, 1H), 8.493 (s, 1H), 8.341 (s, 1H), 8.003 (s, 1H), 7.171 (s, 1H), 6.946 (s, 1H), 4.375 - 4.265 (m, 2H), 3.959 - 3.906 (m, 1H), 3.679 - 3.596 (m, 2H), 3.464 - 3.410Cm, 1H),3.387 (s, 3H), 3.246 - 3.158 (m,1H), 2.250 - 2.191 (m, 2H), 1.882 - 1.8102 (m, 1H), 0.996 (d, *J*=6.4Hz, 3H), 0.872 (d, *J*=6.8Hz,3H).

### Example 35: 10-((2,2-difluoroethoxy)carbonyl)-6-isopropyl-9-(3-methoxypropoxy)-2 -oxo-6,7-dihydro-2H-pyrido[2,1-a]isoquinoline-3-carboxylic acid

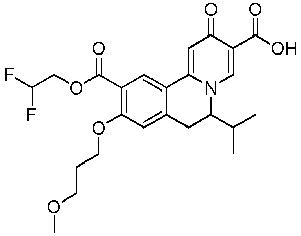

The title compound was prepared according to the synthetic method of step 1 to step 2 in example 3 by using 3-(*tert*-butoxycarbonyl)-6-isopropyl-9-(3-methoxypropoxy) -2-oxo-6,7-dihydro-2*H*-pyrido[2,1-*a*]isoquinoline-10-carboxylic acid (0.350 g, 0.742 mmol) and 2-bromo-1,1-difluoroethane (0.430 g, 2.97 mmol) as raw materials to give a gray solid (0.10 g, 0.21 mmol).
MS (ESI, pos.ion) *m*/*z:* 480.1 [M+H]⁺;
¹H NMR (400 MHz, CDCl₃) δ 15.929 (b, 1H), 8.502 (s, 1H), 8.286 (s, 1H), 7.151 (s, 1H), 6.944 (s, 1H), 6.252 - 5.958 (m,1H), 4.581 - 4.503 (m, 2H), 4.298 - 4.203 (m, 2H), 4.032 - 3.943 (m,1H), 3.670 - 3.586 (m, 2H), 3.502 - 3.422(m, 1H), 3.380 (s, 3H), 3.260 - 3.177 (m,1H), 2.181 - 2.121 (m, 2H), 1.809 - 1.722 (m, 1H), 0.974 (d, *J* = 6.8Hz,3H), 0.849(d, *J* = 6.8Hz,3H).

### Example 36: 6-isopropyl-9-(3-methoxypropoxy)-2-oxo-10-((prop-2-yn-1-yloxy)carbonyl) -6,7-dihydro-2H-pyrido[2,1-a]isoquinoline-3-carboxylic acid

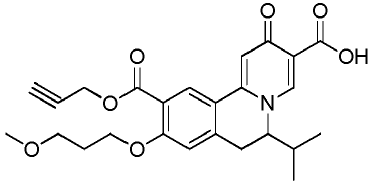

The title compound was prepared according to the synthetic method of step 1 to step 2 in example 3 by using 3-(*tert-*butoxycarbonyl)-6-isopropyl-9-(3-methoxypropoxy) -2-oxo-6,7-dihydro-2*H*-pyrido[2,1-*a*]isoquinoline-10-carboxylic acid (0.31 g, 0.66 mmol) and propargyl bromide (0.31 g, 2.62 mmol) as raw materials to give a gray solid (80 mg, 0.18 mmol).
MS(ESI, pos.ion) *m*/*z:* 454.2 [M+H]⁺;
¹H NMR (400 MHz, CDCl₃) δ 15.915 (b, 1H), 8.473 (s, 1H), 8.289 (s, 1H), 7.164 (s, 1H), 6.924 (s, 1H), 4.946 (d, *J* = 2Hz, 2H), 4.293 - 4.199 (m, 2H), 3.981 - 3.902 (m,1H), 3.696 - 3.612 (m, 2H), 3.450 - 3.385 (m, 4H), 3.245 - 3.168 (m,1H), 2.562 (s, 1H), 2.188 - 2.130 (m, 2H), 1.808 - 1.725 (m, 2H), 0.963 (d, *J* = 6.8Hz,3H), 0.848 (d, *J* = 6.8Hz, 3H).

### Example 39: 6-isopropyl-9-(3-methoxypropoxy)-2-oxo-10-(((1,1,1-trifluoro-2-methylpropan -2-yl)oxy)carbonyl)-6,7-dihydro-2H-pyrido[2,1-a]isoquinoline-3-carboxylic acid

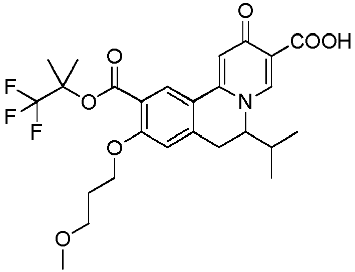

### Step 1: 1,1, 1-trifluoro-2-methylpropan-2-yl 2-(3-methoxypropoxy)-4-(3-methyl-2-oxobutyl) benzoate

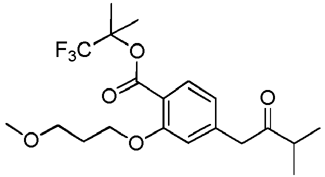

To a 100 mL single-neck flask were added 2-(3-methoxypropoxy)-4-(3-methyl-2-oxobutyl)benzoic acid (2.00 g, 6.80 mmol), 1,1,1-trifluoro-2-methylpropan-2-ol (1.3 g, 10 mmol), DCM (5 mL) and SOCl₂ (0.64 mL, 8.8 mmol). The mixture was heated to 50 °C and stirred overnight. The reaction mixture was cooled to rt, poured into ice-water (20 g), then extracted with DCM (30 mL × 3). The combined organic layers were concentrated *in vacuo* to give the title compound as colorless oil (2.75 g, 6.80 mmol, 100%).
MS (ESI, pos.ion) *m*/*z:* 427.3 [M+ Na]⁺.

### Step 2: 1,1,1-trifluoro-2-methylpropan-2-yl 4-(2-amino-3-methylbutyl)-2-(3-methoxypropoxy) benzoate

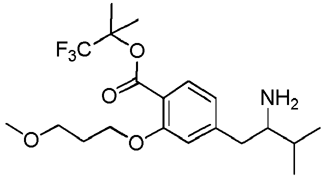

To a 100 mL single-neck flask were added 1,1,1-trifluoro-2-methylpropan-2-yl 2-(3-methoxypropoxy)-4-(3-methyl-2-oxobutyl)benzoate (4.125 g, 10.20 mmol), MeOH (41 mL) and NH₄OAc (9.435 g, 122.4 mmol). The mixture was stirred at rt for 1 h. The mixture was cooled to 0 °C, and NaBH₃CN (1.923 g, 30.60 mmol) was added in portions, then the mixture was heated to rt and stirred overnight. The reaction mixture was worked up by concentrating *in vacuo* to remove the solvent, and the residue was diluted with saturated aqueous ammonium chloride (20 mL); the mixture was extracted with EA (50 mL × 4), and the combined organic layers were dried over anhydrous sodium sulfate, and filtered; the filtrate was concentrated *in vacuo* to give the title compound as colorless oil (4.135 g, 10.20 mmol, 100.0%), which was directly used in the next step.
MS (ESI, pos.ion) *m*/*z:* 406.0 [M+1]⁺.

### Step 3: 1,1,1-trifluoro-2-methylpropan-2-yl 4-(2-formamido-3-methylbutyl)-2-(3 -methoxypropoxy)benzoate

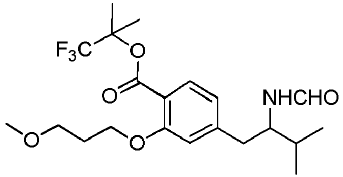

To a 100 mL single-neck flask were added 1,1,1-trifluoro-2-methylpropan-2-yl 4-(2-amino-3-methylbutyl)-2-(3-methoxypropoxy)benzoate (4.13 g, 10.20 mmol), dioxane (28 mL) and formic acid (7.51 g, 163.2 mmol). The mixture was heated to 110 °C and stirred overnight. The mixture was worked up by cooling to rt and concentrating *in vacuo,* to the residue were added EA (100 mL) and water (50 mL); the mixture was extracted with ethyl acetate (100 mL × 3); the combined organic layers were concentrated *in vacuo,* and the residue was purified by silica gel column chromatography (PE/EA (V/V) = 1/1) to give the title compound as a brown thickness product (2.3 g, 5.3 mmol, 52%).
MS (ESI, pos.ion) *m*/*z:* 434.3 [M+1]⁺.

### Step 4: 1,1,1-trifluoro-2-methylpropan-2-yl 3-isopropyl-6-(3-methoxypropoxy) -3,4-dihydroisoquinoline-7-carboxylate

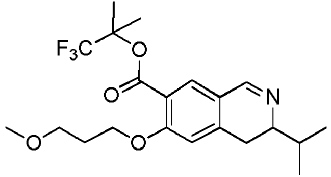

To a 50 mL single-neck flask were added 1,1,1-trifluoro-2-methylpropan-2-yl 4-(2-amino-3-methylbutyl)-2-(3-methoxypropoxy)benzoate (2.3 g, 5.3 mmol), then DCM (23 mL) was added. The mixture was cooled to 0 °C and POCl₃ (0.99 mL, 11 mmol) was added. The mixture was heated to 50 °C and stirred for 4 h. The mixture was worked up by cooling to rt and pouring into ice-water (30 g); the mixture was adjusted with ammonium hydroxide to pH = 10, then the resulting mixture was extracted with EA (30 mL × 3); the combined organic layers were washed with saturated brine (10 mL), dried over anhydrous sodium sulfate and filtered; the filtrate was concentrated *in vacuo,* and the residue was purified by silca gel column chromatography (EA) to give the title comopound as brown oil (1.79 g, 4.31 mmol, 81%).
MS (ESI, pos.ion) *m*/*z:* 416.3 [M+1]⁺.

### Step 5: 3-tert-butyl 10-(1,1,1-trifluoro-2-methylpropan-2-yl) 6-isopro -9-(3-methoxypropoxy)-2-oxo-2,6,7,11b-tetrahydro-1H-pyrido[2,1-a]isoquinoline-3,10-dicarbox ylate

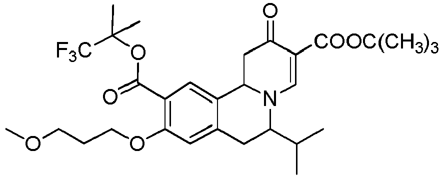

To a 100 mL two-neck flask were added 1,1,1-trifluoro-2-methylpropan-2-yl 3-isopropyl-6-(3-methoxypropoxy)-3,4-dihydroisoquinoline-7-carboxylate (1.79 g, 4.31 mmol) and *tert*-butyl (2*E*)-2-((dimethylamino)methylene)-3-oxobutanoate (1.84 g, 8.62 mmol). Then to the mixture was added *t*-BuOH (5 mL), and the mixture was stirred at 90 °C under nitrogen protection for 48 h. The mixture was worked up by concentrating *in vacuo,* and the residue was purified by silica gel column chromatography (DCM/MeOH (V/V) = 10/1) to give the title compound as black oil (2.2 g, 3.8 mmol, 87%).
MS (ESI, pos.ion) *m*/*z:* 584.4 [M+1]⁺:.

### Step 6: 6-isopropyl-9-(3-methoxypropoxy)-2-oxo-10-(((1,1,1-trifluoro-2-methylpropan -2-yl)oxy)carbonyl)-6,7-dihydro-2H-pyrido[2,1-a]isoquinoline-3-carboxylic acid

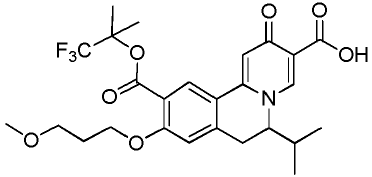

To a 100 mL single-neck flask were added 3-*tert*-butyl 10-(1,1,1-trifluoro-2-methylpropan-2-yl)6-isopropyl-9-(3-methoxypropoxy)-2-oxo-2,6,7,11b-tetrahydro-1*H*-pyrido[2,1-*a*]isoquinoline-3,10-dicarboxylate (0.23 g, 0.39 mmol), DME (6 mL) and chloranil (0.85 mg, 0.39 mmol). The mixture was heated to 90 °C and stirred for 1 h, then cooled to rt, and concentrated *in vacuo.* The residue was purified by silica gel column chromatography (DCM/MeOH (V/V) = 10/1) to give the title compound as a gray solid (150 mg, 0.2854 mmol, 72%).
MS (ESI, pos.ion) *m*/*z:* 525.9 [M+1]⁺;
¹H NMR (400 MHz, CDCl₃) δ 15.971 (br, 1H), 8.490 (s, 1H), 8.186(s, 1H), 7.283 (s, 1H), 6.921 (s, 1H), 4.276 - 4.180 (m, 2H), 4.025 - 3.919 (m, 1H), 3.669 - 3.552 (m,2H), 3.471 - 3.332 (m, 4H), 3.256 - 3.145 (m,1H), 2.195 - 2.119 (m, 2H), 1.846 (s, 6H), 1.806 - 1.739 (m, 1H), 0.969 (d, *J=* 6.4Hz, 3H), 0.847 (d, *J=* 6.4Hz, 3H).

### Example 40: 6-isopropyl-10-(methoxycarbonyl)-2-oxo-9-phenoxy-6,7-dihydro-2H -pyrido[2,1-a]isoquinoline-3-carboxylic acid

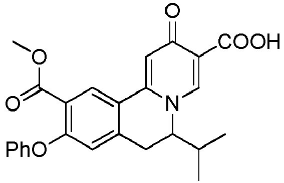

### Step 1: 2-(4-bromo-2-fluorophenyl)-1,3-dioxolane

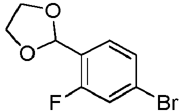

To a 100 mL single-neck flask were added 4-bromo-2-fluorobenzaldehyde (4.71 g, 23.21 mmol), triethyl orthoformate (6.88 g, 46.4 mmol), p-toluenesulfonic acid monohydrate (0.88 g, 4.64 mmol), ethanediol (2.88 g, 46.42 mmol) and n-hexane (50 mL). The mixture was heated to 65 °C and stirred overnight under nitrogen protection. The mixture was worked up by cooling to rt, then water (30 mL) was added; the resulting mixture was extracted with petroleum ether (20 mL × 3), and the combined organic layers were washed with water (10 mL × 2), concentrated *in vacuo;* the residue was purified by silica gel column chromatography (PE/EA (V/V) = 30/1) to give the title compound as colorless oil (2.24 g, 9.07 mmol, 39.1%).
¹H NMR (400Hz, CDCl₃) δ 7.451 - 7.412 (m, 1H), 7.323 (d, *J* =8.4Hz, 1H), 7.277 (d, *J* = 9.6Hz, 1H), 6.047 (s, 1H), 4.170 - 4.111 (m, 2H), 4.085 - 4.007 (m, 2H).

### Step 2: 1-(4-(1,3-dioxolan-2-yl)-3-fluorophenyl)-3-methylbutan-2-one

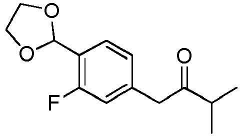

To a 50 mL two-neck flask were added 3-methylbutan-2-one (1.19 g, 13.79 mmol), 2-(4-bromo-2-fluorophenyl)-1,3-dioxolane (1.12 g, 4.60 mmol), Pd(dba)₂ (0.26 g, 0.46 mmol), Xantphos (0.40 g, 0.69 mmol), sodium tert-butoxide (1.33 g, 13.80 mmol) and dioxane (12 mL). The mixture was stirred at 90 °C for 4 h under nitrogen protection. The mixture was cooled to rt, and to the mixture were added EA (50 mL) and water (30 mL). The mixture was partitioned, and the aqueous layer was extracted with ethyl acetate (30 mL × 3). The combined organic layers were concentrated *in vacuo,* and the residue was purified by silica gel column chromatography (EA) to give the title compound as light yellow oil (1.16 g, 4.60 mmol, 100%).
MS (ESI, pos.ion) *m*/*z:* 253.1 [M+H]⁺.

### Step 3: 1-(4-(1,3-dioxolan-2-yl)-3-fluorophenyl)-3-methylbutan-2-amine

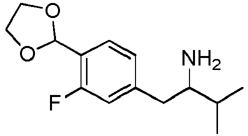

To a 250 mL single-neck flask were added 1-(4-(1,3-dioxolan-2-yl)-3-fluorophenyl)-3-methylbutan-2-one (1.18 g, 4.68 mmol), MeOH (12 mL) and NH₄OAc (4.33 g, 56.2 mmol). The mixture was stirred at rt for 60 min, then cooled to 0 °C and NaBH₃CN (0.882 g, 14.0 mmol) was added. Then the mixture was warmed to rt and stirred ovenight. The mixture was concentrated, and to the residue was added water (30 mL). The mixture was extracted with EA (50 mL × 4). The combined organic layers were concentrated to give the title compound as light yellow oil (1.185 g, 4.678 mmol, 100%), which was used directly in the next step.
MS (ESI, pos.ion) *m*/*z:* 254.3 [M+H]⁺.

### Step 4: N-(1-(4-(1,3-dioxolan-2-yl)-3-fluorophenyl)-3-methylbutan-2-yl)formamide

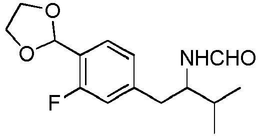

To a 100 mL single-neck flask were added 1-(4-(1,3-dioxolan-2-yl)-3-fluorophenyl)-3-methylbutan-2-amine (1.19 g, 4.68 mmol) and ethyl formate (30 mL). The mixture was heated to 70 °C and stirred for 48 h. The mixture was cooled to rt and concentrated *in vacuo.* The residue was purified by silica gel column chromatography (PE/EA (V/V) = 1/1) to give the title compound as yellow oil (0.89 g, 3.2 mmol, 68%).
MS (ESI, pos.ion) *m*/*z:* 282.2 [M+H]⁺.

### Step 5: N-(1-(3-fluoro-4-formylphenyl)-3-methylbutan-2-yl)formamide

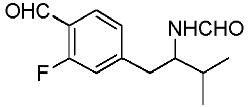

N (1-(4-(1,3-dioxolan-2-yl)-3-fluorophenyl)-3-methylbutan-2-yl)formamide (0.13 g, 0.46 mmol) was dissolved in dioxane (4 mL), then the mixture was cooled to 0 °C, and HCl (2 mL, 6 M) was added dropwise into the mixture. The resulting mixture was warmed to rt and stirred for 4 h. The mixture was cooled to 0 °C, and to the mixture was added water (10 mL). The mixture was extracted with EA (15 mL), and the aqueous layer was extracted with EA (15 mL × 3). The combined organic layers were concentrated *in vacuo,* and the residue was purified by silica gel column chromatography (EA) to give the title compound as colorless oil (108 mg, 0.45 mmol, 100%).
MS (ESI, pos.ion) *m*/*z* : 238.1 [M+H]⁺.

### Step 6: 2-fluoro-4-(2-formamido-3-methylbutyl)benzoic acid

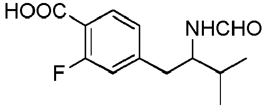

N-(1-(3-fluoro-4-formylphenyl)-3-methylbutan-2-yl)formamide (843.6 mg, 3.56 mmol) was added into a 50 mL single-neck flask, then acetonitrile (10 mL), t-BuOH (2.6 g), CuBr₂ (156 mg) and H₂O (520 mg) were added. The mixture was stirred overnight at rt, and then concentrated. To the residue was added aqueous NaOH solution (1 M, 10 mL), and the mixture was washed with EA (10 mL × 2). The organic layer was washed with aqueous NaOH solution (1 M, 10 mL) once. The combined aqueous layers were adjusted with HCl (2 M) to pH = 3-4, and there was a white solid precipitated out. The mixture was extracted with EA (20 mL × 4). The combined organic layers were concentrated *in vacuo* to give the title compound as a white solid (606 mg, 2.392 mmol, 67.30%).
MS (ESI, pos.ion) *m*/*z:* 254.3 [M+H]⁺.

### Step 7: methyl 2-fluoro-4-(2-formamido-3-methylbutyl)benzoate

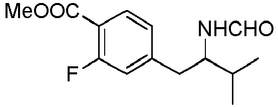

To a 50 mL single-neck flask were added 2-fluoro-4-(2-formamido-3-methylbutyl)benzoic acid (680 mg, 2.69 mmol), K₂CO₃ (740 mg, 5.36 mmol) and DMF (5 mL). The mixture was stirred to dissolve the solid, then CH₃I (571 mg, 4.02 mmol) was added. The mixture was stirred at rt for 2.5 h. To the mixture was added 10 mL of water; the resulting mixture was extracted with ethyl acetate (10 mL × 4); the combined organic layers were concentrated *in vacuo,* and the residue was purified by silica gel column chromatography (EA) to give the title compound as brown oil (0.52 g, 1.9 mmol, 72%).
MS (ESI, pos.ion) *m*/*z:* 268.2 [M+H]⁺.

### Step 8: methyl 8-fluoro-5-isopropyl-2,3-dioxo-3,5,6,10b-tetrahydro-2H-oxazolo[2,3-a] isoquinoline-9-carboxylate

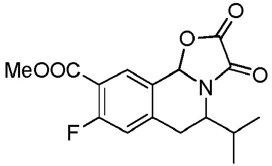

To a 100 mL single-neck flask were added methyl 2-fluoro-4-(2-formamido-3-methylbutyl)benzoate (0.27 g, 1.0 mmol), DCM (10 mL) and oxalyl chloride (190 mg, 1.50 mmol). The mixture was stirred at rt for 30 min. The mixture was cooled to -10 °C, then FeCl₃ (260 mg, 1.62 mmol) was added. The mixture was warmed to rt and stirred overnight. To the mixture was added HCl (5 mL, 2 M), and the mixture was stirred for 30 min. The mixture was extracted with DCM (15 mL ×3), and the combined organic layers were concentrated *in vacuo* to give the title compound as brownness oil (0.29 g, 0.91 mmol, 90%).
MS (ESI, pos.ion) *m*/*z:* 322.1 [M+H]⁺.

### Step 9: methyl 6-fluoro-3-isopropyl-3,4-dihydroisoquinoline-7-carboxylate

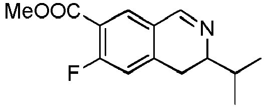

Methyl 8-fluoro-5-isopropyl-2,3-dioxo-3,5,6,10b-tetrahydro-2*H*-oxazolo[2,3-*a*] isoquinoline-9-carboxylate (0.2914 g, 0.9069 mmol), MeOH (10 mL) and H₂SO₄ (0.4 mL) were added into a 50 mL single-neck flask. The mixture was heated to 75 °C and stirred for 1.5 h, then stirred at rt for 10 h. The mixture was concentrated, and the residue was diluted with water (15 mL) and EA (15 mL).The mixture was partitioned, and the organic layer was washed with hydrochloric acid (2 M, 10 mL × 2). The combined aqueous layers were adjusted with ammonium hydroxide to pH = 9-10, and the resulting mixture was extracted with EA (20 mL × 3). The combined organic layers were concentrated *in vacuo* to give the title compound as brown oil (150 mg, 0.60 mmol, 66.34%), which was directly used in the next step.
MS (ESI, pos.ion) *m*/*z:* 250.2 [M+1]⁺.

### Step 10: methyl 3-isopropyl-6-phenoxy-3,4-dihydroisoquinoline-7-carboxylate

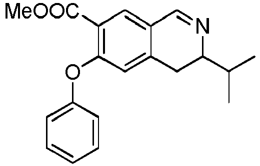

Methyl 6-fluoro-3-isopropyl-3,4-dihydroisoquinoline-7-carboxylate (230 mg, 0.92 mmol) was added into a 50 mL two-neck flask, then K₂CO₃ (254 mg, 1.84 mmol), phenol (130 mg, 1.38 mmol), DMF (10 mL) and KI (5 mg) were added. The mixture was heated to 90 °C and stirred for 5.5 h under nitrogen protection. The mixture was cooled to rt, and to the mixture were added EA (30 mL) and water (15 mL) in turn. The mixture was partitioned, and the aqueous layer was extracted with ethyl acetate (30 mL × 3). The combined organic layers were washed with saturated brine (15 mL × 3), dried over anhydrous sodium sulfate, and concentrated *in vacuo.* The residue was purified by silica gel column chromatography (EA) to give the title compound as brown oil (240 mg, 0.74 mmol, 80%).
MS (ESI, pos.ion) *m*/*z:* 324.1 [M+H]⁺.

### Step 11: 3-tert-butyl 10-methyl 6-isopropyl-2-oxo-9-phenoxy-2,6,7,11b-tetrahydro -1H-pyrido[2,1-a]isoquinoline-3,10-dicarboxylate

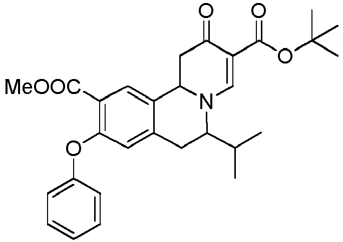

*tert*-Butyl 2-((dimethylamino)methylene)-3-oxobutanoate (0.46 g, 2.13 mmol), methyl 3-isopropyl-6-phenoxy-3,4-dihydroisoquinoline-7-carboxylate (345 mg, 1.067 mmol) and t-BuOH (10 mL) were added into a 100 mL single-neck flask, then the mixture was heated to 100 °C under nigrogen protection and stirred for 6 days. The mixture was concentrated *in vacuo,* and the residue was purified by thin-layer chromatography (PE/acetone (V/V) = 2/1) to give the title compound as brown oil (0.5244 g, 1.07 mmol, 99.99%).
MS (ESI, pos.ion) *m*/*z:* 492.1 [M+H]⁺.

### Step 12: 6-isopropyl-10-(methoxycarbonyl)-2-oxo-9-phenoxy-6,7-dihydro-2H -pyrido[2,1-a]isoquinoline-3-carboxylic acid

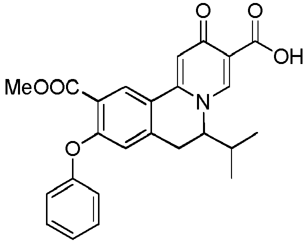

3-tert-Butyl 10-methyl 6-isopropyl-2-oxo-9-phenoxy-2,6,7,11b-tetrahydro-1*H* -pyrido[2,1-a]isoquinoline-3,10-dicarboxylate (524.4 mg, 1.07 mmol) was added into a 100 mL single-neck flask, then dimethoxyethane (20 mL) was added to dissolved the reagent, and chloranil (393 mg, 1.5985 mmol) was added. The mixture was heated to 90 °C and stirred for 3.5 hour under nitrogen protection. The reaction mixture was concentrated *in vacuo.* The residue was purified by thin-layer chromatography (DCM/MeOH (V/V) = 10/1) to give the title compound as a light yellow solid (215 mg, 0.50 mmol, 46.50%).
MS (ESI, pos.ion) *m*/*z:* 434.1 [M+H]⁺;
¹H NMR (400 MHz, CDCl₃) δ 15.837 (b, 1H), 8.487 (s, 1H), 8.363 (s, 1H), 7.470 - 7.431 (m, 2H), 7.261 - 7.221 (m, 2H), 7.099 (d, *J* = 8.0Hz, 2H), 6.802 (s, 1H), 4.103 - 3.874 (m, 4H), 3.404 - 3.295 (m, 1H), 3.154 - 2.990 (m, 1H), 1.807 - 1.704 (m, 1H), 0.917 (d, *J* = 6.4Hz, 3H), 0.849 (d, *J* = 6.4Hz, 3H).

### Example 41: 6-isopropyl-10-(methoxycarbonyl)-9-((R)-3-methoxypyrrolidin-1-yl) -2-oxo-6,7-dihydro-2H-pyrido[2,1-a]isoquinoline-3-carboxylic acid

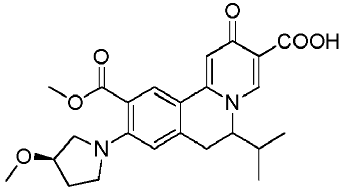

### Step 1: methyl 3-isopropyl-6-((R)-3-methoxypyrrolidin-1-yl)-3,4-dihydroisoquinoline -7-carboxylate

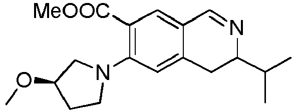

Methyl 6-fluoro-3-isopropyl-3,4-dihydroisoquinoline-7-carboxylate (210 mg, 0.84 mmol), (3*S*)-3-methoxypyrrole (173 mg, 1.26 mmol), K₂CO₃ (348 mg, 2.52 mmol), KI (5 mg) and DMSO (10 mL) was added into a 50 mL two-neck flask. The mixture was heated to 100 °C and stirred for 3.5 h under nitrogen protection. The mixture was cooled to rt, and to the mixture was added water (15 mL); the resulting mixture was extracted with EA (20 mL × 4); the combined organic layers were washed with saturated brine (15 mL × 3), dried over anhydrous sodium sulfate, and concentrated *in vacuo;* the residue was purified by silica gel column chromatography (EA) to give the title compound as brown oil (0.25 g, 0.75 mmol, 89.5%).
MS (ESI, pos.ion) *m*/*z:* 331.2 [M+H]⁺.

### Step 2: 3-tert-butyl 10-methyl 6-isopropyl-9-((R)-3-methoxypyrrolidin-1-yl)-2-oxo -2,6,7,11b-tetrahydro-1H-pyrido[2,1-a]isoquinoline-3,10-dicarboxylate

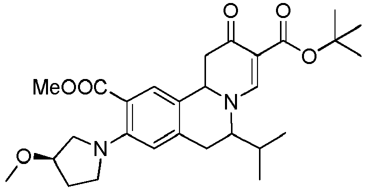

Methyl 3-isopropyl-6-((R)-3-methoxypyrrolidin-1-yl)-3,4-dihydroisoquinoline -7-carboxylate (0.249 g, 0.754 mmol), methyl 3-isopropyl-6-phenoxy-3,4-dihydroisoquinoline -7-carboxylate (0.321 g, 1.51 mmol) and t-BuOH (10 mL) were added into a 50 mL single-neck flask, then the mixture was heated to 95 °C under nigrogen protection and stirred for 5 days. The reaction mixture was concentrated; and the residue was purified by thin-layer chromatography (PE/acetone (V/V) = 1/1) to give the title compound as brown oil (0.376 g, 0.75 mmol, 100%).
MS (ESI, pos.ion) *m*/*z:* 499.5 [M+H]⁺.

### Step 3: 6-isopropyl-10-(methoxycarbonyl)-9-((R)-3-methoxypyrrolidin-1-yl)-2-oxo-6,7-dihydro-2H-pyrido[2,1-a]isoquinoline-3-carboxylic acid

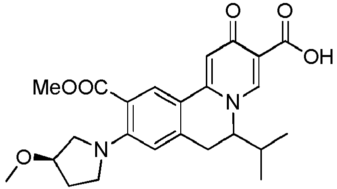

To a 100 mL single-neck flask were added 3-*tert*-butyl 10-methyl 6-isopropyl-9-((*R*)-3-methoxypyrrolidin-1-yl)-2-oxo-2,6,7,11b-tetrahydro-1*H*-pyrido[2,1-*a*]isoqu inoline-3,10-dicarboxylate (0.53 g, 1.07 mmol), dimethoxyethane (15 mL) and chloranil (394 mg, 1.60 mmol). The mixture was stirred at 90 °C for 4 h. The reaction mixture was cooled to rt, and concentrated *in vacuo.* The residue was purified by silica gel column chromatography (DCM/MeOH (V/V) = 15/1) to give the title compound as a light yellow solid (175 mg, 0.40 mmol, 37.11%).
MS (ESI, pos.ion) *m*/*z:* 441.3 [M+H]⁺;
¹H NMR (400 MHz, CDCl₃) δ 16.273 (b, 1H), 8.422 (s, 1H), 8.019(s, 1H), 7.050 (d, *J=* 11.2Hz, 1H), 6.614 (d, *J=* 2.2Hz, 1H), 4.124 - 4.039 (m, 1H), 3.951 (s, 3H), 3.885 - 3.825 (m, 1H), 3.689 - 3.626 (m, 1H), 3.589 - 3.509 (m, 1H), 3.450 - 3.404 (m, 1H), 3.370 - 3.270 (m, 4H), 3.152 - 2.994 (m, 2H), 2.257 - 2.014 (m, 2H), 1.864 - 1.756 (m, 1H), 0.982 - 0.936 (m, 3H), 0.822 (d, *J* = 8 Hz, 3H).

### Example 42: 6-isopropyl-10-(methoxycarbonyl)-9-((S)-3-methoxypyrrolidin-1-yl)-2 -oxo-6,7-dihydro-2H-pyrido[2,1-a]isoquinoline-3-carboxylic acid

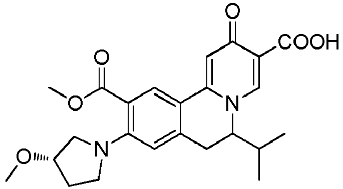

The title compound was prepared according to the sythetic method of example 41 by using methyl 6-fluoro-3-isopropyl-3,4-dihydroisoquinoline-7-carboxylate (300 mg, 1.2 mmol) and (*S*))-3-methoxypyrrolidine (173 mg, 1.26 mmol) as raw materials to give an off-white solid (0.20 g, 0.45 mmol).
MS (ESI, pos.ion) *m*/*z:* 441.1 [M+H]⁺;
¹H NMR (400 MHz, CDCl₃) δ 8.450 (s, 1H), 7.285(s, 1H), 7.269 (d, *J* = 11.6Hz, 1H), 6.615 (s, 1H), 4.151 - 4.028 (m, 1H), 4.022 - 3.831 (m, 4H), 3.665 - 3.631 (m, 1H), 3.568 - 3.532 (m, 1H), 3.393 - 3.263 (m, 4H), 3.202 - 3.006 (m, 2H), 2.296 - 1.230 (m, 4H), 0.956 - 0.936 (d, *J* =9.2Hz, 3H), 0.809 (d, *J* = 0.8Hz, 3H).

### Example 43: 9-(3,3-difluoropyrrolidin-1-yl)-6-isopropyl-10-(methoxycarbonyl)-2-oxo -6,7-dihydro-2H-pyrido[2,1-a]isoquinoline-3-carboxylic acid

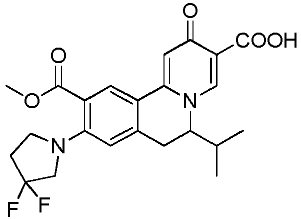

The title compound was prepared according to the sythetic method of example 41 by using methyl 6-fluoro-3-isopropyl-3,4-dihydroisoquinoline-7-carboxylate (300 mg, 1.2 mmol) and 3,3-difluoropyrrolidine hydrochloric acid (259 mg, 1.80 mmol) as raw materials to give an off-white solid (0.25 g, 0.731 mmol).
MS (ESI, pos.ion) *m*/*z:* 447.1 [M+H]⁺;
¹H NMR (400 MHz, CDCl₃) δ 16.066 (b, 1H), 8.440 (d, *J* = 4.4Hz, 1H), 8.093 (d, *J* = 4.8Hz, 1H), 7.090 (d, *J* = 14.4Hz, 1H), 6.640 (s, 1H), 3.979 (s, 3H), 3.935 - 3.786 (m, 1H), 3.764 - 3.497 (m, 4H), 3.445 - 3.320 (m, 1H), 3.229 - 3.094 (m, 1H), 2.598 - 2.444 (m, 2H), 1.847 - 1.729 (m, 1H), 0.971 - 0.941 (d, *J* = 4.8Hz, 3H), 0.841 (d, *J* = 5.2Hz, 3H).

### Example 44: 9-((S)-3-hydroxypyrrolidin-1-yl)-6-isopropyl-10-(methoxycarbonyl) -2-oxo-6,7-dihydro-2H-pyrido[2,1-a]isoquinoline-3-carboxylic acid

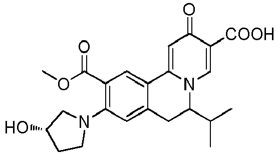

### Step 1: 9-((S)-3-(benzyloxy)pyrrolidin-1-yl)-6-isopropyl-10-(methoxycarbonyl)-2-oxo -6,7-dihydro-2H-pyrido[2,1-a]isoquinoline-3-carboxylic acid

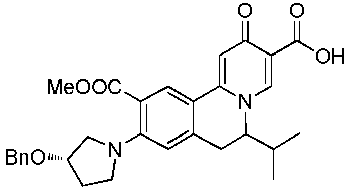

The title compound was prepared according to the sythetic method of example 41 by using methyl 6-fluoro-3-isopropyl-3,4-dihydroisoquinoline-7-carboxylate (200 mg, 0.80 mmol) and (*S*)-3-(benzyloxy)pyrrolidine (213 mg, 1.20 mmol) as raw materials to give brown oil (0.32 g, 0.79 mmol, 98%).
MS (ESI, pos.ion) *m*/*z:* 517.2 [M+H]⁺.

### Step 2: 9-((S)-3-hydroxypyrrolidin-1-yl)-6-isopropyl-10-(methoxycarbonyl)-2-oxo -6,7-dihydro-2H-pyrido[2,1-a]isoquinoline-3-carboxylic acid

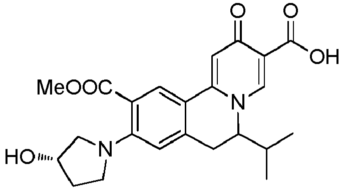

9-((*S*)-3-(Benzyloxy)pyrrolidin-1-yl)-6-isopropyl-10-(methoxycarbonyl)-2-oxo-6,7-dihydro-2H-pyrido[2,1-a]isoquinoline-3-carboxylic acid (200 mg, 0.39 mmol) was added into a 100 mL single-neck flask, then MeOH (20 mL) and Pd/C (40 mg) was added. The mixture was degassed and filled with nitrogen for three times, then stirred at 65 °C for 5 h in a hydrogen atomposphere. The reaction was stopped, and the mixture was filtered. The filter cake was washed with DCM, and the filtrate was concentrated. The residue was purified by silica gel column chromatography (DCM/MeOH (V/V) = 8/1) to give the title compound as a yellow solid (100 mg, 0.23 mmol, 60%).
MS (ESI, pos.ion) *m*/*z:* 427.1 [M+H]⁺;
¹H NMR (400 MHz, CDCl₃) δ 16.2996 (b, 1H), 8.411 (d, *J* = 4.4Hz, 1H), 8.017 (d, *J* = 4.8Hz, 1H), 7.043 (d, *J* = 14.4Hz, 1H), 6.634 (s, 1H), 4.634 (s, 1H), 3.956 (d, *J* = 4.4Hz, 3H), 3.904 - 3.825 (m, 1H), 3.895 - 3.619 (m, 3H), 3.462 - 3.400 (m, 1H), 3.371 - 3.303 (m, 1H), 3.181 - 2.958 (m, 2H), 2.254 - 2.068 (m, 2H), 1.859 - 1.737 (m, 1H), 0.987 - 0.941 (m, 3H), 0.827 (d, *J* =6.4Hz, 3H).

### Example 45: 9-((S)-3-(2,2-difluoroethoxy)pyrrolidin-1-yl)-6-isopropyl-10 -(methoxycarbonyl)-2-oxo-6,7-dihydro-2H-pyrido[2,1-a]isoquinoline-3-carboxylic acid

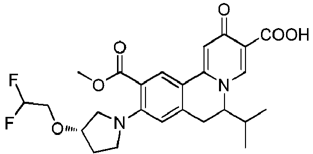

The title compound was prepared according to the sythetic method of example 41 by using methyl 6-fluoro-3-isopropyl-3,4-dihydroisoquinoline-7-carboxylate (300 mg, 1.2 mmol) and (*S*)-3-(2,2-difluoroethoxy)pyrrolidine trifluoroacetate (0.638 g, 2.41 mmol) as raw materials to give an off-white solid (30 mg, 0.879 mmol).
MS (ESI, pos.ion) *m*/*z:* 490.9 [M+H]⁺;
¹H NMR (400 MHz, CDCl₃) δ 8.443 (s, 1H), 8.046 (s, 1H), 7.089 (d, *J* =12.4 Hz, 1H), 6.622 (d, *J* =3.2 Hz, 1H), 5.989 - 5.771 (m, 2H), 4.340 - 4.264 (m, 1H), 9.962 (d, *J* =2.4 Hz, 3H), 3.925 - 3.894 (m, 1H), 3.751 - 3.686 (m, 2H), 3.636 - 3.549 (m, 1H), 3.441 - 3.413 (m, 1H), 3.374 - 3.315 (m, 1H), 3.199 - 3.085 (m, 2H), 2.207 - 2.265 (m, 2H), 2.115 - 2.026 (m, 1H), 0.989 - 0.948 (m, 3H), 0.832 (d, *J* =4.0Hz, 3H).

### Example 48: 10-acetyl-6-isopropyl-9-((S)-3-methoxypyrrolidin-1-yl)-2-oxo-6,7-dihydro -2H-pyrido[2,1-a]isoquinoline-3-carboxylic acid

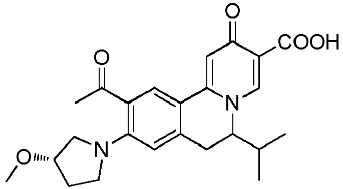

The title compound was prepared according to the synthetic method of step 11 to step 12 in example 30 by using ethyl 10-acetyl-6-isopropyl-2-oxo-9-(((trifluoromethyl) sulfonyl)oxy)-6,7-dihydro-2H-pyrido[2,1-a]isoquinoline-3-carboxylate (250 mg, 0.5 mmol) and (*S*)-(+)-3-methoxypyrrolidine hydrochloride (0.137 g, 1.0 mmol) as raw materials to give a yellow solid (0.110 g).
MS (ESI, pos.ion) *m*/*z:* 425.2 [M+H]⁺;
¹H NMR (400 MHz, CDCl₃) δ 16.18 (s, 1H), 8.43 (s, 1H), 7.92 (d, *J* = 1.9 Hz, 1H), 7.05 (d, *J* = 12.9 Hz, 1H), 6.64 (d, *J* = 2.3 Hz, 1H), 4.09 (s, 1H), 3.92 - 3.83 (m, 1H), 3.52 (ddd, *J* = 20.3, 14.0, 5.5 Hz, 2H), 3.35 (d, *J* = 18.5 Hz, 4H), 3.14 (dd, *J* = 16.1, 5.5 Hz, 1H), 2.97 - 2.74 (m, 1H), 2.68 (d, *J* = 3.7 Hz, 3H), 2.31 - 1.97 (m, 3H), 1.81 (d, *J* = 6.8 Hz, 1H), 0.97 (dd, *J* = 15.8, 6.6 Hz, 3H), 0.89 - 0.77 (m, 3H).

### Example 49: 10-acetyl-6-isopropyl-9-((R)-3-methoxypyrrolidin-1-yl)-2-oxo-6,7-dihydro -2H-pyrido[2,1-a]isoquinoline-3-carboxylic acid

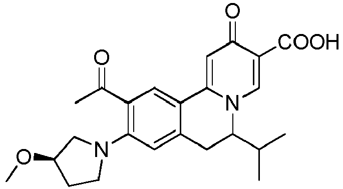

The title compound was prepared according to the synthetic method of step 11 to step 12 in example 30 by using ethyl 10-acetyl-6-isopropyl-2-oxo-9-(((trifluoromethyl) sulfonyl)oxy)-6,7-dihydro-2H-pyrido[2,1-a]isoquinoline-3-carboxylate (250 mg, 0.5 mmol) and (R)-(+)-3-methoxypyrrolidine hydrochloride (0.137 g, 1.0 mmol) as raw materials to give a yellow solid (0.107 g).
MS (ESI, pos.ion) *m*/*z:* 425.2[M+H]⁺;
¹H NMR (400 MHz, CDCl₃) δ 16.18 (s, 1H), 8.43 (s, 1H), 7.92 (d, *J* = 1.9 Hz, 1H), 7.05 (d, *J* = 12.9 Hz, 1H), 6.64 (d, *J* = 2.2 Hz, 1H), 4.09 (s, 1H), 3.92 - 3.82 (m, 1H), 3.37 (s, 3H), 3.32 (s, 2H), 3.14 (dd, *J* = 15.2, 5.8 Hz, 1H), 2.92 (d, *J* = 11.8 Hz, 1H), 2.80 (d, *J* = 11.8 Hz, 1H), 2.68 (d, *J* = 3.7 Hz, 3H), 2.33 - 1.97 (m, 3H), 1.90 - 1.72 (m, 1H), 0.99 - 0.95 (m, 3H), 0.86 - 0.77 (m, 3H).

### Example 50: 10-acetyl-6-isopropyl-9-morpholino-2-oxo-6,7-dihydro-2H-pyrido[2,1-a] isoquinoline-3-carboxylic acid

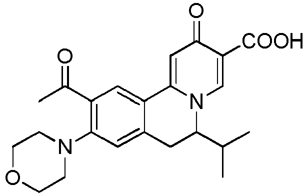

The title compound was prepared according to the synthetic method of step 11 to step 12 in example 30 by using ethyl 10-acetyl-6-isopropyl-2-oxo-9-(((trifluoromethyl) sulfonyl)oxy)-6,7-dihydro-2H-pyrido[2,1-a]isoquinoline-3-carboxylate (250 mg, 0.5 mmol) and morpholine (0.065 g, 0.74 mmol) as raw materials to give a yellow solid (0.030 g).
MS (ESI, pos.ion) *m*/*z:* 411.19 [M+H]⁺;
¹H NMR (400 MHz, CDCl₃) δ 15.90 (s, 1H), 8.44 (s, 1H), 7.82 (s, 1H), 7.10 (s, 1H), 6.88 (s, 1H), 3.97 - 3.81 (m, 5H), 3.38 (dd, *J* = 16.2, 4.6 Hz, 1H), 3.22 - 3.14 (m, 1H), 3.14 - 3.07 (m, 3H), 2.68 (s, 3H), 1.77 (dd, *J* = 16.5, 6.7 Hz, 1H), 0.97 (d, *J* = 6.6 Hz, 3H), 0.83 (d, *J* = 6.7 Hz, 3H).

### Example 51: 10-acetyl-6-isopropyl-9-(4-(2-methoxyethyl)piperazin-1-yl)-2-oxo -6,7-dihydro-2H-pyrido[2,1-a]isoquinoline-3-carboxylic acid

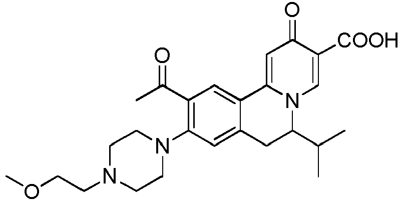

The title compound was prepared according to the synthetic method of step 11 to step 12 in example 30 by using ethyl 10-acetyl-6-isopropyl-2-oxo-9-(((trifluoromethyl) sulfonyl)oxy)-6,7-dihydro-2*H*-pyrido[2,1-*a*]isoquinoline-3-carboxylate (250 mg, 0.5 mmol) and 1-(2-methoxyethyl)piperazine (0.165 g, 1.14 mmol) as raw materials to give a yellow solid (65 mg).
MS (ESI, pos.ion) *m*/*z:* 468.25 [M+H]⁺;
¹H NMR (400 MHz, CDCl₃) δ 16.02 (s, 1H), 8.47 (s, 1H), 7.82 (s, 1H), 7.10 (s, 1H), 6.88 (s, 1H), 3.94 (dd, *J* = 9.6, 4.4 Hz, 1H), 3.59 (t, *J* = 4.9 Hz, 2H), 3.38 (d, *J* = 6.1 Hz, 4H), 3.21 (d, *J* = 5.1 Hz, 4H), 2.81 - 2.69 (m, 5H), 2.67 (s, 3H), 1.86 - 1.69 (m, 2H), 1.27 (d, *J* = 1.5 Hz, 1H), 0.97 (d, *J* = 6.6 Hz, 3H), 0.83 (d, *J* = 6.7 Hz, 3H).

### Example 52: 10-acetyl-9-(4-hydroxypiperidin-1-yl)-6-isopropyl-2-oxo-6.7-dihydro-2H -pyrido[2,1-a]isoquinoline-3-carboxylic acid

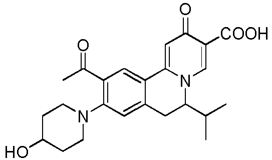

The title compound was prepared according to the synthetic method of step 11 to step 12 in example 30 by using ethyl 10-acetyl-6-isopropyl-2-oxo-9-(((trifluoromethyl) sulfonyl)oxy)-6,7-dihydro-2H-pyrido[2,1-a]isoquinoline-3-carboxylate (550 mg, 1.1 mmol) and piperidin-4-ol (65 m g, 1.6 mmol) as raw materials to give a yellow solid (133 mg).
MS (ESI, pos.ion) *m*/*z:* 425.2066 [M+H]⁺;
¹H NMR (400 MHz, DMSO-*d*₆) δ 8.77 (s, 1H), 7.94 (s, 1H), 7.30 (s, 1H), 7.15 (s, 1H), 4.78 (d, *J* = 3.9 Hz, 1H), 4.47 (d, *J* = 5.8 Hz, 1H), 3.67 (d, *J* = 3.6 Hz, 1H), 3.34 (s, 1H), 3.26 (d, *J* = 8.1 Hz, 2H), 3.17 (d, *J* = 5.1 Hz, 1H), 2.92 (t, *J* = 10.9 Hz, 2H), 2.60 (s, 3H), 1.84 (d, *J* = 10.6 Hz, 2H), 1.64 - 1.43 (m, 3H), 0.89 (d, *J* = 6.6 Hz, 3H), 0.70 (d, *J* = 6.6 Hz, 3H).

### Example 53: 6-isopropyl-9-(3-methoxypropoxy)-2-oxo-10-(4,4,4-trifluorobutanoyl) -6,7-dihydro-2H-pyrido[2,1-a]isoquinoline-3-carboxylic acid

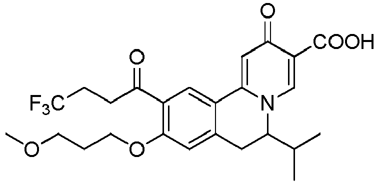

### Step 1: N-methoxy-2-(3-methoxypropoxy)-N-methyl-4-(3-methyl-2-oxobutyl)benzamide

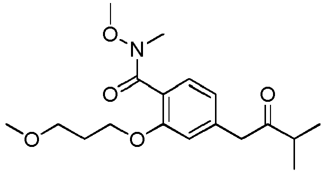

To a 50 mL single-neck flask were added 2-(3-methoxypropoxy)-4-(3-methyl-2-oxobutyl)benzoic acid (1 g, 3.398 mmol), DMF (6 mL), DIPEA (1.78 mL, 10.2 mmol), *N,O*-dimethylhydroxylamine hydrochloride (500 mg, 5.126 mmol) and HATU (2 g, 5.2598 mmol). The mixture was stirred at rt for 24 h. The mixture was adjusted with hydrochloric acid (1 M) to pH = 5, and the resulting mixture was extracted with ethyl acetate (3 × 20 mL); the combined organic layers were dried over anydrous sodium sulfate and filtered; the filtrate was concentrated *in vacuo,* and the residue was purified by silica gel column chromatography (PE/EA (V/V) = 1/1) to give the title compound as colorless oil (200 mg, 0.60 mmol, 17.45%).
MS (ESI, pos.ion) *m*/*z:* 338.3 [M+H]⁺.

### Step 2: 4-(2-amino-3-methylbutyl)-N-methoxy-2-(3-methoxypropoxy)-N-methylbenzamide

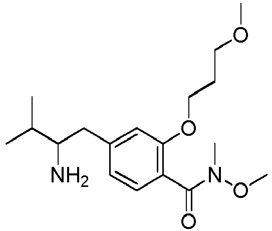

To a 100 mL single-neck flask were added methanol (45 mL), A-methoxy-2-(3-methoxypropoxy)-A-methyl-4-(3-methyl-2-oxobutyl)benzamide (4.5 g, 13 mmol) and ammonium acetate (7.2 g, 93 mmol). The mixture was stirred at rt for 1 h, then cooled to 0 °C, and sodium cyanoborohydride (1.7 g, 27 mmol) was added slowly. The resulting mixture was warmed to rt and stirred for 24 h. The reaction mixture was concentrated by rotary evaporation to remove the methanol; to the residue was added saturated aqueous sodium bicarbonate solution (50 mL) to quenche the reaction, then the mixture was extracted with ethyl acetate (3× 50 mL); the combined organic layers were dried over anydrous sodium sulfate and filtered; the filtrate was concentrated *in vacuo* to give the title compound as colorless oil (4 g, 11.82 mmol, 89%).
MS (ESI, pos.ion) *m*/*z:* 339.1 [M+H]⁺.

### Step 3: tert-butyl (1-(4-(methoxy(methyl)carbamoyl)-3-(3-methoxypropoxy)phenyl)-3 -methylbutan-2-yl)carbamate

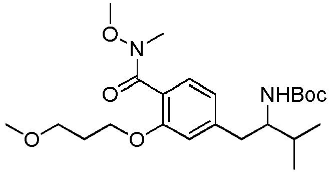

To a 250 mL single-neck flask were added 4-(2-amino-3-methylbutyl)-N-methoxy-2-(3-methoxypropoxy)-N-methylbenzamide (4 g, 11.82 mmol), TEA (3.7 mL, 27 mmol) and DCM (50 mL) in turn, then BoczO (5.8 g, 27 mmol) was added dropwise. The mixture was stirred at rt for 2 h. The reaction mixture was concentrated by rotary evaporation to remove the solvent, and to the residue was added hydrochloric acid (1 M) to pH = 5; the resulting mixture was extracted with ethyl acetate (3 × 20 mL), and the combined organic layers were dried over anydrous sodium sulfate and filtered. The filtrate was concentrated *in vacuo,* and the residue was purified by silica gel column chromatography (PE/EA (V/V) = 1/1) to give the title compound as colorless oil (3.2 g, 7.3 mmol, 62%).
¹H NMR (600 MHz, CDCl₃) δ 7.20 (d, *J* = 7.3 Hz, 1H), 6.83 - 6.77 (m, 2H), 4.37 (d, *J* = 9.3 Hz, 1H), 4.10 (t, *J* = 6.2 Hz, 2H), 3.76 (s, 1H), 3.67 - 3.44 (m, 5H), 3.39 - 3.20 (m, 6H), 2.93 (q, *J* = 7.3 Hz, 1H), 2.83 - 2.76 (m, 1H), 2.72 - 2.65 (m, 1H), 2.06 - 2.01 (m, 1H), 1.79-1.71 (m, 1H), 1.40 (s, 9H), 0.98 (d, *J* = 6.8 Hz, 3H), 0.93 (d, *J* = 6.8 Hz, 3H).

### Step 4: tert-butyl (1-(3-(3-methoxypropoxy)-4-(4,4,4-trifluorobutanoyl)phenyl) -3-methylbutan-2-yl)carbamate

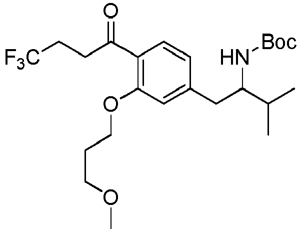

To a dried 50 mL three-neck flask equiped with thermometer and reflux condensor were added magnesium powder (0.3 g, 10 mmol) and THF (8 mL), then a grain of iodine was added, and 3-bromo-1,1,1-trifluoropropane (2 mL) was added. After addition, the mixture was heated to 70 °C and stirred for 2 h, then cooled to rt. To another 50 mL two-neck flask were added tert-butyl (1-(4-(methoxy(methyl)carbamoyl)-3-(3-methoxypropoxy)phenyl) -3-methylbutan-2-yl)carbamate (2 g, 4.560 mmol) and THF (10 mL), and the mixture was degassed and filled with nitrogen for three times, then cooled to 0 °C; to the mixture was added dropwised the Grignard reagent prepared above. The resulting mixture was stirred at 0 °C for 1 h, then waremed to rt and stirred for 4 h. The reaction was quenched wtih saturated aqueous ammonium chloride solution (20 mL), and the mixture was extracted with ethyl acetate (3 × 20 mL); the combined organic layers were dried over anydrous sodium sulfate and filtered; the filtrate was concentrated *in vacuo,* and the residue was purified by silica gel column chromatography (PE/EA (V/V) = 10/1) to give the title compound as colorless oil (200 mg, 0.4206 mmol, 9.224%).
MS (ESI, pos.ion) *m*/*z:* 499.15 [M+Na]⁺.

### Step 5: 1-(4-(2-amino-3-methylbutyl)-2-(3-methoxypropoxy)phenyl)-4,4,4-trifluorobutan-1-one

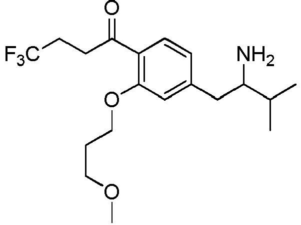

To a 100 mL single-neck flask were added tert-butyl (1-(3-(3-methoxypropoxy)-4-(4,4,4-trifluorobutanoyl)phenyl)-3-methylbutan-2-yl)carbamate (400 mg, 0.8412 mmol), DCM (2 mL) and TFA (2 mL). The mixture was stirred at rt for 4 h. The reaction mixture was concentrated by rotary evaporation; to the residue was added saturated aqueous sodium bicarbonate solution to adjust the pH = 8, then the mixture was extracted with ethyl acetate (3 mL × 20). The combined organic layers were dried over anydrous sodium sulfate and filtered. The filtrate was concentrated *in vacuo* to give the title compound as colorless oil (320 mg, 0.84 mmol, 99%), which was used directly in the next step.
MS (ESI, pos.ion) *m*/*z:* 376.3 [M+H]⁺.

### Step 6: N-(1-(3-(3-methoxypropoxy)-4-(4,4,4-trifluorobutanoyl)phenyl)-3-methylbutan -2-yl)formamide

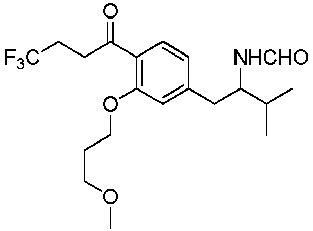

To a 50 mL single-flask were added 1,4-dioxane (6 mL), 1-(4-(2-amino-3-methylbutyl)-2-(3-methoxypropoxy)phenyl)-4,4,4-trifluorobutan-1-one (330 mg, 0.8791 mmol) and formic acid (0.5 mL, 10 mmol). The mixture was heated to reflux and stirred for 12 h under nitrogen protection. The mixture was concentrated *in vacuo,* and the residue was purified by silica gel column chromatography (DCM/MeOH (V/V) = 20/1) to give the title compound as colorless oil (260 mg, 0.6445 mmol, 73.32%).
MS (ESI, pos.ion) *m*/*z:* 404.4 [M+H]⁺.

### Step 7: 4,4,4-trifluoro-1-(3-isopropyl-6-(3-methoxypropoxy)-3,4-dihydroisoguinolin -7-yl)butan-1-one

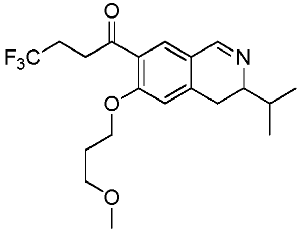

To a 50 mL single-neck flask were added N (1-(3-(3-methoxypropoxy)-4-(4,4,4-trifluorobutanoyl)phenyl)-3-methylbutan-2-yl)formamide (260 mg, 0.6445 mmol) and DCM (5 mL), then the mixture was cooled to 0 °C, and POCl₃ (0.1 mL, 1 mmol) was added. After addition, the mixture was heated to reflux and stirred for 6 h. The reaction mixture was concentrated by rotary evaporation, and to the residue was added saturated aqueous sodium bicarbonate to adjust pH = 8; the resulting mixture was extracted with ethyl acetate (3 × 20 mL), and the combined organic layers were dried over anhydrous sodium sulfate, filtered; the filtrate was concentated *in vacuo,* and the residue was purified by silica gel column chromatography (PE/EA(V/V) = 2/1) to give the title compound as brown oil (138 mg, 0.3581 mmol, 55.56%).
MS (ESI, pos.ion) *m*/*z:* 386.3 [M+H]⁺.

### Step 8: ethyl 6-isopropyl-9-(3-methoxypropoxy)-2-oxo-10-(4,4,4-trifluorobutanoyl) -2,6,7,11b-tetrahydro-1H-pyrido[2,1-a]isoquinoline-3-carboxylate

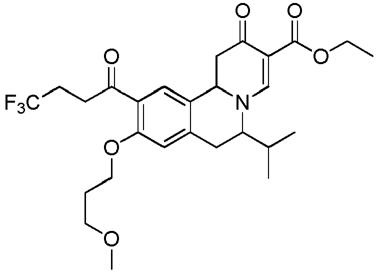

To a 50 mL single-neck flask were added 4,4,4-trifluoro-1-(3-isopropyl-6-(3-methoxypropoxy)-3,4-dihydroisoquinolin-7-yl)butan-1-one (138 mg, 0.361 mmol), ethanol (5 mL) and ethyl 2-(ethoxymethylene)-3-oxo-butyrate (330 mg, 1.77 mmol). The mixture was degassed and filled with nitrogen for three times, then heated and refluxed for 20 h. The mixture was concentrated *in vacuo,* and the residue was purified by silica gel column chromatography (PE/EA (V/V) = 2/1) to give the title compound as brown oil (140 mg, 0.2664 mmol, 74.39%).
MS (ESI, pos.ion) *m*/*z:* 526.4 [M+H]⁺.

### Step 9: ethyl 6-isopropyl-9-(3-methoxypropoxy)-2-oxo-10-(4,4,4-trifluorobutanoyl)-6,7-dihydro-2H-pyrido[2,1-a]isoquinoline-3-carboxylate

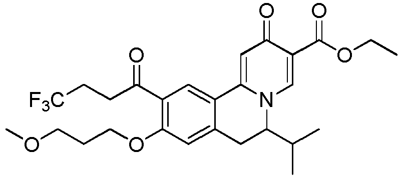

To a 50 mL single-neck flask were added ethyl 6-isopropyl-9-(3-methoxypropoxy)-2-oxo-10-(4,4,4-trifluorobutanoyl)-2,6,7,11b-tetrahydro-1*H-*pyrido[2,1-a]isoquinoline-3-carboxylate (188 mg, 0.3577 mmol), DME (6 mL) and chloranil (87 mg, 0.35383 mmol). The mixture was heated to reflux and stirred for 1 h. The mixture was concentrated *in vacuo,* and the residue was purified by silica gel column chromatography (PE/EA (V/V) = 2/1) to give the title compound as a white solid (140 mg, 0.2674 mmol, 74.77%).

### Step 10: 6-isopropyl-9-(3-methoxypropoxy)-2-oxo-10-(4,4,4-trifluorobutanoyl)-6,7-dihydro-2H -pyrido[2,1-a]isoquinoline-3-carboxylic acid

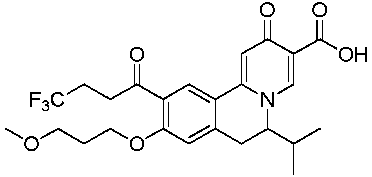

To a 50 mL single-neck flask were added ethyl 6-isopropyl-9-(3-methoxypropoxy)-2-oxo-10-(4,4,4-trifluorobutanoyl)-6,7-dihydro-2H-pyrido[2, 1-a]isoquinoline-3-carboxylate (140 mg, 0.2674 mmol), methanol (5 mL) and lithium hydroxide monohydrate (56 mg, 1.33 mmol). The mixture was stirred at rt for 12 h. The combined organic layers concentrated by rotary evaporation to remove the solvent, and to the residue was added hydrochloric acid (1 M) to pH = 5; the resulting mixture was extracted with ethyl acetate (3 × 20 mL), and the combined organic layers were dried over anydrous sodium sulfate and filtered. The filtrate was concentrated *in vacuo,* and the residue was purified by silica gel column chromatography (DCM/CH₃OH (V/V) = 10/1) to give the title compound as a white solid (50 mg, 0.10 mmol, 37.73%).
MS (ESI, pos.ion) *m*/*z:* 496.3 [M+H]⁺;
1H NMR (600 MHz, CDCl₃) δ 16.03 (s, 1H), 8.53 (s, 1H), 8.21 (s, 1H), 7.14 (s, 1H), 6.97 (s, 1H), 4.33 (s, 2H), 4.06 (s, 1H), 3.61 (s, 2H), 3.51 - 3.18 (m, 7H), 2.58 (d, *J* = 6.4 Hz, 2H), 2.19 (s, 2H), 1.74 (s, 1H), 0.97 (s, 3H), 0.82 (s, 3H).

### Example 54: 6-isopropyl-9-(3-methoxypropoxy)-10-(3-methylbutanoyl)-2-oxo-6,7 -dihydro-2H-pyrido[2,1-a]isoquinoline-3-carboxylic acid

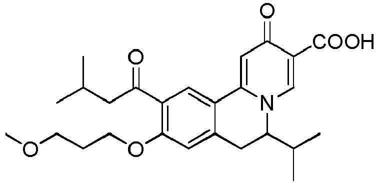

The title compound was prepared according to the sythetic method of example 6 by using 1-(4-bromo-2-hydroxyphenyl)-3-methylbutan-1-one (3.7 g, 14 mmol) as a raw material to give a white solid (200 mg, 0.4391 mmol).
MS (ESI, pos.ion) *m*/*z:* 456.1 [M+H]⁺;
¹H NMR (400 MHz, CDCl₃) δ 15.96 (s, 1H), 8.45 (s, 1H), 8.02 (s, 1H), 7.13 (s, 1H), 6.87 (s, 1H), 4.24 (d, *J* = 5.2 Hz, 2H), 3.92 (s, 1H), 3.59 (t, *J* = 5.2 Hz, 2H), 3.37 (s, 3H), 3.18 (d, *J* = 14.6 Hz, 1H), 2.95 - 2.80 (m, 2H), 2.32 - 2.10 (m, 3H), 1.75 (s, 1H), 1.26 (s, 1H), 1.08 - 0.88 (m, 9H), 0.82 (d, *J* = 6.1 Hz, 3H).

### Example 55: 6-isopropyl-9-(3-methoxypropoxy)-2-oxo-10-(3-phenylpropanoyl)-6,7 -dihydro-2H-pyrido[2,1-a]isoquinoline-3-carboxylic acid

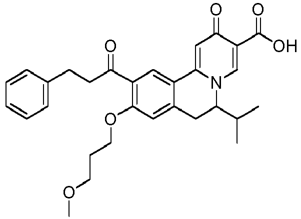

The title compound was prepared according to the sythetic method of example 6 by using 1-(4-bromo-2-hydroxyphenyl)-3-phenylpropan-1-one (1.8 g, 9.83 mmol) as a raw material to give a white solid (190 mg, 0.40 mmol).
MS (ESI, pos.ion) *m*/*z:* 504.2[M+H]⁺;
¹H NMR (400 MHz, CDCl₃) δ 16.01 (s, 1H), 8.48 (s, 1H), 8.08 (s, 1H), 7.40 - 7.09 (m, 6H), 6.90 (s, 1H), 4.40 - 4.19 (m, 2H), 3.60 - 2.92(m, 9H), 2.12 (s, 2H), 1.76 (s, 2H), 1.28 (s, 2H), 1.15 - 0.60 (m, 6H).

### Example 56: 6-isopropyl-10-(methoxycarbamoyl)-9-(3-methoxypropoxy)-2-oxo-6,7 -dihydro-2H-pyrido[2,1-a]isoquinoline-3-carboxylic acid

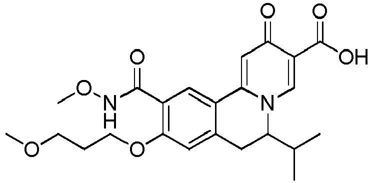

The title compound was prepared according to the synthetic method of example 5 by using 3-(ethoxycarbonyl)-6-isopropyl-9-(3-methoxypropoxy)-2-oxo-6,7-dihydro-2*H* -pyrido[2,1-a]isoquinoline-10-carboxylic acid (0.1 g, 0.2 mmol) and O-methylhydroxylamine hydrochloride (60 mg, 0.72 mmol) as raw materials to give a yellow solid (50 mg).
MS (ESI, pos.ion) *m*/*z:* 445.6 [M+1]⁺;
¹H NMR (400 MHz, CDCl₃) δ 11.20 (s, 1H), 8.60 (s, 1H), 8.53 (s, 1H), 7.19 (s, 1H), 6.90 (s, 1H), 4.26 - 4.40 (m, 2H), 3.99 - 4.12 (m,1H), 3.90 (s, 3H), 3.64 - 3.76 (m, 3H), 3.40 - 3.57 (s, 4H), 3.17 - 3.28 (m,1H), 2.13 - 2.31 (m, 2H), 0.96 (d, *J* = 8Hz, 3H), 0.80 (d, *J* = 8Hz, 3H).

### Example 59: 6-isopropyl-10-(5-isopropylthiazol-2-yl)-9-(3-methoxypropoxy)-2-oxo-6,7 -dihydro-2H-pyrido[2,1-a]isoquinoline-3-carboxylic acid

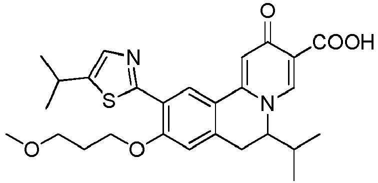

To a 25 mL single-neck flask were added ethyl 6-isopropyl-9-(3-methoxypropoxy)-2-oxo-10-(((trifluoromethyl)sulfonyl)oxy)-6,7-dihydro-2H-p yrido[2,1-a]isoquinoline-3-carboxylate (0.15 g, 0.27 mmol), 5-isopropyl-2-(tributylstannyl)thiazole (285 mg, 0.69 mmol), anhydrous dioxane (10 mL) and bis(triphenylphosphine)palladium(II) chloride (57 mg, 0.082 mmol). The mixture was heated to 110 °C and stirred overnight in nitrogen atomposhere. The reaction mixture was concentrated by rotary evaporation, and the residue was purified by silica gel column chromatography (DCM/MeOH (V/V) = 15/1) to give the title compound as a gray solid (15 mg, 0.274 mmol).
MS (ESI, pos.ion) *m*/*z:* 497.2 [M+H] ⁺;
¹H NMR (400 MHz, CDCl₃) δ 16.13 (s, 1H), 8.84 (s, 1H), 8.47 (s, 1H), 7.65 (s, 1H), 7.32 (s, 1H), 6.94 (s, 1H), 4.46 - 4.33 (m, 2H), 3.99 - 3.85 (m, 1H), 3.78 - 3.64 (m, 2H), 3.48 - 3.38 (m, 4H), 3.35 - 3.26 (m, 1H), 3.24 - 3.15 (m, 1H), 2.33 - 2.20 (m, 2H), 2.10 - 1.95 (m, 1H), 1.5 - 1.40 (m, 6H), 0.98 (d, *J* = 6.5 Hz, 3H), 0.85 (d, *J* = 6.7 Hz, 3H).

### Example 60: 6-isopropyl-10-(4-isopropylthiazol-2-yl)-9-(3-methoxypropoxy)-2-oxo-6,7 -dihydro-2H-pyrido[2,1-a]isoquinoline-3-carboxylic acid

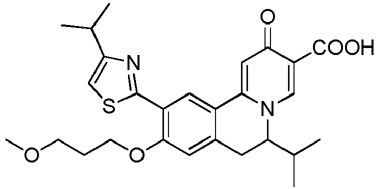

The title compound was prepared according to the synthetic method of example 59 by using ethyl 6-isopropyl-9-(3-methoxypropoxy)-2-oxo-10-(((trifluoromethyl)sulfonyl)oxy) -6,7-dihydro-2*H*-pyrido[2,1-*a*]isoquinoline-3-carboxylate (0.15 g, 0.27 mmol) and 4-isopropyl-2-(tributylstannyl)thiazole (285 mg, 0.69 mmol) as raw materials to give a gray solid (20 mg, 0.27 mmol).
MS (ESI, pos.ion) *m*/*z:* 497.1 [M+H] ⁺;
1H NMR (400 MHz, CDCl₃) δ 16.129 (br, 1H), 8.907 (s, 1H), 8.408 (s, 1H), 7.328 (d, J=7.6Hz, 1H), 7.012 (s, 1H), 6.945 (s, 1H), 4.425 - 4.340 (m, 2H), 3.989 - 3.900 (m, 1H), 3.748 - 3.665 (m, 2H), 3.495 - 3.422 (m, 1H), 3.3952 (s, 3H), 3.257 - 3.154 (m, 2H), 2.320 - 2.264 (m, 2H), 1.873 - 1.775 (m, 1H), 1.411 (d, *J* = 2.4Hz, 3H), 1.396 (d, *J* = 2.4Hz, 3H), 0.976 (d, *J* = 6.4Hz, 3H), 0.836 (d, *J* = 6.8Hz, 3H).

### Example 61: 6-isopropyl-9-(3-methoxypropoxy)-2-oxo-10-(5-phenylthiazol-2-yl)-6,7 -dihydro-2H-pyrido[2,1-a]isoquinoline-3-carboxylic acid

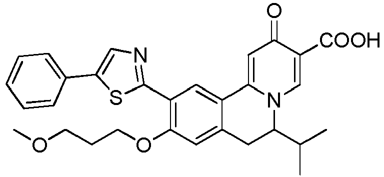

### Step 1: N-(1-(3-(3-methoxypropoxy)-4-(5-phenylthiazol-2-yl)phenyl)-3-methylbutan -2-yl)formamide

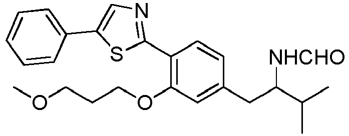

To a 50 mL two-neck flask were added *N*-(1-(4-(5-bromothiazol-2-yl)-3-(3-methoxypropoxy)phenyl)-3-methylbutan-2-yl)formamide (1.0 g, 2.26 mmol), phenylboronic acid (414 mg, 3.40 mmol), tetrakis(triphenylphosphine)palladium (261 mg, 0.23 mmol), H₂O (2 mL), K₂CO₃ (937 mg, 6.79 mmol) and dioxane (20 mL), then the mixture was heated to 100 °C and stirred overnight in nitrogen atomposphere. The mixture was cooled to rt and concentrated *in vacuo,* and to the mixture was added EA (30 mL) and water (20 mL). The mixture was partitioned, and the aqueous layer was extracted with EA (30 mL × 3). The combined organic layers were concentrated *in vacuo,* and the residue was purified by silica gel column chromatography (EA) to give the title compound as a white solid (990 mg, 2.26 mmol, 99.63%).

### Step 2: 6-isopropyl-9-(3-methoxypropoxy)-2-oxo-10-(5-phenylthiazol-2-yl)-6,7-dihydro -2H-pyrido[2,1-a]isoquinoline-3-carboxylic acid

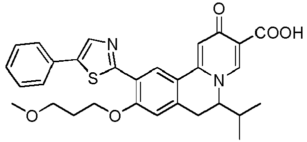

The title compound was prepared according to sythetic method of step 4 to step 8 in example 24 by using *N-*(1-(3-(3-methoxypropoxy)-4-(5-phenylthiazol-2-yl)phenyl) -3-methylbutan-2-yl)formamide (990 mg, 2.26 mmol) as a raw material to give a white solid (430 mg, 0.81 mmol).
MS (ESI, pos.ion) *m*/*z:* 531.3 [M+H]⁺;
¹H NMR (400 MHz, CDCl₃) δ 8.903 (s, 1H), 8.479 (s, 1H), 8.123 (s, 1H), 7.652 (d, *J* = 4.8Hz, 2H), 7.462 (t, *J* =5.2 Hz, 5.2Hz, 1H), 7.393 - 7.369 (m, 1H), 7.335(d, *J* = 4.0Hz, 1H), 6.984 (s, 1H), 4.485 - 4.369 (m, 2H), 3.991 - 3.906 (m, 1H), 3.767 - 3.707 (m, 2H), 3.448 - 3.384 (m, 4H), 3.265 - 3.179 (m, 1H), 2.365 - 2.312 (m, 2H), 1.866 - 1.807 (m, 1H), 0.988 (d, *J* = 4.4Hz, 3H), 0.858 (d, *J* = 6.0Hz, 3H).

### Example 62: 6-isopropyl-9-(3-methoxypropoxy)-2-oxo-10-(2-phenylthiazol-4-yl)-6,7 -dihydro-2H-pyrido[2,1-a]isoquinoline-3-carboxylic acid

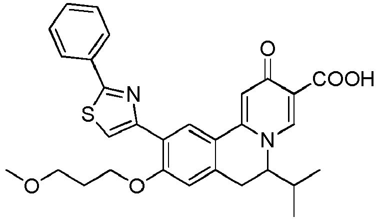

The title compound was prepared according to sythetic method of step 10 to step 14 in example 14 by using thiobenzamide (288 mg, 2.10 mmol) and N (1-(4-(2-bromoacetyl)-3-(3-methoxypropoxy)phenyl)-3-methylbutan-2-yl)formamide (0.8 g, 2 mmol) as raw materials to give a white solid (200 mg, 0.57 mmol).
MS (ESI, pos.ion) *m*/*z:* 531.1 [M+H] ⁺;
¹H NMR (400 MHz, CDCl₃) δ 16.192 (br, 1H), 8.929 (s, 1H), 8.467 (s, 1H), 8.096 (d, *J*= 6.4Hz, 2H), 8.109 (s, 1H), 7.542 - 7.490 (m, 3H), 7.314 (s, 1H), 6.930 (s, 1H), 4.390 - 4.303 (m, 2H), 3.933 - 3.899 (m, 1H), 3.666 - 3.638 (m, 2H), 3.501 - 3.429 (m, 1H), 3.397 (s, 3H), 3.25117 - 3.138 (m, 1H), 2.291 - 2.215 (m, 2H), 1.894 - 1.790 (m, 1H), 0.973 (d, *J* = 6.4Hz, 3H), 0.837 (d, *J* = 6.8Hz, 3H).

### Example 68: 6-isopropyl-9-((3-methoxycyclobutyl)methoxy)-2-oxo-10-(thiazol-2-yl) -6,7-dihydro-2H-pyrido[2,1-a]isoquinoline-3-carboxylic acid

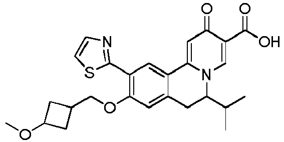

The title compound was prepared according to the synthetic method of example 19 by using 9-hydroxy-6-isopropyl-2-oxo-10-(thiazol-2-yl)-6,7-dihydro-2*H*-pyrido[2,1-*a*]isoquinoline -3-carboxylic acid (200 mg, 0.49 mmol) and (3-methoxycyclobutyl)methyl methanesulfonate (200 mg, 0.3653 mmol) as raw materials to give a gray solid (60 mg, 0.49 mmol).
MS (ESI, pos.ion) *m*/*z:* 481.30 [M+H] ⁺;
¹H NMR (400 MHz, CDCl₃) δ 8.72 (s, 1H), 8.39 (s, 1H), 7.80 (s, 1H), 7.38 (s, 1H), 7.22 (s, 1H), 6.86 (s, 1H), 4.19 - 4.10 (m, 2H), 3.84 - 3.75 (m, 1H), 3.37 - 3.24 (m, 2H), 3.16 - 3.08 (m, 4H), 2.53 - 2.41 (m, 2H), 1.84 - 1.62 (m, 3H), 1.25 - 1.20 (m, 1H), 0.84 (d, *J* = 6.5 Hz, 3H), 0.70 (d, *J* = 6.7 Hz, 3H).

### Example 69: 6-isopropyl-2-oxo-9-(pyrrolidin-1-yl)-10-(thiazol-2-yl)-6,7-dihydro-2H-pyrido[2,1-a]isoquinoline-3-carboxylic acid

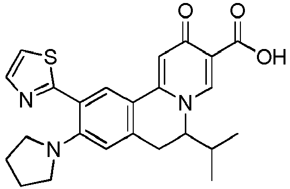

### Step 1: 4-bromo-2-(pyrrolidin-1-yl)benzonitrile

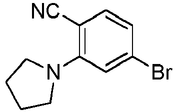

4-Bromo-2-fluorobenzonitrile (1.0 g, 5.0 mmol), DMSO (20 mL), KI (0.14 mg, 0.00084 mmol), K₂CO₃ (1.4 g, 10 mmol) and pyrrolidine (0.5 g, 7 mmol) was added into a 50 mL two-neck flask. The mixture was heated to 100 °C and stirred overnight under nitrogen protection. The mixture was cooled to rt, and to the mixture was added water (20 mL); the resulting mixture was extracted with EA (30 mL × 3); the combined organic layers were washed with water (30 mL), and the organic layers were concentrated *in vacuo* to give the title compound as a light yellow solid (1.26 g, 5.02 mmol, 100%).
MS (ESI, pos.ion) *m*/*z:* 251.0 [M+H] ⁺.

### Step 2: 4-bromo-2-(pyrrolidin-1-yl)benzothioamide

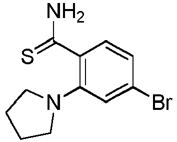

To a 100 mL single-neck flask were added 4-bromo-2-(pyrrolidin-1-yl)benzonitrile (7.04 g, 28.0 mmol), pyridine (10 mL), triethylamine (5.8 mL, 42 mmol) and an aqueous solution of ammonium sulfide (13.9 g, 42.1 mmol, mass percent: 40 %). The mixture was heated to 65 °C and stirred overnight. The mixture was cooled to rt, and to the mixture was added water (20 mL). The resulting mixture was extracted with EA (50 mL × 3). The combined organic layers were washed with saturated brine (20 mL × 3), and concentrated *in vacuo.* The residue was purified by silica gel column chromatography (PE/EA (V/V) = 4/1) to give the title compound as a red brown solid (2.0 g, 7.0 mmol, 25%).
MS (ESI, pos.ion) *m*/*z:* 285.0 [M+H] ⁺.

### Step 3: 2-(4-bromo-2-(pyrrolidin-1-yl)phenyl)thiazole

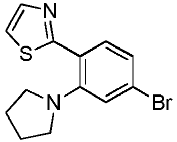

4-Bromo-2-(pyrrolidin-1-yl)benzothioamide (0.57 g, 2.0 mmol), 2-bromo-1,1-diethoxyethane (0.394 g, 2.00 mmol), EtOH (12 mL), water (1 mL) and PTSA•H₂O (0.190 g, 1.00 mmol) were added into a 100 mL single-neck flask. The mixture was heated to 100 °C and stirred overnight in nitrogen atomosphere. The mixture was cooled to rt and concentrated *in vacuo;* and the residue was purified by silica gel column chromatography (PE/EA (V/V) = 30/1) to give the title compound as brown oil (0.60 g, 1.9 mmol, 97%).
MS (ESI, pos.ion) *m*/*z:* 309.0 [M+H] ⁺.

### Step 4: 3-methyl-1-(3-(pyrrolidin-1-yl)-4-(thiazol-2-yl)phenyl)butan-2-one

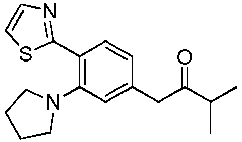

To a 100 mL two-neck flask were added 3-methylbutan-2-one (1.813 g, 21.05 mmol), 2-(4-bromo-2-(pyrrolidin-1-yl)phenyl)thiazole (2.170 g, 7.018 mmol), Pd(dba)₂ (100 mg, 0.17 mmol), Xantphos (162 mg, 0.28 mmol), sodium tert-butoxide (2.31 g, 24.07 mmol) and dixoane (20 mL). The mixture was stirred at 90 °C for 24 h under nitrogen protection. The mixture was cooled to rt and concentrated *in vacuo;* and the residue was purified by silica gel column chromatography (PE/EA (V/V) = 30/1) to give the title compound as brown oil (2.21 g, 7.02 mmol, 99.98%).
MS (ESI, pos.ion) *m*/*z:* 315.2 [M+H] ⁺.

### Step 5: 3-methyl-1-(3-(pyrrolidin-1-yl)-4-(thiazol-2-yl)phenyl)butan-2-amine

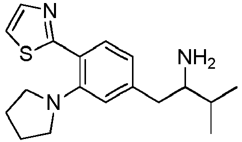

To a 250 mL single-neck flask were added 3-methyl-1-(3-(pyrrolidin-1-yl)-4-(thiazol-2-yl)phenyl)butan-2-one (2.7 g, 8.6 mmol), MeOH (30 mL) and NH₄OAc (7.9 g, 100 mmol). The mixture was stirred at rt for 1 h. The mixture was cooled to 0 °C, then NaBH₃CN (1.6 g, 25 mmol) was added. The mixture was warmed to rt and stirred overnight. To the mixture was added water (20 mL); the resulting mixture was extracted with EA (30 mL × 3); the combined organic layers were washed with saturated aqueous sodium bicarbonate (20 mL), dried over anhydous sodium sulfate and concentrated *in vacuo* to give the title compound as brown oil (2.7 g, 8.6 mmol, 100%).
MS (ESI, pos.ion) *m*/*z:* 316.1 [M+H] ⁺.

### Step 6: N-(3-methyl-1-(3-(pyrrolidin-1-yl)-4-(thiazol-2-yl)phenyl)butan-2-yl)formamide

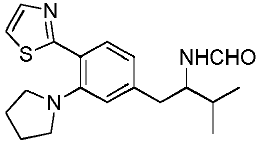

3-Methyl-1-(3-(pyrrolidin-1-yl)-4-(thiazol-2-yl)phenyl)butan-2-amine (2.7 g, 8.6 mmol) was added into a 100 mL single-neck flask, then ethyl formate (27 mL) was added. The mixture was heated to 60 °C and stirred overnight. The mixture was cooled to rt and concentrated *in vacuo;* and the residue was purified by silica gel column chromatography (PE/EA (V/V) = 30/1) to give the title compound as brown oil (1.0 g, 2.9 mmol, 34%).
MS (ESI, pos.ion) *m*/*z:* 344.2 [M+H] ⁺.

### Step 7: 5-isopropyl-8-(pyrrolidin-1-yl)-9-(thiazol-2-yl)-5,6-dihydro-2H-oxazolo[2,3-a] isoquinoline-2,3 (10bH)-dione

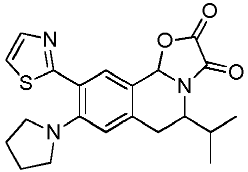

*N*-(3-Methyl-1-(3-(pyrrolidin-1-yl)-4-(thiazol-2-yl)phenyl)butan-2-yl)formamide (730 mg, 2.125 mmol) and DCM (30 mL) were added into a 50 mL single-neck flask, then the mixture was cooled to 0 °C. To the mixture was added oxalyl chloride (0.36 mL, 4.3 mmol), and the resulting mixture was stirred for 30 min. The mixture was cooled to -10 °C, and FeCl₃ (546 mg, 3.39 mmol) was added. The mixture was heated to rt and stirred overnight. The mixture was cooled to 0 °C, and quenched with water (10 mL). The mixture was extracted with DCM (30 mL × 4). The combined organic layers were dried over anhydrous sodium sulfate, filtered, and concentrated *in vacuo* to give the title compound as brown oil (760 mg, 1.912 mmol, 90 %), which was used directly in the next step.
MS (ESI, pos.ion) *m*/*z:* 398.1 [M+H] ⁺.

### Step 8: 2-(3-isopropyl-6-(pyrrolidin-1-yl)-3,4-dihydroisoquinolin-7-yl)thiazole

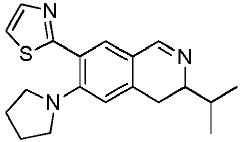

5-Isopropyl-8-(pyrrolidin-1-yl)-9-(thiazol-2-yl)-5,6-dihydro-2H-oxazolo[2,3-a] isoquinoline-2,3(10b*H*)-dione (760 mg, 1.91 mmol), MeOH (40 mL) and H₂SO₄ (0.9 mL) was added in to a 100 mL single-neck flask. The mixture was heated to 70 °C and stirred for 3 h under nitrogen protection. The mixture was cooled to rt, and concentrated *in vacuo.* To the residue were added water (15 mL) and EA (20 mL), and the mixture was partitioned. The organic layers were washed with HCl (20 mL × 4, 1 M), and the combined aqueous layers were adjusted with ammonium hydroxide to pH = 9-10. The mixture was extracted with EA (30 mL × 4), and the combined organic layers were concentrated *in vacuo* to give the title compound as brown oil (600 mg, 1.843 mmol, 96%).
MS (ESI, pos.ion) *m*/*z:* 326.4 [M+H] ⁺.

### Step 9: ethyl 6-isopropyl-2-oxo-9-(pyrrolidin-1-yl)-10-(thiazol-2-yl)-2,6,7,11b-tetrahydro -1H-pyrido[2,1-a]isoquinoline-3-carboxylate

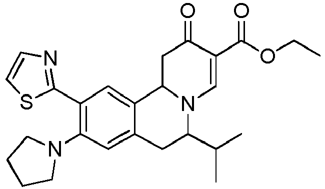

To a 100 mL single-neck flask were added ethyl 2-(ethoxymethylene)-3-oxobutanoate (543 mg, 2.919 mmol) and 2-(3-isopropyl-6-(pyrrolidin-1-yl)-3,4-dihydroisoquinolin -7-yl)thiazole (500 mg, 1.54 mmol) and EtOH (10 mL). The mixture was heated to 85 °C and stirred overnight under nitrogen protection. The reaction mixture was concentrated *in vacuo.* The residue was purified by thin-layer chromatography (PE/EA (V/V) = 1/1) to give the title compound as brown oil (80 mg, 0.17 mmol, 11%).
MS (ESI, pos.ion) *m*/*z:* 466.4 [M+H] ⁺.

### Step 10: ethyl 6-isopropyl-2-oxo-9-(pyrrolidin-1-yl)-10-(thiazol-2-yl)-6,7-dihydro-2H -pyrido[2,1-a]isoquinoline-3-carboxylate

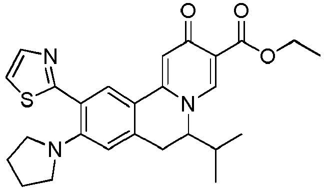

To a 50 mL single-neck flask were added ethyl 6-isopropyl-2-oxo-9-(pyrrolidin-1-yl)-10-(thiazol-2-yl)-2,6,7,11b-tetrahydro-1*H*-pyrido[2,1-*a*] isoquinoline-3-carboxylate (86 mg, 0.185 mmol), dimethoxyethane (9 mL) and chloranil (45 mg, 0.18 mmol). The mixture was stirred at 90 °C for 1 h under nitrogen protection, then cooled to rt. The reaction mixture was concentrated *in vacuo,* and the residue was purified by thin-layer chromatography (DCM/MeOH (V/V) = 10/1) to give the title compound as brown oil (53 mg, 0.11 mmol, 62%).
MS (ESI, pos.ion) *m*/*z:* 464.4 [M+H]⁺.

### Step 11: 6-isopropyl-2-oxo-9-(pyrrolidin-1-yl)-1O-(thiazol-2-yl)-6,7-dihydro-2H -pyrido[2,1-a]isoquinoline-3-carboxylic acid

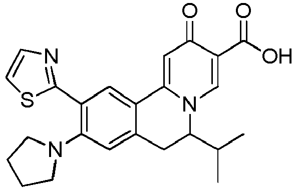

Ethyl 6-isopropyl-2-oxo-9-(pyrrolidin-1-yl)-10-(thiazol-2-yl)-6,7-dihydro-2*H* -pyrido[2,1-a]isoquinoline-3-carboxylate (53 mg, 0.1143 mmol) was dissolved in the mixed solvent of EtOH (2 mL), THF(4 mL) and H₂O (1 mL). To the mixture was added LiOH H₂O (14 mg, 0.3333 mmol), then the mixture was stirred at rt for 1.5 h. The mixture was adjusted to pH 3-4, then concentrated; to the mixture was added water (5 mL). The mixture was extracted with DCM (10 mL × 3); the combined organic layers were concentrated, and the residue was purified by preparative chromatography to give the title compound as a yellow solid (10 mg, 0.023 mmol, 20%).
MS (ESI, pos.ion) *m*/*z:* 436.2 [M+H] ⁺;
¹H NMR (400 MHz, CDCl₃) δ 8.427 (s, 1H), 7.887 (d, *J* = 3.2Hz, 1H), 7.75 (s, 1H), 7.460 (d, *J* =3.2Hz, 1H), 7.036 (s, 1H), 6.656 (s, 1H), 3.894 - 3.8519 (m, 1H), 3.776 - 3.664 (m, 1H), 3.405 - 3.327 (m, 1H), 3.215 - 3.103 (m, 4H), 2.064 - 2.000 (m, 1H), 1.976 - 1.846 (m, 4H), 0.989 (d, *J* = 6.4Hz, 3H), 0.840 (d, *J* = 6.8Hz, 3H).

### Example 70: 6-isopropyl-10-(methoxycarbonyl)-2-oxo-9-phenyl-6,7-dihydro-2H -pyrido[2,1-a]isoquinoline-3-carboxylic acid

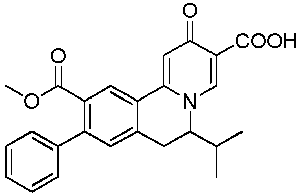

The title compound was prepared according to the sythetic method of step 8 to step 10 in example 32 by using methyl 6-bromo-3-isopropyl-3,4-dihydroisoquinoline-7-carboxylate (0.230 g, 0.741 mmol) and phenylboronic acid (149 mg, 1.2220 mmol) as raw materials to give a white solid (150 mg, 0.631 mmol).
MS (ESI, pos.ion) *m*/*z:* 418.3 [M+H]⁺;
¹H NMR (400 MHz, CDCl₃) δ 15.825 (b, 1H), 8.557 (s, 1H), 8.281 (s, 1H), 7.533 - 7.373 (m, 5H), 7.304 (s, 1H), 7.287 (s, 1H), 4.077 - 3.960 (m, 1H), 3.740 (s, 3H), 3.513 - 3.426 (m, 1H), 3.320 - 3.255 (m, 1H), 1.880 - 1.756 (m, 1H), 0.989 (d, *J=* 5.2Hz, 3H), 0.880 (d, *J=* 6.4Hz, 3H).

### Example 71: 6-isopropyl-10-(methoxycarbonyl)-9-(4-methoxyphenyl)-2-oxo-6,7-dihydro -2H-pyrido[2,1-a]isoquinoline-3-carboxylic acid

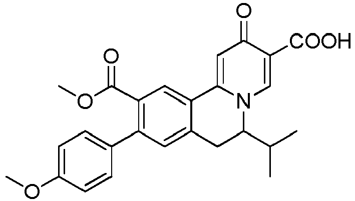

The title compound was prepared according to the sythetic method of step 8 to step 10 in example 32 by using methyl 6-bromo-3-isopropyl-3,4-dihydroisoquinoline-7-carboxylate (0.400 g, 1.29 mmol) and 4-methoxyphenylboronic acid (0.294 g, 1.93 mmol) as raw materials to give a white solid (0.30 g, 0.67 mmol).
MS (ESI, pos.ion) *m*/*z:* 448.2 [M+H]⁺;
¹H NMR (400 MHz, CDCl₃) 15.909 (b, 1H), 8.562 (s, 1H), 8.234 (s, 1H), 7.354 - 7.290 (m, 4H), 6.997 (d, *J =* 6.8Hz, 2H), 4.128 - 3.969 (m, 1H), 3.888 - 3.845 (m, 3H), 3.774 (s, 3H), 3.581 - 3.20 (m, 2H), 1.896 - 1.804 (m, 1H), 0.984 (d, *J=* 3.2, 3H), 0.869 (d, J=3.2, 3H).

### Example 72: 6-isopropyl-10-(methoxycarbonyl)-9-(1-(3-methoxypropyl)-1H-pyrazol-4-yl) -2-oxo-6,7-dihydro-2H-pyrido[2,1-a]isoquinoline-3-carboxylic acid

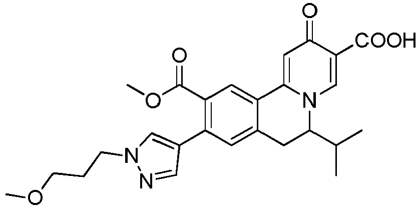

### Step 1: 4-bromo-1-(3-methoxypropyl)-1H-pyrazole

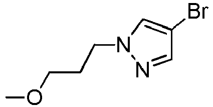

To a 100 mL single-neck flask were added 4-bromo-1*H*-pyrazole (3.0 g, 20 mmol), 1-bromo-3-methoxypropane (3.7 g, 24 mmol), cesium carbonate (13 g, 39.90 mmol) and DMF (30 mL). The mixture was heated to 50 °C and stirred overnight. The mixture was cooled to rt and to the mixture was added water (50 mL). The mixture was extracted with DCM (50 mL × 4). The combined organic layers were concentrated *in vacuo,* and the residue was purified by silica gel column chromatography (PE/EA (V/V) = 1/1) to give the title compound as colorless oil (4.3 g, 20 mmol, 96%).
MS (ESI, pos.ion) *m*/*z:* 219.0 [M+H]⁺.

### Step 2: 1-(3-methoxypropyl)-4-(4,4,5,5-tetramethyl-1,3,2-dioxaborolan-2-yl)-1H-pyrazole

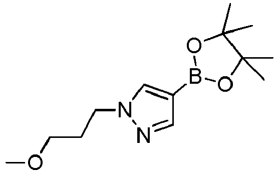

Bis(pinacolato)diboron (1.39 g, 5.47 mmol), 4-bromo-1-(3 -methoxypropyl)-1*H*-pyrazole (1.00 g, 4.56 mmol), Pd(dppf)Cl₂ (334 mg, 0.46 mmol), AcOK (1.34 g, 13.7 mmol) and 1,4-dioxane (20 mL) were added into a 50 mL two-neck flask. The mixture was heated to 85 °C and stirred for 3 h under nitrogen protection. The mixture was cooled to rt, and to the mixture was added 30 mL of water. The resulting mixture was extracted with ethyl acetate (50 mL × 3), and the combined organic layers were dried over anhydrous sodium sulfate and concentrated *in vacuo.* The residue was purified by silica gel column chromatography (PE/EA (V/V) = 4/1) to give the title compound as colorless oil (1.39 g, 5.47 mmol).
MS (ESI, pos.ion) *m*/*z:* 267.3 [M+H]⁺.

### Step 3: 6-isopropyl-10-(methoxycarbonyl)-9-(1-(3-methoxypropyl)-1H-pyrazol-4-yl) -2-oxo-6,7-dihydro-2H-pyrido[2,1-a]isoquinoline-3-carboxylic acid

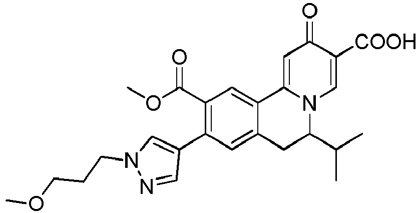

The title compound was prepared according to the sythetic method of step 8 to step 10 in example 32 by using 1-(3-methoxypropyl)-4-(4,4,5,5-tetramethyl-1,3,2 -dioxaborolan-2-yl)-1*H*-pyrazole (272 mg, 1.022 mmol) and methyl 6-bromo-3-isopropyl-3,4-dihydroisoquinoline-7-carboxylate (212 mg, 0.6834 mmol) as raw materials to give a white solid (126 mg, 0.26 mmol).
MS (ESI, pos.ion) *m*/*z:* 480.2 [M+H]⁺;
¹H NMR (400 MHz, CDCl₃) δ 15.797 (b, 1H), 8.517 (s, 1H), 8.166 (s, 1H), 7.695 (d, *J* = 4.2Hz, 2H), 7.391 (s, 1H), 7.244 (s, 1H), 4.298 (t, *J* = 6.8, 6.8Hz, 2H), 4.030 - 3.960 (m, 1H), 3.907 (s, 3H), 3.495 - 3.350 (m, 6H), 3.308 - 3.231 (m, 1H), 2.238 - 2.127 (m, 2H), 1.840 - 1.733 (m, 1H), 0.984 (d, *J* = 6.4Hz, 3H), 0.864 (d, *J* = 6.4Hz, 3H).

### Example 73: 9-(5-chlorothiophen-2-yl)-6-isopropyl-10-(methoxycarbonyl)-2-oxo-6,7 -dihydro-2H-pyrido[2,1-a]isoquinoline-3-carboxylic acid

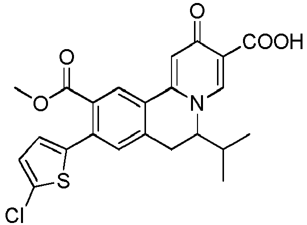

The title compound was prepared according to the sythetic method of step 8 to step 10 in example 32 by using 2-(5-chlorothiophen-2-yl)-4,4,5,5-tetramethyl-1,3,2-dioxaborolane (236 mg, 0.97 mmol) and methyl 6-bromo-3-isopropyl-3,4-dihydroisoquinoline-7-carboxylate (200 mg, 0.64 mmol) as raw materials to give a light yellow solid (92 mg, 0.20 mmol).
MS (ESI, pos.ion) *m*/*z:* 458.0 [M+H]⁺;
¹H NMR (400 MHz, CDCl₃) δ 15.682 (b, 1H), 8.529 (s, 1H), 8.177 (s, 1H), 7.407 (s, 1H), 7.262 (s, 1H), 6.958 - 6.935 (m, 2H), 4.035 - 3.963 (m, 1H), 3.882 (s, 3H), 3.495 - 3.414 (m, 1H), 3.322 - 3.236 (m, 1H), 1.827 - 1.748 (m, 1H), 0.989 (d, *J* = 4.4Hz, 3H), 0.873 (d, *J* = 4.4Hz, 3H).

### Example 74: 9-(5-chlorofuran-2-yl)-6-isopropyl-10-(methoxycarbonyl)-2-oxo-6,7-dihydro -2H-pyrido[2,1-a]isoquinoline-3-carboxylic acid

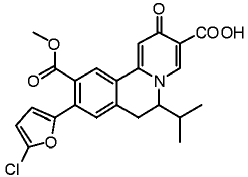

The title compound was prepared according to the sythetic method of example 32 by using methyl 6-bromo-3-isopropyl-3,4-dihydroisoquinoline-7-carboxylate (200 mg, 0.64 mmol) as a raw material to give a light yellow solid (30 mg, 0.20 mmol).
MS (ESI, pos.ion) *m*/*z:* 442.0 [M+H]⁺;
¹H NMR (600 MHz, CDCl₃) δ 15.71 (s, 1H), 8.51 (s, 1H), 8.08 (s, 1H), 7.63 (s, 1H), 7.24 (s, 1H), 6.82 (d, *J* = 3.5 Hz, 1H), 6.35 (d, *J* = 3.5 Hz, 1H), 4.00-3.93 (m, 4H), 3.45 (dd, *J* = 16.3, 5.1 Hz, 1H), 3.31 (d, *J* = 15.9 Hz, 1H), 1.83 - 1.72 (m, 1H), 0.99 (d, *J* = 6.6 Hz, 3H), 0.87 (d, *J* = 6.7 Hz, 3H).

### Example 75: 6-isopropyl-10-methoxy-2-oxo-9-(thiazol-2-yl)-6,7-dihydro-2H -pyrido[2,1-a]isoquinoline-3-carboxylic acid

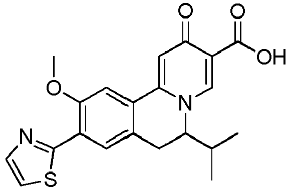

### Step 1: 1-(4-methoxyphenyl)-3-methylbutan-2-one

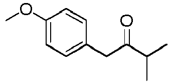

To a reaction flask were added 1-iodo-4-methoxybenzene (53 g, 226.47 mmol), 3-methylbutan-2-one (59 g, 685.0 mmol), Xantphos (13 g, 22.47 mmol), Pd₂(dba)₃ (10 g, 10.92 mmoll), t-BuONa (43 g, 447.43 mmol) and tetrahydrofuran (500 mL). The mixture was degassed and filled with nitrogen for three times, then heated to 60 °C and stirred for 12 h. The mixture was cooled to rt, filtered, and the filtrate was concentrated *in vacuo.* The residue was purified by silica gel column chromatography (PE/EA (V/V) = 30/1) to give the title compound as yellow oil (36 g, 187.26 mmol, 83%).
MS (ESI, pos.ion) *m*/*z:* 193.1 [M+H]⁺.

### Step 2: 1-(4-methoxyphenyl)-3-methylbutan-2-amine

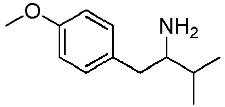

To a reaction flask were added 1-(4-methoxyphenyl)-3-methylbutan-2-one (36 g, 187.26 mmol), methanol (360 mL) and ammonium acetate (101 g, 1310.29 mmol). The mixture was stirred at rt for 1 h, then cooled to 0 °C, and sodium cyanoborohydride (24 g, 381.91 mmol) was added in portions, and the resulting mixture was stirred at rt for 24 h. The reaction mixture was concentrated *in vacuo* to remove methanol, and to the residue was added saturated aqueous sodium bicarbonate (300 mL) to quench the reaction. The resulting mixture was extracted with EA (300 mL × 2). The combined organic layers were washed with saturated aqueous sodium chloride (200 mL), dried over anhydous sodium sulfate and concentrated *in vacuo* to give the title compound as yellow oil (31.4 g, 162 mmol, 87%).
MS (ESI, pos.ion) *m*/*z:* 194.3[M+H]⁺.

### Step 3: tert-butyl (1-(4-methoxyphenyl)-3-methylbutan-2-yl)carbamate

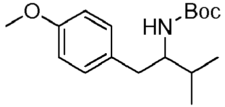

To a reaction flask were added 1-(4-methoxyphenyl)-3-methylbutan-2-amine (31 g, 163 mmol) and dichloromethane (300 mL). The mixture was cooled to 0 °C, then Boc₂O (43 g, 195 mmol) and triethylamine (33 g, 330 mmol) were added. After addition, the mixture was warmed to rt and stirred for 12 h, and saturated aqueous ammonium chloride (200 mL) was added. The mixture was partitoned, and the organic layers were concentrated *in vacuo.* The residue was purified by silica gel column chromatography (PE/EA (V/V) = 20/1) to give the title compound as a white solid (43 g, 146.6 mmol, 90%).
MS (ESI, pos.ion) *m*/*z:* 317.3[M+Na]⁺.

### Step 4: tert-butyl (1-(3-iodo-4-methoxyphenyl)-3-methylbutan-2-yl)carbamate

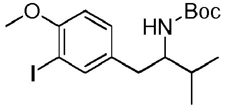

To a reaction flask were added *tert*-butyl (1-(4-methoxyphenyl)-3-methylbutan-2-yl)carbamate (30 g, 102.2 mmol) and methanol (400 mL). The mixture was stirred to dissolve the reagent, then silver sulfate (35 g, 113 mmol) and iodine (28.6 g, 113 mmol) were added. The mixture was stirred at rt for 1 h. The mixture was filtered and concentrated *in vacuo.* The residue was dissolved in ethyl acetate (400 mL), and the mixture was washed with 20% Na₂S₂O₃ solution (200 mL × 2) and saturated sodium chloride (200 mL) in turn. The organic layer was concentrated *in vacuo,* and the residue was purified by ilica gel column chromatography (PE/EA (V/V) = 15/1) to give the title compound as a white solid (25 g, 59.62 mmol, 58%).
MS (ESI, pos.ion) *m*/*z:* 342.0[M+Na]⁺; 354.0[M-tBu]⁺.

### Step 5: 1-(3-iodo-4-methoxyphenyl)-3-methylbutan-2-amine

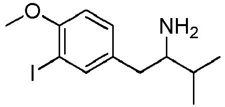

To a reaction flask were added tert-butyl (1-(3-iodo-4-methoxyphenyl)-3-methylbutan-2-yl)carbamate (10 g, 23.85 mmol) and dichloromethane (50 mL). The mixture was stirred to dissolve the reagent, then trifluoroacetic acid (50 mL) was added. The mixture was stirred at rt for 4 h. The mixture was concentrated *in vacuo,* and the residue was dissolved in ethyl acetate (100 mL). The mixture was adjusted with 20% aqueous potassium carbonate till the pH value of the aqueous layer was 8, and the resulting mixture was partitioned. The aqueous layer was extracted with ethyl acetate (100 mL), and the organic layer was washed with saturated aqueous sodium chloride (100 mL), dried over anhydrous sodium sulfate, then concentrated *in vacuo* to remove the solvent and give the title compound as a yellow oil (7.6 g, 24 mmol, 100%), which was used directly in the next step.

### Step 6: N-(1-(3-iodo-4-methoxyphenyl)-3-methylbutan-2-yl)formamide

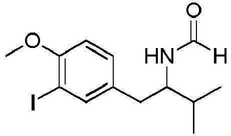

To a reaction flask were added 1-(3-iodo-4-methoxyphenyl)-3-methylbutan-2-amine (7.5 g, 23 mmol) and ethyl acetate (200 mL). The mixture was heated to reflux and stirred for 12 h. The reaction mixture was washed with saturated aqueous sodium bicarbonate (100 mL) and sodium chloride (100 mL) in turn, dried over anydrous sodium sulfate and concentrated *in vacuo* to remove the solvent and give the title compound as a light yellow solid (6.3 g, 18 mmol, 77%).
MS (ESI, pos.ion) *m*/*z:* 348.1 [M+1]⁺.

### Step 7: 6-iodo-3-isopropyl-7-methoxy-3,4-dihydroisoquinoline

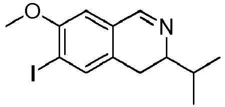

To a reaction flask were added *N-*(1-(3-iodo-4-methoxyphenyl) -3-methylbutan-2-yl)formamide (6.0 g, 17.2 mmol) and dichloromethane (150 mL). The mixture was degassed and filled with nitrogen for three times, then the mixture was stirred to dissolve the reagent. Oxalyl chloride (2.8 g, 24 mol) was added. The mixture was stirred at rt for 1 h. The reaction mixture was cooled to -10 °C, and ferric trichloride (1.7 g, 20 mol) was added. The mixture was warmed to rt and stirred for 12 h. The reaction was quenched with HCl (2 M, 100 mL), and the mixture was partitioned. The aqueous layer was extracted with dichloromethane (50 mL), then the organic layer was washed with saturated aqueous sodium chloride (50 mL), dried over anhydrous sodium sulfate and concentrated *in vacuo* to remove the solvent. Methanol (150 mL) was added to dissolve the residue, and concentrated sulfuric acid (7.5 mL) was added. The resulting mixture was refluxed for 12 h. The mixture was cooled to rt, and concentrated *in vacuo.* The residue was adjusted with saturated aqueous sodium bicarbonate to pH = 8, then the mixture was extracted with ethyl acetate (60 mL × 2). The combined organic layers were concentrated *in vacuo,* and the residue was purified by silica gel column chromatography (PE/EA (V/V) = 3/1) to give the title compound as red oil (3.0 g, 8.6 mmol, 50%).
MS (ESI, pos.ion) *m*/*z:* 330.1[M+1]⁺;

### Step 8: ethyl 9-iodo-6-isopropyl-10-methoxy-2-oxo-2,6,7,11b-tetrahydro-1H-pyrido[2,1-a] isoquinoline-3-carboxylate

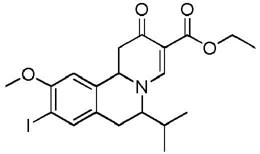

To a reaction flask were added 6-iodo-3-isopropyl-7-methoxy-3,4-dihydroisoquinoline (3 g, 9.1 mmol), ethyl 2-(ethoxymethylene)-3-oxobutanoate (8.5 g, 45.5 mol) and ethanol (30 mL). The reaction mixture was refluxed for 12 h. The reaction mixture was concentrated *in vacuo* to remove the solvent, and the residue was used directly in the next step.
MS (ESI, pos.ion) *m*/*z:* 470.1[M+1]⁺.

### Step 9: ethyl 9-iodo-6-isopropyl-10-methoxy-2-oxo-6,7-dihydro-2H-pyrido[2,1-a]isoquinoline -3-carboxylate

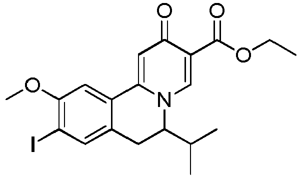

To a reaction flask were added ethyl 9-iodo-6-isopropyl-10-methoxy -2-oxo-2,6,7,11b-tetrahydro-1*H*-pyrido[2,1-*a*]isoquinoline-3-carboxylate (4.3 g, 9.2 mmol), chloranil (2.3 g, 9.4 mmol) and dimethoxyethane (80 mL). The mixture was refluxed for 8 h. The mixture was concentrated *in vacuo,* and the residue was purified by silica gel column chromatography (DCM/MeOH (V/V) = 15/1) to give the title compound as a brown solid (3.6 g, 7.7 mmol, 84%).
MS (ESI, pos.ion) *m*/*z:* 468.1 [M+1]⁺.

### Step 10: 6-isopropyl-10-methoxy-2-oxo-9-(thiazol-2-yl)-6,7-dihydro-2H-pyrido[2,1-a] isoquinoline-3-carboxylic acid

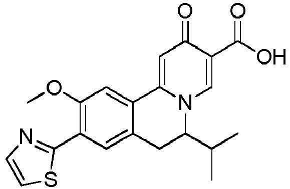

To a dried reaction flask were added ethyl 9-iodo-6-isopropyl-10-methoxy -2-oxo-6,7-dihydro-2*H*-pyrido[2,1-*a*]isoquinoline-3-carboxylate (300 mg, 0.64 mmol), 2-(tributylstannyl)thiazole (0.48 g, 1.28 mmol), bis(triphenylphosphine)palladium(II) chloride (0.1 g, 0.1 mmol) and 1,4-dioxane (10 mL). After addition, the mixture was degassed and filled with nitrogen for three times, then heated to 110 °C and stirred for 12 h. The reaction mixture was concentrated *in vacuo,* and the residue was purified by silica gel column chromatography (DCM/MeOH (V/V) = 15/1) to give the title compound as a light yellow solid (170 mg, 0.43 mmol, 67%).
MS (ESI, pos.ion) *m*/*z:* 397.0[M+1]⁺;
¹H NMR (600 MHz, DMSO-*d*₆) δ 8.86 (s, 1H), 8.35 (s, 1H), 8.02 (d, *J* = 3.0 Hz, 1H), 7.89 (d, *J* = 3.0 Hz, 1H), 7.83 (s, 1H), 7.71 (s, 1H), 4.53 (d, *J* = 9.8 Hz, 1H), 4.16 (s, 3H), 3.35 - 3.15 (m, 2H), 1.75 - 1.44 (m, 1H), 0.88 (d, *J* = 6.6 Hz, 3H), 0.71 (d, *J* = 6.6 Hz, 3H).

### Example 76: 9-(4-bromothiazol-2-yl)-6-isopropyl-10-methoxy-2-oxo-6.7-dihydro-2H -pyrido[2,1-a]isoquinoline-3-carboxylic acid

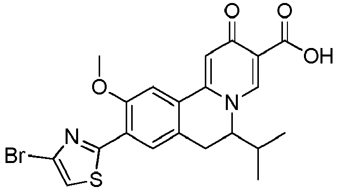

The title compound was prepared according to the synthetic method of step 10 in example 76 by using ethyl 9-iodo-6-isopropyl-10-methoxy-2-oxo-6,7-dihydro-2*H*-pyrido[2,1-*a*] isoquinoline-3-carboxylate (300 mg, 0.64 mmol) and 4-bromo-2-(tributylstannyl)thiazole (0.44 g, 0.96 mmol) as raw materials to give a white solid (80 mg, 0.17 mmol).
MS (ESI, pos.ion) *m*/*z:* 475.1[M+1]⁺;
¹H NMR (400 MHz, DMSO-*d*₆) δ 9.26 (s, 1H), 8.85 (s, 1H), 7.92 - 7.43 (m, 3H), 4.51 (s, 1H), 3.95 (s, 3H), 3.32 - 3.24 (m, 2H), 1.75 - 1.50 (m, 1H), 0.80 (dd, *J* = 59.0, 5.3 Hz, 6H).

### Example 77: 6-isopropyl-9-(5-isopropylthiazol-2-yl)-10-methoxy-2-oxo-6,7-dihydro-2H-pyrido[2,1-a]isoquinoline-3-carboxylic acid

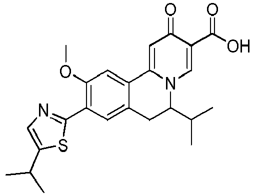

The title compound was prepared according to the synthetic method of step 10 in example 76 by using ethyl 9-iodo-6-isopropyl-10-methoxy-2-oxo-6,7-dihydro-2*H*-pyrido[2,1-*a*] isoquinoline-3-carboxylate (300 mg, 0.64 mmol) and 4-bromo-2-(tributylstannyl)thiazole (0.44 g, 0.96 mmol) as raw materials to give a white solid (80 mg, 0.17 mmol).
MS (ESI, pos.ion) *m*/*z:* 439.1[M+1]⁺;
¹H NMR (600 MHz, DMSO-*d*₆) δ 8.86 (s, 1H), 8.28 (s, 1H), 7.80 (s, 1H), 7.74 (s, 1H), 7.69 (s, 1H), 4.52 (d, *J* = 9.8 Hz, 1H), 4.14 (s, 3H), 3.33 - 3.23 (m, 2H), 3.18 (d, *J* = 5.1 Hz, 1H), 1.65 - 1.51 (m, 1H), 1.34 (d, *J* = 6.9 Hz, 6H), 0.87 (d, *J* = 7.4 Hz, 3H), 0.71 (d, *J* = 6.7 Hz, 3H).

### Example 78: 6-isopropyl-10-methoxy-9-(4-(methoxymethyl)thiazol-2-yl)-2-oxo-6,7 -dihydro-2H-pyrido[2,1-a]isoquinoline-3-carboxylic acid

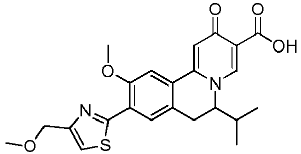

The title compound was prepared according to the synthetic method of step 10 in example 76 by using ethyl 9-iodo-6-isopropyl-10-methoxy-2-oxo-6,7-dihydro -2*H*-pyrido[2,1-*a*]isoquinoline-3-carboxylate (300 mg, 0.64 mmol) and 4-(methoxymethyl)-2-(tributylstannyl)thiazole (0.67 g, 1.605 mmol) as raw materials to give a white solid (100 mg, 0.2270 mmol).
MS (ESI, pos.ion) *m*/*z:* 441.3[M+1]⁺;
¹H NMR (400 MHz, CDCl₃) δ 15.78 (s, 1H), 8.54 (s, 1H), 8.42 (s, 1H), 7.43 (s, 1H), 7.39 (s, 1H), 7.28 (s, 1H), 4.69 (s, 2H), 4.14 (s, 3H), 4.01 - 3.92 (m, 1H), 3.53 (s, 3H), 3.39 (dd, *J* = 16.3, 4.1 Hz, 1H), 3.30 (d, *J* = 16.1 Hz, 1H), 1.84 - 1.78 (m, 1H), 0.92 (dd, *J* = 52.3, 6.6 Hz, 6H).

### Example 79: 9-(5-chlorothiophen-2-yl)-6-isopropyl-10-methoxy-2-oxo-6,7-dihydro -2H-pyrido[2,1-a]isoquinoline-3-carboxylic acid

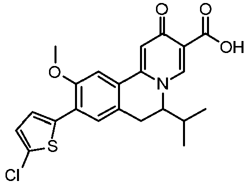

The title compound was prepared according to the synthetic method of example 16 by using ethyl 9-iodo-6-isopropyl-10-methoxy-2-oxo-6,7-dihydro-2*H*-pyrido[2,1-*a*]isoquinoline-3-carboxylate (300 mg, 0.64 mmol) and 2-(5-chlorothiophen-2-yl)-4,4,5,5-tetramethyl-1,3,2 -dioxaborolane (235 mg, 0.96 mmol) as raw materials to give a gray solid (210 mg, 0.49 mmol).
MS (ESI, pos.ion) *m*/*z:* 430.0[M+1]⁺;
¹H NMR (400 MHz, DMSO-*d*₆) δ 8.83 (s, 1H), 7.94 (s, 1H), 7.76 - 7.59 (m, 3H), 7.21 (d, *J* = 3.2 Hz, 1H), 4.57 - 4.45 (m, 1H), 4.07 (s, 3H), 3.29 - 3.21 (m, 2H), 1.61-1.50 (m,1H), 0.80 (dd, *J* = 69.3, 6.0 Hz, 6H).

### Example 80: 9-(5-bromothiazol-2-yl)-6-isopropyl-10-methoxy-2-oxo-6,7-dihydro -2H-pyrido[2,1-a]isoquinoline-3-carboxylic acid

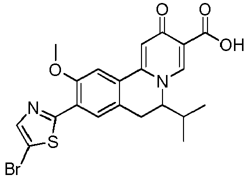

### Step 1: tert-butyl (1-(4-methoxy-3-(thiazol-2-yl)phenyl)-3-methylbutan-2-yl)carbamate

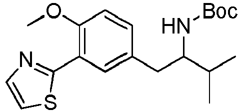

To a dried reaction flask were added 1-(3-iodo-4-methoxyphenyl) -3-methylbutan-2-amine (1.30 g, 3.10 mmol), 2-(tributylstannyl)thiazole (1.74 g, 4.65 mmol), bis(triphenylphosphine)palladium(II) chloride (0.54 g, 0.78 mmol) and 1,4-dioxane (30 mL). After addition, the mixture was degassed and filled with nitrogen for three times, then heated to 110 °C and stirred for 12 h. The reaction mixture was concentrated *in vacuo,* and the residue was purified by silica gel column chromatography (PE/EA (V/V) = 6/1) to give the title compound as a yellow solid (1.0 g, 2.66 mmol, 86%).
MS (ESI, pos.ion) *m*/*z:* 377.4 [M+1]⁺.

### Step 2: tert-butyl (1-(3-(5-bromothiazol-2-yl)-4-methoxyphenyl)-3-methylbutan-2-yl)carbamate

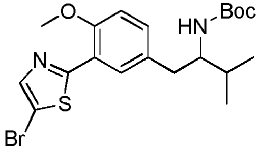

To a reaction flask were added tert-butyl (1-(4-methoxy-3-(thiazol-2-yl)phenyl)-3-methylbutan-2-yl)carbamate (0.90 g, 2.39 mmol), dichloromethane (20 mL) and NBS (0.425 g, 2.39 mmol). The mixture was stirred at rt for 30 min. The reaction mixture was concentrated *in vacuo,* and the residue was purified by silica gel column chromatography (PE/EA (V/V) = 6/1) to give the title compound as a light yellow solid (0.90 g, 1.98 mmol, 82.68%).
MS (ESI, pos.ion) *m*/*z:* 455.1[M+1]⁺.

### Step 3: 1-(3-(5-bromothiazol-2-yl)-4-methoxyphenyl)-3-methylbutan-2-amine

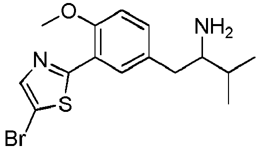

To a reaction flask were added tert-butyl (1-(3-(5-bromothiazol-2-yl) -4-methoxyphenyl)-3-methylbutan-2-yl)carbamate (0.9 g, 2 mmol) and dichloromethane (5 mL). The mixture was stirred to dissolve the reagent, then trifluoroacetic acid (5 mL) was added. The mixture was stirred at rt for 4 h. The mixture was concentrated *in vacuo,* and the residue was dissolved in ethyl acetate (30 mL). The mixture was adjusted with 20% aqueous potassium carbonate till the pH value of the aqueous layer was 8, and the resulting mixture was partitioned. The aqueous layer was extracted with ethyl acetate (20 mL), then the combined organic layers were washed with saturated aqueous sodium chloride (30 mL), dried over anhydrous sodium sulfate and concentrated *in vacuo* to remove the solvent and give the title compound as yellow oil (0.7 g, 2 mmol, 100%), which was used directly in the next step.
MS (ESI, pos.ion) *m*/*z:* 355.1[M+1]⁺.

### Step 4: N-(1-(3-(5-bromothiazol-2-yl)-4-methoxyphenyl)-3-methylbutan-2-yl)formamide

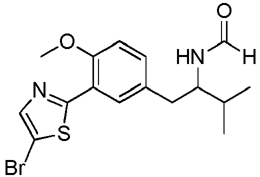

To a reaction flask were added 1-(3-(5-bromothiazol-2-yl)-4-methoxyphenyl) -3-methylbutan-2-amine (0.7 g, 2 mmol) and ethyl formate (200 mL). The mixture was heated to reflux and stirred for 12 h. The reaction mixture was washed with saturated aqueous sodium bicarbonate (200 mL) and sodium chloride (200 mL) in turn, dried over anydrous sodium sulfate and concentrated *in vacuo* to remove the solvent and give the title compound as a light yellow solid (0.8 g, 2 mmol, 100%).
MS (ESI, pos.ion) *m*/*z:* 383.1 [M+1]⁺.

### Step 5: 5-bromo-2-(3-isopropyl-7-methoxy-3,4-dihydroisoquinolin-6-yl)thiazole

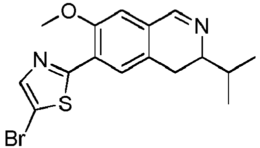

To a reaction flask were added *N-*(1-(3-(5-bromothiazol-2-yl) -4-methoxyphenyl)-3-methylbutan-2-yl)formamide (0.9 g, 2 mmol) and dichloromethane (20 mL). The mixture was degassed and filled with nitrogen for three times, then the mixture was stirred to dissolve the reagent and oxalyl chloride (0.3 g, 2.2 mmol) was added. The mixture was stirred at rt for 1 h. The reaction mixture was cooled to -10 °C, and ferric trichloride (0.4 g, 2.4 mmol) was added. The mixture was warmed to rt and stirred for 12 h. The reaction was quenched with HCl (2 M, 20 mL), and the mixture was partitioned. The aqueous layer was extracted with dichloromethane (20 mL), then the organic layer was washed with saturated aqueous sodium chloride (30 mL), dried over anhydrous sodium sulfate and concentrated *in vacuo.* The residue was dissolved in methanol (30 mL), and to the mixture was added concentrated sulfuric acid (1.5 mL). The mixture was refluxed for 12 h, then concentrated *in vacuo,* and to the residue was added saturated aqueous sodium bicarbonate to adjust pH = 8. The mixture was extracted with ethyl acetate (20 mL × 2). The combined organic layers were concentrated *in vacuo,* and the residue was purified by silica gel column chromatography (PE/EA (V/V) = 3/1) to give the title compound as red oil (90 mg, 0.2464 mmol, 10%).
MS (ESI, pos.ion) *m*/*z:* 365.0[M+1]⁺.

### Step 6: ethyl 9-(5-bromothiazol-2-yl)-6-isopropyl-10-methoxy-2-oxo-2,6,7,11b-tetrahydro-1H-pyrido[2,1-a]isoquinoline-3-carboxylate

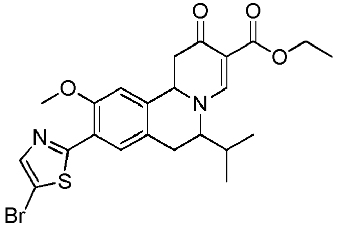

To a reaction flask were added 5-bromo-2-(3-isopropyl-7-methoxy -3,4-dihydroisoquinolin-6-yl)thiazole (90 mg, 0.2464 mmol), ethyl 2-(ethoxymethylene)-3-oxobutanoate (0.25 g, 1.3 mmol) and ethanol (2 mL). The reaction mixture was refluxed for 12 h. The reaction mixture was concentrated *in vacuo* to remove the solvent, and the residue was used directly in the next step.
MS (ESI, pos.ion) *m*/*z:* 505.1[M+1]⁺.

### Step 7 : ethyl 9-(5-bromothiazol-2-yl)-6-isopropyl-10-methoxy-2-oxo-6,7-dihydro -2H-pyrido[2,1-a]isoquinoline-3-carboxylate

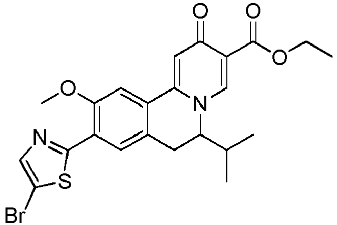

To a reaction flask were added ethyl 9-(5-bromothiazol-2-yl) -6-isopropyl-10-methoxy-2-oxo-2,6,7,11b-tetrahydro-1*H*-pyrido[2,1-*a*]isoquinoline-3-carboxylate (0.13 g, 0.26 mmol), chloranil (0.063 g, 0.26 mmol) and dimethoxyethane (80 mL). The mixture was refluxed for 8 h. The reaction mixture was concentrated *in vacuo,* and the residue was purified by silica gel column chromatography (DCM/MeOH (V/V) = 15/1) to give the title compound as a brown solid (76 mg, 0.15 mmol, 59%).
MS (ESI, pos.ion) *m*/*z:* 503.0 [M+1]⁺.

### Step 8: 9-(5-bromothiazol-2-yl)-6-isopropyl-10-methoxy-2-oxo-6,7-dihydro-2H-pyrido[2,1-a] isoquinoline-3-carboxylic acid

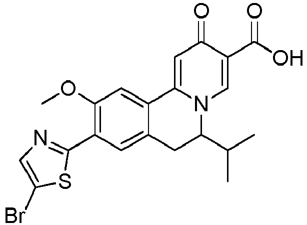

Ethyl 9-(5-bromothiazol-2-yl)-6-isopropyl-10-methoxy-2-oxo-6,7-dihydro -2*H*-pyrido[2,1-*a*]isoquinoline-3-carboxylate (76 mg, 0.1510 mmol) was dissolved in methanol (3 mL, 74.2 mmol), and lithium hydroxide (32 mg, 0.7619 mmol) was added. The mixture was stirred at rt for 12 h. The reaction mixture was adjusted with hydrochloric acid (1 M) to pH =2, and there was a solid precipitated out. The mixture was filtered, and the filter cake was recrystallized from methanol (5 mL) to give the title compound as a gray solid (42 mg, 0.088 mmol, 58.52%).
MS (ESI, pos.ion) *m*/*z:* 475.0[M+1]⁺;
¹H NMR (400 MHz, DMSO-*d*₆) δ 8.86 (s, 1H), 8.28 (s, 1H), 8.09 (s, 1H), 7.85 (s, 1H), 7.71 (s, 1H), 4.53 (d, *J* = 9.8 Hz, 1H), 4.17 (s, 3H), 3.39 - 3.35 (m, 2H), 1.65 - 1.53 (m, 1H), 0.88 (d, *J* = 6.6 Hz, 3H), 0.71 (d, *J* = 6.6 Hz, 3H).

### Example 81: 6-isopropyl-10-(2-(4-methoxyphenyl)-2-oxoethoxy)-9-(3-methoxypropoxy) -2-oxo-6,7-dihydro-2H-pyrido[2,1-a]isoquinoline-3-carboxylic acid

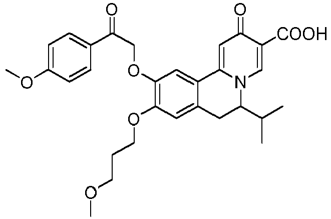

The title compound was prepared according to the synthetic method of example 29 by using ethyl 10-hydroxy-6-isopropyl-9-(3-methoxypropoxy)-2-oxo-6,7-dihydro-2*H-*pyrido[2,1-a]isoquinoline-3-carboxylate (0.400 g, 1.03 mmol) and 2-bromo-1-(4-methoxyphenyl)ethanone (130 mg, 0.57 mmol) as raw materials to give a white solid (0.28 g, 0.6 mmol).
MS (ESI, pos.ion) *m*/*z:* 536.3 [M+H]⁺;
¹H NMR (400 MHz, CDCl₃) δ 16.005 (br, 1H), 8.436 (s, 1H), 8.029 (d, *J* = 8.8Hz, 1H), 8.341 (s, 2H), 7.181 (s, 1H), 7.019 (d, *J* = 8.8Hz, 1H), 6.942 (s, 1H), 6.823 (s, 1H), 5.373 (s, 2H), 4.252 - 4.186 (m, 2H), 3.928 (s, 3H), 3.873 - 3.830 (m, 1H), 3.614 - 3.574 (m, 2H), 3.376 (s, 3H), 3.329 - 3.294 (m, 1H), 3.108 - 3.056 (m, 1H), 2.183 - 2.114 (m, 2H), 1.879 - 1.793 (m, 1H), 0.959 (d, *J* = 6.4Hz, 3H), 0.834 (d, *J* = 6.8Hz, 3H).

### Biological assays

### HBV cell line

The chromosomes of HepG2.2.15 cells (SELLS, PNAS, 1987 and SELLS, JV, 1988) integrate the complete HBV genome and stably express viral RNA and viral proteins. HepG2.2.15 cells are able to secrete mature HBV particles and HBsAg into the culture medium. Viral particle DNA and HBsAg secreted by HepG 2.2.15 cells can be quantified by qPCR and ELISA methods, and the effects of the compound on virus replication and HBsAg secretion are thus examined.

### Assay 1: Inhibition of HBV Virus replication by compounds of the invention

### Test method

HepG 2.2.15 Cells (8,000 per well) were seeded into 96-well cell culture plates in duplicate and cultured for 3 days until the cells grew to fill in pores. Cells were treated with 4-fold serial diluted compound solution for 10 days, and the compound solution was exchanged once every other day, the final concentration of DMSO in all wells was 0.5% and DMSO was used as a drug-free control. On the eleventh day, the supernatant was collected for quantitative detection of HBV DNA.

qPCR method was used for detection of viral genomic DNA, HBV primers were as follows:
HBV-For-202, CAGGCGGGGTTTTTCTTGTTGA (SEQ ID NO:1);
HBV-Rev-315, GTGATTGGAGGTTGGGGACTGC (SEQ ID NO:2).

Using the SYBR Premix Ex Taq II - Takara DRR081S kit, 1 µL of the cell culture supernatant was used as a template, a plasmid containing the HBV genome was used for ploting a standard curve, and virus copy number was calculated using the standard curve. Concentration-virus copy number was treated with Graphpad Prism 5 software and the IC₅₀ of inhibition of virus replication by compounds was calculated by a four-parameter nonlinear regression model.

Conclusion: The inhibition experiment of HBV virus replication by the compound of the present invention shows that the IC₅₀ of the inhibitory activity of the compound of the present invention against HBV DNA is less than 0.5 µM, and the IC₅₀ of the inhibitory activity of most compounds against HBV DNA replication is less than 0.1 µM. The inhibitory activities against HBV DNA replication of part of the compounds were as shown in table 2.

**Table 2: inhibitory activities against HBV DNA replication of part of the compounds in the invention**

| **Example** | **DNA IC₅₀ (nM)** | **Example** | **DNA IC₅₀ (nM)** |
|---|---|---|---|
| Example 1 | 6.30 | Example 5 | 10.60 |
| Example 6 | 3.02 | Example 7 | 8.16 |
| Example 8 | 4.46 | Example 9 | 0.18 |
| Example 10 | 7.29 | Example 11 | 1.90 |
| Example 13 | 0.64 | Example 14 | 3.36 |
| Example 15 | 0.39 | Example 16 | 1.99 |
| Example 17 | 3.60 | Example 18 | 8.09 |
| Example 20 | 8.79 | Example 21 | 0.30 |
| Example 22 | 0.84 | Example 24 | 8.63 |
| Example 30 | 7.68 | Example 31 | 6.26 |
| Example 32 | 7.57 | Example 33 | 1.93 |
| Example 34 | 4.74 | Example 35 | 7.59 |
| Example 36 | 2.55 | Example 47 | 6.35 |
| Example 53 | 7.99 | Example 67 | 4.36 |
| Example 69 | 10.18 | Example 73 | 3.88 |
| Example 74 | 2.97 | Example 75 | 2. 10 |
| Example 77 | 2.79 | Example 78 | 2.84 |
| Example 79 | 2.01 | Example 80 | 2.57 |

### Assay 2: Inhibition of HBsAg secretion by compounds of the invention

### Test method

HepG 2.2.15 Cells (8,000 per well) were seeded into 96-well cell culture plates in duplicate and cultured for 3 days until the cells grew to fill in pores. Cells were treated with 4-fold serial diluted compound solution for 10 days, and the compound solution was exchanged once every other day, the final concentration of DMSO in all wells was 0.5% and DMSO was used as a drug-free control. On the eleventh day, the supernatant was collected for quantitative detection of HBsAg.

The level of HBsAg secreted by the cells after treatment with the compound was detected by ELISA. The method used a hepatitis B surface antigen diagnostic kit (Shanghai Kehua Biotechnology Co., Ltd. S10910113). 25 µL of the supernatant to be examined (diluted to 75 µL in PBS) was added to each well of the ELISA plate, and a kit-positive control and a negative control were set. The ELISA plates were blocked with mounting paper and incubated at 37 °C for 60 minutes. The ELISA plate was took out, and the mounting paper was removed, then 50 µL of enzyme conjugate was added into each well. After shaking for 10 seconds, the ELISA plates were blocked with mounting paper and incubated at 37 °C for 30 minutes. The ELISA plate was took out, and the mounting paper was removed, and the ELISA plate was repeated washing five times: liquid in the wells was discard each time; the wells was filled with washing solution, then stood for 60 seconds and spined dry; and liquid residue on blotting paper was patted dry. After the washing was completed, to all wells was immediately added a mixture of freshly prepared developer A and developer B: 100 µL per well. After shaking for 10 seconds on oscillator, the ELISA plates were blocked with mounting paper and incubated at 37 °C for 30 minutes. To all wells were added 50 µL of stop solution. Data were read at a wavelength of 450 nm on an Envision plate reader. Concentration-HBsAg OD450 value was treated with Graphpad Prism 5 software and the IC₅₀ of inhibition of HBsAg secretion by compounds was calculated by a four-parameter nonlinear regression model.

Conclusion: Inhibitory test of compounds in the invention aganist HBV Virus replication shows that the IC₅₀ value of the inhibitory activity of the compound in the present invention against HBsAg secretion is less than 0.5 µM, and the IC₅₀ value of the inhibitory activity of most compounds against HBsAg secretion is less than 0.1 µM. The inhibitory activities against HBsAg secretion of part of the compounds were as shown in table 3.

**Table 3: inhibitory activities againstHBsAg secretion of part of the compounds**

| **Example** | **HbsAg IC₅₀ (nM)** | **Example** | **HbsAg IC₅₀ (nM)** |
|---|---|---|---|
| Example 1 | 2.25 | Example 5 | 9.88 |
| Example 6 | 3.63 | Example 7 | 13.46 |
| Example 8 | 6.28 | Example 9 | 0.25 |
| Example 10 | 10.50 | Example 11 | 1.60 |
| Example 13 | 1.48 | Example 14 | 7.20 |
| Example 15 | 0.77 | Example 16 | 3.20 |
| Example 17 | 2.14 | Example 18 | 5.53 |
| Example 20 | 8.12 | Example 21 | 0.35 |
| Example 22 | 0.65 | Example 24 | 9.99 |
| Example 30 | 7.83 | Example 31 | 8.20 |
| Example 32 | 9.62 | Example 33 | 3.08 |
| Example 34 | 9.85 | Example 35 | 9.25 |
| Example 36 | 4.22 | Example 67 | 7.77 |
| Example 69 | 15.22 | Example 73 | 12.68 |
| Example 74 | 3.68 | Example 75 | 2.41 |
| Example 77 | 2.87 | Example 78 | 3.26 |
| Example 79 | 2.95 | Example 80 | 2.16 |

### Assay 3: Pharmacokinetic test of compounds of the invention in beagle dogs, mice and rats

### (1) Pharmacokinetic test of compounds of the invention in beagle dogs

Pharmacokinetic test of compounds of the invention in beagle dogs (weigh: 10-12 kg, male, age: 10-12 months, three mumbers in each oral group and intravenous group) was shown as follows.

### Test method

Beagle dogs received test compound of the invention at a dose of 2.5 mg/kg or 5 mg/kg by oral gavage or at a dose of 1 mg/kg or 2 mg/kg by intravenous injection.

Blood samples of vein were taken at 0.083, 0.25, 0.5, 1, 2, 4, 6, 8 and 24 hours after the administration, and collected in anticoagulation tube with EDTA-K₂. The test compounds were extracted from plasma samples by liquid-liquid extraction. Then quantitative analysis was performed on a triple-quadrupole tandem mass spectrometer using multiple reaction monitoring (MRM). Pharmacokinetic parameters were calculated using a noncompartmental method by WinNonLin 6.3 software.

Conclusion: the pharmacokinetic data show that the compounds of the invention have good pharmacokinetic properties in vivo of beagle dogs, and have a good application prospect in anti-HBV virus.

### (2) Pharmacokinetic test in mice

Pharmacokinetic test of compounds of the invention in mice (weigh: 20-25 g, male, age: 45-60 days, three mumbers in each oral group and intravenous group) was shown as follows.

### Test method

ICR mice received test compound of the invention at a dose of 10 mg/kg by oral gavage or at a dose of 2 mg/kg or 10 mg/kg by tail intravenous injection. Blood samples of orbital vein were taken at 0.083, 0.25, 0.5, 1, 2, 4, 6, 8 and 24 hours after the administration, and collected in anticoagulation tube with EDTA-K₂. The test compounds were extracted from plasma samples by liquid-liquid extraction. Then quantitative analysis was performed on a triple-quadrupole tandem mass spectrometer using multiple reaction monitoring (MRM). Pharmacokinetic parameters were calculated using a noncompartmental method by WinNonLin 6.1 software.

Conclusion: the pharmacokinetic data show that the compounds of the invention have good pharmacokinetic properties in vivo of mice, and have a good application prospect in anti-HBV virus.

### (3) Pharmacokinetic test in SD rat

Pharmacokinetic test of compounds of the invention in SD rats (weigh: 200-250 kg, male, age: 2-3 months, three mumbers in each oral group and intravenous group) was shown as follows.

### Test method

Rats received test compound at a dose of 2.5 mg/kg or 5 mg/kg by oral gavage or at a dose of 1 mg/kg by intravenous injection.

Blood samples of vein were taken at 0.083, 0.25, 0.5, 1, 2, 5, 7 and 24 hours after the administration, and collected in anticoagulation tube with EDTA-K₂. The test compounds were extracted from plasma samples by liquid-liquid extraction. Then quantitative analysis was performed on a triple-quadrupole tandem mass spectrometer using multiple reaction monitoring (MRM). Pharmacokinetic parameters were calculated using a noncompartmental method by WinNonLin 6.3 software.

Conclusion: the pharmacokinetic data show that the compounds of the invention have good pharmacokinetic properties in vivo of SD rats, and have a good application prospect in anti-HBV virus.

### Assay 4: The stability test of the compounds of the invention in different species of liver microsomes

The stability test of the compounds in different species of liver microsomes was shown as follows.

### Test method

To a 96-well plate was added 30 µL of a mixed solution of a blank solution and liver microsomes, and to each well was added 15 µL of a buffer containing the test compound. Two samples were taken in parallel. After pre-incubating at 37 °C for 10 min, 15 µL of NADPH solution (8 mM) was added according to the time point. The final concentration of the test compound was 1 µM, the concentration of liver microsomes was 0.1 mg/mL, and the final concentration of NADPH was 2 mM. After incubation for 0, 15, 30 and 60 min, respectively, 150 L acetonitrile (with internal standard) was added to the mixed system. The acetonitrile diluted sample was centrifuged at 4000 rpm for 5 min and 150 µL of the supernatant was analyzed by LC-MS/MS.

Conclusion: The data of stability test in liver microsomes show that the compounds of the present invention have better stability in different species of liver microsomes.

## Claims

1. A compound having Formula (I) or a stereoisomer, a tautomer, an N-oxide, a solvate or a pharmaceutically acceptable salt thereof, wherein, 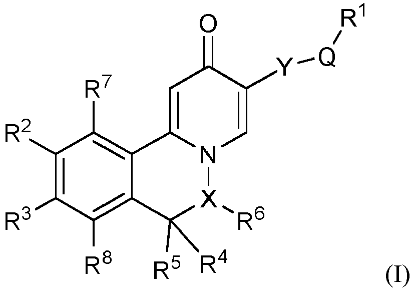
X is N or -CR^{6a}-;
Y is -C(=O)-;
Q is a single bond, -O- or -N(R⁹)-;
R¹ is H, deuterium, methyl, ethyl, n-propyl or isopropyl, wherein each of the methyl, ethyl, n-propyl and isopropyl is independently unsubstituted or substituted with 1, 2, 3 or 4 R^{v};
R⁹ is H, deuterium, methyl, ethyl, n-propyl, isopropyl or C₁₋₆ haloalkyl;
each of R⁴ and R⁵ is independently H, deuterium, C₁₋₄ alkyl, C₁₋₄ alkylamino or C₁₋₄ alkoxy, wherein each of the C₁₋₄ alkyl, C₁₋₄ alkylamino and C₁₋₄ alkoxy is independently unsubstituted or substituted with 1, 2, 3 or 4 R^{y};
R⁶ is H, deuterium, C₁₋₄ alkyl, C₂₋₄ alkenyl or C₃₋₆ cycloalkyl, wherein each of the C₁₋₄ alkyl, C₂₋₄ alkenyl and C₃₋₆ cycloalkyl is independently unsubstituted or substituted with 1, 2, 3 or 4 R^{z};
R^{6a} is H, deuterium, F, Cl, Br, CN, OH, C₁₋₄ alkyl or C₂₋₄ alkenyl, wherein each of the C₁₋₄ alkyl and C₂₋₄ alkenyl is independently unsubstituted or substituted with 1, 2, 3 or 4 R^{z};
R² is phenyl, 5- to 6-membered heteroaryl, R¹³-O-C(=O)-(CR^{e}R^{f})ₘ-, wherein m is 0, R¹⁴-S(=O)₂-N(R^{g})-C(=O)-, R^{c}C(=O)-, R^{a}R^{b}NC(=O)- or R^{d}OC(=O)-, wherein each of the phenyland 5- to 6-membered heteroaryl is independently unsubstituted or substituted with 1, 2, 3 or 4 R^{w};
each of R⁷ and R⁸ is independently H, deuterium, F, Cl, Br, hydroxy, cyano or C₁₋₆ alkyl;
R³ is 5- to 6-membered heterocyclyl, phenyl, 5- to 6-membered heteroaryl R¹³-(CR^{e}R^{f})ₘ-O- or R¹⁰O-, wherein each of the 5- to 6-membered heterocyclyl, phenyl and 5- to 6-membered heteroaryl is independently unsubstituted or substituted with 1, 2, 3 or 4 R^{w};
R¹⁰ is H, deuterium, C₁₋₆ alkyl, C₂₋₆ alkynyl, C₃₋₆ cycloalkyl, 5- to 6-membered heterocyclyl, phenyl, naphthyl or 5- to 6-membered heteroaryl, wherein each of the C₁₋₆ alkyl, C₂₋₆ alkynyl, C₃₋₆ cycloalkyl, 5- to 6-membered heterocyclyl, phenyl, naphthyl and 5- to 6-membered heteroaryl is independently unsubstituted or substituted with 1, 2, 3 or 4 R^{x};
each R¹³ and R¹⁴ is independently C₁₋₁₂ alkyl, C₁₋₁₂ alkoxy, C₃₋₇ cycloalkyl, 5- to 10-membered heteroaryl, 3- to 12-membered heterocyclyl or C₆₋₁₀ aryl, wherein each of the C₁₋₁₂ alkyl, C₁₋₁₂ alkoxy, C₃₋₇ cycloalkyl, 5- to 10-membered heteroaryl, 3- to 12-membered heterocyclyl and C₆₋₁₀ aryl is independently unsubstituted or substituted with 1, 2, 3 or 4 R^{h};
each R^{a}, R^{b}, R^{c}, R^{d}, R^{e}, R^{f} and R^{g} is independently H, deuterium, OH, C₁₋₄ haloalkyl, C₁₋₆ alkyl, C₁₋₄ alkoxy, C₂₋₄ alkenyl, C₂₋₄ alkynyl, C₃₋₆ cycloalkyl, phenyl, naphthyl, 5- to 6-membered heterocyclyl or 5- to 6-membered heteroaryl, wherein each of the C₁₋₄ haloalkyl, C₁₋₆ alkyl, C₁₋₄ alkoxy, C₂₋₄ alkenyl, C₂₋₄ alkynyl, C₃₋₆ cycloalkyl, phenyl, naphthyl, 5- to 6-membered heterocyclyl and 5- to 6-membered heteroaryl is independently unsubstituted or substituted with 1, 2, 3 or 4 substituents independently selected from F, Cl, Br, CN, OH, amino, C₁₋₄ alkyl, C₁₋₄ haloalkyl, C₁₋₄ alkoxy or C₁₋₄ alkylamino;
or R^{a} and R^{b}, together with the nitrogen atom to which they are attached, form a 5- to 6-membered heterocyclyl or 5- to 6-membered heteroaryl, wherein each of the 5-to 6-membered heterocyclyl and 5- to 6-membered heteroaryl is independently unsubstituted or substituted with 1, 2, 3 or 4 substituents independently selected from F, Cl, Br, CN, OH, amino, C₁₋₄ alkyl, C₁₋₄ haloalkyl, C₁₋₄ alkoxy or C₁₋₄ alkylamino;
each R^{v}, R^{w}, R^{x}, R^{y}, R^{z} and R^{h} is independently F, Cl, Br, CN, OH, -COOH, amino, C₁₋₆ alkyl, C₁₋₆ haloalkyl, C₁₋₆ alkoxy, C₁₋₆ haloalkoxy, C₁₋₆ alkylthio, C₁₋₆ alkylamino, C₂₋₄ alkenyl, C₂₋₄ alkynyl, C₃₋₆ cycloalkyl, 5- to 6-membered heterocyclyl, phenyl, naphthyl or 5- to 6-membered heteroaryl, wherein each of the amino, C₁₋₆ alkyl, C₁₋₆ haloalkyl, C₁₋₆ alkoxy, C₁₋₆ haloalkoxy, C₁₋₆ alkylthio, C₁₋₆ alkylamino, C₂₋₄ alkenyl, C₂₋₄ alkynyl, C₃₋₆ cycloalkyl, 5- to 6-membered heterocyclyl, phenyl, naphthyl and 5- to 6-membered heteroaryl is independently unsubstituted or substituted with 1, 2, 3 or 4 substituents independently selected from F, Cl, Br, CN, OH, =O, -COOH, amino, C₁₋₄ alkyl, C₁₋₄ haloalkyl, C₁₋₄ alkoxy, C₁₋₄ alkylthio, C₁₋₄ haloalkoxy, C₁₋₄ alkylamino, C₂₋₄ alkenyl, C₂₋₄ alkynyl, phenyl, naphthyl, C₃₋₆ cycloalkyl, 5- to 6-membered heteroaryl, 5- to 6-membered heterocyclyl, C₁₋₄ alkoxy-C₁₋₄-alkyl or C₁₋₄ alkylamino-C₁₋₄-alkyl;
m is 0, 1, 2, 3, 4, 5, 6, 7, 8, 9 or 10.

2. The compound according to any one of claim 1, wherein R² is furyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidinyl, phenyl, R¹³-O-C(=O)-(CR^{e}R^{f})ₘ-, wherein m is 0, R^{c}-C(=O)-(CR^{e}R^{f})ₘ-O-C(=O)-, R¹⁴-S(=O)₂-N(R^{g})-C(=O)-, R^{c}C(=O)-, R^{a}R^{b}NC(=O)- or R^{d}OC(=O)-, wherein each of the furyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidinyl and phenyl is independently unsubstituted or substituted with 1, 2, 3 or 4 R^{w}.

3. The compound according to claim 1 or 2, wherein each of R⁷ and R⁸ is independently H, deuterium, F, Cl, Br, hydroxy, cyano, methyl, ethyl, *n*-propyl, isopropyl, *n*-butyl, isobutyl, *n*-pentyl or *n*-hexyl;
R³ is pyrrolidinyl, pyrazolidinyl, imidazolidinyl, tetrahydrofuranyl, tetrahydrothiophenyl, tetrahydropyranyl, tetrahydrothiapyranyl, piperidyl, morpholinyl, thiomorpholinyl, piperazinyl, furyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidinyl, phenyl, R¹³-(CR^{e}R^{f})ₘ-O-, or R¹⁰O-, and wherein each of the pyrrolidinyl, pyrazolidinyl, imidazolidinyl, tetrahydrofuranyl, tetrahydrothiophenyl, tetrahydropyranyl, tetrahydrothiapyranyl, piperidyl, morpholinyl, thiomorpholinyl, piperazinyl, furyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidinyl and phenyl is independently unsubstituted or substituted with 1, 2, 3 or 4 R^{w};
R¹⁰ is H, deuterium, methyl, ethyl, *n-*propyl, isopropyl, *n*-butyl, isobutyl, *n*-pentyl, *n*-hexyl, ethynyl, propynyl, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidyl, morpholinyl, thioxomorpholinyl, piperazinyl, furyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidinyl, phenyl or naphthyl, wherein each of the methyl, ethyl, *n*-propyl, isopropyl, *n*-butyl, isobutyl, *n*-pentyl, *n*-hexyl, ethynyl, propynyl, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidyl, morpholinyl, thioxomorpholinyl, piperazinyl, furyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidinyl, phenyl and naphthyl is independently unsubstituted or substituted with 1, 2, 3 or 4 R^{x}.

4. The compound according to any one of claims 1 to 3, wherein each of R⁴ and R⁵ is independently H, deuterium, C₁₋₃ alkyl, C₁₋₃ alkylamino or C₁₋₃ alkoxy, , wherein each of the C₁₋₃ alkyl, C₁₋₃ alkylamino and C₁₋₃ alkoxy is independently unsubstituted or substituted with 1, 2, 3, or 4 R^{y};
R⁶ is H, deuterium, methyl, ethyl, *n*-propyl, isopropyl, *n*-butyl, isobutyl, *tert*-butyl, vinyl, propenyl, cyclopropyl, cyclobutyl, cyclopentyl or cyclohexyl, wherein each of the methyl, ethyl, *n*-propyl, isopropyl, *n*-butyl, isobutyl, *tert*-butyl, vinyl, propenyl, cyclopropyl, cyclobutyl, cyclopentyl and cyclohexyl is independently unsubstituted or substituted with 1, 2, 3, or 4 R^{z};
R^{6a} is H, deuterium, F, Cl, Br, CN, OH, methyl, ethyl, *n*-propyl, isopropyl, *n*-butyl, isobutyl, *tert*-butyl, vinyl or propenyl, wherein each of the methyl, ethyl, *n*-propyl, isopropyl, *n*-butyl, isobutyl, *tert*-butyl, vinyl and propenyl is independently unsubstituted or substituted with 1, 2, 3, or 4 R^{z}.

5. The compound according to any one of claims 1 to 4, wherein each R¹³ and R¹⁴ is independently C₁₋₆ alkyl, C₁₋₆ alkoxy, C₃₋₆ cycloalkyl, 5- to 6-membered heteroaryl, 5- to 6-membered heterocyclyl, phenyl or naphthyl, wherein each of the C₁₋₆ alkyl, C₁₋₆ alkoxy, C₃₋₆ cycloalkyl, 5- to 6-membered heteroaryl, 5- to 6-membered heterocyclyl, phenyl and naphthyl is independently unsubstituted or substituted with 1, 2, 3 or 4 R^{h}.

6. The compound according to any one of claims 1 to 5, wherein each R¹³ and R¹⁴ is independently methyl, ethyl, *n*-propyl, isopropyl, *n*-butyl, isobutyl, *tert*-butyl, methoxy, ethoxy, propoxy, isopropoxy, *n*-butoxy, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidyl, morpholinyl, thiomorpholinyl, piperazinyl, furyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidinyl, phenyl or naphthyl, wherein each of the methyl, ethyl, *n*-propyl, isopropyl, *n*-butyl, isobutyl, *tert*-butyl, methoxy, ethoxy, propoxy, isopropyl, *n*-butoxy, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidyl, morpholinyl, thiomorpholinyl, piperazinyl, furyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidinyl, phenyl and naphthyl is independently unsubstituted or substituted with 1, 2, 3, or 4 R^{h}.

7. The compound according to any one of the claims 1 to 6, wherein each R^{a}, R^{b}, R^{c}, R^{d}, R^{e}, R^{f} and R^{g} is independently H, deuterium, OH, C₁₋₃ haloalkyl, methyl, ethyl, *n*-propyl, isopropyl, *n*-butyl, isobutyl, *tert*-butyl, *n*-pentyl, 3-pentyl, methoxy, ethoxy, propoxy, isopropoxy, *n*-butoxy, ethenyl, propenyl, ethynyl, propynyl, propargyl, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidyl, morpholinyl, thiomorpholinyl, piperazinyl, furyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidinyl, phenyl or naphthyl, wherein each of the C₁₋₃ haloalkyl, methyl, ethyl, *n*-propyl, isopropyl, *n*-butyl, isobutyl, *tert*-butyl, methoxy, ethoxy, propoxy, isopropoxy, *n*-butoxy, ethenyl, propenyl, ethynyl, propynyl, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidyl, morpholinyl, thiomorpholinyl, piperazinyl, furyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidinyl, phenyl and naphthyl is independently unsubstituted or substituted with 1, 2, 3, or 4 substituents independently selected from F, Cl, Br, CN, OH, amino, C₁₋₄ alkyl, C₁₋₄ haloalkyl, C₁₋₄ alkoxy or C₁₋₄ alkylamino;
or, R^{a} and R^{b}, together with the nitrogen atom to which they are attached, form pyrrolidinyl, pyrazolidinyl, imidazolidinyl, piperidyl, morpholinyl, thiomorpholinyl, piperazinyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, pyrazinyl, pyridazinyl or pyrimidinyl, wherein each of the pyrrolidinyl, pyrazolidinyl, imidazolidinyl, piperidyl, morpholinyl, thiomorpholinyl, piperazinyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, pyrazinyl, pyridazinyl and pyrimidinyl is independently unsubstituted or substituted with 1, 2, 3, or 4 substituents independently selected from F, Cl, Br, CN, OH, amino, C₁₋₄ alkyl, C₁₋₄ haloalkyl, C₁₋₄ alkoxy or C₁₋₄ alkylamino.

8. The compound according to any one of claims 1 to 7, wherein each R^{v}, R^{w}, R^{x}, R^{y}, R^{z} and R^{h} is independently F, Cl, Br, CN, OH, -COOH, amino, C₁₋₄ alkyl, C₁₋₄ haloalkyl, C₁₋₄ alkoxy, C₁₋₄ haloalkoxy, C₁₋₄ alkylthio, C₁₋₄ alkylamino, C₂₋₄ alkenyl, C₂₋₄ alkynyl, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidyl, morpholinyl, thiomorpholinyl, piperazinyl, furyl, pyrrolyl, pyridyl, pyrazolyl, imidazolyl, triazolyl, tetrazolyl, oxazolyl, isoxazolyl, dioxazolyl, 1,3,5-triazinyl, thiazolyl, thienyl, pyrazinyl, pyridazinyl, pyrimidinyl, phenyl or naphthyl, wherein each of the cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidyl, morpholinyl, thiomorpholinyl, piperazinyl, furyl, pyrrolyl, pyridyl, pyrazolyl, imidazolyl, triazolyl, tetrazolyl, oxazolyl, isoxazolyl, dioxazolyl, 1,3,5-triazinyl, thiazolyl, thienyl, pyrazinyl, pyridazinyl, pyrimidinyl, phenyl and naphthyl is independently unsubstituted or substituted with 1, 2, 3 or 4 substituents independently selected from F, Cl, Br, CN, OH, =O, -COOH, amino, C₁₋₄ alkyl, C₁₋₄ haloalkyl, C₁₋₄ alkoxy, C₁₋₄ alkylthio, C₁₋₄ haloalkoxy, C₁₋₄ alkylamino, C₂₋₄ alkenyl, C₂₋₄ alkynyl, cyclopropyl, cyclobutyl, cyclopentyl, cyclohexyl, pyrrolidinyl, pyrazolidinyl, imidazolidinyl, tetrahydrofuranyl, tetrahydrothiophenyl, tetrahydropyranyl, tetrahydrothiopyranyl, piperidyl, morpholinyl, thiomorpholinyl, piperazinyl, furyl, pyrrolyl, pyridyl, pyrazolyl, imidazolyl, triazolyl, tetrazolyl, oxazolyl, isoxazolyl, oxadiazolyl, 1,3,5-triazinyl, thiazolyl, thienyl, pyrazinyl, pyridazinyl, pyrimidinyl, phenyl, naphthyl, C₁₋₃ alkoxy-C₁₋₃-alkyl and C₁₋₃ alkylamino-C₁₋₃-alkyl.

9. The compound according to any one of claims 1 to 8 having one of the following structures, 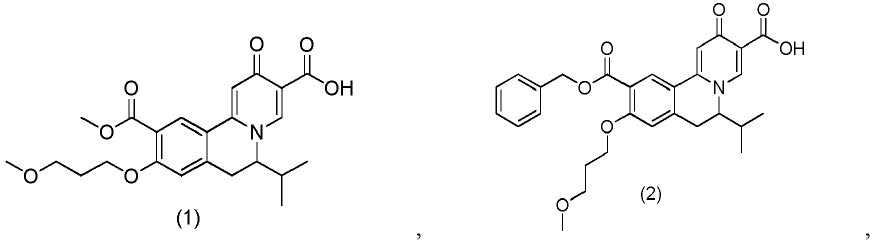 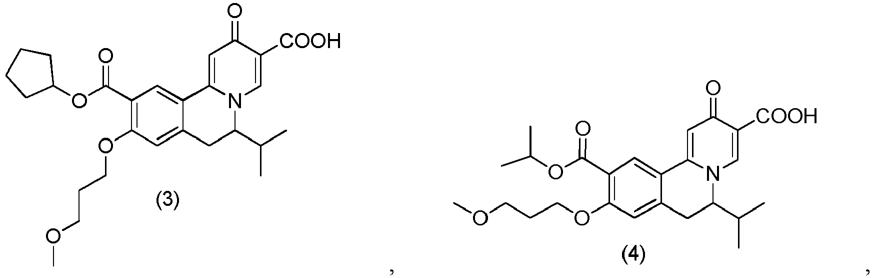 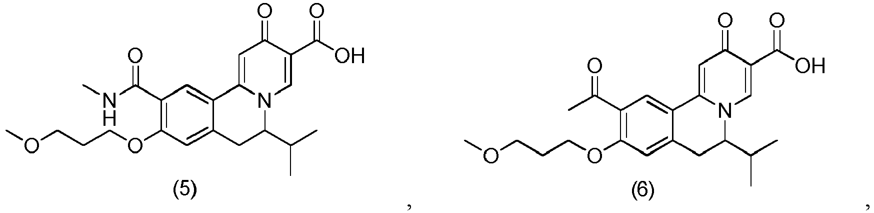 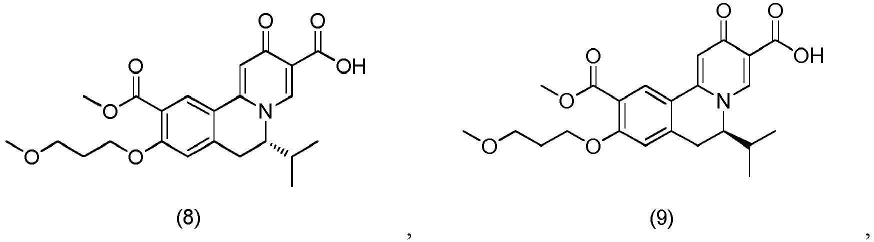 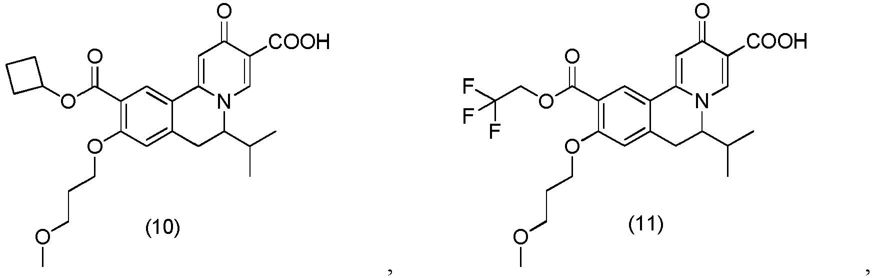 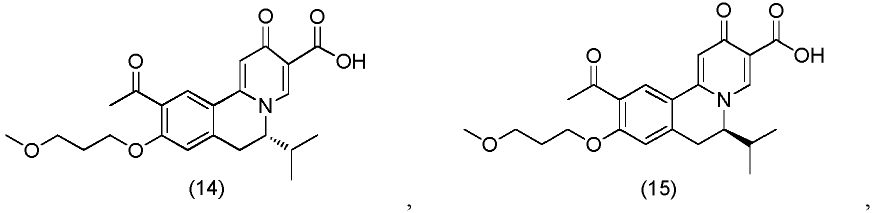 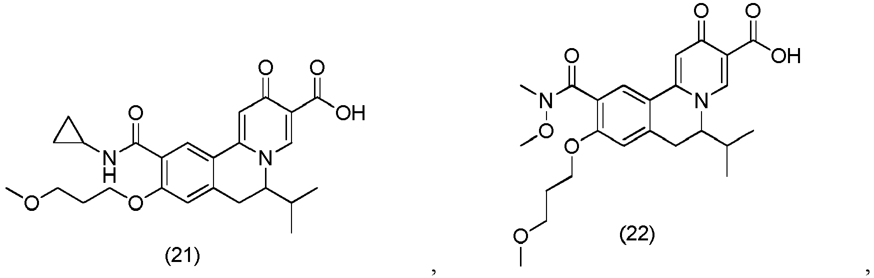 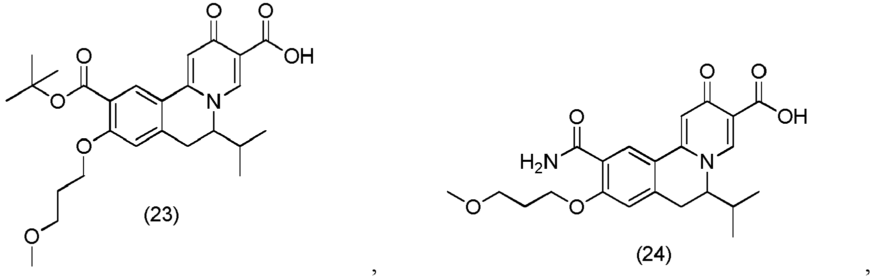 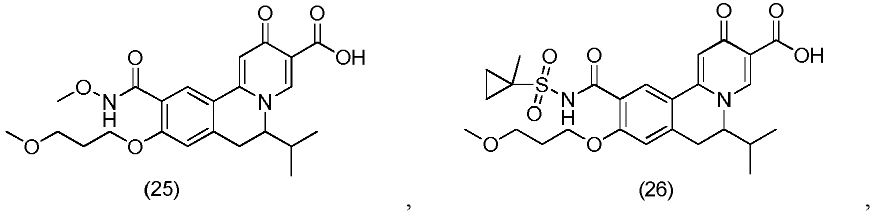 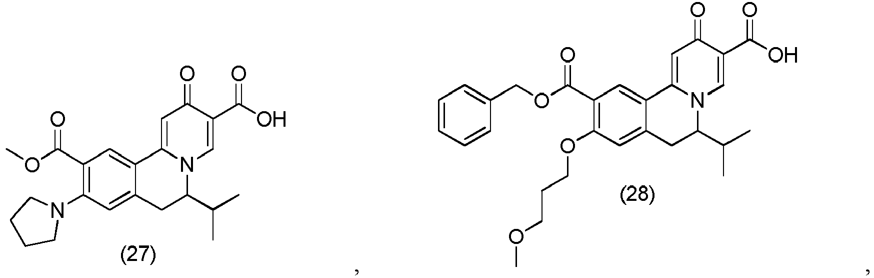 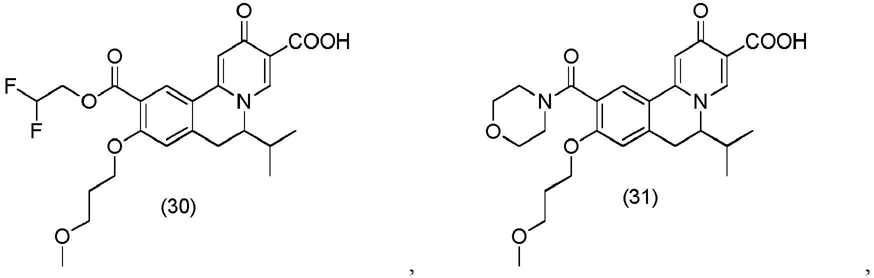 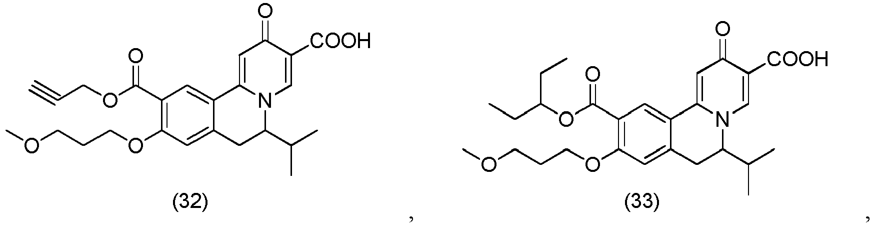 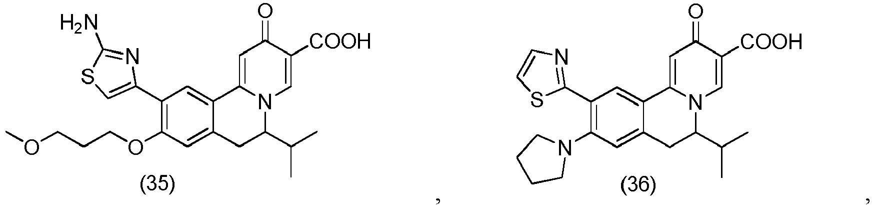 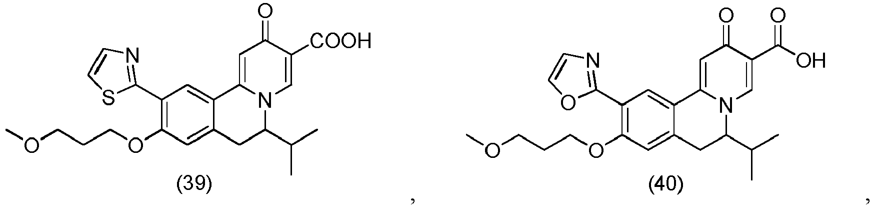 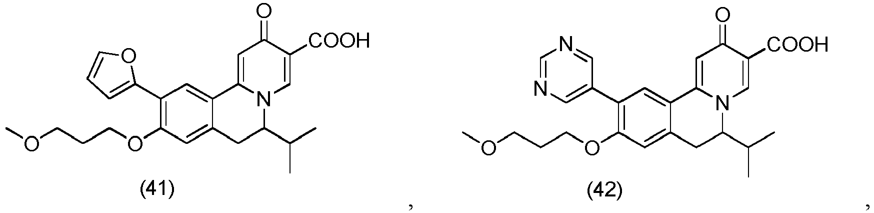 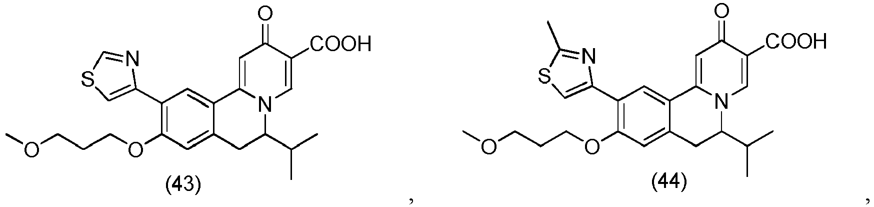 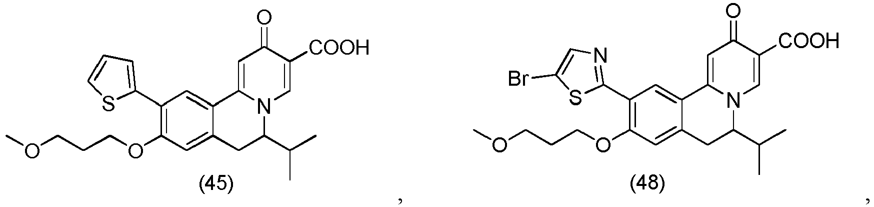 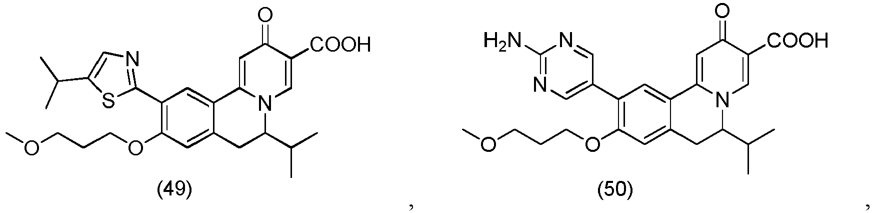 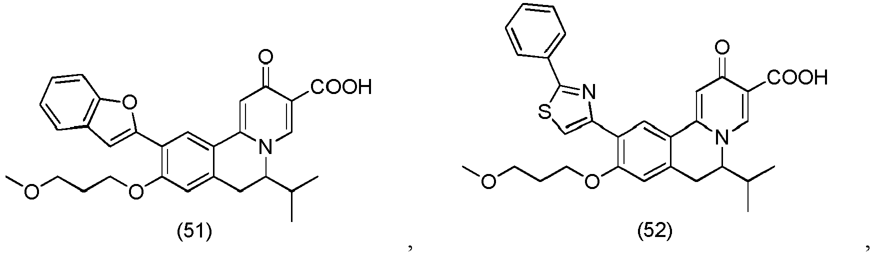 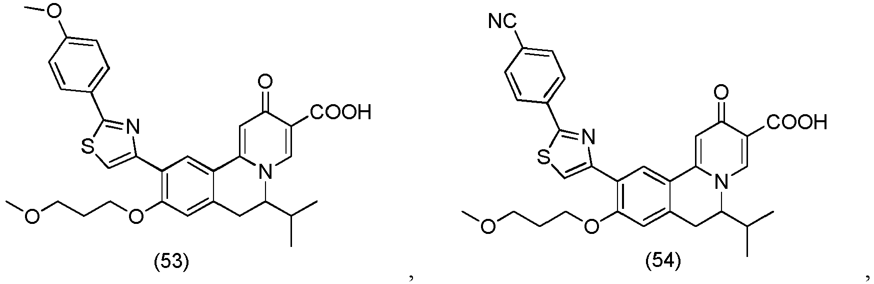 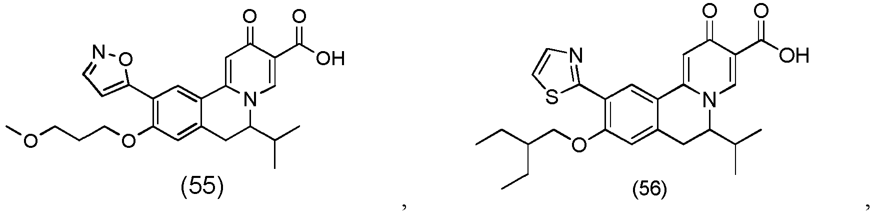 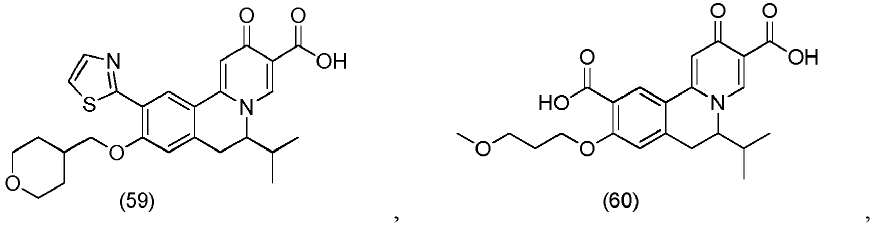 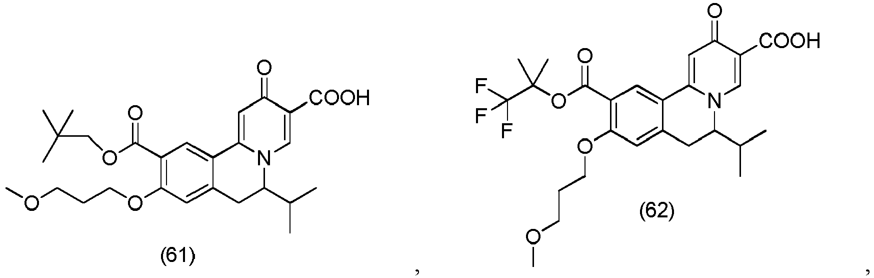 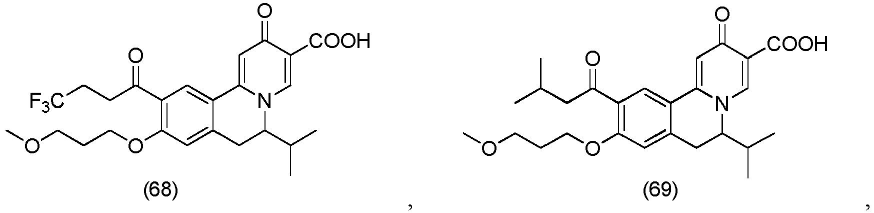 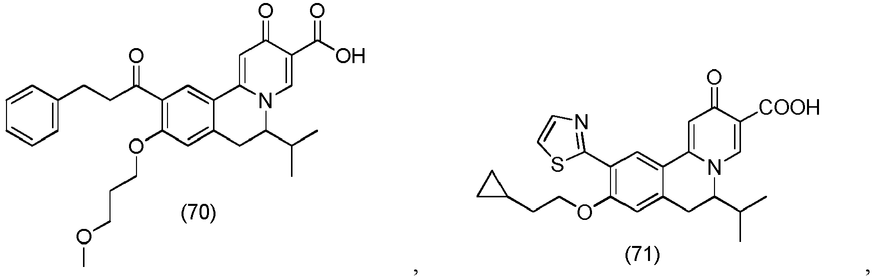 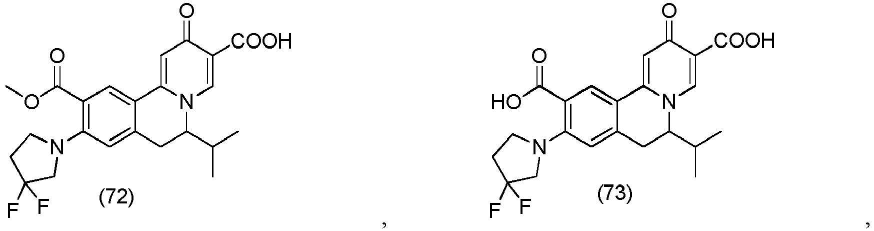 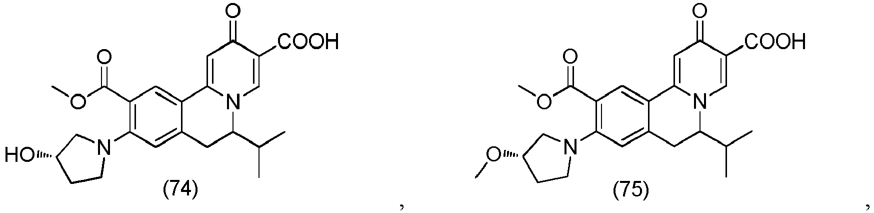 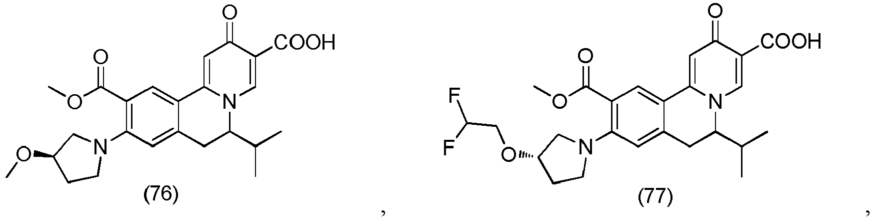 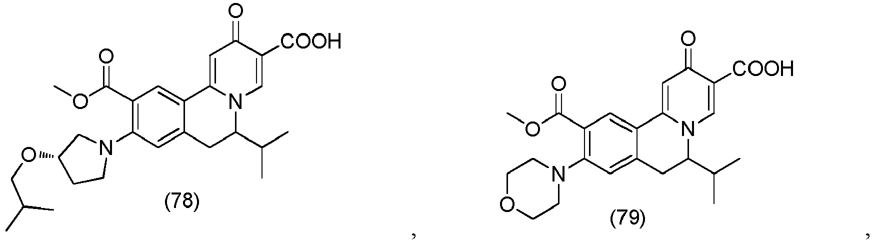 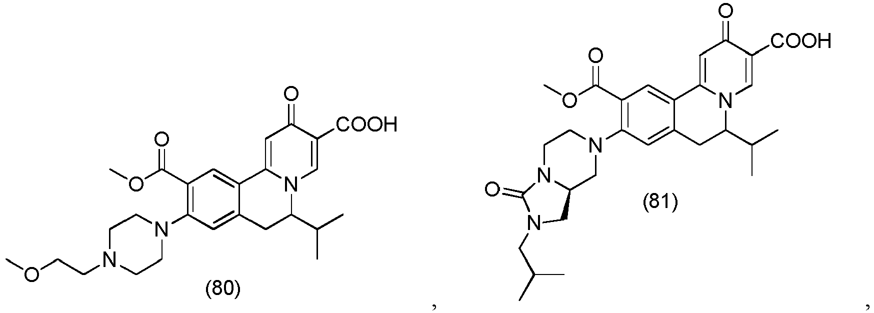 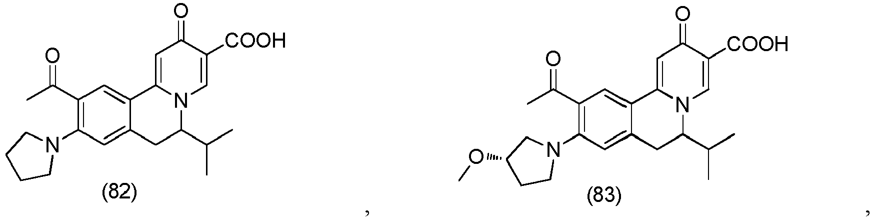 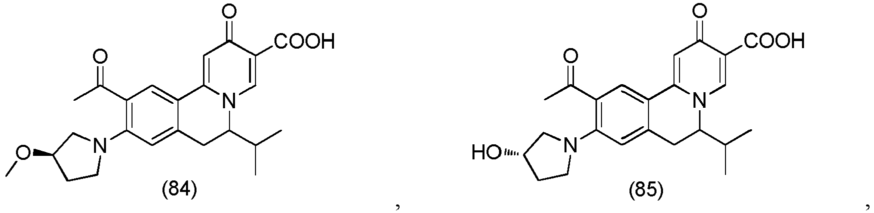 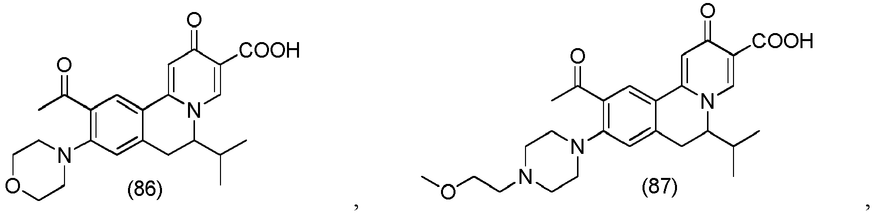 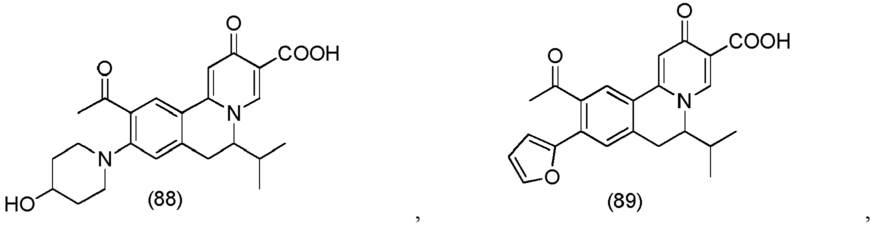 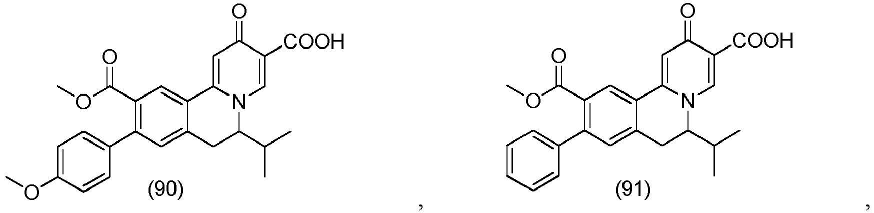 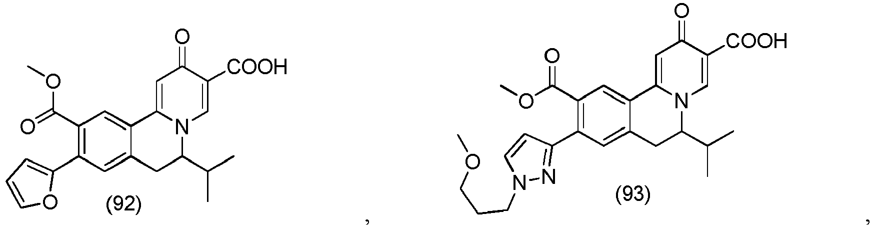 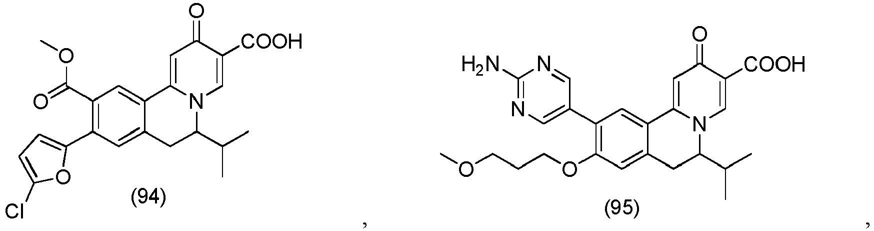 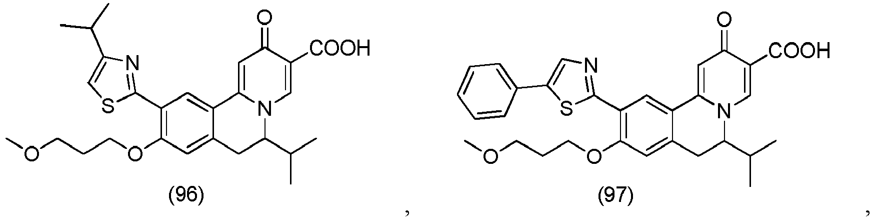 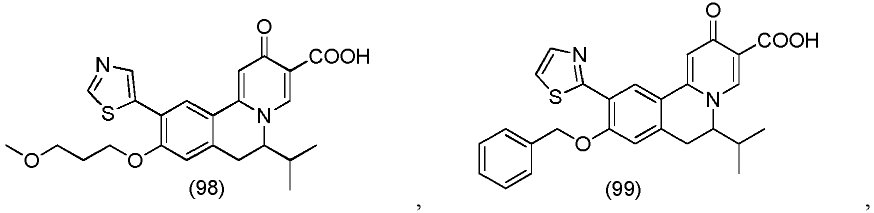 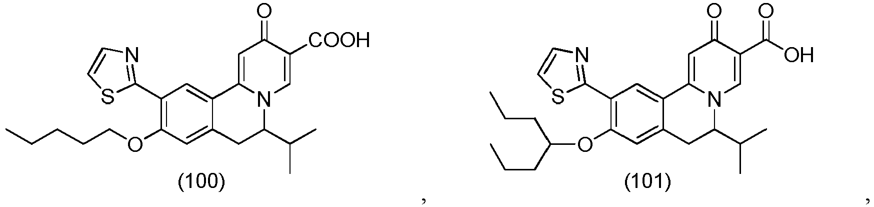 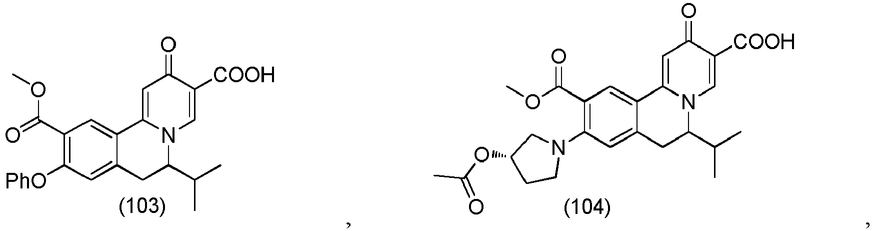 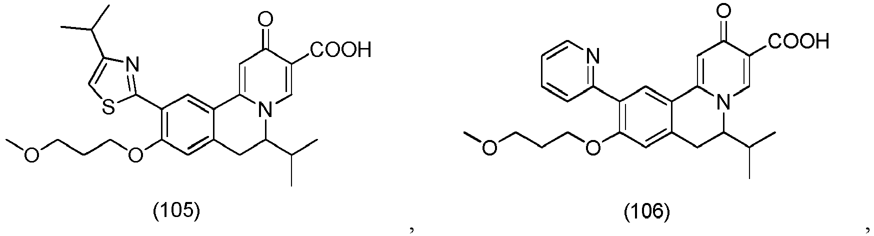 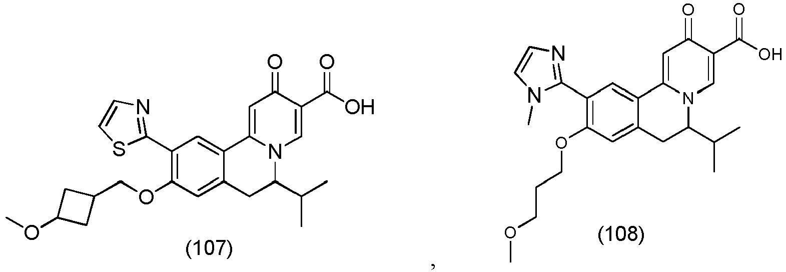 or 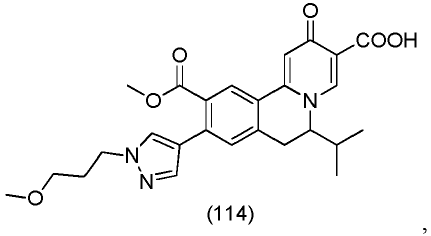 or a stereoisomer, a tautomer, an N-oxide or a pharmaceutically acceptable salt.

10. A pharmaceutical composition comprising the compound according to any one of claims 1 to 9; optionally, wherein the pharmaceutical composition further comprises a pharmaceutically acceptable excipient, or a combination of the excipients.

11. The pharmaceutical composition according to claim 10 further comprising other anti-HBV drug, wherein the other anti-HBV drug is a HBV polymerase inhibitor, an immunomodulator or an interferon.

12. The pharmaceutical composition according to claim 10 or 11, wherein the other anti-HBV agent is lamivudine, telbivudine, tenofovir, entecavir, adefovir dipivoxil, alfaferone, alloferon, celmoleukin, clevudine, emtricitabine, famciclovir, interferon, hepatect CP, intefen, interferon α-1b, interferon α, interferon α-2a, interferon β-1a, interferon α-2, interleukin-2, mivotilate, nitazoxanide, peginterferon alfa-2a, ribavirin, roferon-A, sizofiran, euforavac, ampligen, phosphazid, heplisav, interferon α-2b, levamisole or propagermanium.

13. The compound according to any one of claims 1 to 9 or the pharmaceutical composition according to claim 10 or 12 for use in preventing, treating or lessening a disorder or disease caused by a virus infection in a patient, wherein the virus disease is hepatitis B infection or a disease caused by hepatitis B infection.

14. The compound for use or the pharmaceutical composition for use according to claim 13, wherein the disease caused by hepatitis B infection is cirrhosis or hepatocellular carcinogenesis.

15. The compound according to any one of claims 1 to 9 or the pharmaceutical composition according to claim 10 or 12 for use in inhibiting the production or secretion of HBsAg, and/or in inhibiting the production of HBV DNA in a patient.

## Patentansprüche

1. Verbindung der Formel (I) oder ein Stereoisomer, ein Tautomer, ein N-Oxid, ein Solvat oder ein pharmazeutisch akzeptables Salz davon, wobei 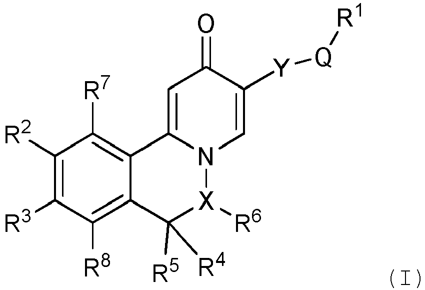
X N oder -CR^{6a}- ist;
Y -C(=O)- ist;
Q eine Einfachbindung, -O- oder -N(R⁹)- ist;
R¹ H, Deuterium, Methyl, Ethyl, n-Propyl oder Isopropyl ist, wobei jedes von Methyl, Ethyl, n-Propyl und Isopropyl unabhängig unsubstituiert oder mit 1, 2, 3 oder 4 R^{v} substituiert ist;
R⁹ H, Deuterium, Methyl, Ethyl, n-Propyl, Isopropyl oder C₁₋₆-Haloalkyl ist;
jeder von R⁴ und R⁵ unabhängig H, Deuterium, C₁₋₄-Alkyl, C₁₋₄-Alkylamino oder C₁₋₄-Alkoxy ist, wobei jedes von C₁₋₄-Alkyl, C₁₋₄-Alkylamino und C₁₋₄-Alkoxy unabhängig unsubstituiert oder mit 1, 2, 3 oder 4 R^{y} substituiert ist;
R⁶ H, Deuterium, C₁₋₄-Alkyl, C₂₋₄-Alkenyl oder C₃₋₆-Cycloalkyl ist, wobei jedes von C₁₋₄-Alkyl, C₂₋₄-Alkenyl und C₃₋₆-Cycloalkyl unabhängig unsubstituiert oder mit 1, 2, 3 oder 4 R^{z} substituiert ist;
R^{6a} H, Deuterium, F, Cl, Br, CN, OH, C₁₋₄-Alkyl oder C₂₋₄-Alkenyl ist, wobei jedes von C₁₋₄-Alkyl und C₂₋₄-Alkenyl unabhängig unsubstituiert oder mit 1, 2, 3 oder 4 R^{z} substituiert ist;
R² Phenyl, 5- bis 6-gliedriges Heteroaryl, R¹³-O-C(=O)-(CR^{e}R^{f})ₘ- ist, wobei m 0, R¹⁴-S(=O)₂-N(R^{g})-C(=O)-, R^{c}C(=O)-, R^{a}R^{b}NC(=O)- oder R^{d}OC(=O)- ist, wobei jedes von Phenyl und 5- bis 6-gliedrigem Heteroaryl unabhängig unsubstituiert oder mit 1, 2, 3 oder 4 R^{w} substituiert ist;
jeder von R⁷ und R⁸ unabhängig H, Deuterium, F, Cl, Br, Hydroxy, Cyano oder C₁₋₆-Alkyl ist;
R³ 5- bis 6-gliedriges Heterocyclyl, Phenyl, 5- bis 6-gliedriges Heteroaryl R¹³-(CR^{e}R^{f})ₘ-O- oder R¹⁰O- ist, wobei jedes von 5- bis 6-gliedrigem Heterocyclyl, Phenyl und 5- bis 6-gliedrigem Heteroaryl unabhängig unsubstituiert oder mit 1, 2, 3 oder 4 R^{w} substituiert ist;
R¹⁰ H, Deuterium, C₁₋₆-Alkyl, C₂₋₆-Alkynyl, C₃₋₆-Cycloalkyl, 5- bis 6-gliedriges Heterocyclyl, Phenyl, Naphthyl oder 5- bis 6-gliedriges Heteroaryl ist, wobei jedes von C₁₋₆-Alkyl, C₂₋₆-Alkynyl, C₃₋₆-Cycloalkyl, 5- bis 6-gliedrigem Heterocyclyl, Phenyl, Naphthyl und 5- bis 6-gliedrigem Heteroaryl unabhängig unsubstituiert oder mit 1, 2, 3 oder 4 R^{x} substituiert ist;
jeder R¹³ und R¹⁴ unabhängig C₁₋₁₂-Alkyl, C₁₋₁₂-Alkoxy, C₃₋₇-Cycloalkyl, 5- bis 10-gliedriges Heteroaryl, 3- bis 12-gliedriges Heterocyclyl oder C₆₋₁₀-Aryl ist, wobei jedes von C₁₋₁₂-Alkyl, C₁₋₁₂-Alkoxy, C₃₋₇-Cycloalkyl, 5- bis 10-gliedrigem Heteroaryl, 3- bis 12-gliedrigem Heterocyclyl und C₆₋₁₀-Aryl unabhängig unsubstituiert oder mit 1, 2, 3 oder 4 R^{h} substituiert ist;
jeder R^{a}, R^{b}, R^{c}, R^{d}, R^{e}, R^{f} und R^{g} unabhängig H, Deuterium, OH, C₁₋₄-Haloalkyl, C₁₋₆-Alkyl, C₁₋₄-Alkoxy, C₂₋₄-Alkenyl, C₂₋₄-Alkynyl, C₃₋₆-Cycloalkyl, Phenyl, Naphthyl, 5- bis 6-gliedriges Heterocyclyl oder 5- bis 6-gliedriges Heteroaryl ist, wobei jedes von C₁₋₄-Haloalkyl, C₁₋₆-Alkyl, C₁₋₄-Alkoxy, C₂₋₄-Alkenyl, C₂₋₄-Alkynyl, C₃₋₆-Cycloalkyl, Phenyl, Naphthyl, 5- bis 6-gliedrigem Heterocyclyl und 5- bis 6-gliedrigem Heteroaryl unabhängig unsubstituiert oder mit 1, 2, 3 oder 4 Substituenten substituiert ist, unabhängig ausgewählt aus F, Cl, Br, CN, OH, Amino, C₁₋₄-Alkyl, C₁₋₄-Haloalkyl, C₁₋₄-Alkoxy oder C₁₋₄-Alkylamino;
oder R^{a} und R^{b} zusammen mit dem Stickstoffatom, an das sie angebunden sind, ein 5- bis 6-gliedriges Heterocyclyl oder 5- bis 6-gliedriges Heteroaryl bilden, wobei jedes von 5- bis 6-gliedrigem Heterocyclyl und 5-bis 6-gliedrigem Heteroaryl unabhängig unsubstituiert oder mit 1, 2, 3 oder 4 Substituenten substituiert ist, unabhängig ausgewählt aus F, Cl, Br, CN, OH, Amino, C₁₋₄-Alkyl, C₁₋₄-Haloalkyl, C₁₋₄-Alkoxy oder C₁₋₄-Alkylamino;
jeder R^{v}, R^{w}, R^{x}, R^{y}, R^{z} und R^{h} unabhängig F, Cl, Br, CN, OH, -COOH, Amino, C₁₋₆-Alkyl, C₁₋₆-Haloalkyl, C₁₋₆-Alkoxy, C₁₋₆-Haloalkoxy, C₁₋₆-Alkylthio, C₁₋₆-Alkylamino, C₂₋₄-Alkenyl, C₂₋₄-Alkynyl, C₃₋₆-Cycloalkyl, 5- bis 6-gliedriges Heterocyclyl, Phenyl, Naphthyl oder 5- bis 6-gliedriges Heteroaryl ist, wobei jedes von Amino, C₁₋₆-Alkyl, C₁₋₆-Haloalkyl, C₁₋₆-Alkoxy, C₁₋₆-Haloalkoxy, C₁₋₆-Alkylthio, C₁₋₆-Alkylamino, C₂₋₄-Alkenyl, C₂₋₄-Alkynyl, C₃₋₆-Cycloalkyl, 5- bis 6-gliedrigem Heterocyclyl, Phenyl, Naphthyl und 5- bis 6-gliedrigem Heteroaryl unabhängig unsubstituiert oder mit 1, 2, 3 oder 4 Substituenten substituiert ist, unabhängig ausgewählt aus F, Cl, Br, CN, OH, =O, -COOH, Amino, C₁₋₄-Alkyl, C₁₋₄-Haloalkyl, C₁₋₄-Alkoxy, C₁₋₄-Alkylthio, C₁₋₄-Haloalkoxy, C₁₋₄-Alkylamino, C₂₋₄-Alkenyl, C₂₋₄-Alkynyl, Phenyl, Naphthyl, C₃₋₆-Cycloalkyl, 5- bis 6-gliedrigem Heteroaryl, 5- bis 6-gliedrigem Heterocyclyl, C₁₋₄-Alkoxy-C₁₋₄-Alkyl oder C₁₋₄-Alkylamino-C₁₋₄-Alkyl;
m 0, 1, 2, 3, 4, 5, 6, 7, 8, 9 oder 10 ist.

2. Verbindung gemäß irgendeinem von Anspruch 1, wobei R² Furyl, Pyrrolyl, Pyridyl, Pyrazolyl, Imidazolyl, Triazolyl, Tetrazolyl, Oxazolyl, Isoxazolyl, Oxadiazolyl, 1,3,5-Triazinyl, Thiazolyl, Thienyl, Pyrazinyl, Pyridazinyl, Pyrimidinyl, Phenyl, R¹³-O-C(=O)-(CR^{e}R^{f})ₘ- ist, wobei m 0, R^{c}-C(=O)-(CR^{e}R^{f})ₘ-O-C(=O)-, R¹⁴-S(=O)₂-N(R^{g})-C(=O)-, R^{c}C(=O)-, R^{a}R^{b}NC(=O)- oder R^{d}OC(=O)- ist, wobei jedes von Furyl, Pyrrolyl, Pyridyl, Pyrazolyl, Imidazolyl, Triazolyl, Tetrazolyl, Oxazolyl, Isoxazolyl, Oxadiazolyl, 1,3,5-Triazinyl, Thiazolyl, Thienyl, Pyrazinyl, Pyridazinyl, Pyrimidinyl und Phenyl unabhängig unsubstituiert oder mit 1, 2, 3 oder 4 R^{w} substituiert ist.

3. Verbindung gemäß Anspruch 1 oder 2, wobei jeder von R⁷ und R⁸ unabhängig H, Deuterium, F, Cl, Br, Hydroxy, Cyano, Methyl, Ethyl, n-Propyl, Isopropyl, n-Butyl, Isobutyl, n-Pentyl oder n-Hexyl ist;
R³ Pyrrolidinyl, Pyrazolidinyl, Imidazolidinyl, Tetrahydrofuranyl, Tetrahydrothiophenyl, Tetrahydropyranyl, Tetrahydrothiapyranyl, Piperidyl, Morpholinyl, Thiomorpholinyl, Piperazinyl, Furyl, Pyrrolyl, Pyridyl, Pyrazolyl, Imidazolyl, Triazolyl, Tetrazolyl, Oxazolyl, Isoxazolyl, Oxadiazolyl, 1,3,5-Triazinyl, Thiazolyl, Thienyl, Pyrazinyl, Pyridazinyl, Pyrimidinyl, Phenyl, R¹³-(CR^{e}Rf)ₘ-O- oder R¹⁰O- ist, und wobei jedes von Pyrrolidinyl, Pyrazolidinyl, Imidazolidinyl, Tetrahydrofuranyl, Tetrahydrothiophenyl, Tetrahydropyranyl, Tetrahydrothiapyranyl, Piperidyl, Morpholinyl, Thiomorpholinyl, Piperazinyl, Furyl, Pyrrolyl, Pyridyl, Pyrazolyl, Imidazolyl, Triazolyl, Tetrazolyl, Oxazolyl, Isoxazolyl, Oxadiazolyl, 1,3,5-Triazinyl, Thiazolyl, Thienyl, Pyrazinyl, Pyridazinyl, Pyrimidinyl und Phenyl unabhängig unsubstituiert oder mit 1, 2, 3 oder 4 R^{w} substituiert ist;
R¹⁰ H, Deuterium, Methyl, Ethyl, n-Propyl, Isopropyl, n-Butyl, Isobutyl, n-Pentyl, n-Hexyl, Ethynyl, Propynyl, Cyclopropyl, Cyclobutyl, Cyclopentyl, Cyclohexyl, Pyrrolidinyl, Pyrazolidinyl, Imidazolidinyl, Tetrahydrofuranyl, Tetrahydrothiophenyl, Tetrahydropyranyl, Tetrahydrothiopyranyl, Piperidyl, Morpholinyl, Thioxomorpholinyl, Piperazinyl, Furyl, Pyrrolyl, Pyridyl, Pyrazolyl, Imidazolyl, Triazolyl, Tetrazolyl, Oxazolyl, Isoxazolyl, Oxadiazolyl, 1,3,5-Triazinyl, Thiazolyl, Thienyl, Pyrazinyl, Pyridazinyl, Pyrimidinyl, Phenyl oder Naphthyl ist, wobei jedes von Methyl, Ethyl, n-Propyl, Isopropyl, n-Butyl, Isobutyl, n-Pentyl, n-Hexyl, Ethynyl, Propynyl, Cyclopropyl, Cyclobutyl, Cyclopentyl, Cyclohexyl, Pyrrolidinyl, Pyrazolidinyl, Imidazolidinyl, Tetrahydrofuranyl, Tetrahydrothiophenyl, Tetrahydropyranyl, Tetrahydrothiopyranyl, Piperidyl, Morpholinyl, Thioxomorpholinyl, Piperazinyl, Furyl, Pyrrolyl, Pyridyl, Pyrazolyl, Imidazolyl, Triazolyl, Tetrazolyl, Oxazolyl, Isoxazolyl, Oxadiazolyl, 1,3,5-Triazinyl, Thiazolyl, Thienyl, Pyrazinyl, Pyridazinyl, Pyrimidinyl, Phenyl und Naphthyl unabhängig unsubstituiert oder mit 1, 2, 3 oder 4 R^{x} substituiert ist.

4. Verbindung gemäß irgendeinem der Ansprüche 1 bis 3, wobei jeder von R⁴ und R⁵ unabhängig H, Deuterium, C₁₋₃-Alkyl, C₁₋₃-Alkylamino oder C₁₋₃-Alkoxy ist, wobei jedes von C₁₋₃-Alkyl, C₁₋₃-Alkylamino und C₁₋₃-Alkoxy unabhängig unsubstituiert oder mit 1, 2, 3, oder 4 R^{y} substituiert ist;
R⁶ H, Deuterium, Methyl, Ethyl, n-Propyl, Isopropyl, n-Butyl, Isobutyl, tert-Butyl, Vinyl, Propenyl, Cyclopropyl, Cyclobutyl, Cyclopentyl oder Cyclohexyl ist, wobei jedes von Methyl, Ethyl, *n*-Propyl, Isopropyl, n-Butyl, Isobutyl, tert-Butyl, Vinyl, Propenyl, Cyclopropyl, Cyclobutyl, Cyclopentyl und Cyclohexyl unabhängig unsubstituiert oder mit 1, 2, 3, oder 4 R^{z} substituiert ist;
R^{6a} H, Deuterium, F, Cl, Br, CN, OH, Methyl, Ethyl, *n-*Propyl, Isopropyl, *n*-Butyl, Isobutyl, *tert*-Butyl, Vinyl oder Propenyl ist, wobei jedes von Methyl, Ethyl, *n-*Propyl, Isopropyl, *n*-Butyl, Isobutyl, *tert*-Butyl, Vinyl und Propenyl unabhängig unsubstituiert oder mit 1, 2, 3, oder 4 R^{z} substituiert ist.

5. Verbindung gemäß irgendeinem der Ansprüche 1 bis 4, wobei jeder R¹³ und R¹⁴ unabhängig C₁₋₆-Alkyl, C₁₋₆-Alkoxy, C₃₋₆-Cycloalkyl, 5- bis 6-gliedriges Heteroaryl, 5- bis 6-gliedriges Heterocyclyl, Phenyl oder Naphthyl ist, wobei jedes von C₁₋₆-Alkyl, C₁₋₆-Alkoxy, C₃₋₆-Cycloalkyl, 5- bis 6-gliedrigem Heteroaryl, 5- bis 6-gliedrigem Heterocyclyl, Phenyl und Naphthyl unabhängig unsubstituiert oder mit 1, 2, 3 oder 4 R^{h} substituiert ist.

6. Verbindung gemäß irgendeinem der Ansprüche 1 bis 5, wobei jeder R¹³ und R¹⁴ unabhängig Methyl, Ethyl, *n-*Propyl, Isopropyl, *n*-Butyl, Isobutyl, *tert*-Butyl, Methoxy, Ethoxy, Propoxy, Isopropoxy, n-Butoxy, Cyclopropyl, Cyclobutyl, Cyclopentyl, Cyclohexyl, Pyrrolidinyl, Pyrazolidinyl, Imidazolidinyl, Tetrahydrofuranyl, Tetrahydrothiophenyl, Tetrahydropyranyl, Tetrahydrothiopyranyl, Piperidyl, Morpholinyl, Thiomorpholinyl, Piperazinyl, Furyl, Pyrrolyl, Pyridyl, Pyrazolyl, Imidazolyl, Triazolyl, Tetrazolyl, Oxazolyl, Isoxazolyl, Oxadiazolyl, 1,3,5-Triazinyl, Thiazolyl, Thienyl, Pyrazinyl, Pyridazinyl, Pyrimidinyl, Phenyl oder Naphthyl ist, wobei jedes von Methyl, Ethyl, n-Propyl, Isopropyl, n-Butyl, Isobutyl, tert-Butyl, Methoxy, Ethoxy, Propoxy, Isopropyl, n-Butoxy, Cyclopropyl, Cyclobutyl, Cyclopentyl, Cyclohexyl, Pyrrolidinyl, Pyrazolidinyl, Imidazolidinyl, Tetrahydrofuranyl, Tetrahydrothiophenyl, Tetrahydropyranyl, Tetrahydrothiopyranyl, Piperidyl, Morpholinyl, Thiomorpholinyl, Piperazinyl, Furyl, Pyrrolyl, Pyridyl, Pyrazolyl, Imidazolyl, Triazolyl, Tetrazolyl, Oxazolyl, Isoxazolyl, Oxadiazolyl, 1,3,5-Triazinyl, Thiazolyl, Thienyl, Pyrazinyl, Pyridazinyl, Pyrimidinyl, Phenyl und Naphthyl unabhängig unsubstituiert oder mit 1, 2, 3, oder 4 R^{h} substituiert ist.

7. Verbindung gemäß irgendeinem der Ansprüche 1 bis 6, wobei jeder R^{a}, R^{b}, R^{c}, R^{d}, R^{e}, R^{f} und R^{g} unabhängig H, Deuterium, OH, C₁₋₃-Haloalkyl, Methyl, Ethyl, *n*-Propyl, Isopropyl, *n*-Butyl, Isobutyl, *tert*-Butyl, *n*-Pentyl, 3-Pentyl, Methoxy, Ethoxy, Propoxy, Isopropoxy, *n*-Butoxy, Ethenyl, Propenyl, Ethynyl, Propynyl, Propargyl, Cyclopropyl, Cyclobutyl, Cyclopentyl, Cyclohexyl, Pyrrolidinyl, Pyrazolidinyl, Imidazolidinyl, Tetrahydrofuranyl, Tetrahydrothiophenyl, Tetrahydropyranyl, Tetrahydrothiopyranyl, Piperidyl, Morpholinyl, Thiomorpholinyl, Piperazinyl, Furyl, Pyrrolyl, Pyridyl, Pyrazolyl, Imidazolyl, Triazolyl, Tetrazolyl, Oxazolyl, Isoxazolyl, Oxadiazolyl, 1,3,5-Triazinyl, Thiazolyl, Thienyl, Pyrazinyl, Pyridazinyl, Pyrimidinyl, Phenyl oder Naphthyl ist, wobei jedes von C₁₋₃-Haloalkyl, Methyl, Ethyl, n-Propyl, Isopropyl, n-Butyl, Isobutyl, tert-Butyl, Methoxy, Ethoxy, Propoxy, Isopropoxy, n-Butoxy, Ethenyl, Propenyl, Ethynyl, Propynyl, Cyclopropyl, Cyclobutyl, Cyclopentyl, Cyclohexyl, Pyrrolidinyl, Pyrazolidinyl, Imidazolidinyl, Tetrahydrofuranyl, Tetrahydrothiophenyl, Tetrahydropyranyl, Tetrahydrothiopyranyl, Piperidyl, Morpholinyl, Thiomorpholinyl, Piperazinyl, Furyl, Pyrrolyl, Pyridyl, Pyrazolyl, Imidazolyl, Triazolyl, Tetrazolyl, Oxazolyl, Isoxazolyl, Oxadiazolyl, 1,3,5-Triazinyl, Thiazolyl, Thienyl, Pyrazinyl, Pyridazinyl, Pyrimidinyl, Phenyl und Naphthyl unabhängig unsubstituiert oder mit 1, 2, 3, oder 4 Substituenten substituiert ist, unabhängig ausgewählt aus F, Cl, Br, CN, OH, Amino, C₁₋₄-Alkyl, C₁₋₄-Haloalkyl, C₁₋₄-Alkoxy oder C₁₋₄-Alkylamino ;
oder R^{a} und R^{b} zusammen mit dem Stickstoffatom, an das sie angebunden sind, Pyrrolidinyl, Pyrazolidinyl, Imidazolidinyl, Piperidyl, Morpholinyl, Thiomorpholinyl, Piperazinyl, Pyrrolyl, Pyridyl, Pyrazolyl, Imidazolyl, Triazolyl, Tetrazolyl, Oxazolyl, Isoxazolyl, Oxadiazolyl, 1,3,5-Triazinyl, Thiazolyl, Pyrazinyl, Pyridazinyl oder Pyrimidinyl bilden, wobei jedes von Pyrrolidinyl, Pyrazolidinyl, Imidazolidinyl, Piperidyl, Morpholinyl, Thiomorpholinyl, Piperazinyl, Pyrrolyl, Pyridyl, Pyrazolyl, Imidazolyl, Triazolyl, Tetrazolyl, Oxazolyl, Isoxazolyl, Oxadiazolyl, 1,3,5-Triazinyl, Thiazolyl, Pyrazinyl, Pyridazinyl und Pyrimidinyl unabhängig unsubstituiert oder mit 1, 2, 3, oder 4 Substituenten substituiert ist, unabhängig ausgewählt aus F, Cl, Br, CN, OH, Amino, C₁₋₄-Alkyl, C₁₋₄-Haloalkyl, C₁₋₄-Alkoxy oder C₁₋₄-Alkylamino.

8. Verbindung gemäß irgendeinem der Ansprüche 1 bis 7, wobei jeder R^{v}, R^{w}, R^{x}, R^{y}, R^{z} und R^{h} unabhängig F, Cl, Br, CN, OH, -COOH, Amino, C₁₋₄-Alkyl, C₁₋₄-Haloalkyl, C₁₋₄-Alkoxy, C₁₋₄-Haloalkoxy, C₁₋₄-Alkylthio, C₁₋₄-Alkylamino, C₂₋₄-Alkenyl, C₂₋₄-Alkynyl, Cyclopropyl, Cyclobutyl, Cyclopentyl, Cyclohexyl, Pyrrolidinyl, Pyrazolidinyl, Imidazolidinyl, Tetrahydrofuranyl, Tetrahydrothiophenyl, Tetrahydropyranyl, Tetrahydrothiopyranyl, Piperidyl, Morpholinyl, Thiomorpholinyl, Piperazinyl, Furyl, Pyrrolyl, Pyridyl, Pyrazolyl, Imidazolyl, Triazolyl, Tetrazolyl, Oxazolyl, Isoxazolyl, Dioxazolyl, 1,3,5-Triazinyl, Thiazolyl, Thienyl, Pyrazinyl, Pyridazinyl, Pyrimidinyl, Phenyl oder Naphthyl ist, wobei jedes von Cyclopropyl, Cyclobutyl, Cyclopentyl, Cyclohexyl, Pyrrolidinyl, Pyrazolidinyl, Imidazolidinyl, Tetrahydrofuranyl, Tetrahydrothiophenyl, Tetrahydropyranyl, Tetrahydrothiopyranyl, Piperidyl, Morpholinyl, Thiomorpholinyl, Piperazinyl, Furyl, Pyrrolyl, Pyridyl, Pyrazolyl, Imidazolyl, Triazolyl, Tetrazolyl, Oxazolyl, Isoxazolyl, Dioxazolyl, 1,3,5-Triazinyl, Thiazolyl, Thienyl, Pyrazinyl, Pyridazinyl, Pyrimidinyl, Phenyl und Naphthyl unabhängig unsubstituiert oder mit 1, 2, 3 oder 4 Substituenten substituiert ist, unabhängig ausgewählt aus F, Cl, Br, CN, OH, =O, -COOH, Amino, C₁₋₄-Alkyl, C₁₋₄-Haloalkyl, C₁₋₄-Alkoxy, C₁₋₄-Alkylthio, C₁₋₄-Haloalkoxy, C₁₋₄-Alkylamino, C₂₋₄-Alkenyl, C₂₋₄-Alkynyl, Cyclopropyl, Cyclobutyl, Cyclopentyl, Cyclohexyl, Pyrrolidinyl, Pyrazolidinyl, Imidazolidinyl, Tetrahydrofuranyl, Tetrahydrothiophenyl, Tetrahydropyranyl, Tetrahydrothiopyranyl, Piperidyl, Morpholinyl, Thiomorpholinyl, Piperazinyl, Furyl, Pyrrolyl, Pyridyl, Pyrazolyl, Imidazolyl, Triazolyl, Tetrazolyl, Oxazolyl, Isoxazolyl, Oxadiazolyl, 1,3,5-Triazinyl, Thiazolyl, Thienyl, Pyrazinyl, Pyridazinyl, Pyrimidinyl, Phenyl, Naphthyl, C₁₋₃-Alkoxy-C₁₋₃-Alkyl und C₁₋₃-Alkylamino-C₁₋₃-Alkyl.

9. Verbindung gemäß irgendeinem der Ansprüche 1 bis 8 mit einer der folgenden Strukturen 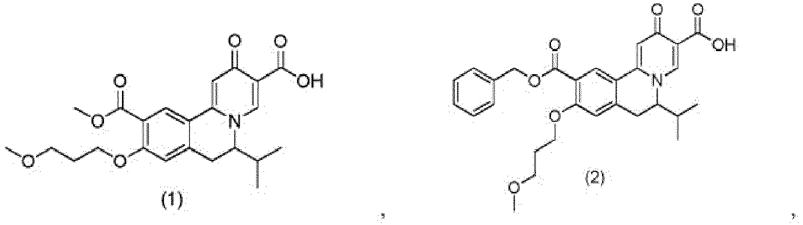 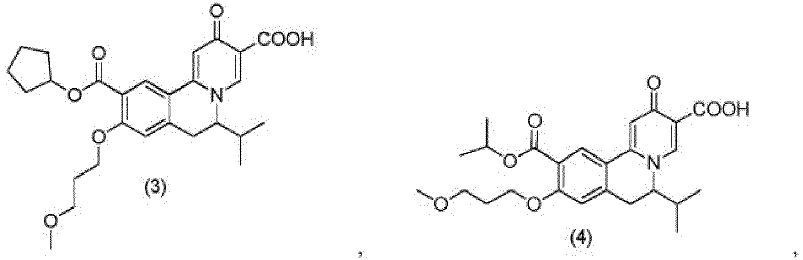 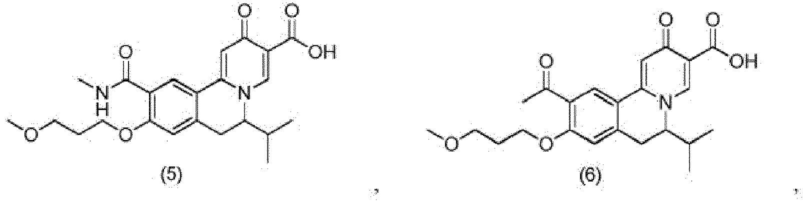 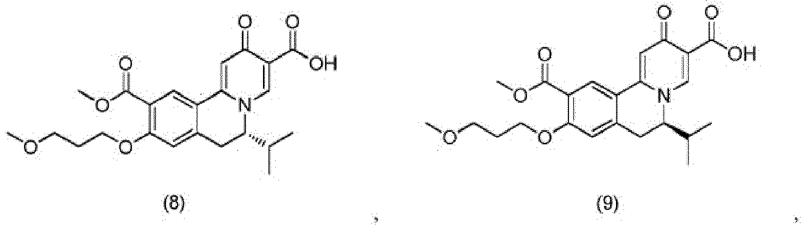 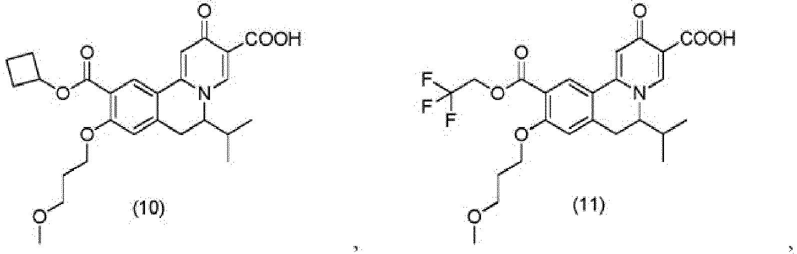 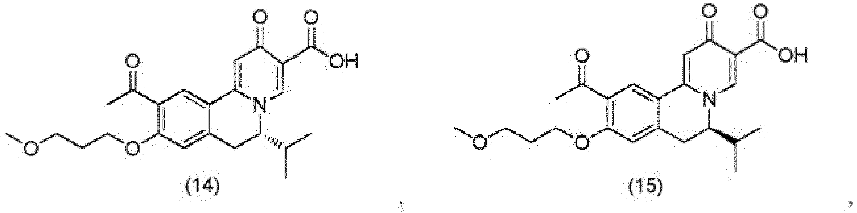 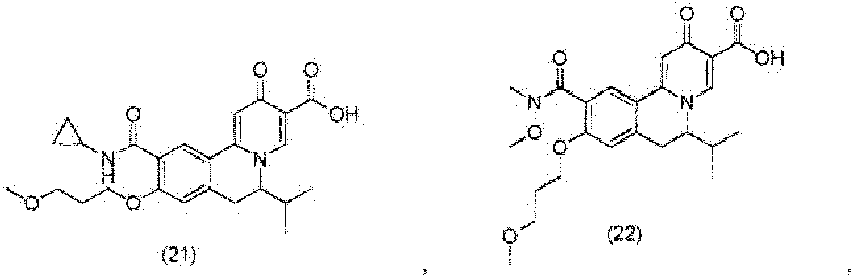 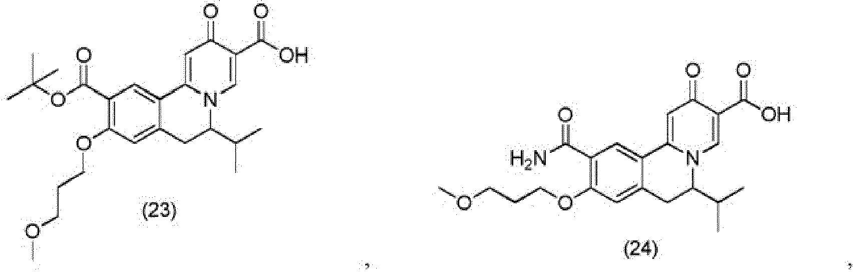 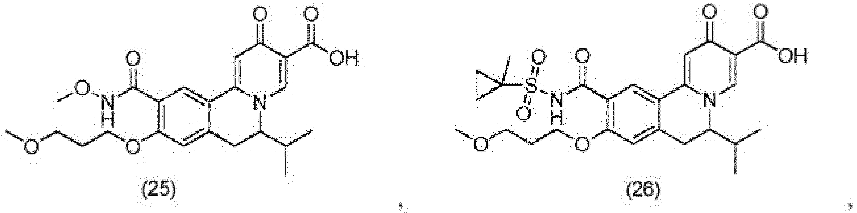 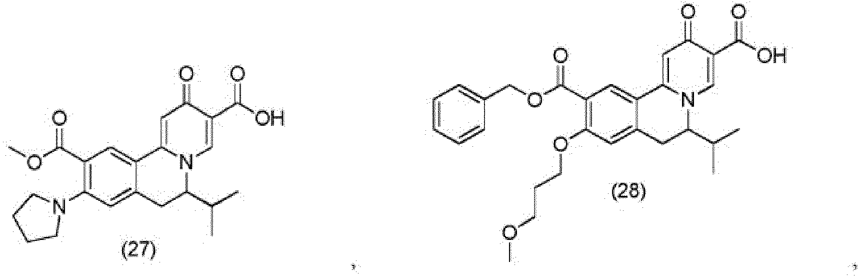 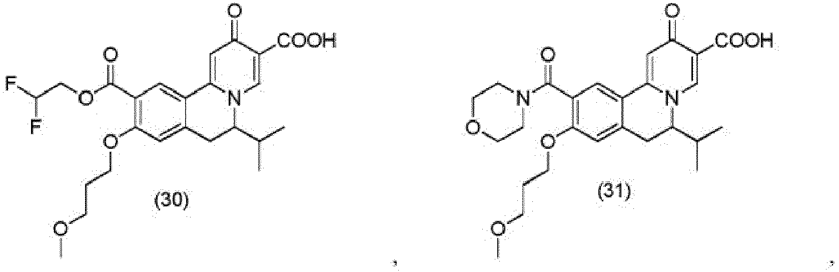 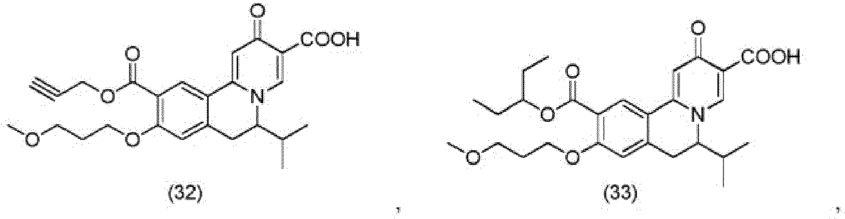 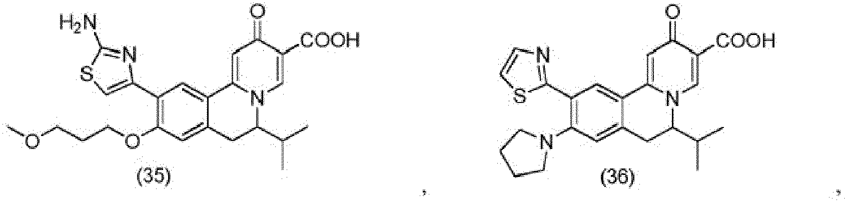 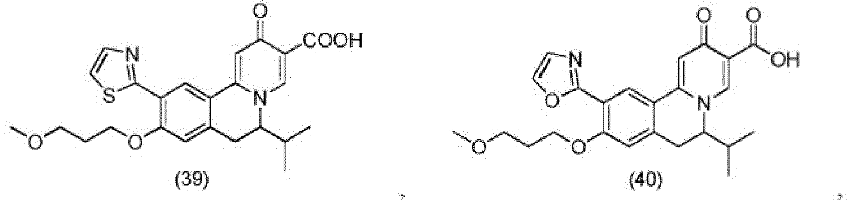 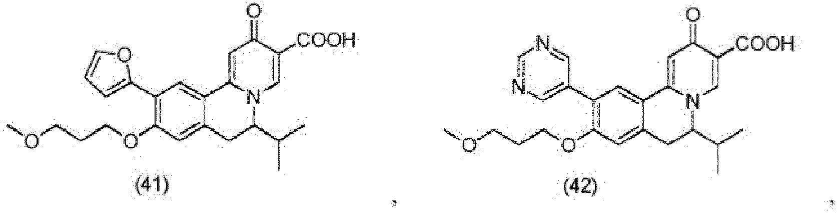 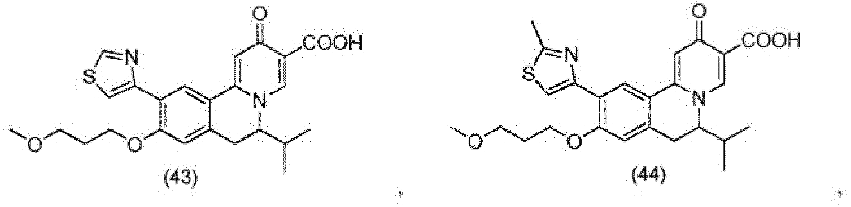 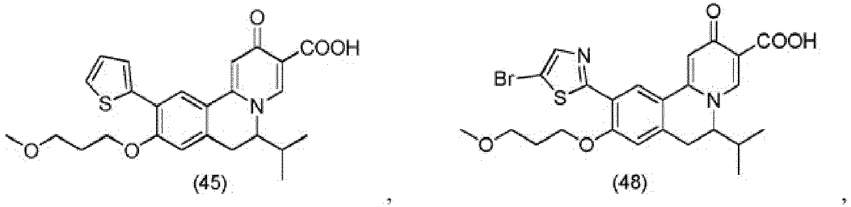 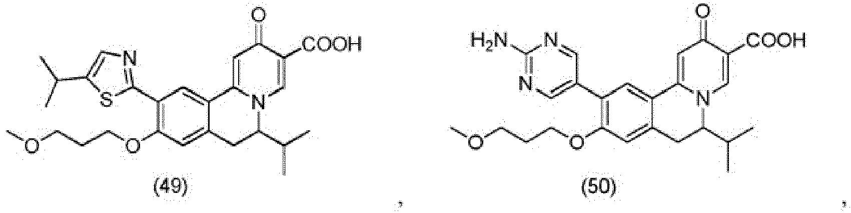 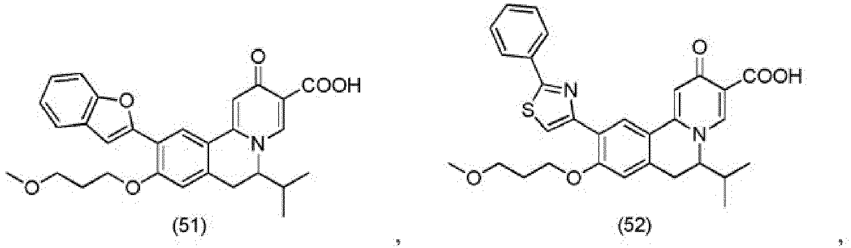 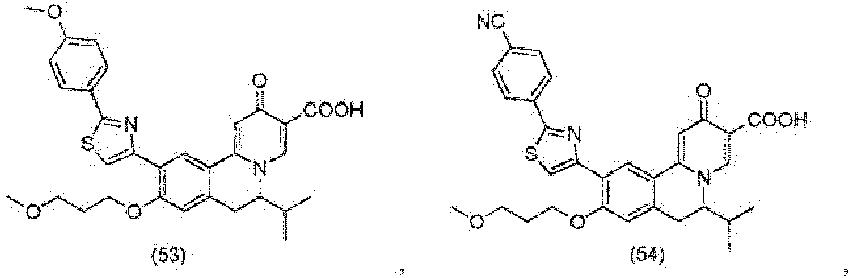 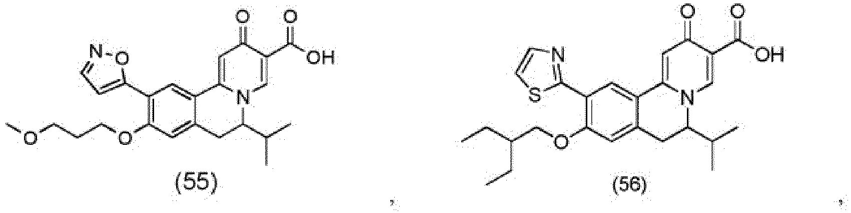 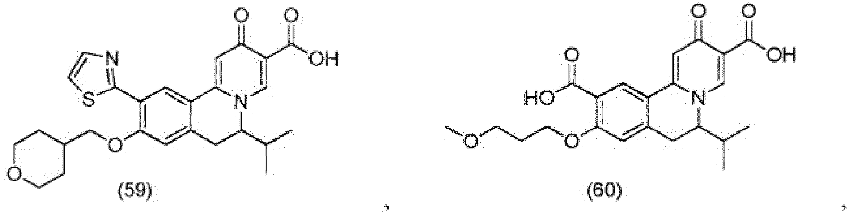 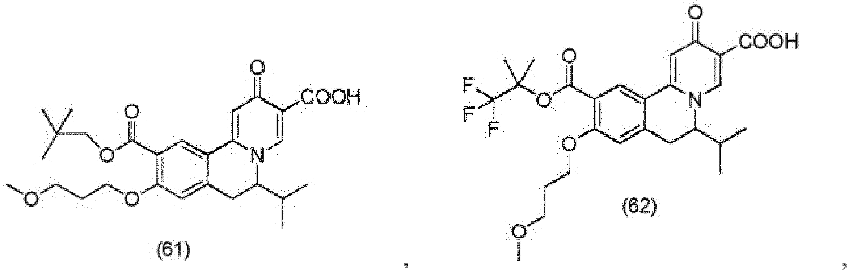 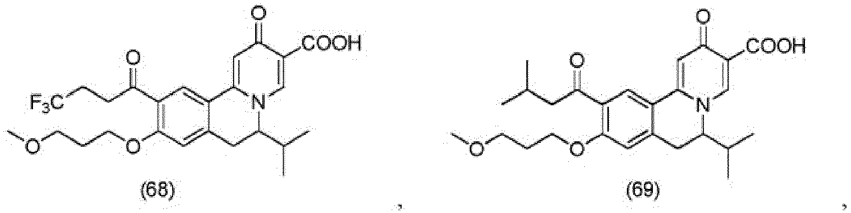 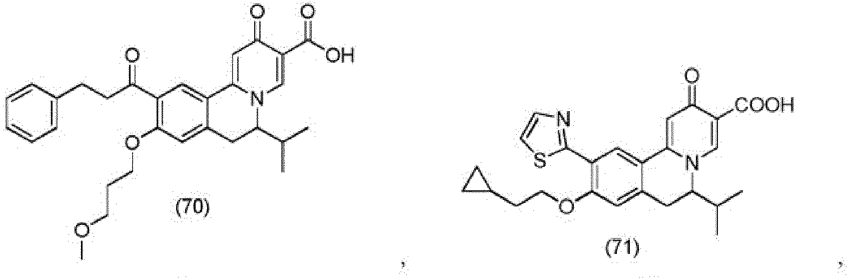 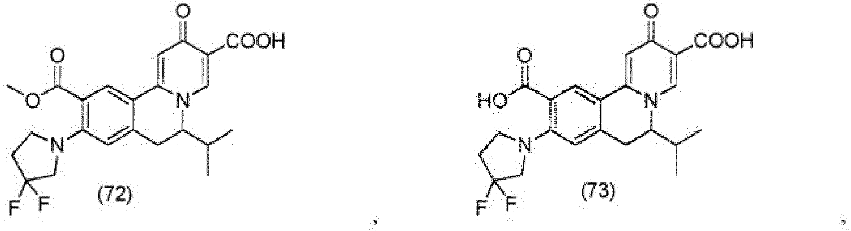 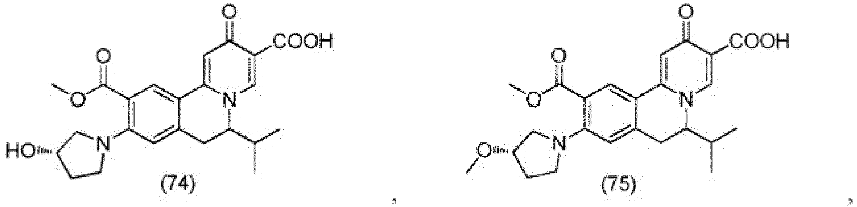 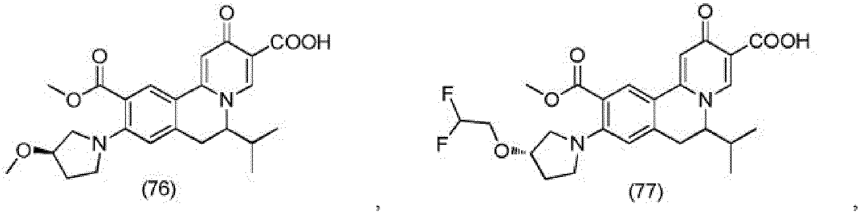 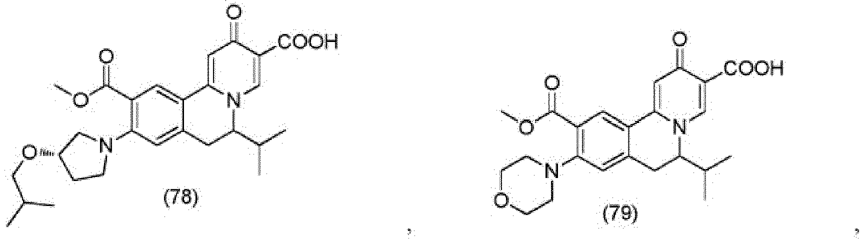 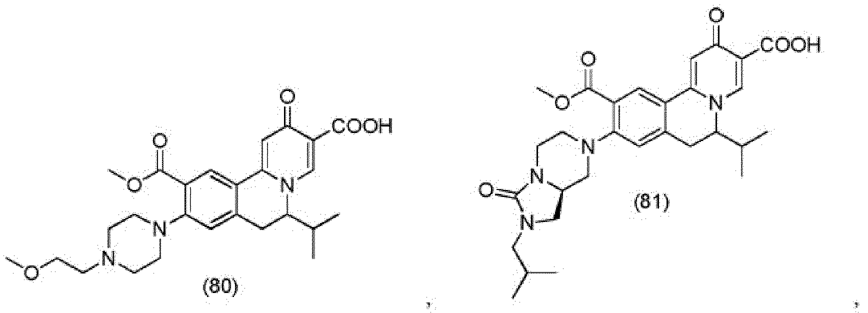 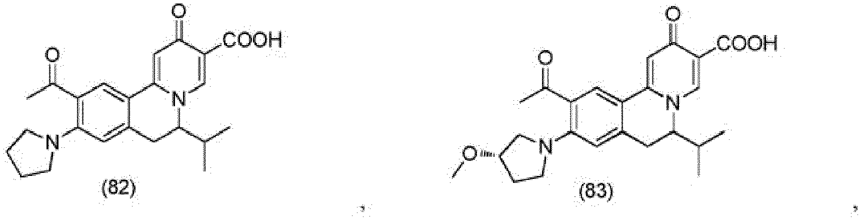 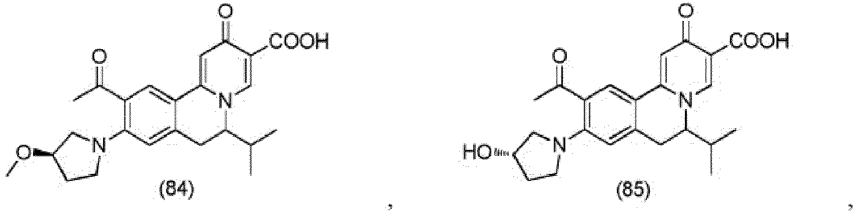 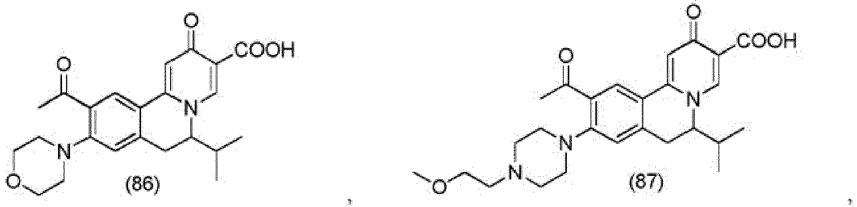 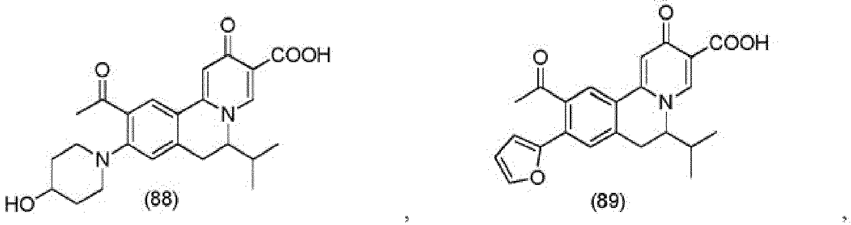 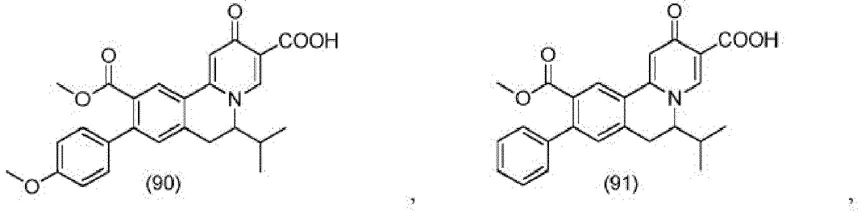 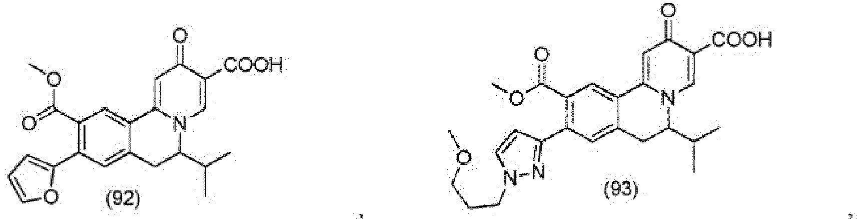 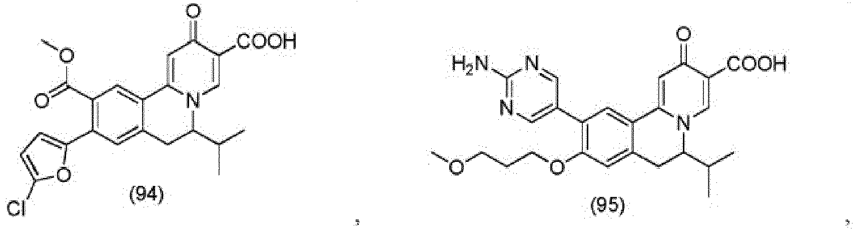 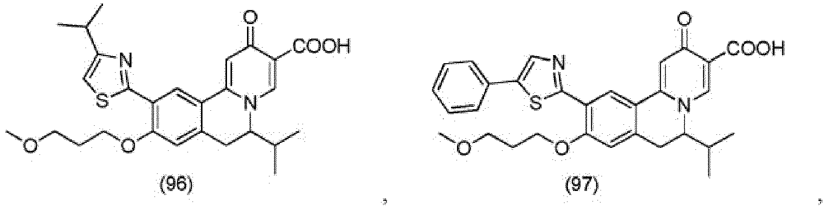 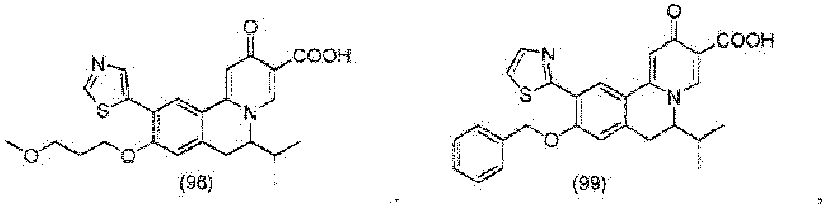 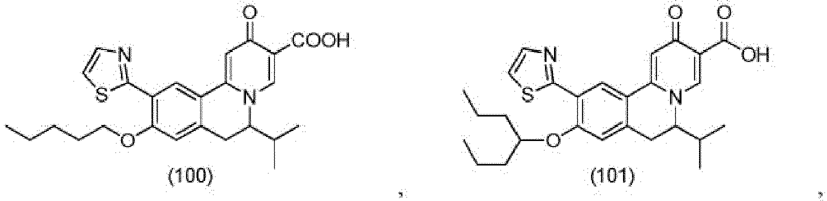 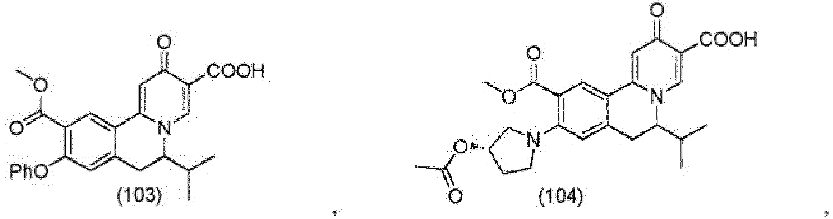 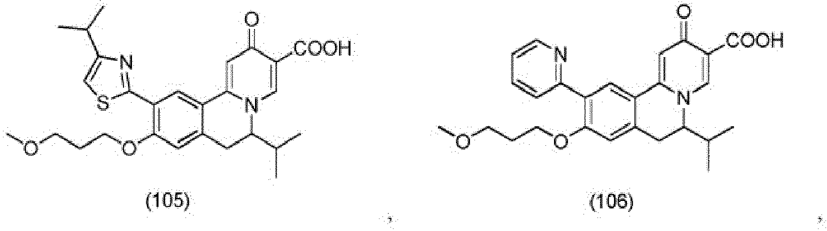 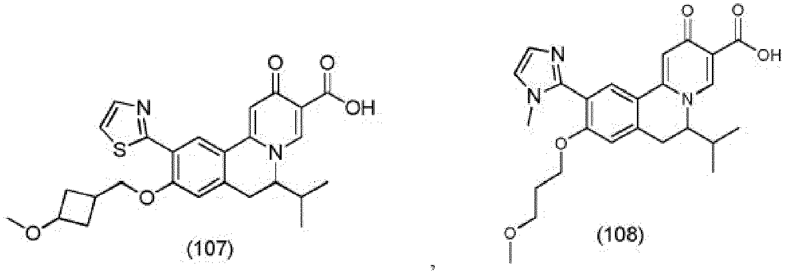 oder 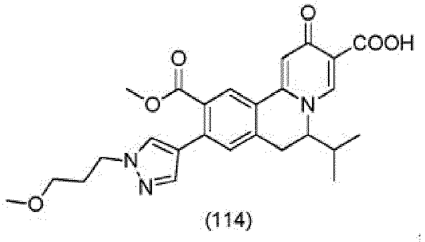 oder ein Stereoisomer, ein Tautomer, ein *N*-Oxid oder ein pharmazeutisch akzeptables Salz.

10. Pharmazeutische Zusammensetzung, umfassend die Verbindung gemäß irgendeinem der Ansprüche 1 bis 9; wobei die pharmazeutische Zusammensetzung optional weiterhin einen pharmazeutisch akzeptablen Hilfsstoff oder eine Kombination der Hilfsstoffe umfasst.

11. Pharmazeutische Zusammensetzung gemäß Anspruch 10, welche weiterhin einen anderen Anti-HBV-Arzneistoff umfasst, wobei der andere Anti-HBV-Arzneistoff ein HBV-Polymeraseinhibitor, ein Immunmodulator oder ein Interferon ist.

12. Pharmazeutische Zusammensetzung gemäß Anspruch 10 oder 11, wobei das andere Anti-HBV-Agens Lamivudin, Telbivudin, Tenofovir, Entecavir, Adefovir Dipivoxil, Alfaferon, Alloferon, Celmoleukin, Clevudin, Emtricitabin, Famciclovir, Interferon, Hepatect CP, Intefen, Interferon α-1b, Interferon α, Interferon α-2a, Interferon β-1a, Interferon α-2, Interleukin-2, Mivotilat, Nitazoxanid, Peginterferon alfa-2a, Ribavirin, Roferon-A, Sizofiran, Euforavac, Ampligen, Phosphazid, Heplisav, Interferon α-2b, Levamisol oder Propagermanium ist.

13. Verbindung gemäß irgendeinem der Ansprüche 1 bis 9 oder pharmazeutische Zusammensetzung gemäß Anspruch 10 oder 12 zur Verwendung bei der Prävention, Behandlung oder Verminderung einer durch eine Virusinfektion verursachten Störung oder Erkrankung in einem Patienten, wobei die Viruserkrankung Hepatitis B-Infektion oder eine durch Hepatitis B-Infektion verursachte Erkrankung ist.

14. Verbindung zur Verwendung oder pharmazeutische Zusammensetzung zur Verwendung gemäß Anspruch 13, wobei die durch Hepatitis B-Infektion verursachte Erkrankung Zirrhose oder hepatozelluläre Karzinogenese ist.

15. Verbindung gemäß irgendeinem der Ansprüche 1 bis 9 oder pharmazeutische Zusammensetzung gemäß Anspruch 10 oder 12 zur Verwendung bei der Inhibition der Produktion oder Sekretion von HBsAg und/oder bei der Inhibition der Produktion von HBV-DNA in einem Patienten.

## Revendications

1. Composé présentant la Formule (I) ou un stéréoisomère, un tautomère, un *N-*oxyde, un solvate ou un sel pharmaceutiquement acceptable de ceux-ci, dans lequel 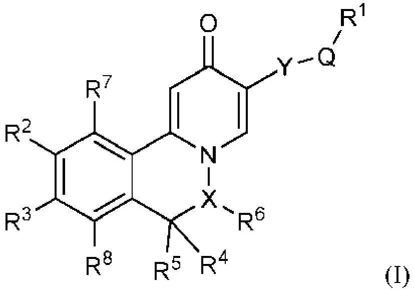
X est du N ou du -CR^{6a}- ;
Y est du -C(=O)- ;
Q est une liaison simple, -O- ou -N(R⁹)- ;
R¹ est du H, du deutérium, du méthyle, de l'éthyle, du n-propyle ou de l'isopropyle, dans lequel chacun parmi le méthyle, l'éthyle, le n-propyle et l'isopropyle est indépendamment non substitué ou substitué par 1, 2, 3 ou 4 R^{v} ;
R⁹ est du H, du deutérium, du méthyle, de l'éthyle, du n-propyle, de l'isopropyle ou un haloalkyle en C₁₋₆ ;
chacun des R⁴ et R⁵ est indépendamment du H, du deutérium, un alkyle en C₁₋₄, un alkylamino en C₁₋₄ ou un alcoxy en C₁₋₄, dans lequel chacun des alkyle en C₁₋₄, alkylamino en C₁₋₄ et alcoxy en C₁₋₄ est indépendamment non substitué ou substitué par 1, 2, 3 ou 4 R^{y} ;
R⁶ est du H, du deutérium, un alkyle en C₁₋₄, un alkényle en C₂₋₄ ou un cycloalkyle en C₃₋₆, dans lequel chacun des alkyle en C₁₋₄, alkényle en C₂₋₄ et cycloalkyle en C₃₋₆ est indépendamment non substitué ou substitué par 1, 2, 3 ou 4 R^{z} ;
R^{6a} est du H, du deutérium, du F, du Cl, du Br, du CN, du OH, un alkyle en C₁₋₄ ou un alkényle en C₂₋₄, dans lequel chacun des alkyle en C₁₋₄ et alkényle en C₂₋₄ est indépendamment non substitué ou substitué par 1, 2, 3 ou 4 R^{z} ;
R² est du phényle, un hétéroaryle à 5 à 6 chaînons, du R¹³-O-C(=O)-(CR^{e}R^{f})ₘ-, dans lequel m vaut 0, R¹⁴-S(=O)₂-N(R^{g})-C(=O)-, R^{c}C(=O)-, R^{a}R^{b}NC(=O)- ou R^{d}OC(=O)-, dans lequel chacun parmi le phényle et l'hétéroaryle à 5 à 6 chaînons est indépendamment non substitué ou substitué par 1, 2, 3 ou 4 R^{w} ;
chacun des R⁷ et R⁸ est indépendamment du H, du deutérium, du F, du Cl, du Br, un hydroxy, un cyano ou un alkyle en C₁₋₆ ;
R³ est un hétérocyclyle à 5 à 6 chaînons, du phényle, un hétéroaryle à 5 à 6 chaînons R¹³-(CR^{e}R^{f})ₘ-O- ou R¹⁰O-, dans lequel chacun des hétérocyclyle à 5 à 6 chaînons, phényle et hétéroaryle à 5 à 6 chaînons est indépendamment non substitué ou substitué par 1, 2, 3 ou 4 R^{w} ;
R¹⁰ est du H, du deutérium, un alkyle en C₁₋₆, un alkynyle en C₂₋₆, un cycloalkyle en C₃₋₆, un hétérocyclyle à 5 à 6 chaînons, du phényle, du naphtyle ou un hétéroaryle à 5 à 6 chaînons, dans lequel chacun des alkyle en C₁₋₆, alkynyle en C₂₋₆, cycloalkyle en C₃₋₆, hétérocyclyle à 5 à 6 chaînons, phényle, naphtyle et hétéroaryle à 5 à 6 chaînons est indépendamment non substitué ou substitué par 1, 2, 3 ou 4 R^{x} ;
chaque R¹³ et R¹⁴ est indépendamment un alkyle en C₁₋₁₂, un alcoxy en C₁₋₁₂, un cycloalkyle en C₃₋₇, un hétéroaryle à 5 à 10 chaînons, un hétérocyclyle à 3 à 12 chaînons ou un aryle en C₆₋₁₀, dans lequel chacun des alkyle en C₁₋₁₂, alcoxy en C₁₋₁₂, cycloalkyle en C₃₋₇, hétéroaryle à 5 à 10 chaînons, hétérocyclyle à 3 à 12 chaînons et aryle en C₆₋₁₀ est indépendamment non substitué ou substitué par 1, 2, 3 ou 4 R^{h} ;
chaque R^{a}, R^{b}, R^{c}, R^{d}, R^{e}, R^{f} et R^{g} est indépendamment du H, du deutérium, du OH, un haloalkyle en C₁₋₄, un alkyle en C₁₋₆, un alcoxy en C₁₋₄, un alkényle en C₂₋₄, un alkynyle en C₂₋₄, un cycloalkyle en C₃₋₆, du phényle, du naphtyle, un hétérocyclyle à 5 à 6 chaînons ou un hétéroaryle à 5 à 6 chaînons, dans lequel chacun des haloalkyle en C₁₋₄, alkyle en C₁₋₆, alcoxy en C₁₋₄, alkényle en C₂₋₄, alkynyle en C₂₋₄, cycloalkyle en C₃₋₆, phényle, naphtyle, hétérocyclyle à 5 à 6 chaînons et hétéroaryle en 5 à 6 chaînons est indépendamment non substitué ou substitué par 1, 2, 3 ou 4 substituants indépendamment sélectionnés parmi F, Cl, Br, CN, OH, un amino, un alkyle en C₁₋₄, un haloalkyle en C₁₋₄, un alcoxy en C₁₋₄ ou un alkylamino en C₁₋₄ ;
ou R^{a} et R^{b}, avec l'atome d'azote auquel ils sont attachés, forment un hétérocyclyle à 5 à 6 chaînons ou un hétéroaryle à 5 à 6 chaînons, dans lequel chacun parmi l'hétérocyclyle à 5 à 6 chaînons et l'hétéroaryle à 5 à 6 chaînons est indépendamment non substitué ou substitué par 1, 2, 3 ou 4 substituants indépendamment sélectionnés parmi F, Cl, Br, CN, OH, un amino, un alkyle en C₁₋₄, un haloalkyle en C₁₋₄, un alcoxy en C₁₋₄ ou un alkylamino en C₁₋₄ ;
chaque R^{v}, R^{w}, R^{x}, R^{y}, R^{z} et R" est indépendamment du F, du Cl, du Br, du CN, du OH, du -COOH, un amino, un alkyle en C₁₋₆, un haloalkyle en C₁₋₆, un alcoxy en C₁₋₆, un haloalcoxy en C₁₋₆, un alkylthio en C₁₋₆, un alkylamino en C₁₋₆, un alkényle en C₂₋₄, un alkynyle en C₂₋₄, un cycloalkyle en C₃₋₆, un hétérocyclyle à 5 à 6 chaînons, du phényle, du naphtyle ou un hétéroaryle à 5 à 6 chaînons, dans lequel chacun des amino, alkyle en C₁₋₆, haloalkyle en C₁₋₆, alcoxy en C₁₋₆, haloalcoxy en C₁₋₆, alkylthio en C₁₋₆, alkylamino en C₁₋₆, alkényle en C₂₋₄, alkynyle en C₂₋₄, cycloalkyle en C₃₋₆, hétérocyclyle à 5 à 6 chaînons, phényle, naphtyle et hétéroaryle à 5 à 6 chaînons est indépendamment non substitué ou substitué par 1, 2, 3 ou 4 substituants indépendamment sélectionnés parmi F, Cl, Br, CN, OH, =O, -COOH, un amino, un alkyle en C₁₋₄, un haloalkyle en C₁₋₄, un alcoxy en C₁₋₄, un alkylthio en C₁₋₄, un haloalcoxy en C₁₋₄, un alkylamino en C₁₋₄, un alkényle en C₂₋₄, un alkynyle en C₂₋₄, du phényle, du naphtyle, un cycloalkyle en C₃₋₆, un hétéroaryle à 5 à 6 chaînons, un hétérocyclyle à 5 à 6 chaînons, un alcoxy en C₁₋₄-alkyle en C₁₋₄ ou un alkylamino en C₁₋₄-alkyle en C₁₋₄ ;
m vaut 0, 1, 2, 3, 4, 5, 6, 7, 8, 9 ou 10.

2. Composé selon l'une quelconque de la revendication 1, dans lequel R² est du furyle, du pyrrolyle, du pyridyle, du pyrazolyle, de l'imidazolyle, du triazolyle, du tétrazolyle, de l'oxazolyle, de l'isoxazolyle, de l'oxadiazolyle, du 1,3,5-triazinyle, du thiazolyle, du thiényle, du pyrazinyle, du pyridazinyle, du pyrimidinyle, du phényle, du R¹³-O-C(=O)-(CR^{e}R^{f})_{M}-, dans lequel m vaut 0, R^{c}-C(=O)-(CR^{e}R^{f})ₘ-O-C(=O)-, R¹⁴-S(=O)₂-N(R^{g})-C(=O)-, R^{c}C(=O)-, R^{a}R^{b}NC(=O)- ou R^{d}OC(=O)-, dans lequel chacun des furyle, pyrrolyle, pyridyle, pyrazolyle, imidazolyle, triazolyle, tétrazolyle, oxazolyle, isoxazolyle, oxadiazolyle, 1,3,5-triazinyle, thiazolyle, thiényle, pyrazinyle, pyridazinyle, pyrimidinyle et phényle est indépendamment non substitué ou substitué par 1, 2, 3 ou 4 R^{w}.

3. Composé selon la revendication 1 ou la revendication 2, dans lequel chacun des R⁷ et R⁸ est indépendamment du H, du deutérium, du F, du Cl, du Br, un hydroxy, un cyano, du méthyle, de l'éthyle, du n-propyle, de l'isopropyle, du *n*-butyle, de l'isobutyle, du *n*-pentyle ou du *n*-hexyle ;
R³ est du pyrrolidinyle, du pyrazolidinyle, de l'imidazolidinyle, du tétrahydrofuranyle, du tétrahydrothiophényle, du tétrahydropyranyle, du tétrahydrothiapyranyle, du pipéridyle, du morpholinyle, du thiomorpholinyle, du pipérazinyle, du furyle, du pyrrolyle, du pyridyle, du pyrazolyle, de l'imidazolyle, du triazolyle, du tétrazolyle, de l'oxazolyle, de l'isoxazolyle, de l'oxadiazolyle, du 1,3,5-triazinyle, du thiazolyle, du thiényle, du pyrazinyle, du pyridazinyle, du pyrimidinyle, du phényle, du R¹³-(CR^{e}R^{f})ₘ-O- ou du R¹⁰O-, et dans lequel chacun des pyrrolidinyle, pyrazolidinyle, imidazolidinyle, tétrahydrofuranyle, tétrahydrothiophényle, tétrahydropyranyle, tétrahydrothiapyranyle, pipéridyle, morpholinyle, thiomorpholinyle, pipérazinyle, furyle, pyrrolyle, pyridyle, pyrazolyle, imidazolyle, triazolyle, tétrazolyle, oxazolyle, isoxazolyle, oxadiazolyle, 1,3,5-triazinyle, thiazolyle, thiényle, pyrazinyle, pyridazinyle, pyrimidinyle et phényle est indépendamment non substitué ou substitué par 1, 2, 3 ou 4 R^{w} ;
R¹⁰ est du H, du deutérium, du méthyle, de l'éthyle, du n-propyle, de l'isopropyle, du n-butyle, de l'isobutyle, du n-pentyle, du n-hexyle, de l'éthynyle, du propynyle, du cyclopropyle, du cyclobutyle, du cyclopentyle, du cyclohexyle, du pyrrolidinyle, du pyrazolidinyle, de l'imidazolidinyle, du tétrahydrofuranyle, du tétrahydrothiophényle, du tétrahydropyranyle, du tétrahydrothiopyranyle, du pipéridyle, du morpholinyle, du thioxomorpholinyle, du pipérazinyle, du furyle, du pyrrolyle, du pyridyle, du pyrazolyle, de l'imidazolyle, du triazolyle, du tétrazolyle, de l'oxazolyle, de l'isoxazolyle, de l'oxadiazolyle, du 1,3,5-triazinyle, du thiazolyle, du thiényle, du pyrazinyle, du pyridazinyle, du pyrimidinyle, du phényle ou du naphtyle, dans lequel chacun des méthyle, éthyle, n-propyle, isopropyle, n-butyle, isobutyle, n-pentyle, n-hexyle, éthynyle, propynyle, cyclopropyle, cyclobutyle, cyclopentyle, cyclohexyle, pyrrolidinyle, pyrazolidinyle, imidazolidinyle, tétrahydrofuranyle, tétrahydrothiophényle, tétrahydropyranyle, tétrahydrothiopyranyle, pipéridyle, morpholinyle, thioxomorpholinyle, pipérazinyle, furyle, pyrrolyle, pyridyle, pyrazolyle, imidazolyle, triazolyle, tétrazolyle, oxazolyle, isoxazolyle, oxadiazolyle, 1,3,5-triazinyle, thiazolyle, thiényle, pyrazinyle, pyridazinyle, pyrimidinyle, phényle et naphtyle est indépendamment non substitué ou substitué par 1, 2, 3 ou 4 R^{x}.

4. Composé selon l'une quelconque des revendications 1 à 3, dans lequel chacun des R⁴ et R⁵ est indépendamment du H, du deutérium, un alkyle en C₁₋₃, un alkylamino en C₁₋₃ ou un alcoxy en C₁₋₃, dans lequel chacun des alkyle en C₁₋₃, alkylamino en C₁₋₃ et alcoxy en C₁₋₃ est indépendamment non substitué ou substitué par 1, 2, 3 ou 4 R^{y} ;
R⁶ est du H, du deutérium, du méthyle, de l'éthyle, du *n*-propyle, de l'isopropyle, du *n*-butyle, de l'isobutyle, du *tert*-butyle, du vinyle, du propényle, du cyclopropyle, du cyclobutyle, du cyclopentyle ou du cyclohexyle, dans lequel chacun des méthyle, éthyle, *n*-propyle, isopropyle, *n*-butyle, isobutyle, *tert*-butyle, vinyle, propényle, cyclopropyle, cyclobutyle, cyclopentyle et cyclohexyle est indépendamment non substitué ou substitué par 1, 2, 3 ou 4 R^{z} ;
R^{6a} est du H, du deutérium, du F, du Cl, du Br, du CN, du OH, du méthyle, de l'éthyle, du *n*-propyle, de l'isopropyle, du *n*-butyle, de l'isobutyle, du *tert*-butyle, du vinyle ou du propényle, dans lequel chacun des méthyle, éthyle, *n*-propyle, isopropyle, *n*-butyle, isobutyle, *tert*-butyle, vinyle et propényle est indépendamment non substitué ou substitué par 1, 2, 3 ou 4 R^{z}.

5. Composé selon l'une quelconque des revendications 1 à 4, dans lequel chaque R¹³ et R¹⁴ est indépendamment un alkyle en C₁₋₆, un alcoxy en C₁₋₆, un cycloalkyle en C₃₋₆, un hétéroaryle en 5 à 6 chaînons, un hétérocyclyle à 5 à 6 chaînons, du phényle ou du naphtyle, dans lequel chacun des alkyle en C₁₋₆, alcoxy en C₁₋₆, cycloalkyle en C₃₋₆, hétéroaryle à 5 à 6 chaînons, hétérocyclyle à 5 à 6-chaînons, phényle et naphtyle est indépendamment non substitué ou substitué par 1, 2, 3 ou 4 R^{h}.

6. Composé selon l'une quelconque des revendications 1 à 5, dans lequel chaque R¹³ et R¹⁴ est indépendamment du méthyle, de l'éthyle, du n-propyle, de l'isopropyle, du *n*-butyle, de l'isobutyle, du *tert*-butyle, un méthoxy, un éthoxy, un propoxy, un isopropoxy, un *n*-butoxy, du cyclopropyle, du cyclobutyle, du cyclopentyle, du cyclohexyle, du pyrrolidinyle, du pyrazolidinyle, de l'imidazolidinyle, du tétrahydrofuranyle, du tétrahydrothiophényle, du tétrahydropyranyle, du tétrahydrothiopyranyle, du pipéridyle, du morpholinyle, du thiomorpholinyle, du pipérazinyle, du furyle, du pyrrolyle, du pyridyle, du pyrazolyle, de l'imidazolyle, du triazolyle, du tétrazolyle, de l'oxazolyle, de l'isoxazolyle, de l'oxadiazolyle, du 1,3,5-triazinyle, du thiazolyle, du thiényle, du pyrazinyle, du pyridazinyle, du pyrimidinyle, du phényle ou du naphtyle, dans lequel chacun des méthyle, éthyle, *n*-propyle, isopropyle, *n*-butyle, isobutyle, *tert*-butyle, méthoxy, éthoxy, propoxy, isopropyle, *n-*butoxy, cyclopropyle, cyclobutyle, cyclopentyle, cyclohexyle, pyrrolidinyle, pyrazolidinyle, imidazolidinyle, tétrahydrofuranyle, tétrahydrothiophényle, tétrahydropyranyle, tétrahydrothiopyranyle, pipéridyle, morpholinyle, thiomorpholinyle, pipérazinyle, furyle, pyrrolyle, pyridyle, pyrazolyle, imidazolyle, triazolyle, tétrazolyle, oxazolyle, isoxazolyle, oxadiazolyle, 1,3,5-triazinyle, thiazolyle, thiényle, pyrazinyle, pyridazinyle, pyrimidinyle, phényle et naphtyle est indépendamment non substitué ou substitué par 1, 2, 3 ou 4 R^{h}.

7. Composé selon l'une quelconque des revendications 1 à 6, dans lequel R^{a}, R^{b}, R^{c}, R^{d}, R^{e}, R^{f} et R^{g} est indépendamment du H, du deutérium, du OH, un haloalkyle en C₁₋₃, du méthyle, de l'éthyle, du *n*-propyle, de l'isopropyle, du *n*-butyle, de l'isobutyle, du *tert*-butyle, du *n*-pentyle, du 3-pentyle, un méthoxy, un éthoxy, un propoxy, un isopropoxy, un *n*-butoxy, de l'éthényle, du propényle, de l'éthynyle, du propynyle, du propargyle, du cyclopropyle, du cyclobutyle, du cyclopentyle, du cyclohexyle, du pyrrolidinyle, du pyrazolidinyle, de l'imidazolidinyle, du tétrahydrofuranyle, du tétrahydrothiophényle, du tétrahydropyranyle, du tétrahydrothiopyranyle, du pipéridyle, du morpholinyle, du thiomorpholinyle, du pipérazinyle, du furyle, du pyrrolyle, du pyridyle, du pyrazolyle, de l'imidazolyle, du triazolyle, du tétrazolyle, de l'oxazolyle, de l'isoxazolyle, de l'oxadiazolyle, du 1,3,5-triazinyle, du thiazolyle, du thiényle, du pyrazinyle, du pyridazinyle, du pyrimidinyle, du phényle ou du naphtyle, dans lequel chacun des haloalkyle en C₁₋₃, méthyle, éthyle, *n*-propyle, isopropyle, *n-*butyle, isobutyle, *tert*-butyle, méthoxy, éthoxy, propoxy, isopropoxy, *n*-butoxy, éthényle, propényle, éthynyle, propynyle, cyclopropyle, cyclobutyle, cyclopentyle, cyclohexyle, pyrrolidinyle, pyrazolidinyle, imidazolidinyle, tétrahydrofuranyle, tétrahydrothiophényle, tétrahydropyranyle, tétrahydrothiopyranyle, pipéridyle, morpholinyle, thiomorpholinyle, pipérazinyle, furyle, pyrrolyle, pyridyle, pyrazolyle, imidazolyle, triazolyle, tétrazolyle, oxazolyle, isoxazolyle, oxadiazolyle, 1,3,5-triazinyle, thiazolyle, thiényle, pyrazinyle, pyridazinyle, pyrimidinyle, phényle et naphtyle est indépendamment non substitué ou substitué par 1, 2, 3 ou 4 substituants indépendamment sélectionnés par F, Cl, Br, CN, OH, un amino, un alkyle en C₁₋₄, un haloalkyle en C₁₋₄, un alcoxy en C₁₋₄ ou un alkylamino en C₁₋₄ ;
ou, R^{a} et R^{b}, avec l'atome d'azote auquel ils sont attachés, forment du pyrrolidinyle, du pyrazolidinyle, de l'imidazolidinyle, du pipéridyle, du morpholinyle, du thiomorpholinyle, du pipérazinyle, du pyrrolyle, du pyridyle, du pyrazolyle, de l'imidazolyle, du triazolyle, du tétrazolyle, de l'oxazolyle, de l'isoxazolyle, de l'oxadiazolyle, du 1,3,5-triazinyle, du thiazolyle, du pyrazinyle, du pyridazinyle ou du pyrimidinyle, dans lequel chacun des pyrrolidinyle, pyrazolidinyle, imidazolidinyle, pipéridyle, morpholinyle, thiomorpholinyle, pipérazinyle, pyrrolyle, pyridyle, pyrazolyle, imidazolyle, triazolyle, tétrazolyle, oxazolyle, isoxazolyle, oxadiazolyle, 1,3,5-triazinyle, thiazolyle, pyrazinyle, pyridazinyle et pyrimidinyle est indépendamment non substitué ou substitué par 1, 2, 3 ou 4 substituants indépendamment sélectionnés parmi F, Cl, Br, CN, OH, un amino, un alkyle en C₁₋₄, un haloalkyle en C₁₋₄, un alcoxy en C₁₋₄ ou un alkylamino en C₁₋₄.

8. Composé selon l'une quelconque des revendications 1 à 7, dans lequel chaque R^{v}, R^{w}, R", R^{y}, R^{z} et R^{h} est indépendamment du F, du CI, du Br, du CN, du OH, du - COOH, un amino, un alkyle en C₁₋₄, un haloalkyle en C₁₋₄, un alcoxy en C₁₋₄, un haloalcoxy en C₁₋₄, un alkylthio en C₁₋₄, un alkylamino en C₁₋₄, un alkényle en C₂₋₄, un alkynyle en C₂₋₄, du cyclopropyle, du cyclobutyle, du cyclopentyle, du cyclohexyle, du pyrrolidinyle, du pyrazolidinyle, de l'imidazolidinyle, du tétrahydrofuranyle, du tétrahydrothiophényle, du tétrahydropyranyle, du tétrahydrothiopyranyle, du pipéridyle, du morpholinyle, du thiomorpholinyle, du pipérazinyle, du furyle, du pyrrolyle, du pyridyle, du pyrazolyle, de l'imidazolyle, du triazolyle, du tétrazolyle, de l'oxazolyle, de l'isoxazolyle, de l'oxadiazolyle, du 1,3,5-triazinyle, du thiazolyle, du thiényle, du pyrazinyle, du pyridazinyle, du pyrimidinyle, du phényle ou du naphtyle, dans lequel chacun des cyclopropyle, cyclobutyle, cyclopentyle, cyclohexyle, pyrrolidinyle, pyrazolidinyle, imidazolidinyle, tétrahydrofuranyle, tétrahydrothiophényle, tétrahydropyranyle, tétrahydrothiopyranyle, pipéridyle, morpholinyle, thiomorpholinyle, pipérazinyle, furyle, pyrrolyle, pyridyle, pyrazolyle, imidazolyle, triazolyle, tétrazolyle, oxazolyle, isoxazolyle, oxadiazolyle,1,3,5-triazinyle, thiazolyle, thiényle, pyrazinyle, pyridazinyle, pyrimidinyle, phényle et naphtyle est indépendamment non substitué ou substitué par 1, 2, 3 ou 4 substituants indépendamment sélectionnés parmi F, Cl, Br, CN, OH, =O, -COOH, un amino, un alkyle en C₁₋₄, un haloalkyle en C₁₋₄, un alcoxy en C₁₋₄, un alkylthio en C₁₋₄, un haloalcoxy en C₁₋₄, un alkylamino en C₁₋₄, un alkényle en C₂₋₄, un alkynyle en C₂₋₄, du cyclopropyle, du cyclobutyle, du cyclopentyle, du cyclohexyle, du pyrrolidinyle, du pyrazolidinyle, de l'imidazolidinyle, du tétrahydrofuranyle, du tétrahydrothiophényle, du tétrahydropyranyle, du tétrahydrothiopyranyle, du pipéridyle, du morpholinyle, du thiomorpholinyle, du pipérazinyle, du furyle, du pyrrolyle, du pyridyle, du pyrazolyle, de l'imidazolyle, du triazolyle, du tétrazolyle, de l'oxazolyle, de l'isoxazolyle, de l'oxadiazolyle, du 1,3,5-triazinyle, du thiazolyle, du thiényle, du pyrazinyle, du pyridazinyle, du pyrimidinyle, du phényle, du naphtyle, un alcoxy en C₁₋₃-alkyle en C₁₋₃ et un alkylamino en C₁₋₃-alkyle en C₁₋₃.

9. Composé selon l'une quelconque des revendications 1 à 8 présentant l'une des structures suivantes, 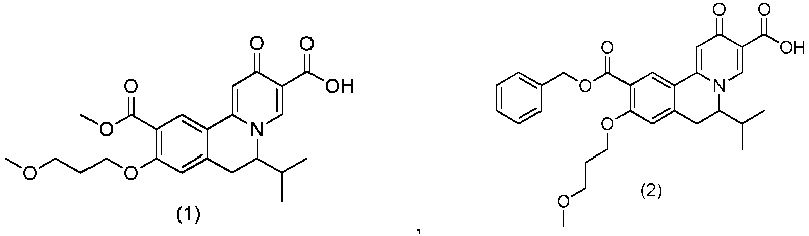 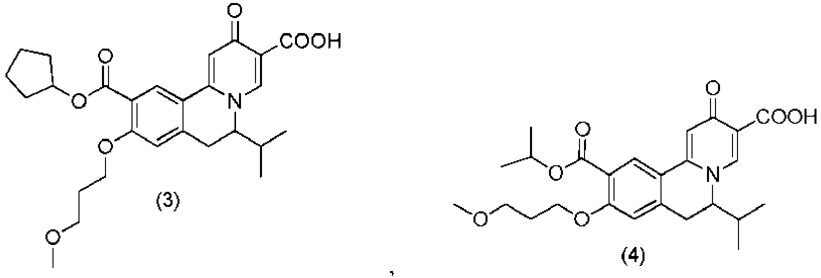 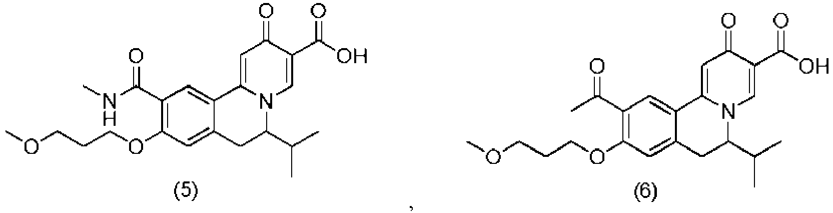 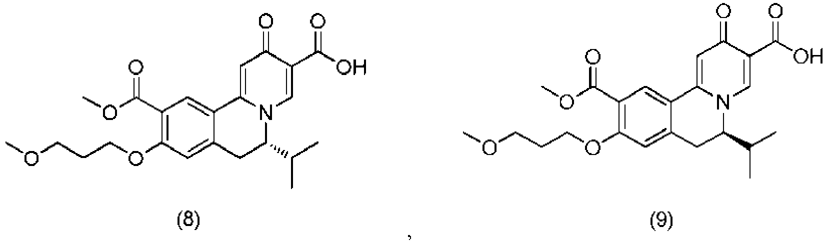 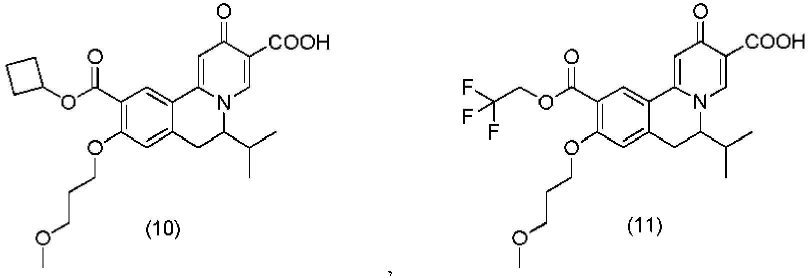 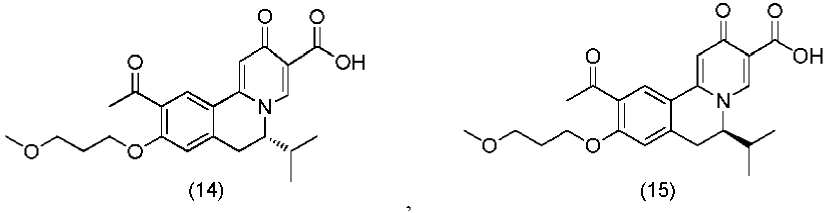 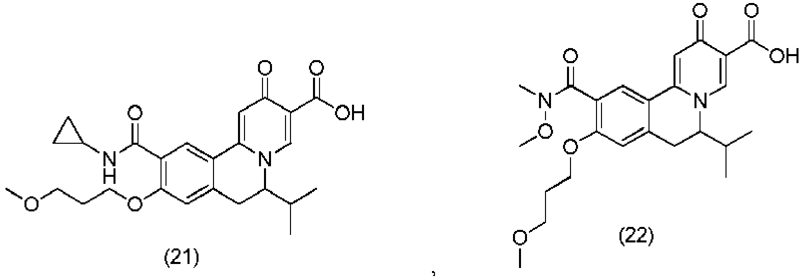 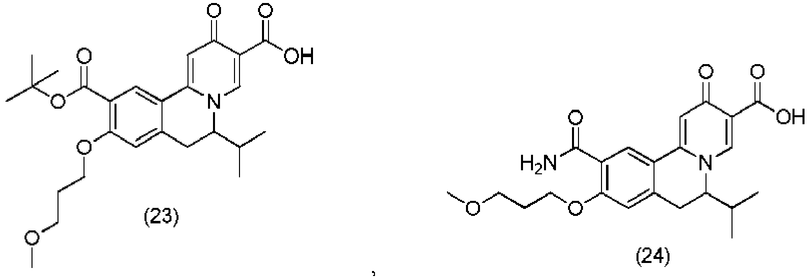 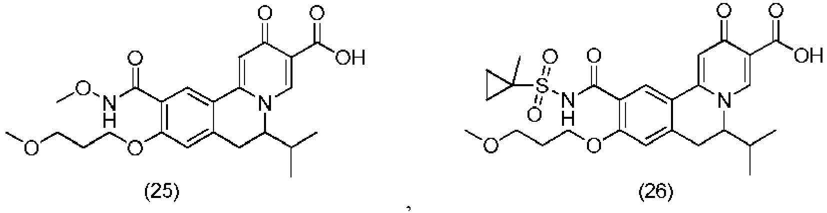 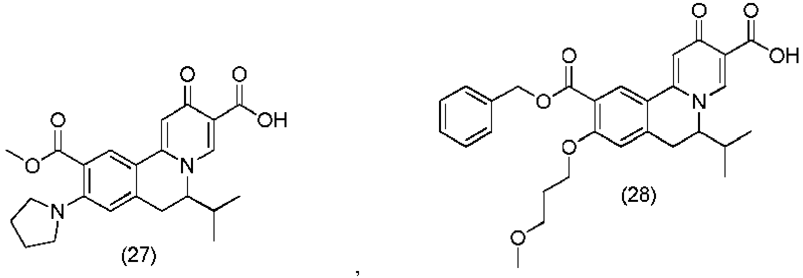 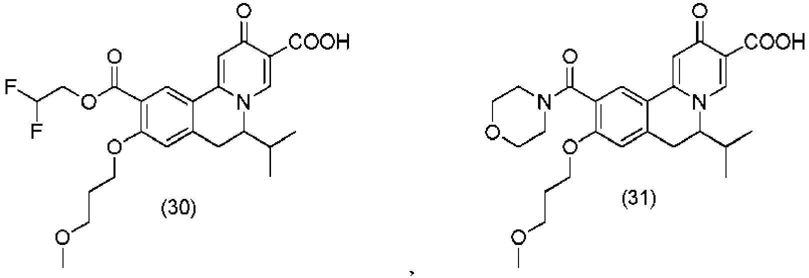 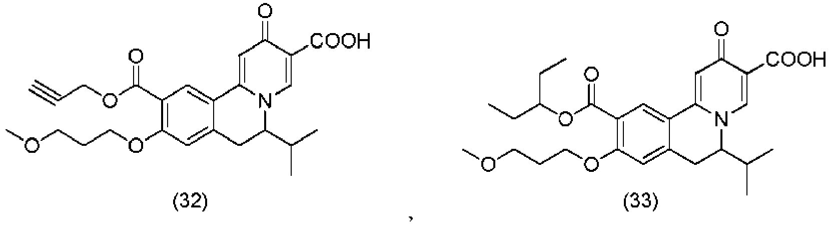 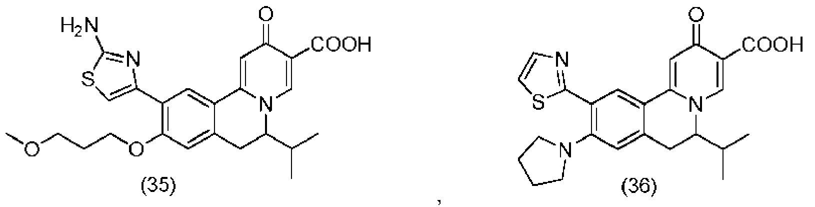 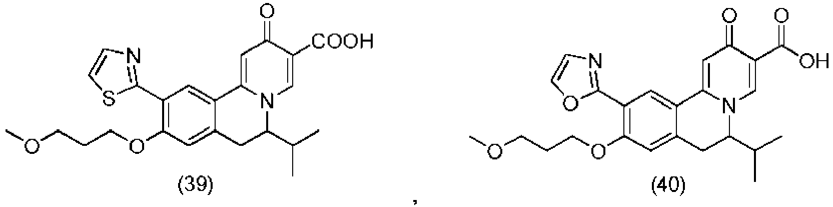 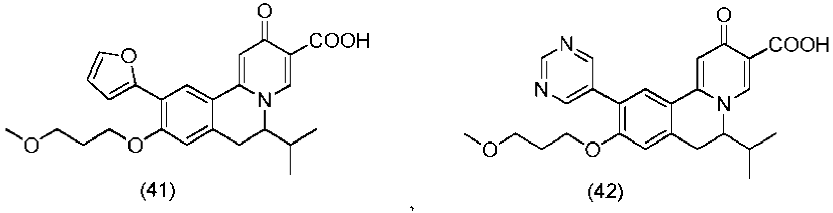 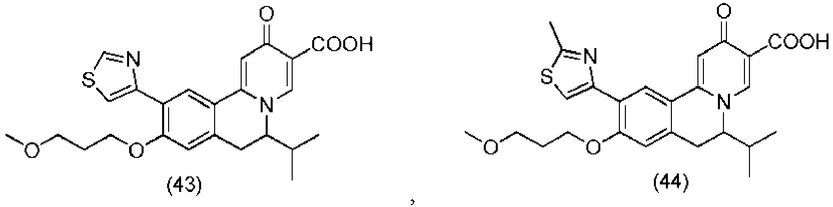 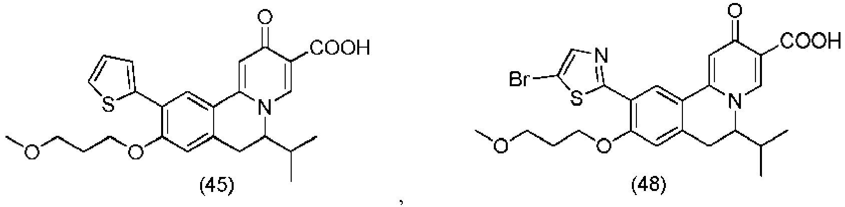 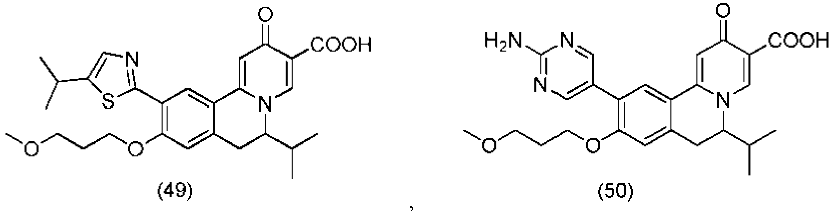 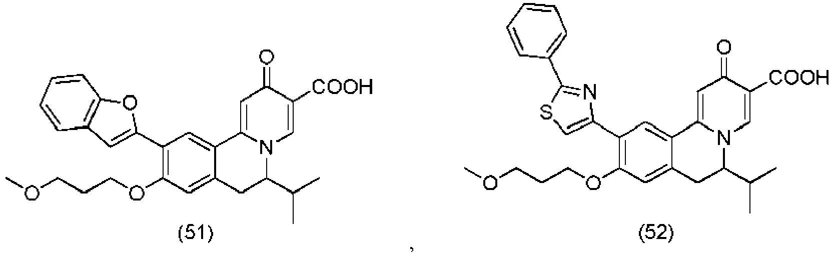 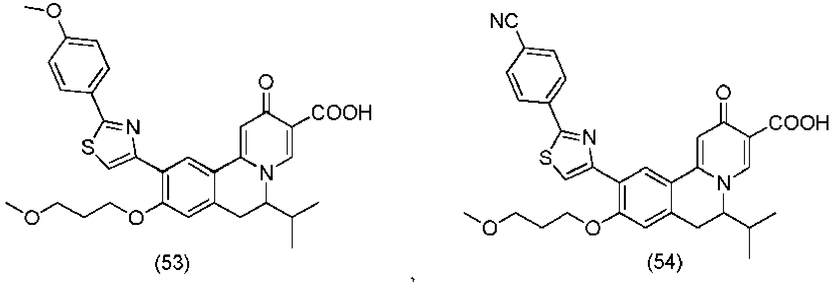 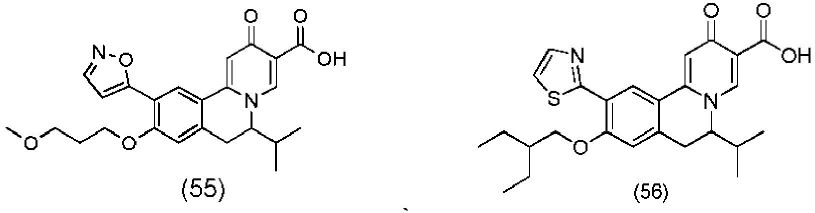 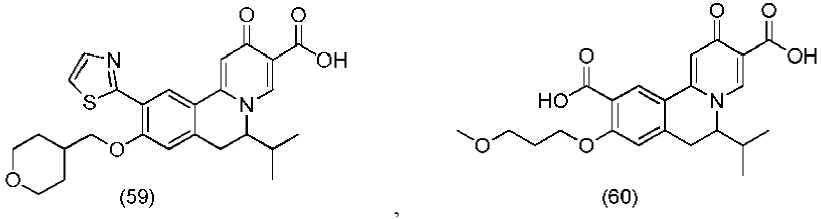 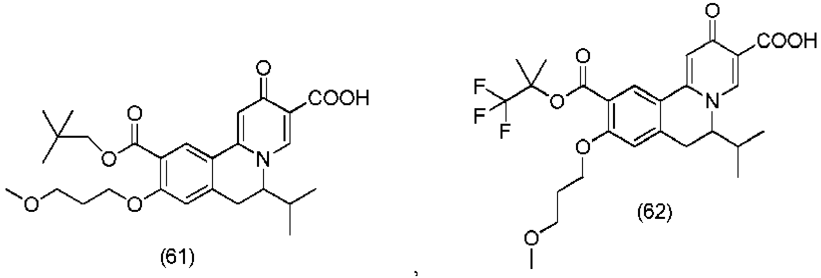 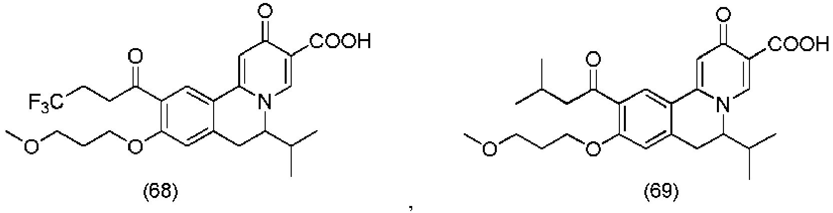 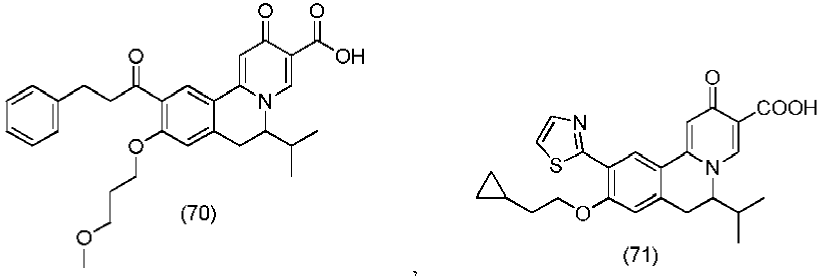 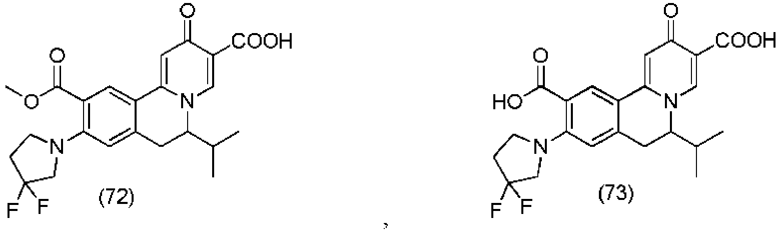 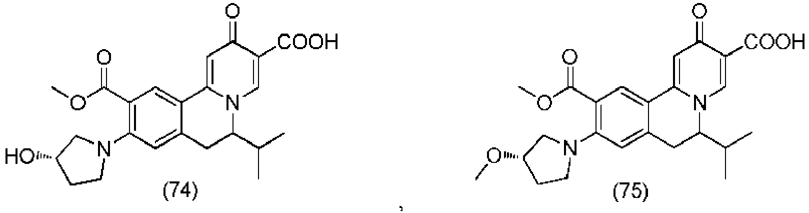 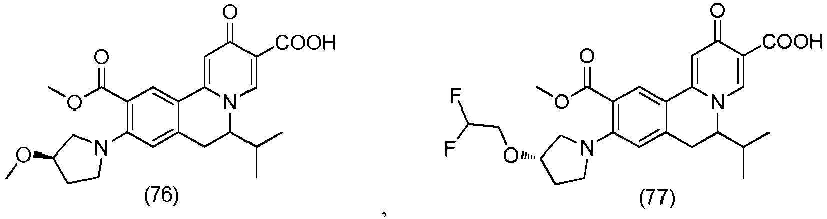 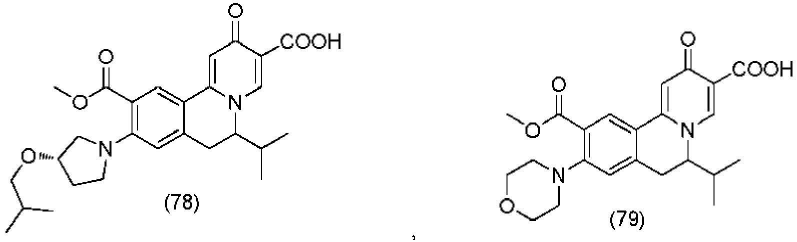 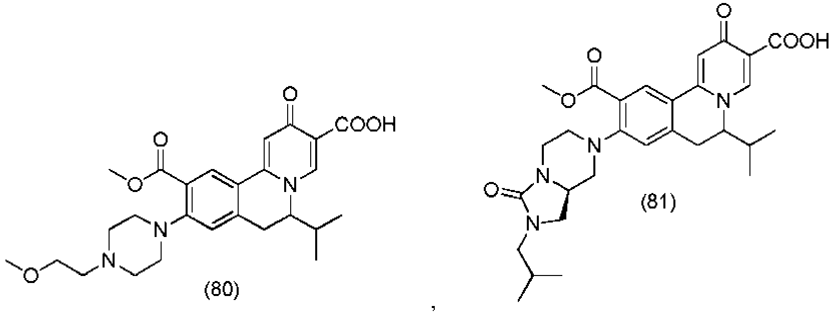 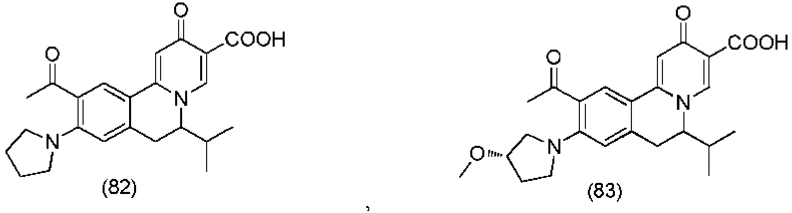 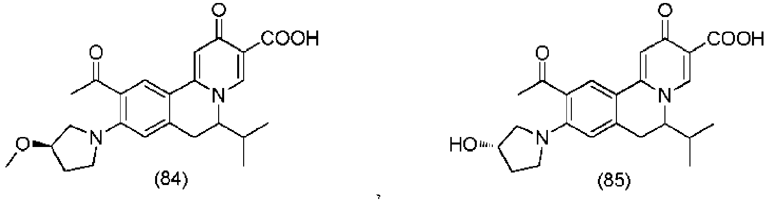 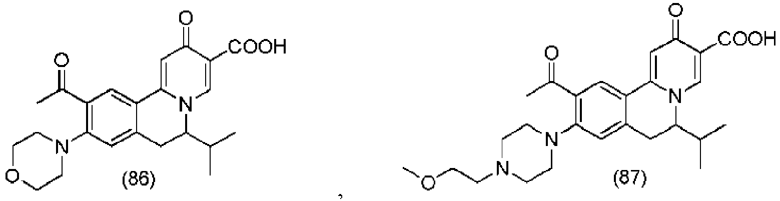 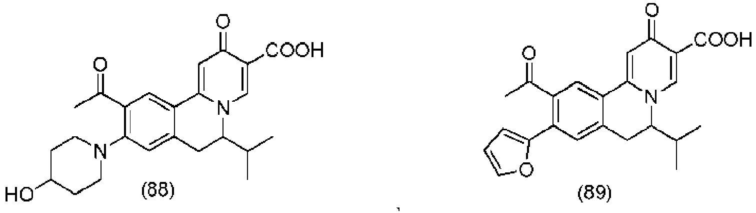 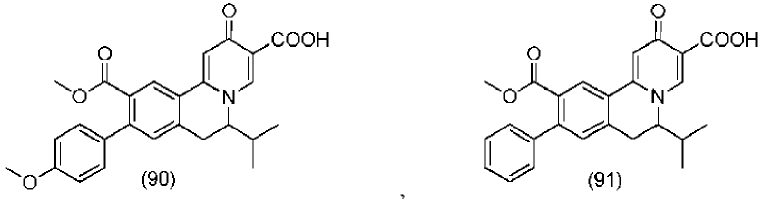 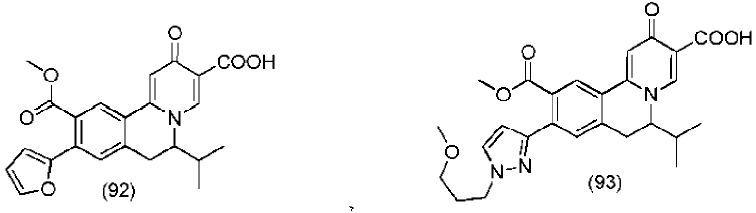 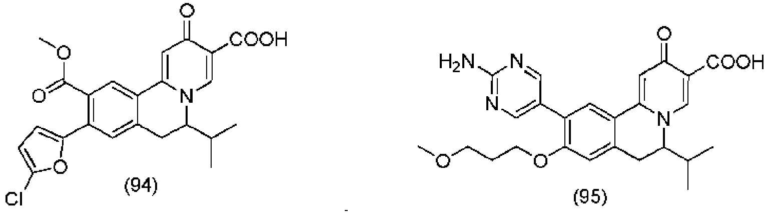 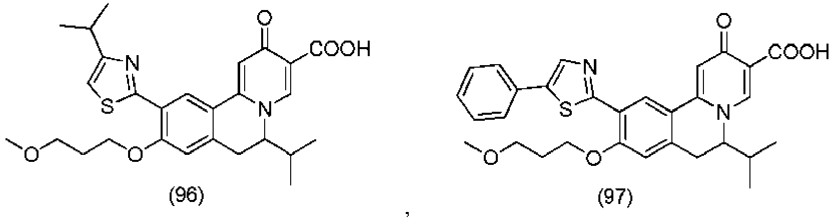 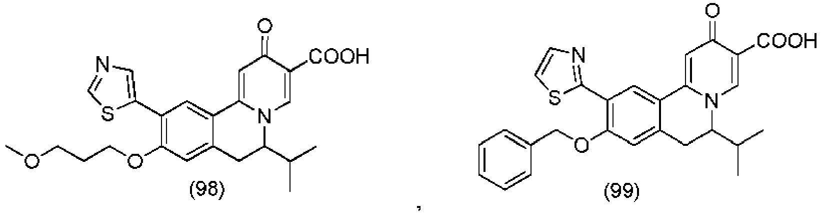 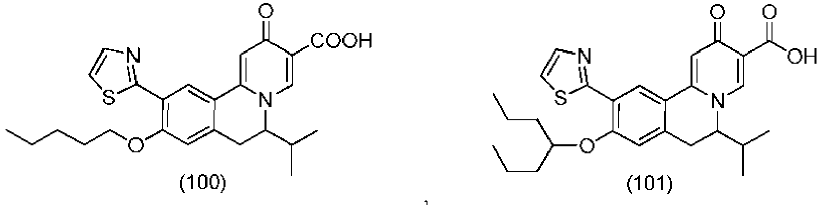 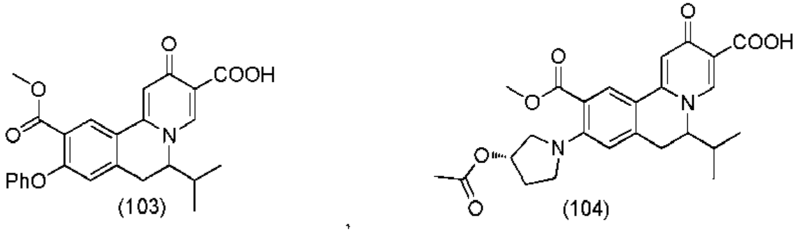 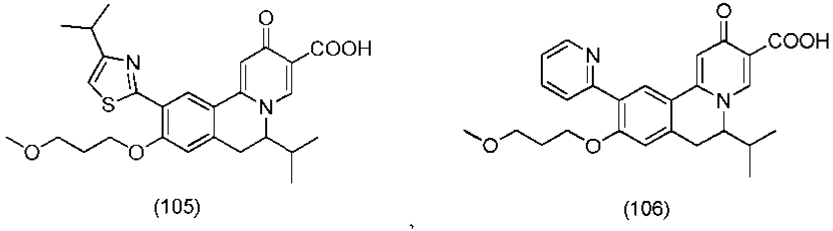 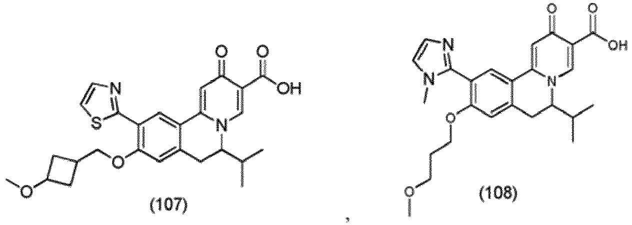 ou 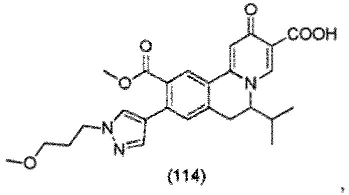 ou un stéréoisomère, un tautomère, un N-oxyde ou un sel pharmaceutiquement acceptable.

10. Composition pharmaceutique comprenant le composé selon l'une quelconque des revendications 1 à 9 ; facultativement, dans laquelle la composition pharmaceutique comprend en outre un excipient pharmaceutiquement acceptable, ou une combinaison des excipients.

11. Composition pharmaceutique selon la revendication 10 comprenant en outre un autre médicament anti-HBV, dans laquelle l'autre médicament anti-HBV est un inhibiteur de la polymérase HBV, un immunomodulateur ou un interféron.

12. Composition pharmaceutique selon la revendication 10 ou la revendication 11, dans laquelle l'autre agent anti-HBV est la lamivudine, la telbivudine, le ténofovir, l'entecavir, l'adéfovir dipivoxil, l'alfaferone, l'alloferon, la celmoleukine, la clévudine, l'emtricitabine, le famciclovir, l'interféron, l'hepatect CP, l'intefen, l'interféron α-1b, l'interféron α, l'interféron α-2a, l'interféron β-1a, l'interféron α-2, l'interleukin-2, le mivotilate, le nitazoxanide, le peginterféron alfa-2a, la ribavirine, le roféron-A, le sizofiran, l'euforavac, l'ampligen, le phosphazide, l'heplisav, l'interféron α-2b, le lévamisole ou le propagermanium.

13. Composé selon l'une quelconque des revendications 1 à 9 ou composition pharmaceutique selon la revendication 10 ou la revendication 12 pour utilisation dans la prévention, le traitement ou l'atténuation d'un trouble ou d'une maladie causé par une infection virale chez un patient, dans lequel la maladie virale est une infection par hépatite B ou une maladie causée par une infection par hépatite B.

14. Composé pour utilisation ou composition pharmaceutique pour utilisation selon la revendication 13, dans lequel la maladie causée par une infection par hépatite B est la cirrhose ou une carcinogenèse hépatocellulaire.

15. Composé selon l'une quelconque des revendications 1 à 9 ou composition pharmaceutique selon la revendication 10 ou revendication 12 pour utilisation dans l'inhibition de la production ou de la sécrétion de HBsAg, et/ou l'inhibition de la production d'ADN de HBV chez un patient.
